# EUROPEAN PATENT APPLICATION

(11) **EP 4 786 456 A1**
(43) Date of publication of application: **05.08.2026**
(21) Application number: 25155552.0
(22) Date of filing: 03.02.2025
(51) Int. Cl.: C07D 285/12, A01N 43/00, C07C 235/00, C07C 233/27, C07C 233/88, A01P 13/00

(54) **HERBICIDAL ACTIVE THIADIAZOL COMPOUNDS**

(71) Applicant: Globachem NV, 3800 Sint-Truiden (BE)
(72) Inventor: CLOHESSY, Thomas Aidan, 3800 Sint-Truiden (BE); CEBAN, Victor, 3800 Sint-Truiden (BE); GROOM, Bethany Jade, 3800 Sint-Truiden (BE); JACKSON, Victoria Elizabeth, 3800 Sint-Truiden (BE); JORDAN, Linda, 3800 Sint-Truiden (BE); PHILLIPS, Max, 3800 Sint-Truiden (BE); DIETERICH, Simon, 3800 Sint-Truiden (BE); CATLING, Jacob, 3800 Sint-Truiden (BE)
(74) Representative: Hoffmann Eitle

(57) **Abstract**

The present invention relates to novel herbicidally active thiadiazol compounds and in particular chlorinated thiadiazol compounds according to Formula I, methods for producing these compounds, as well as to compositions comprising them. In addition, the present invention relates to the use of these compounds and composition comprising these compounds in agriculture, in particular as a herbicide.

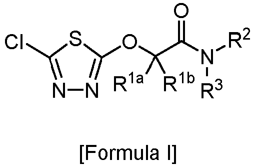

## Description

### FIELD OF THE INVENTION

The present invention relates to novel herbicidally active thiadiazol compounds and in particular chlorinated thiadiazol compounds, methods for producing these compounds, as well as to compositions comprising them. In addition, the present invention relates to the use of these compounds and composition comprising these compounds in agriculture, in particular as a herbicide.

### BACKGROUND OF THE INVENTION

In view of the increasing world population and the associated increased demand for nourishments, the productivity of cultivated growth needs to be improved. However, unwanted plants reduce crop yield due to resource competition and are considered to also alter developmental trajectories of crops early in the growing season. Therefore, herbicides are used to control the growth of unwanted plants.

The present invention therefore aims for the provision of herbicidally active compounds and to compositions comprising the same which are useful in agriculture. In particular, the present invention aims to have selective herbicides that reduce unwanted damage and/or have a toxicologically favourable profile, particularly in crop plants, especially in cereal crops such as maize, barley and/or wheat.

It is also an aim of the present invention to provide a broad-spectrum herbicide - preferably a broad-spectrum, pre-emergence or post-emergence herbicide - which exhibits improved or comparable efficacy to flufenacet (/V-(4-fluorophenyl)-/V-(propan-2-yl)-2-{[5-(trifluoromethyl)-1 ,3,4-thiadiazol-2-yl]oxy}acetamide) against monocotyledonous plants (i.e. from the plant clade Monocots) and/or dicotyledonous plants (i.e. from the plant clade Eudicots).

It is another aim of the present invention to provide a herbicide - preferably a pre-emergence herbicide - which provides broad-spectrum efficacy against perennial monocotyledonous plants which is comparable to flufenacet, but is more effective against dicotyledonous plants, especially plants from the clade Eudicotidae such as the pests *Chenopodium album, Solanum nigrum* and/or *Geranium dissectum.*

It is another aim of the present invention to provide a pre-emergence herbicide which is effective against *Lolium multiflorum* and/or *Alopecurus myosuroides,* but is also more effective against monocotyledonous plants than flufenacet, especially against *Apera spica-venti, Digitaria sanguinalis* and/or *Echinochloa crus-galli,* particularly at lower application rates.

Another aim of the present invention is to provide a post-emergence herbicide which is more selective for monocotyledonous plants than is flufenacet (i.e. it exhibits lower post-emergence herbicidal activity against dicotyledonous plants than does flufenacet).

It is another aim of the present invention to provide a post-emergence herbicide which is more effective than flufenacet against *Lolium multiflorum, Apera spica-venti* and/or *Setaria viridis.*

### SUMMARY OF THE INVENTION

The present invention is based on the finding that the above object can be solved by novel compounds having a specific structure that includes a chlorinated thiadiazol moiety.

Specifically, according to a first aspect of the invention, the present invention provides a compound corresponding to the following formula I, or an agrochemically acceptable salt thereof, a stereoisomer thereof, an enantiomer thereof, a deuteromer thereof, or a tautomer thereof: 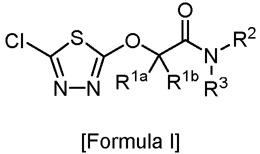 wherein
each of R^{1a} and R^{1b} is H;
R² is selected from C₁₋₃ alkyl, C₄₋₈ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₁₋₆ alkylsulfonyl, C₂₋₈ heterocyclyl and (C₂₋₈ heterocyclyl)C₁₋₃ alkyl, each of which may be optionally substituted; and
R³ is C₆₋₁₀ aryl which is substituted;
or an agrochemically acceptable salt thereof, a stereoisomer thereof, an enantiomer thereof, a deuteromer thereof, or a tautomer thereof,
   wherein:
   - when R^{1a} = H, R^{1b} = H and R² = isopropyl, R³ is not phenyl, 4-fluorophenyl, 2-chlorophenyl, 3-chlorophenyl, 4-chlorophenyl, 3-methylphenyl, 4-methylphenyl, 2-methoxyphenyl, 4-methoxyphenyl, 4-ethoxyphenyl, 4-thiomethylphenyl, 3-chloro-4-thiomethylphenyl, 3,5-dimethylphenyl, 3,5-dichlorophenyl, 3-chloro-4-methoxyphenyl, 3,5-di(trifluoromethyl)phenyl, 3,4-dichlorophenyl, 5-chloro-2-methylphenyl; or
   - when R^{1a} = H, R^{1b} = H and R² = methyl, R³ is not phenyl, 4-(trifluoromethyl)phenyl, 3-(trifluoromethyl)phenyl, 2-methyl-5-nitrophenyl, 4-thiomethylphenyl, 3-thiomethylphenyl, 4-fluorophenyl, 3-fluorophenyl, 3,4-dichlorophenyl, 2-methylphenyl, 3-methylphenyl, 4-methylphenyl, 3-chlorophenyl.

The present invention also covers compounds which include isotopes of the above-mentioned groups, and it specifically includes deuterated compounds of the above defined compound of formula I and any of the compounds defined in the following.

In a second aspect, the present invention provides a composition comprising a compound according to the first aspect.

In a third aspect, the present invention provides the use of the compound according to the first aspect or a composition according to the second aspect in agriculture.

In a fourth aspect, the present invention provides methods for producing a compound according to the first aspect.

In a fifth aspect, the present invention provides compounds which may be useful intermediates in the preparation of the compounds according to the first aspect, as well as the use of any of these compounds in the preparation of a compound according to the first aspect, and processes for preparing a compound according to the first aspect that involves any of these compounds.

In a further aspect, the present invention also provides combinations comprising a compound according to the first aspect or a composition according to the second aspect and one or more additional herbicides and/or plant growth regulators. These compositions and agrochemical combinations may also be used in accordance with the third aspect.

In another aspect, the present invention further provides a plant propagation material which comprises or is treated with or adheres thereto a compound according to the first aspect or a corresponding composition.

In the following, the present invention and definitions as used herein to describe the present invention will be set out in more detail.

### DEFINITIONS

In the present disclosure, the following definitions are applicable.

The term "Cₙ-Cₘ alkyl" as used herein refers to a saturated straight-chain or branched hydrocarbon radical attached via any of the carbon atoms having n to m carbon atoms (herein n and m is each a natural number independently selected from 1 to 10, where n<m), for example, any one of the radicals methyl, ethyl, n-propyl, 1-methylbutyl, 2-methylbutyl, 3-methylbutyl, 2,2-dimethylpropyl, 1-ethylpropyl, n-hexyl, n-pentyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl, but are not limited thereto.

The term "Cₙ-Cₘ alkoxy" as used herein refers to a straight-chain or branched saturated alkyl radical having n to m carbon atoms (as mentioned above) which is attached via an oxygen atom, i.e., for example, any one of the radicals methoxy, ethoxy, n-propoxy, 1-methylethoxy, n-butoxy, 1-methylpropoxy, 2-methylpropoxy or 1,1-dimethylethoxy, but are not limited thereto.

The term "(Cₙ-Cₘ alkoxy)Cₙ-Cₘalkyl" refers to an alkyl group where one or more of the hydrogens is substituted by an alkoxy group. n and m in the alkoxy group and the alkyl group are independently selected as appropriate.

The term "Cₙ-Cₘ (cyano)alkyl" as used herein refers to a straight chain or branched saturated Cₙ-Cₘ alkyl radical having n to m carbon atoms (as mentioned above), where one or more of the hydrogen atoms in these radicals is replaced by a cyano group: for example, cyanomethyl, 2-cyanoethyl, 2-cyanopropyl, 3-cyanopropyl, 1-(cyanomethyl)-2-ethyl, 1-(methyl)-2-cyanoethyl, 4-cyanobutyl, and the like.

The term "C₃-Cₘ cycloalkyl" as used herein refers to 3 to m membered cycloalkyl groups such as cyclopropane, cyclobutane, cyclopentane, cyclohexane and the like.

The term "C₂-Cₘ alkenyl" as used herein refers to a straight or branched alkenyl chain having from two to m carbon atoms and one or more double bonds, for example, ethenyl, prop-1-enyl, and but-2-enyl, but are not limited thereto.

The term "C₂-Cₘ alkynyl" as used herein refers to a straight or branched alkynyl chain having from two to m carbon atoms and one or more triple bond, for example, ethynyl, prop-2-ynyl, but-3-ynyl.

The term "C₆-C₁₀ aryl" as used herein refers to a carbocyclic ring system comprising 6 to 10 carbon atoms and at least one aromatic ring. For purposes of embodiments of this invention, the aryl group is a monocyclic or bicyclic ring system. Aryl groups include, but are not limited to, aryl residues derived from benzene or naphthalene (i.e., phenyl or naphthyl). Unless stated otherwise specifically in the specification, the term "aryl" or the prefix "ar" (such as in "aralkyl") is meant to include aryl groups that are optionally substituted.

The term "carbocyclic group" as used herein refers to a closed ring structure which consists of carbon atoms and hydrogen atoms. The term "heterocyclic group" as used herein refers to closed ring structures analogous to carbocyclic groups in which one or more of the carbon atoms in the ring is an element other than carbon, for example, nitrogen (N), sulfur (S) or oxygen (O).

The term "heterocyclyl" as used herein refers to a radical of a heterocyclic group which is attached via any of the carbon atoms. Analogous to the foregoing, "heterocyclyl(alkyl)" refers to heterocyclyl which is substituted with an alkyl group.

The term "oxo" as used herein means an oxygen atom that is double bonded to a carbon atom or to another element.

Halogen or "halo" generally refers to fluorine (F), chlorine (Cl), bromine (Br) or iodine (I). This also applies, correspondingly, to halogen in combination with other meanings, such as haloalkyl.

The term "optionally substituted" as used herein means that the group referenced is either unsubstituted or is substituted by one or more substituents. For example, "C₁-C₃ alkyl is optionally substituted with one or two F" means a group selected from C₁-C₃ alkyl, C₁-C₃ alkyl substituted with one F, and C₁-C₃ alkyl substituted with two F.

Unless specified otherwise, the term "substituted" as used herein means substitution by any of the above or other groups (*e.g*., alkyl, alkylene, alkylcycloalkyl, alkoxy, alkylphosphoryl, alkylphosphorylaminyl, amidinylalkyloxy, guanidinylalkyloxy, alkylcarbonylaminylalkyloxy, heterocyclylalkyloxy, heteroarylalkyloxy, aminylalkyloxy, alkoxyalkyl, alkoxycarbonyl, haloalkylaminyl, hydroxylalkylaminyl, amidinylalkylaminyl, guanidinylalkylaminyl, aminylalkyl, aminylalkylaminyl, aminylalkoxy, alkylaminylalkoxy aryloxy, alkylaminyl, alkylcarbonylaminyl, alkylaminylalkyl, aminylcarbonyl, alkylaminylcarbonyl, alkylcarbonylaminylalkoxy, aminylcarbonylalkyl, aminylcarbonycycloalkylalkyl, thioalkyl, aryl, aralkyl, arylalkyloxy, arylalkylaminyl, carboxyalkyl, cyanoalkyl, cycloalkyl, cycloalkyloxy, cycloalkylaminyl, cyanocycloalkyl, cycloalkylaminylcarbonyl, cycloalkylalkyl, haloalkyl, haloalkoxy, heterocyclyl, heterocyclyloxy, heterocyclylaminyl, *N*-heterocyclyl, heterocyclylalkyl, heterocyclylalkyloxy, heterocyclylalkylaminyl, heteroaryl, *N*-heteroaryl, heteroarylalkyl, heteroarylalkyloxy, heteroarylalkylaminyl, hydroxylalkylaminyl, phosphoalkoxy and/or hydroxylalkyl) wherein at least one hydrogen atom (*e*.*g*., 1, 2, 3 or more or all hydrogen atoms) is replaced by a bond to a non-hydrogen atom such as, but not limited to: a halogen atom such as F, Cl, Br, and I; an oxygen atom in groups such as hydroxyl groups, alkoxy groups, and ester groups; a sulfur atom in groups such as thiol groups, thioalkyl groups, sulfonyl groups, and sulfoxide groups; a nitrogen atom in groups such as amines, amides, alkylamines, dialkylamines, arylamines, alkylarylamines, diarylamines, *N*-oxides, imides, and enamines; a silicon atom in groups such as trialkylsilyl groups, dialkylarylsilyl groups, alkyldiarylsilyl groups, and triarylsilyl groups; and other heteroatoms in various other groups. "Substituted" also means any of the above groups in which one or more hydrogen atoms are replaced by a higher-order bond (*e*.*g*., a double- or triple-bond) to a heteroatom such as oxygen in oxo, carbonyl, carboxyl, and ester groups; and nitrogen in groups such as imines, oximes, hydrazones, and nitriles. For example, "substituted" includes any of the above groups in which one or more hydrogen atoms are replaced with -NR_{g}Rₕ, -NR_{g}C(=O)Rₕ, -NR_{g}C(=O)NR_{g}Rₕ, -NR_{g}C(=O)ORₕ, -NR_{g}SO₂Rₕ, -OC(=O)NR_{g}Rₕ, -OR_{g}, -SR_{g}, -SOR_{g}, -SO₂R_{g}, -OSO₂R_{g}, -SO₂OR_{g}, =NSO₂R_{g}, and -SO₂NR_{g}Rₕ. "Substituted" also means any of the above groups in which one or more hydrogen atoms are replaced with -C(=O)R_{g}, -C(=O)OR_{g}, -C(=O)NR_{g}Rₕ, -CH₂SO₂R_{g}, -CH₂SO₂NR_{g}Rₕ. In the foregoing, R_{g} and Rₕ are the same or different and independently hydrogen, alkyl, alkoxy, alkylaminyl, thioalkyl, aryl, aralkyl, cycloalkyl, cycloalkylalkyl, haloalkyl, heterocyclyl, *N-*heterocyclyl, heterocyclylalkyl, heteroaryl, *N*-heteroaryl and/or heteroarylalkyl. "Substituted" further means any of the above groups in which one or more hydrogen atoms are replaced by a bond to an aminyl, cyano, hydroxyl, imino, nitro, oxo, thioxo, halo, alkyl, alkoxy, alkylaminyl, thioalkyl, aryl, aralkyl, cycloalkyl, cycloalkylalkyl, haloalkyl, heterocyclyl, *N*-heterocyclyl, heterocyclylalkyl, heteroaryl, *N-*heteroaryl and/or heteroarylalkyl group. In addition, each of the foregoing substituents may also be optionally substituted with one or more of the above substituents.

The term "leaving group" as used herein refers to a group capable of being displaced from a molecule when said molecule undergoes reaction with a nucleophile.

The term "stereoisomer" as used herein includes any enantiomer, diastereomer, geometric isomer (E/Z) or tautomer of a given compound.

The term "deuteromer" as used herein, refers to a given compound in which at least one hydrogen atom (¹H), preferably at least two, more preferably 3, 4, 5, and most preferably all hydrogen atoms are replaced with a deuterium isotope (²H or D). For example, one or both H-atoms of a -CH₂- moiety in a given compound may be replaced with D (i.e. -CHD- or CD₂-).

As used herein, the term "agrochemically acceptable salt" refers to a salt which can be applied as an agrochemical.

As used herein, the term "herbicide" refers to an active ingredient that kills, controls or otherwise inhibits plant growth. The preferred amount or concentration of the herbicide is an "effective amount" or "effective concentration".

As used herein, the term "controlling" means killing, reducing or retarding growth or preventing or reducing germination of plants. Generally, the plants to be controlled are unwanted plants, also called weeds. "Locus" means the area in which the plants are growing or will grow.

As used herein, the term "pre-emergence" refers to the period of time before the germinated plant is established (i.e. before the germinated plant emerges from the soil). On the other hand, "post-emergence" refers to the period of time once the germinated plant has established itself (i.e. once the germinated plant emerges from the soil).

As used herein, the terms "effective amount" and "effective concentration" refer to the amount or concentration of the compound, respectively, or an agrochemically acceptable salt thereof, which, upon single or multiple applications provides the desired effect.

An effective amount or an effective concentration is readily determined by the skilled person in the art, by the use of known techniques and by observing results obtained under analogous circumstances. In determining the effective amount or the effective concentration, a number of factors are considered including, but not limited to, the type of plant or derived product to be applied; the unwanted plants to be controlled and its lifecycle; the particular compound applied; the type of application; and other relevant circumstances.

In each of the following aspects and embodiments of the invention, "consisting essentially" and inflections thereof are a preferred embodiment of "comprising" and its inflections, and "consisting of" and inflections thereof are a preferred embodiment of "consisting essentially of" and its inflections.

### DETAILED DESCRIPTION OF THE INVENTION

Embodiments according to the invention are provided as set out below. The disclosure in the present application makes available each and every combination of these embodiments disclosed in the following.
1. A compound of formula I: 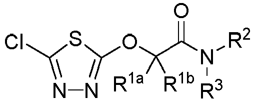 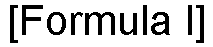 wherein each of R^{1a} and R^{1b} is H;
   R² is selected from C₁₋₃ alkyl, C₄₋₈ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₁₋₆ alkylsulfonyl, C₂₋₈ heterocyclyl and (C₂₋₈ heterocyclyl)C₁₋₃ alkyl, each of which may be optionally substituted; and
   R³ is C₆₋₁₀ aryl which is substituted;
   or an agrochemically acceptable salt thereof, a stereoisomer thereof, an enantiomer thereof, a deuteromer thereof, or a tautomer thereof,
   wherein when:
      - R^{1a} = H, R^{1b} = H and R² = isopropyl, R³ is not phenyl, 4-fluorophenyl, 2-chlorophenyl, 3-chlorophenyl, 4-chlorophenyl, 3-methylphenyl, 4-methylphenyl, 2-methoxyphenyl, 4-methoxyphenyl, 4-ethoxyphenyl, 4-thiomethylphenyl, 3-chloro-4-thiomethylphenyl, 3,5-dimethylphenyl, 3,5-dichlorophenyl, 3-chloro-4-methoxyphenyl, 3,5-di(trifluoromethyl)phenyl, 3,4-dichlorophenyl, 5-chloro-2-methylphenyl; or
      - R^{1a} = H, R^{1b} = H and R² = methyl, R³ is not phenyl, 4-(trifluoromethyl)phenyl, 3-(trifluoromethyl)phenyl, 2-methyl-5-nitrophenyl, 4-thiomethylphenyl, 3-thiomethylphenyl, 4-fluorophenyl, 3-fluorophenyl, 3,4-dichlorophenyl, 2-methylphenyl, 3-methylphenyl, 4-methylphenyl, 3-chlorophenyl.
2. The compound of item 1, wherein:
   each of the groups listed for R² is optionally substituted by one or more substituents selected from F, C!, Br, !, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiRs, wherein each R is independently selected from H, OH, C₁₋₆ alkoxy and C₁₋₆ alkyl, and wherein two Rs and the atom to which they are bound may form a cyclic group;
   each of the groups listed for R³ is substituted by one or more substituents selected from F, C!, Br, !, CN, NO₂, C₁₋₆ alkyl, C₁₋₆ alkoxy and C₃₋₆ cycloalkyl;
   or an agrochemically acceptable salt thereof, a stereoisomer thereof, an enantiomer thereof, a deuteromer thereof, or a tautomer thereof.
3. The compound of item 1, with the proviso that R³ is C₆ aryl substituted by at least one of halogen, CN, NO₂, C₁₋₆ alkyl, halogeno-C₁₋₆ alkyl, C₁₋₆ alkoxy, halogeno-C₁₋₆ alkoxy, C₁₋₆ alkylthio and halogeno-C₁₋₆ alkylthio, preferably C₆ aryl substituted by at least one of F, Cl, CH₃, OCH₃, OC₂H₅, SCH₃ and CF₃;
   or an agrochemically acceptable salt thereof, a stereoisomer thereof, an enantiomer thereof, a deuteromer thereof, or a tautomer thereof.
4. The compound of any one of items 1 to 3, wherein R² is selected from C₄₋₈ alkyl, (cyano)C₁₋₆ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₂₋₈ heterocyclyl and (C₂₋₈ heterocyclyl)C₁₋₃ alkyl, each of which may be optionally substituted by one or more groups selected from F, Cl, Br, I, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiRs, wherein each R is independently selected from H, OH and C₁₋₆ alkyl,
   or an agrochemically acceptable salt thereof, a stereoisomer thereof, an enantiomer thereof, a deuteromer thereof, or a tautomer thereof.
5. The compound of any one of items 1 to 4, wherein R² is selected from C₄₋₈ alkyl, (cyano)C₁₋₆ alkyl, C₂₋₆ alkenyl, C₂₋₆ alkynyl, (C₃₋₆ cycloalkyl)C₁₋₃ alkyl and C₃₋₆ cycloalkyl, each of which may be optionally substituted by one or more groups selected from F, C!, Br, !, CN, oxo, C₁₋₆ alkyl, C₁₋₆ alkoxy, (C₁₋₆alkoxy)C₁₋₆alkyl, SO₂R, C(O)R, CO₂R, C(O)NR₂, NR₂ and SiRs, wherein each R is independently selected from H, OH and C₁₋₆ alkyl; or an agrochemically acceptable salt thereof, a stereoisomer thereof, an enantiomer thereof, a deuteromer thereof, or a tautomer thereof.
6. The compound of any one of items 1 to 5, wherein R² is selected from R²-1 to R²-63 as defined in Table 1; or an agrochemically acceptable salt thereof, a stereoisomer thereof, an enantiomer thereof, a deuteromer thereof, or a tautomer thereof.
7. The compound of any one of items 1 to 6, wherein R² is selected from cyanomethyl, cyclopropyl, sec-butyl, 1-butyn-4-yl, 2-butyn-1-yl, prop-1-yl, 3-methyl-1-butyn-3-yl, cyclopropylmethyl, 1-trimethylsilyl-2-butyn-4-yl, 1-buten-4-yl, 1-buten-3-yl, 2-methyl-1-propen-3-yl, allyl, tert-butyl, cyclopentyl, propargyl, iso-butyl, iso-propyl, 1-butyn-3-yl and 3-methylbut-2-yl; or an agrochemically acceptable salt thereof, a stereoisomer thereof, an enantiomer thereof, a deuteromer thereof, or a tautomer thereof.
8. The compound of any one of items 1 to 7, wherein R² is selected from cyanomethyl, cyclopropyl, sec-butyl, 1-butyn-4-yl, 2-butyn-1-yl, prop-1-yl, 3-methyl-1-butyn-3-yl, cyclopropylmethyl, 1-trimethylsilyl-2-butyn-4-yl, 1-buten-4-yl, 1-buten-3-yl, 2-methyl-1-propen-3-yl, allyl, tert-butyl, cyclopentyl, propargyl, iso-butyl, 1-butyn-3-yl and 3-methylbut-2-yl; or an agrochemically acceptable salt thereof, a stereoisomer thereof, an enantiomer thereof, a deuteromer thereof, or a tautomer thereof.
9. The compound of any one of items 1 to 4, wherein R² is selected from C₂₋₈ alkyl, (cyano)C₁₋₆ alkyl, C₂₋₆ alkenyl, C₂₋₆ alkynyl, (C₃₋₆ cycloalkyl)C₁₋₃ alkyl and C₃₋₆ cycloalkyl, each of which may be optionally substituted by one or more groups selected from F, C!, Br, !, CN, oxo, C₁₋₆ alkyl, C₁₋₆ alkoxy, (C₁₋₆alkoxy)C₁₋₆alkyl, SO₂R, C(O)R, CO₂R, C(O)NR₂, NR₂ and SiRs, wherein each R is independently selected from H, OH and C₁₋₆ alkyl; or an agrochemically acceptable salt thereof, a stereoisomer thereof, an enantiomer thereof, a deuteromer thereof, or a tautomer thereof.
10. The compound of any one of items 1 to 9, wherein R³ is C₆₋₁₀ aryl substituted by at least one F, preferably in the 3-position or 4-position relative to the attachment point of R³ to the remainder of the molecule, and optionally further substituted by one or more groups selected from F, Cl, Br, I, CN, NO₂, C₁₋₆ alkyl, C₁₋₆ alkoxy and C₃₋₆ cycloalkyl; or an agrochemically acceptable salt thereof, a stereoisomer thereof, an enantiomer thereof, a deuteromer thereof, or a tautomer thereof.
11. The compound of any one of items 1 to 10, wherein R³ is C₆ aryl substituted by at least one F, preferably in the 3-position or 4-position relative to the attachment point of R³ to the remainder of the molecule, and optionally further substituted by one or more groups selected from F, Cl, Br, I, CN, NO₂, C₁₋₆ alkyl, C₁₋₆ alkoxy and C₃₋₆ cycloalkyl; or an agrochemically acceptable salt thereof, a stereoisomer thereof, an enantiomer thereof, a deuteromer thereof, or a tautomer thereof.
12. The compound of any one of items 1 to 11, wherein R³ is selected from R³-1 to R³-92 as defined in Table 2 below; or an agrochemically acceptable salt thereof, a stereoisomer thereof, an enantiomer thereof, a deuteromer thereof, or a tautomer thereof.
13. The compound of any of items 1 to 12, wherein R³ is selected from 4-fluorophenyl, 4-chlorophenyl, 4-nitrophenyl, 4-(trifluoromethoxy)phenyl, 2-fluorophenyl, 3-fluorophenyl, 2,5-difluorophenyl, 3,4-difluorophenyl, 2,4-difluorophenyl, 2,3-difluorophenyl, 3,5-difluorophenyl, 2,6-difluorophenyl, 3-fluoro-4-chlorophenyl, 3-chloro-4-fluorophenyl, 2-chloro-4-fluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl, 3-chloro-2,4-difluorophenyl, 2,6-dichloro-4-fluorophenyl and 2-chloro-4-fluoro-6-methylphenyl; or an agrochemically acceptable salt thereof, a stereoisomer thereof, an enantiomer thereof, a deuteromer thereof, or a tautomer thereof.
14. The compound of any one of items 1 to 13, wherein R³ is selected from 4-fluorophenyl, 3,4-difluorophenyl, 2,4-difluorophenyl, 3-fluoro-4-chlorophenyl, 3-chloro-4-fluorophenyl, 4-chlorophenyl, 4-nitrophenyl, 4-(trifluoromethoxy)phenyl, 2,5-difluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl, 3-chloro-2,4-difluorophenyl, 2,6-dichloro-4-fluorophenyl and 2-chloro-4-fluoro-6-methylphenyl; or an agrochemically acceptable salt thereof, a stereoisomer thereof, an enantiomer thereof, a deuteromer thereof, or a tautomer thereof.
15. The compound of any one of items 1 to 14, wherein R² is selected from iso-butyl, tert-butyl, cyclopentyl and propargyl, preferably propargyl; or an agrochemically acceptable salt thereof, a stereoisomer thereof, an enantiomer thereof, a deuteromer thereof, or a tautomer thereof.
16. The compound of any one of items 1 to 14, wherein R² is selected from C₁₋₃ alkyl; or an agrochemically acceptable salt thereof, a stereoisomer thereof, an enantiomer thereof, a deuteromer thereof, or a tautomer thereof.
17. The compound of any one of items 1 to 14 and 16, wherein R² is isopropyl; or an agrochemically acceptable salt thereof, a stereoisomer thereof, an enantiomer thereof, a deuteromer thereof, or a tautomer thereof.
18. The compound of any one of items 1 to 14, wherein R² is selected from C₁₋₃ alkyl, C₄₋₈ alkyl, (cyano)C₁₋₆ alkyl, C₂₋₆ alkenyl, C₂₋₆ alkynyl, (C₃₋₆ cycloalkyl)C₁₋₃ alkyl and C₃₋₆ cycloalkyl, each of which may be optionally substituted; and R³ is 4-fluorophenyl; or an agrochemically acceptable salt thereof, a stereoisomer thereof, an enantiomer thereof, a deuteromer thereof, or a tautomer thereof.
19. The compound of any one of items 1 to 14 and 18, wherein R² is selected from C₄₋₈ alkyl, (cyano)C₁₋₆ alkyl, C₂₋₆ alkenyl, C₂₋₆ alkynyl, (C₃₋₆ cycloalkyl)C₁₋₃ alkyl and C₃₋₆ cycloalkyl, each of which may be optionally substituted; and R³ is 4-fluorophenyl; or an agrochemically acceptable salt thereof, a stereoisomer thereof, an enantiomer thereof, a deuteromer thereof, or a tautomer thereof.
20. The compound of any one of items 1 to 14 and 18, wherein R² is selected from cyanomethyl, cyclopropyl, sec-butyl, 1-butyn-4-yl, 2-butyn-1-yl, prop-1-yl, 3-methyl-1-butyn-3-yl, cyclopropylmethyl, 1-trimethylsilyl-2-butyn-4-yl, 1-buten-4-yl, 1-buten-3-yl, 2-methyl-1-propen-3-yl, allyl, tert-butyl, cyclopentyl, propargyl, iso-butyl, iso-propyl, 1-butyn-3-yl and 3-methylbut-2-yl, and R³ is 4-fluorophenyl; or an agrochemically acceptable salt thereof, a stereoisomer thereof, an enantiomer thereof, a deuteromer thereof, or a tautomer thereof.
21. The compound of any one of items 1 to 14, 18 and 20, wherein R² is selected from cyanomethyl, cyclopropyl, sec-butyl, 1-butyn-4-yl, 2-butyn-1-yl, prop-1-yl, 3-methyl-1-butyn-3-yl, cyclopropylmethyl, 1-trimethylsilyl-2-butyn-4-yl, 1-buten-4-yl, 1-buten-3-yl, 2-methyl-1-propen-3-yl, allyl, tert-butyl, cyclopentyl, propargyl, iso-butyl, 1-butyn-3-yl and 3-methylbut-2-yl, and R³ is 4-fluorophenyl; or an agrochemically acceptable salt thereof, a stereoisomer thereof, an enantiomer thereof, a deuteromer thereof, or a tautomer thereof.
22. The compound of any one of items 1 to 21, having a structure selected from Table 3 or 4; or an agrochemically acceptable salt thereof, a stereoisomer thereof, an enantiomer thereof, a deuteromer thereof, or a tautomer thereof, with the proviso that said compound is not compound 1-9, 1-20, 1-108, I-141, 1-174 or I-900.
23. The compound of any one of items 1 to 22, represented by the following Formula la', 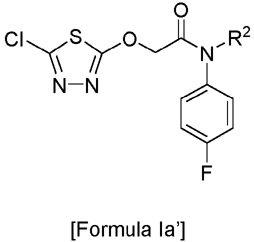 wherein R² is: (i) allyl; (ii) propargyl; (iii) 1-butyn-3-yl; (iv) 1-butyn-4-yl; (v) 2-butyn-1-yl; (vi) prop-1-yl; (vii) 3-methyl-1-butyn-3-yl; or an agrochemically acceptable salt thereof, a stereoisomer thereof, an enantiomer thereof, a deuteromer thereof, or a tautomer thereof.
24. The compound of any one of items 1 to 23, represented by the following Formula la', 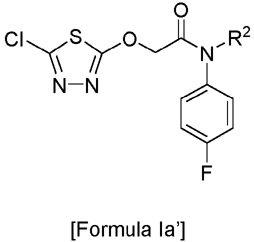 wherein R² is:
   (i) 1-butyn-4-yl;
   (ii) 2-butyn-1-yl;
   (iii) propargyl; or
   (iv) allyl;
   or an agrochemically acceptable salt thereof, a stereoisomer thereof, an enantiomer thereof, a deuteromer thereof, or a tautomer thereof.
25. A composition comprising a compound according to any one of items 1 to 24 or an agrochemically acceptable salt thereof, a stereoisomer thereof, an enantiomer thereof, a deuteromer thereof, or a tautomer thereof, and one or more auxiliary and/or adjuvant, and optionally one or more further active ingredients.
26. The composition of item 25, which is an agrochemical composition.
27. Use of a compound according to any one of items 1 to 24 or an agrochemically acceptable salt thereof, a stereoisomer thereof, an enantiomer thereof, a deuteromer thereof, or a tautomer thereof, or a composition according to item 25 or 26 in agriculture.
28. Use of the compound of any one of items 1 to 24 or an agrochemically acceptable salt thereof, a stereoisomer thereof, an enantiomer thereof, a deuteromer thereof, or a tautomer thereof, or a composition according to item 25 or 26 as a herbicide on crops selected from cereals including barley and wheat, cotton, oilseed rape, sunflower, maize, rice, soybeans, sugar beet, sugar cane, turf, trees including fruit trees, palm trees, coconut trees or other nuts, vines including grapes, fruit bushes, fruit plants, and vegetables including potatoes and tomatoes.
29. The use of item 28, wherein the compound or composition is used against a monocotyledonous plant (i.e. a plant from the clade Monocots) and/or a dicotyledonous plant (i.e. a plant from the clade Eudicot), preferably against a species selected from blackgrass, wild oat, Fat Hen, Crabgrass, Barnyard grass, cut-leaved crane's-bill, Italian ryegrass, Common Millet, green bristlegrass and Black nightshade.
30. The use of item 28 or 29 wherein the compound or composition is used against a monocotyledonous plant (i.e. a plant from the clade Monocots) and/or a dicotyledonous plant (i.e. a plant from the clade Eudicot), preferably against a species selected from *Alopecurus myosuroidpes, Avena fatua, Chenopodium album, Digitaria sanguinalis, Echinochloa crus-galli, Geranium dissectum, Lolium multiflorum, Panicum miliaceum, Setaria viridis* and *Solanum nigrum,* more preferably against both a monocotyledonous plant and a dicotyledonous plant, the latter preferably selected from *Chenopodium album, Geranium dissectum* and *Solanum nigrum.*
31. Agrochemical combination comprising the compound of any one of items 1 to 24 or the composition of item 25 or 26 and one or more additional herbicides and/or plant growth regulators.
32. Use of the compound of any one of items 1 to 24 or the composition of item 25 or 26 in combination with one or more additional herbicides.
33. A composition comprising the compound of any one of items 1 to 24 and one or more additional herbicides and/or plant growth regulators.
34. A method for producing compound according to any one of items 1 to 24, comprising a step selected from the following (i) to (iv):
   (i) 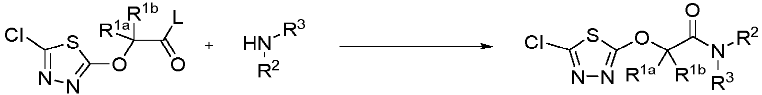
   (ii) 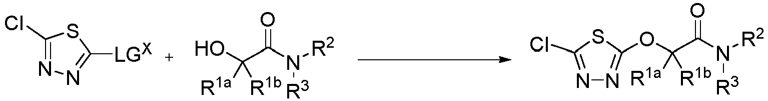
   (iii) 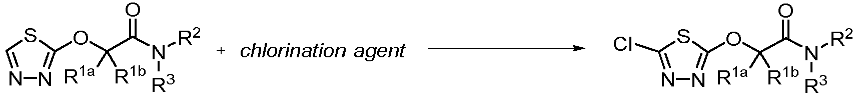
   (iv) 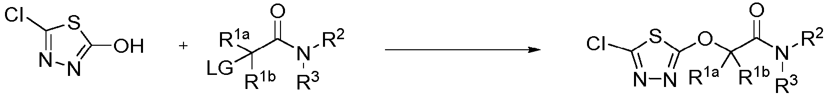
   wherein L, LG and LG^{X} are each independently leaving groups, and the remaining groups are as defined hereinabove.
35. An intermediate selected from: 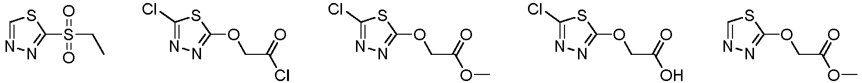 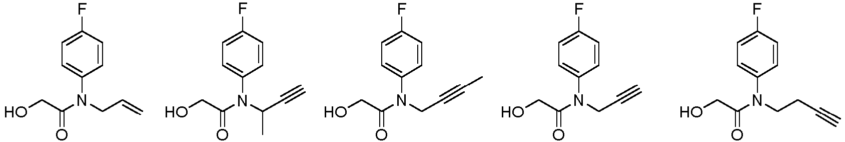 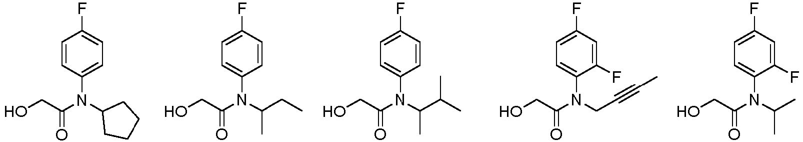 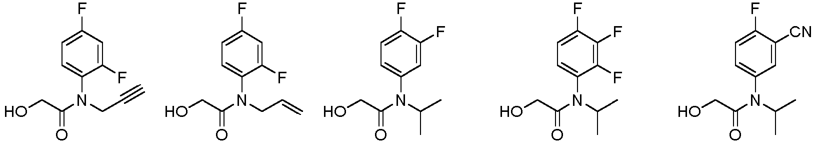 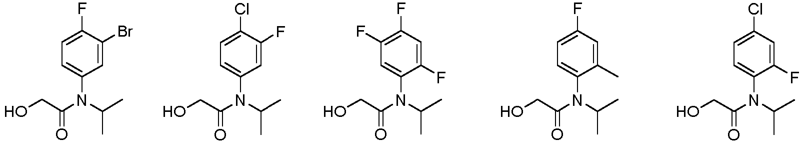 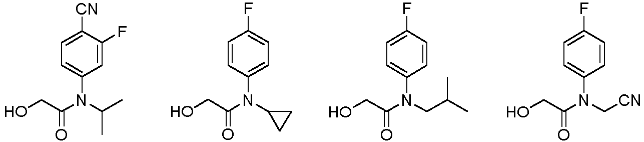 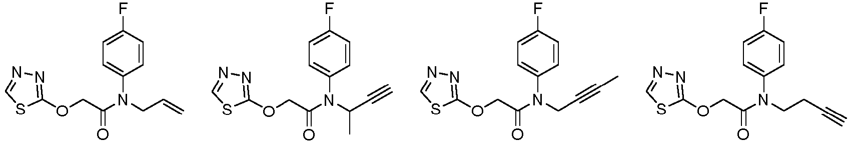 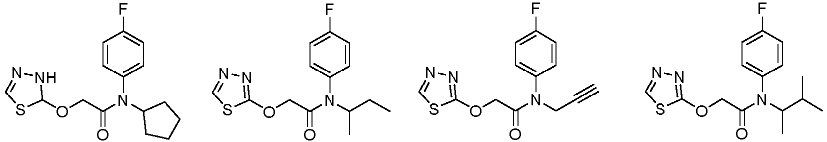 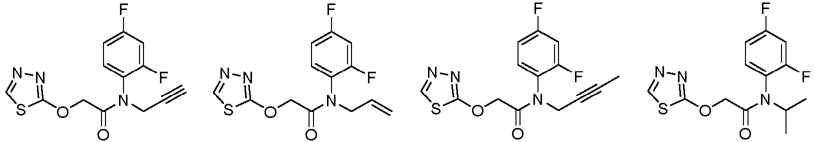 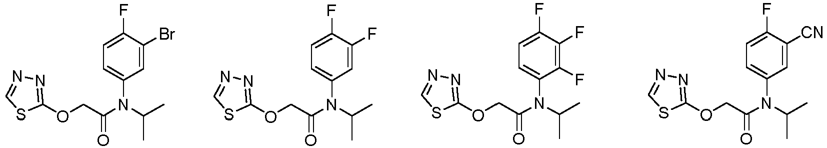 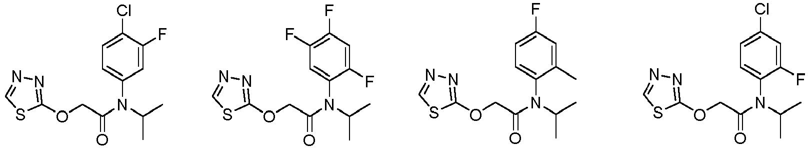 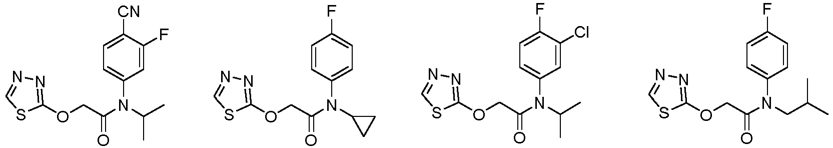 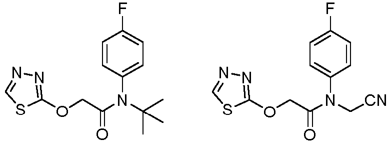 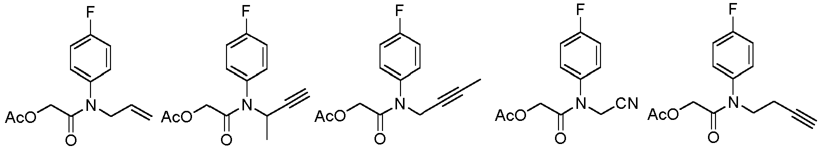 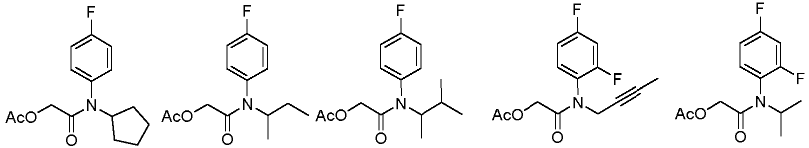 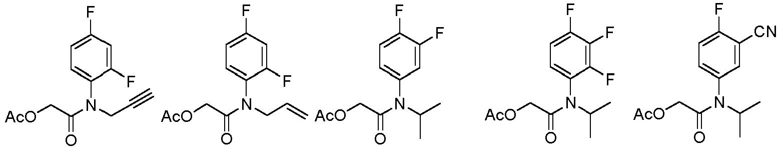 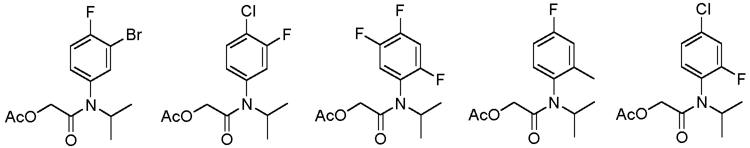 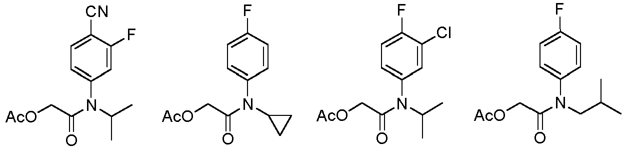 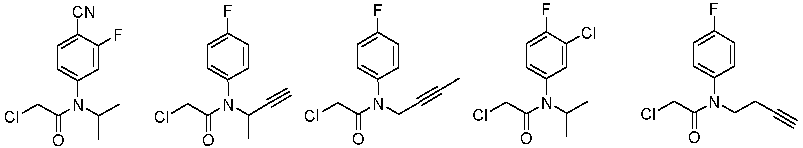 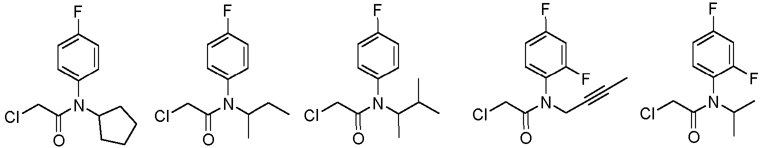 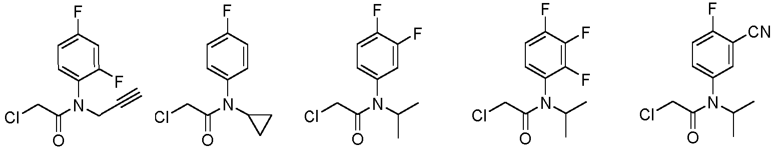 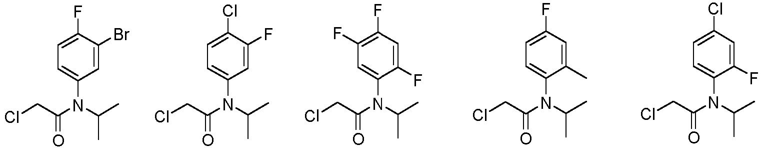
36. A method for producing a compound according to item 1 to 24, involving using one or more intermediates according to item 35 in at least one step.
37. A plant propagation material, such as a seed, comprising, or treated with or adhered thereto, a compound of any one of items 1 to 24 or a composition of item 25 or 26.

### Compounds of formula I

According to a first aspect of the invention, there is provided a compound of formula I: 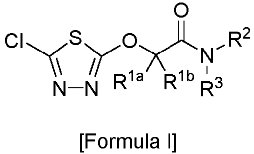 wherein each of R^{1a} and R^{1b} is H;
R² is selected from C₁₋₃ alkyl, C₄₋₈ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₁₋₆ alkylsulfonyl, C₂₋₈ heterocyclyl and (C₂₋₈ heterocyclyl)C₁₋₃ alkyl, each of which may be optionally substituted; and
R³ is C₆₋₁₀ aryl which is substituted, wherein:
   - when R^{1a} = H, R^{1b} = H and R² = isopropyl, R³ is not phenyl, 4-fluorophenyl, 2-chlorophenyl, 3-chlorophenyl, 4-chlorophenyl, 3-methylphenyl, 4-methylphenyl, 2-methoxyphenyl, 4-methoxyphenyl, 4-ethoxyphenyl, 4-thiomethylphenyl, 3-chloro-4-thiomethylphenyl, 3,5-dimethylphenyl, 3,5-dichlorophenyl, 3-chloro-4-methoxyphenyl, 3,5-di(trifluoromethyl)phenyl, 3,4-dichlorophenyl, 5-chloro-2-methylphenyl; or
   - when R^{1a} = H, R^{1b} = H and R² = methyl, R³ is not phenyl, 4-(trifluoromethyl)phenyl, 3-(trifluoromethyl)phenyl, 2-methyl-5-nitrophenyl, 4-thiomethylphenyl, 3-thiomethylphenyl, 4-fluorophenyl, 3-fluorophenyl, 3,4-dichlorophenyl, 2-methylphenyl, 3-methylphenyl, 4-methylphenyl, 3-chlorophenyl.

In a preferred embodiment, each of the groups listed for R² is optionally substituted by one or more substituents selected from F, C!, Br, !, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiRs, wherein each R is independently selected from H, OH, C₁₋₆ alkoxy and C₁₋₆ alkyl, and wherein two Rs and the atom to which they are bound may form a cyclic group.

In another preferred embodiment, R³ is substituted by one or more substituents selected from F, Cl, Br, I, CN, NO₂, C₁₋₆ alkyl, C₁₋₆ alkoxy and C₃₋₆ cycloalkyl.

In another embodiment, the present invention provides a compound of any of the preceding embodiments with the proviso that R³ is C₆ aryl substituted by at least one of halogen, CN, NO₂, C₁₋₆ alkyl, halogeno-C₁₋₆ alkyl, C₁₋₆ alkoxy, halogeno-C₁₋₆ alkoxy, C₁₋₆ alkylthio and halogeno-C₁₋₆ alkylthio, preferably C₆ aryl substituted by at least one of F, Cl, CH₃, OCH₃, OC₂H₅, SCH₃ and CF₃.

In a further embodiment, the present invention provides a compound of any of the preceding embodiments wherein R² is selected from C₄₋₈ alkyl, (cyano)C₁₋₆ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₂₋₈ heterocyclyl and (C₂₋₈ heterocyclyl)C₁₋₃ alkyl, each of which may be optionally substituted by one or more groups selected from F, Cl, Br, I, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiRs, wherein each R is independently selected from H, OH and C₁₋₆ alkyl. Preferably, R² may be selected from C₄₋₈ alkyl, (cyano)C₁₋₆ alkyl, C₂₋₆ alkenyl, C₂₋₆ alkynyl and C₃₋₆ cycloalkyl.

In a preferred embodiment, R² is selected from R²-1 to R²-63 as defined in Table 1 below, and R^{1a}, R^{1b} and R³ are defined as in the preceding embodiments.

**Table 1. Preferred R² groups**

| | | | | | | | |
|---|---|---|---|---|---|---|---|
| R²-1 | 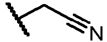 | R²-12 | 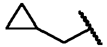 | R²-23 | 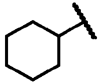 | R²-34 | 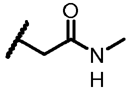 |
| R²-2 | 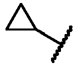 | R²-13 | 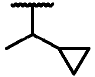 | R²-24 | 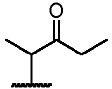 | R²-35 | 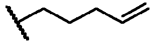 |
| R²-3 | 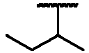 | R²-14 | 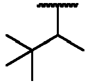 | R²-25 | 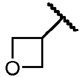 | R²-36 | 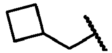 |
| R²-4 | 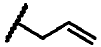 | R²-15 | 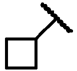 | R²-26 | 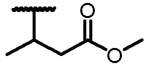 | R²-37 | 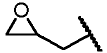 |
| R²-5 | 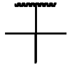 | R²-16 | 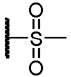 | R²-27 | 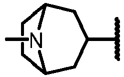 | R²-38 | 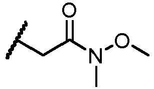 |
| R²-6 | 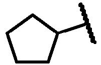 | R²-17 | 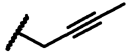 | R²-28 | 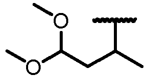 | R²-39 | 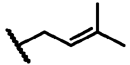 |
| R²-7 | 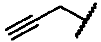 | R²-18 | 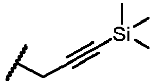 | R²-29 | 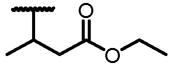 | R²-40 | 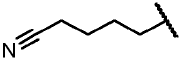 |
| R²-8 | 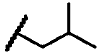 | R²-19 | 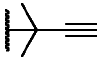 | R²-30 | 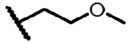 | R²-41 | 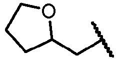 |
| R²-9 | 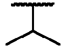 | R²-20 | 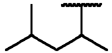 | R²-31 | 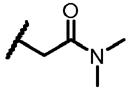 | R²-42 | 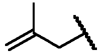 |
| R²-10 | 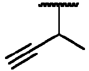 | R²-21 | 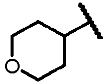 | R²-32 | 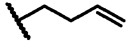 | R²-43 | 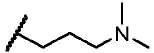 |
| R²-11 | 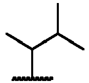 | R²-22 | 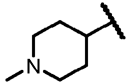 | R²-33 | 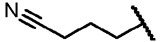 | R²-44 | 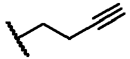 |
| R²-45 | 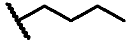 | R²-50 | 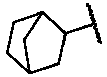 | R²-55 | 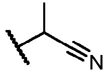 | R²-60 | 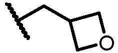 |
| R²-46 | 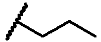 | R²-51 | 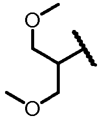 | R²-56 | 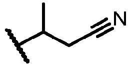 | R²-61 | 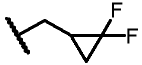 |
| R²-47 | 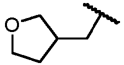 | R²-52 | 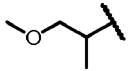 | R²-57 | 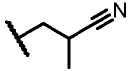 | R²-62 | 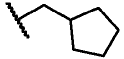 |
| R²-48 | 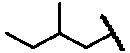 | R²-53 | 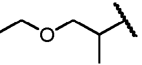 - | R²-58 | 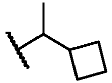 | R²-63 | 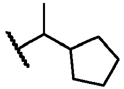 |
| R²-49 | 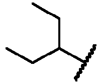 | R²-54 | 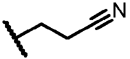 | R²-59 | 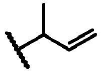 | | |

In a further preferred embodiment, R² is selected from cyanomethyl, cyclopropyl, sec-butyl, 1-butyn-4-yl, 2-butyn-1-yl, prop-1-yl, 3-methyl-1-butyn-3-yl, cyclopropylmethyl, 1-trimethylsilyl-2-butyn-4-yl, 1-buten-4-yl, 1-buten-3-yl, 2-methyl-1-propen-3-yl, allyl, tert-butyl, cyclopentyl, propargyl, iso-butyl, iso-propyl, 1-butyn-3-yl and 3-methylbut-2-yl, and R^{1a}, R^{1b} and R³ are defined as in the preceding embodiments. More preferably, R² is selected from allyl, propargyl, 1-butyn-3-yl, 1-butyn-4-yl, 2-butyn-1-yl and 3-methyl-1-butyn-3-yl.

In a further more preferred embodiment, R³ is 4-fluorophenyl and R² is selected from R² is selected from cyanomethyl, cyclopropyl, sec-butyl, 1-butyn-4-yl, 2-butyn-1-yl, prop-1-yl, 3-methyl-1-butyn-3-yl, cyclopropylmethyl, 1-trimethylsilyl-2-butyn-4-yl, 1-buten-4-yl, 1-buten-3-yl, 2-methyl-1-propen-3-yl, allyl, tert-butyl, cyclopentyl, propargyl, iso-butyl, iso-propyl, 1-butyn-3-yl and 3-methylbut-2-yl, and R^{1a}, R^{1b} and R³ are defined as in the preceding embodiments. Even more preferably, R³ is 4-fluorophenyl and R² is selected from allyl, propargyl, 1-butyn-3-yl, 1-butyn-4-yl, 2-butyn-1-yl and 3-methyl-1-butyn-3-yl.

In the compound of any of the preceding embodiments, R³ is preferably C₆₋₁₀ aryl substituted by at least one F. More preferably, R³ is C₆ aryl and the substituent is present in the 2-position, 3-position or 4-position relative to the attachment point of R³ to the remainder of the molecule. R³ may optionally be further substituted by one or more groups selected from F, C!, Br, !, CN, NO₂, C₁₋₆ alkyl, C₁₋₆ alkoxy and C₃₋₆ cycloalkyl.

In a preferred embodiment, R³ is C₆ aryl substituted by at least one F, preferably in the 3-position or 4-position relative to the attachment point of R³ to the remainder of the molecule, and optionally further substituted by one or more groups selected from F, C!, Br, !, CN, NO₂, C₁₋₆ alkyl, C₁₋₆ alkoxy and C₃₋₆ cycloalkyl.

In another preferred embodiment, R³ is C₆ aryl substituted by at least one F, preferably in the 3-position or 4-position relative to the attachment point of R³ to the remainder of the molecule, and optionally further substituted by one or more groups selected from halogen, CN, NO₂, C₁₋₆ alkyl, halogeno-C₁₋₆ alkyl, C₁₋₆ alkoxy, halogeno-C₁₋₆ alkoxy, C₁₋₆ alkylthio and halogeno-C₁₋₆ alkylthio, preferably wherein R³ is unsubstituted C₆ aryl or C₆ aryl substituted by at least one of F, Cl, CH₃, OCH₃, OC₂H₅, SCH₃ and CF₃, and

R² is selected from C₄₋₈ alkyl, (cyano)C₁₋₆ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₂₋₈ heterocyclyl and (C₂₋₈ heterocyclyl)C₁₋₃ alkyl, each of which may be optionally substituted by one or more groups selected from F, Cl, Br, I, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiRs, wherein each R is independently selected from H and C₁₋₆ alkyl.

In another preferred embodiment, R³ may be C₆ aryl substituted by at least one F, preferably the substitution is in the 3-position or 4-position relative to the attachment point of R³ to the remainder of the molecule, and R³ may be optionally further substituted by one or more groups selected from F, Cl, Br, I, CN, NO₂, C₁₋₆ alkyl, C₁₋₆ alkoxy and C₃₋₆ cycloalkyl, and R² is selected from C₄₋₈ alkyl, (cyano)C₁₋₆ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₂₋₈ heterocyclyl and (C₂₋₈ heterocyclyl)C₁₋₃ alkyl, each of which may be optionally substituted by one or more groups selected from F, Cl, Br, I, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiRs, wherein each R is independently selected from H, OH and C₁₋₆ alkyl.

In a preferred embodiment, R³ is selected from R³-1 to R³-99 as defined in Table 2, and R², R^{1a} and R^{1b} are defined as in any of the preceding embodiments.

**Table 2. Preferred R³ groups**

| | | | | | | | |
|---|---|---|---|---|---|---|---|
| R³-1 | 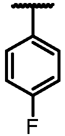 | R³-2 | 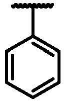 | R³-3 | 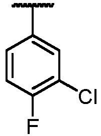 | R³-4 | 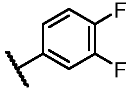 |
| R³-5 | 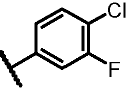 | R³-6 | 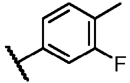 | R³-7 | 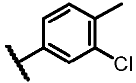 | R³-8 | 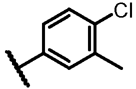 |
| R³-9 | 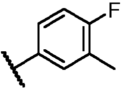 | R³-10 | 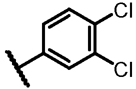 | | | | |
| R³-11 | 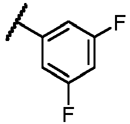 | R³-21 | 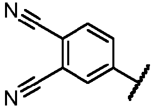 | R³-31 | 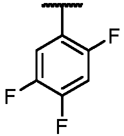 | R³-41 | 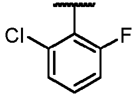 |
| R³-12 | 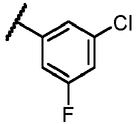 | R³-22 | 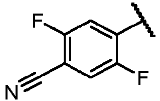 | R³-32 | 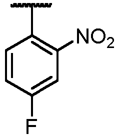 | R³-42 | 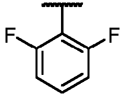 |
| R³-13 | 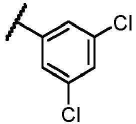 | R³-23 | 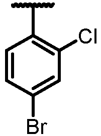 | R³-33 | 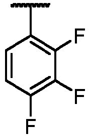 | R³-43 | 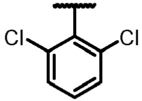 |
| R³-14 | 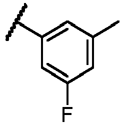 | R³-24 | 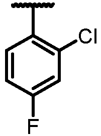 | R³-34 | 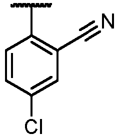 | R³-44 | 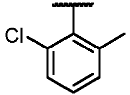 |
| R³-15 | 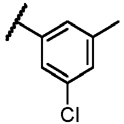 | R³-25 | 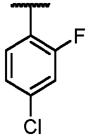 | R³-35 | 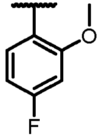 | R³-45 | 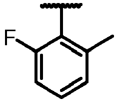 |
| R³-16 | 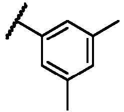 | R³-26 | 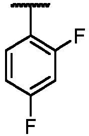 | R³-36 | 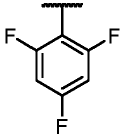 | R³-46 | 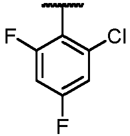 |
| R³-17 | 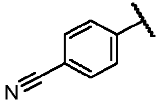 | R³-27 | 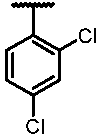 | R³-37 | 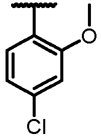 | R³-47 | 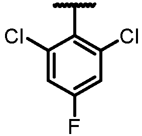 |
| R³-18 | 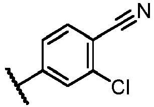 | R³-28 | 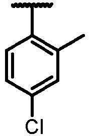 | R³-38 | 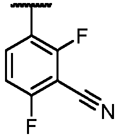 | R³-48 | 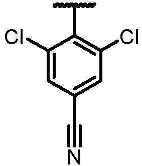 |
| R³-19 | 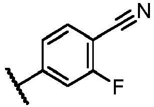 | R³-29 | 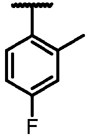 | R³-39 | 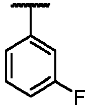 | R³-49 | 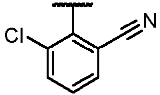 |
| R³-20 | 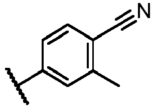 | R³-30 | 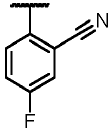 | R³-40 | 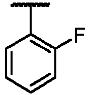 | R³-50 | 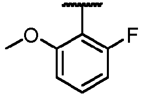 |

| | | | | | | | |
|---|---|---|---|---|---|---|---|
| R³-51 | 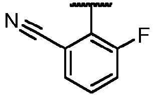 | R³-61 | 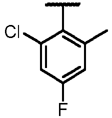 | R³-71 | 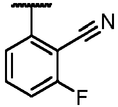 | R³-81 | 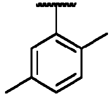 |
| R³-52 | 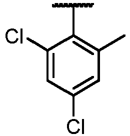 | R³-62 | 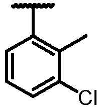 | R³-72 | 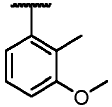 | R³-82 | 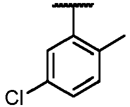 |
| R³-53 | 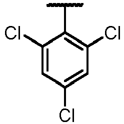 | R³-63 | 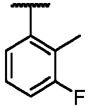 | R³-73 | 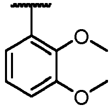 | R³-83 | 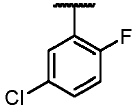 |
| R³-54 | 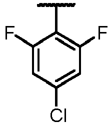 | R³-64 | 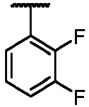 | R³-74 | 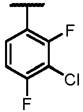 | R³-84 | 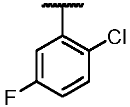 |
| R³-55 | 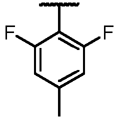 | R³-65 | 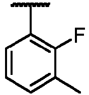 | R³-75 | 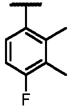 | R³-85 | 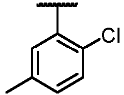 |
| R³-56 | 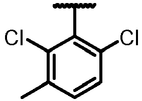 | R³-66 | 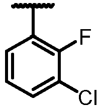 | R³-76 | 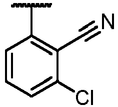 | R³-86 | 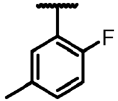 |
| R³-57 | 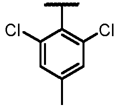 | R³-67 | 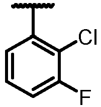 | R³-77 | 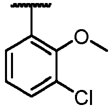 | R³-87 | 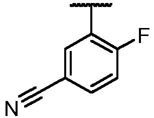 |
| R³-58 | 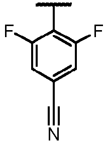 | R³-68 | 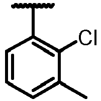 | R³-78 | 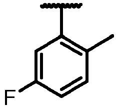 | R³-88 | 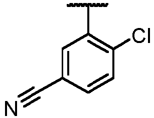 |
| R³-59 | 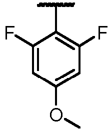 | R³-69 | 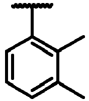 | R³-79 | 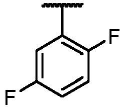 | R³-89 | 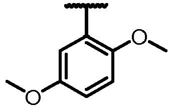 |
| R³-60 | 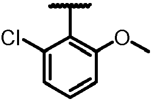 | R³-70 | 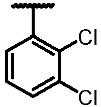 | R³-80 | 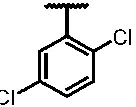 | R³-90 | 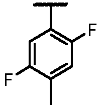 |
| R³-91 | 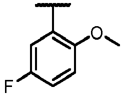 | R³-94 | 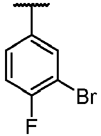 | R³-97 | 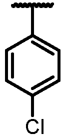 | | |
| R³-92 | 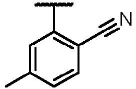 | R³-95 | 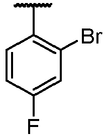 | R³-98 | 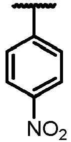 | | |
| R³-93 | 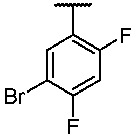 | R³-96 | 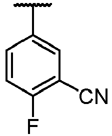 | R³-99 | 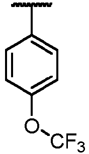 | | |

More preferably, R³ is 4-fluorophenyl and R^{1a}, R^{1b} and R² are defined as in any of the preceding embodiments.

In one preferred embodiment, R³ is selected from 4-fluorophenyl, 4-chlorophenyl, 4-nitrophenyl, 4-(trifluoromethoxy)phenyl, 3,4-difluorophenyl, 2,4-difluorophenyl, 3-fluoro-4-chlorophenyl, 3-chloro-4-fluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl, 3-chloro-2,4-difluorophenyl, 2,6-dichloro-4-fluorophenyl and 2-chloro-4-fluoro-6-methylphenyl, preferably R³ is selected from 4-fluorophenyl, 4-chlorophenyl, 4-nitrophenyl, 4-(trifluoromethoxy)phenyl, 3,4-difluorophenyl, 2,4-difluorophenyl, 3-fluoro-4-chlorophenyl and 3-chloro-4-fluorophenyl, more preferably R³ is selected from 4-fluorophenyl, 3,4-difluorophenyl, 2,4-difluorophenyl, 3-fluoro-4-chlorophenyl and 3-chloro-4-fluorophenyl, and

R² is selected from C₄₋₈ alkyl, (cyano)C₁₋₆ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₂₋₈ heterocyclyl and (C₂₋₈ heterocyclyl)C₁₋₃ alkyl, each of which may be optionally substituted by one or more groups selected from F, C!, Br, !, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiRs, wherein each R is independently selected from H and C₁₋₆ alkyl, and R^{1a} and R^{1b} are defined as in any of the preceding embodiments.

In another embodiment, R³ is 4-fluorophenyl, and R² is defined as in any of the preceding embodiments.

In one embodiment, R² is selected from C₄₋₈ alkyl, (cyano)C₁₋₆ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₂₋₈ heterocyclyl and (C₂₋₈ heterocyclyl)C₁₋₃ alkyl, each of which may be optionally substituted by one or more groups selected from F, Cl, Br, I, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiRs, wherein each R is independently selected from H and C₁₋₆ alkyl, and R³ is selected from 4-fluorophenyl, 3,4-difluorophenyl, 2,4-difluorophenyl, 3-fluoro-4-chlorophenyl and 3-chloro-4-fluorophenyl, preferably 4-fluorophenyl.

In another embodiment, R² is selected from C₄₋₈ alkyl, (cyano)C₁₋₆ alkyl, C₂₋₆ alkenyl, C₂₋₆ alkynyl, (C₃₋₆ cycloalkyl)C₁₋₃ alkyl and C₃₋₆ cycloalkyl, preferably R² is selected from C₄₋₈ alkyl, C₂₋₆ alkenyl and C₂₋₆ alkynyl, and R³ is defined as in any of the preceding embodiments. More preferably, R² is selected from cyanomethyl, cyclopropyl, sec-butyl, 1-butyn-4-yl, 2-butyn-1-yl, prop-1-yl, 3-methyl-1-butyn-3-yl, cyclopropylmethyl, 1-trimethylsilyl-2-butyn-4-yl, 1-buten-4-yl, 1-buten-3-yl, 2-methyl-1-propen-3-yl, allyl, iso-butyl, tert-butyl, cyclopentyl, propargyl and 1-butyn-3-yl, even more preferably R² is selected from 1-butyn-4-yl, 2-butyn-1-yl, prop-1-yl, 3-methyl-1-butyn-3-yl, allyl and propargyl, and R³ is defined as in any of the preceding embodiments. Alternatively, R² is selected from C₁₋₃ alkyl and R³ is defined as in any of the preceding embodiments. For example, R² is isopropyl and R³ is defined as in any of the preceding embodiments.

In one embodiment, R² is selected from C₁₋₃ alkyl, C₄₋₈ alkyl, (cyano)C₁₋₆ alkyl, C₂₋₆ alkenyl, C₂₋₆ alkynyl, (C₃₋₆ cycloalkyl)C₁₋₃ alkyl and C₃₋₆ cycloalkyl, R³ is selected from 4-fluorophenyl, 4-chlorophenyl, 4-nitrophenyl, 4-(trifluoromethoxy)phenyl, 3,4-difluorophenyl, 2,4-difluorophenyl, 3-fluoro-4-chlorophenyl, 3-chloro-4-fluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl, 3-chloro-2,4-difluorophenyl, 2,6-dichloro-4-fluorophenyl and 2-chloro-4-fluoro-6-methylphenyl, preferably R³ is selected from 4-fluorophenyl, 4-chlorophenyl, 4-nitrophenyl, 4-(trifluoromethoxy)phenyl, 3,4-difluorophenyl, 2,4-difluorophenyl, 3-fluoro-4-chlorophenyl and 3-chloro-4-fluorophenyl, more preferably R³ is selected from 4-fluorophenyl, 3,4-difluorophenyl, 2,4-difluorophenyl, 3-fluoro-4-chlorophenyl and 3-chloro-4-fluorophenyl. For example, in one embodiment, (i) R² is selected from cyanomethyl, cyclopropyl, sec-butyl, 1-butyn-4-yl, 2-butyn-1-yl, 3-methyl-1-butyn-3-yl, allyl, tert-butyl, cyclopentyl, propargyl, iso-butyl, 1-butyn-3-yl and 3-methylbut-2-yl, preferably R² is selected from 1-butyn-4-yl, 2-butyn-1-yl, 3-methyl-1-butyn-3-yl, allyl, propargyl and 1-butyn-3-yl, and R³ is 4-fluorophenyl; or (ii) R² is isopropyl, and R³ is selected from 3,4-difluorophenyl, 2,4-difluorophenyl, 3-fluoro-4-chlorophenyl and 3-chloro-4-fluorophenyl.

In a preferred embodiment, R² is selected from C₄₋₈ alkyl, (cyano)C₁₋₆ alkyl, C₂₋₆ alkenyl, C₂₋₆ alkynyl, (C₃₋₆ cycloalkyl)C₁₋₃ alkyl and C₃₋₆ cycloalkyl, R³ is selected from 4-fluorophenyl, 4-chlorophenyl, 4-nitrophenyl, 4-(trifluoromethoxy)phenyl, 3,4-difluorophenyl, 2,4-difluorophenyl, 3-fluoro-4-chlorophenyl, 3-chloro-4-fluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl, 3-chloro-2,4-difluorophenyl, 2,6-dichloro-4-fluorophenyl and 2-chloro-4-fluoro-6-methylphenyl, preferably R³ is selected from 4-fluorophenyl, 4-chlorophenyl, 4-nitrophenyl, 4-(trifluoromethoxy)phenyl, 3,4-difluorophenyl, 2,4-difluorophenyl, 3-fluoro-4-chlorophenyl and 3-chloro-4-fluorophenyl, more preferably R³ is selected from 4-fluorophenyl, 3,4-difluorophenyl, 2,4-difluorophenyl, 3-fluoro-4-chlorophenyl and 3-chloro-4-fluorophenyl. For example, R² is selected from cyanomethyl, cyclopropyl, sec-butyl, 1-butyn-4-yl, 2-butyn-1-yl, 3-methyl-1-butyn-3-yl, allyl, tert-butyl, cyclopentyl, propargyl, iso-butyl, 1-butyn-3-yl and 3-methylbut-2-yl, preferably R² is selected from 1-butyn-4-yl, 2-butyn-1-yl, 3-methyl-1-butyn-3-yl, allyl, propargyl and 1-butyn-3-yl, and R³ is 4-fluorophenyl.

In another preferred embodiment, the compound of formula I corresponds to Formula Ia and has a structure selected from the following Table 3.

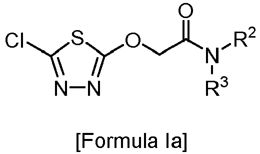

**Table 3. Preferred compounds of Formula Ia (the 6 compounds identified by * are excluded from the scope of the present invention.**

| R³ | R² | | | | | | | | | | |
|---|---|---|---|---|---|---|---|---|---|---|---|
| | R²-1 | R²-2 | R²-3 | R²-4 | R²-5 | R²-6 | R²-7 | R²-8 | R²-9 | R²-10 | R²-11 |
| R³-1 | **I-1** | **I-2** | **I-3** | **I-4** | **I-5** | **I-6** | **I-7** | **I-8** | **I-9*** | **I-10** | **I-11** |
| R³-2 | **I-12** | **I-13** | **I-14** | **I-15** | **I-16** | **I-17** | **I-18** | **I-19** | **I-20*** | **I-21** | **I-22** |
| R³-3 | **I-23** | **I-24** | **I-25** | **I-26** | **I-27** | **I-28** | **I-29** | **I-30** | **I-31** | **I-32** | **I-33** |
| R³-4 | **I-34** | **I-35** | **I-36** | **I-37** | **I-38** | **I-39** | **I-40** | **I-41** | **I-42** | **I-43** | **I-44** |
| R³-5 | **I-45** | **I-46** | **I-47** | **I-48** | **I-49** | **I-50** | **I-51** | **I-52** | **I-53** | **I-54** | **I-55** |
| R³-6 | **I-56** | **I-57** | **I-58** | **I-59** | **I-60** | **I-61** | **I-62** | **I-63** | **I-64** | **I-65** | **I-66** |
| R³-7 | **I-67** | **I-68** | **I-69** | **I-70** | **I-71** | **I-72** | **I-73** | **I-74** | **I-75** | **I-76** | **I-77** |
| R³-8 | **I-78** | **I-79** | **I-80** | **I-81** | **I-82** | **I-83** | **I-84** | **I-85** | **I-86** | **I-87** | **I-88** |
| R³-9 | **I-89** | **I-90** | **I-91** | **I-92** | **I-93** | **I-94** | **I-95** | **I-96** | **I-97** | **I-98** | **I-99** |
| R³-10 | **I-100** | **I-101** | **I-102** | **I-103** | **I-104** | **I-105** | **I-106** | **I-107** | **I-108*** | **I-109** | **I-110** |
| R³-11 | **I-111** | **I-112** | **I-113** | **I-114** | **I-115** | **I-116** | **I-117** | **I-118** | **I-119** | **I-120** | **I-121** |
| R³-12 | **I-122** | **I-123** | **I-124** | **I-125** | **I-126** | **I-127** | **I-128** | **I-129** | **I-130** | **I-131** | **I-132** |
| R³-13 | **I-133** | **I-134** | **I-135** | **I-136** | **I-137** | **I-138** | **I-139** | **I-140** | **I-141*** | **I-142** | **I-143** |
| R³-14 | **I-144** | **I-145** | **I-146** | **I-147** | **I-148** | **I-149** | **I-150** | **I-151** | **I-152** | **I-153** | **I-154** |
| R³-15 | **I-155** | **I-156** | **I-157** | **I-158** | **I-159** | **I-160** | **I-161** | **I-162** | **I-163** | **I-164** | **I-165** |
| R³-16 | **I-166** | **I-167** | **I-168** | **I-169** | **I-170** | **I-171** | **I-172** | **I-173** | **I-174*** | **I-175** | **I-176** |
| R³-17 | **I-177** | **I-178** | **I-179** | **I-180** | **I-181** | **I-182** | **I-183** | **I-184** | **I-185** | **I-186** | **I-187** |
| R³-18 | **I-188** | **I-189** | **I-190** | **I-191** | **I-192** | **I-193** | **I-194** | **I-195** | **I-196** | **I-197** | **I-198** |
| R³-19 | **I-199** | **I-200** | **I-201** | **I-202** | **I-203** | **I-204** | **I-205** | **I-206** | **I-207** | **I-208** | **I-209** |
| R³-20 | **I-210** | **I-211** | **I-212** | **I-213** | **I-214** | **I-215** | **I-216** | **I-217** | **I-218** | **I-219** | **I-220** |
| R³-21 | **I-221** | **I-222** | **I-223** | **I-224** | **I-225** | **I-226** | **I-227** | **I-228** | **I-229** | **I-230** | **I-231** |
| R³-22 | **I-232** | **I-233** | **I-234** | **I-235** | **I-236** | **I-237** | **I-238** | **I-239** | **I-240** | **I-241** | **I-242** |
| R³-23 | **I-243** | **I-244** | **I-245** | **I-246** | **I-247** | **I-248** | **I-249** | **I-250** | **I-251** | **I-252** | **I-253** |
| R³-24 | **I-254** | **I-255** | **I-256** | **I-257** | **I-258** | **I-259** | **I-260** | **I-261** | **I-262** | **I-263** | **I-264** |
| R³-25 | **I-265** | **I-266** | **I-267** | **I-268** | **I-269** | **I-270** | **I-271** | **I-272** | **I-273** | **I-274** | **I-275** |
| R³-26 | **I-276** | **I-277** | **I-278** | **I-279** | **I-280** | **I-281** | **I-282** | **I-283** | **I-284** | **I-285** | **I-286** |
| R³-27 | **I-287** | **I-288** | **I-289** | **I-290** | **I-291** | **I-292** | **I-293** | **I-294** | **I-295** | **I-296** | **I-297** |
| R³-28 | **I-298** | **I-299** | **I-300** | **I-301** | **I-302** | **I-303** | **I-304** | **I-305** | **I-306** | **I-307** | **I-308** |
| R³-29 | **I-309** | **I-310** | **I-311** | **I-312** | **I-313** | **I-314** | **I-315** | **I-316** | **I-317** | **I-318** | **I-319** |
| R³-30 | **I-320** | **I-321** | **I-322** | **I-323** | **I-324** | **I-325** | **I-326** | **I-327** | **I-328** | **I-329** | **I-330** |
| R³-31 | **I-331** | **I-332** | **I-333** | **I-334** | **I-335** | **I-336** | **I-337** | **I-338** | **I-339** | **I-340** | **I-341** |
| R³-32 | **I-342** | **I-343** | **I-344** | **I-345** | **I-346** | **I-347** | **I-348** | **I-349** | **I-350** | **I-351** | **I-352** |
| R³-33 | **I-353** | **I-354** | **I-355** | **I-356** | **I-357** | **I-358** | **I-359** | **I-360** | **I-361** | **I-362** | **I-363** |
| R³-34 | **I-364** | **I-365** | **I-366** | **I-367** | **I-368** | **I-369** | **I-370** | **I-371** | **I-372** | **I-373** | **I-374** |
| R³-35 | **I-375** | **I-376** | **I-377** | **I-378** | **I-379** | **I-380** | **I-381** | **I-382** | **I-383** | **I-384** | **I-385** |
| R³-36 | **I-386** | **I-387** | **I-388** | **I-389** | **I-390** | **I-391** | **I-392** | **I-393** | **I-394** | **I-395** | **I-396** |
| R³-37 | **I-397** | **I-398** | **I-399** | **I-400** | **I-401** | **I-402** | **I-403** | **I-404** | **I-405** | **I-406** | **I-407** |
| R³-38 | **I-408** | **I-409** | **I-410** | **I-411** | **I-412** | **I-413** | **I-414** | **I-415** | **I-416** | **I-417** | **I-418** |
| R³-39 | **I-419** | **I-420** | **I-421** | **I-422** | **I-423** | **I-424** | **I-425** | **I-426** | **I-427** | **I-428** | **I-429** |
| R³-40 | **I-430** | **I-431** | **I-432** | **I-433** | **I-434** | **I-435** | **I-436** | **I-437** | **I-438** | **I-439** | **I-440** |
| R³-41 | **I-441** | **I-442** | **I-443** | **I-444** | **I-445** | **I-446** | **I-447** | **I-448** | **I-449** | **I-450** | **I-451** |
| R³-42 | **I-452** | **I-453** | **I-454** | **I-455** | **I-456** | **I-457** | **I-458** | **I-459** | **I-460** | **I-461** | **I-462** |
| R³-43 | **I-463** | **I-464** | **I-465** | **I-466** | **I-467** | **I-468** | **I-469** | **I-470** | **I-471** | **I-472** | **I-473** |
| R³-44 | **I-474** | **I-475** | **I-476** | **I-477** | **I-478** | **I-479** | **I-480** | **I-481** | **I-482** | **I-483** | **I-484** |
| R³-45 | **I-485** | **I-486** | **I-487** | **I-488** | **I-489** | **I-490** | **I-491** | **I-492** | **I-493** | **I-494** | **I-495** |
| R³-46 | **I-496** | **I-497** | **I-498** | **I-499** | **I-500** | **I-501** | **I-502** | **I-503** | **I-504** | **I-505** | **I-506** |
| R³-47 | **I-507** | **I-508** | **I-509** | **I-510** | **I-511** | **I-512** | **I-513** | **I-514** | **I-515** | **I-516** | **I-517** |
| R³-48 | **I-518** | **I-519** | **I-520** | **I-521** | **I-522** | **I-523** | **I-524** | **I-525** | **I-526** | **I-527** | **I-528** |
| R³-49 | **I-529** | **I-530** | **I-531** | **I-532** | **I-533** | **I-534** | **I-535** | **I-536** | **I-537** | **I-538** | **I-539** |
| R³-50 | **I-540** | **I-541** | **I-542** | **I-543** | **I-544** | **I-545** | **I-546** | **I-547** | **I-548** | **I-549** | **I-550** |
| R³-51 | **I-551** | **I-552** | **I-553** | **I-554** | **I-555** | **I-556** | **I-557** | **I-558** | **I-559** | **I-560** | **I-561** |
| R³-52 | **I-562** | **I-563** | **I-564** | **I-565** | **I-566** | **I-567** | **I-568** | **I-569** | **I-570** | **I-571** | **I-572** |
| R³-53 | **I-573** | **I-574** | **I-575** | **I-576** | **I-577** | **I-578** | **I-579** | **I-580** | **I-581** | **I-582** | **I-583** |
| R³-54 | **I-584** | **I-585** | **I-586** | **I-587** | **I-588** | **I-589** | **I-590** | **I-591** | **I-592** | **I-593** | **I-594** |
| R³-55 | **I-595** | **I-596** | **I-597** | **I-598** | **I-599** | **I-600** | **I-601** | **I-602** | **I-603** | **I-604** | **I-605** |
| R³-56 | **I-606** | **I-607** | **I-608** | **I-609** | **I-610** | **I-611** | **I-612** | **I-613** | **I-614** | **I-615** | **I-616** |
| R³-57 | **I-617** | **I-618** | **I-619** | **I-620** | **I-621** | **I-622** | **I-623** | **I-624** | **I-625** | **I-626** | **I-627** |
| R³-58 | **I-628** | **I-629** | **I-630** | **I-631** | **I-632** | **I-633** | **I-634** | **I-635** | **I-636** | **I-637** | **I-638** |
| R³-59 | **I-639** | **I-640** | **I-641** | **I-642** | **I-643** | **I-644** | **I-645** | **I-646** | **I-647** | **I-648** | **I-649** |
| R³-60 | **I-650** | **I-651** | **I-652** | **I-653** | **I-654** | **I-655** | **I-656** | **I-657** | **I-658** | **I-659** | **I-660** |
| R³-61 | **I-661** | **I-662** | **I-663** | **I-664** | **I-665** | **I-666** | **I-667** | **I-668** | **I-669** | **I-670** | **I-671** |
| R³-62 | **I-672** | **I-673** | **I-674** | **I-675** | **I-676** | **I-677** | **I-678** | **I-679** | **I-680** | **I-681** | **I-682** |
| R³-63 | **I-683** | **I-684** | **I-685** | **I-686** | **I-687** | **I-688** | **I-689** | **I-690** | **I-691** | **I-692** | **I-693** |
| R³-64 | **I-694** | **I-695** | **I-696** | **I-697** | **I-698** | **I-699** | **I-700** | **I-701** | **I-702** | **I-703** | **I-704** |
| R³-65 | **I-705** | **I-706** | **I-707** | **I-708** | **I-709** | **I-710** | **I-711** | **I-712** | **I-713** | **I-714** | **I-715** |
| R³-66 | **I-716** | **I-717** | **I-718** | **I-719** | **I-720** | **I-721** | **I-722** | **I-723** | **I-724** | **I-725** | **I-726** |
| R³-67 | **I-727** | **I-728** | **I-729** | **I-730** | **I-731** | **I-732** | **I-733** | **I-734** | **I-735** | **I-736** | **I-737** |
| R³-68 | **I-738** | **I-739** | **I-740** | **I-741** | **I-742** | **I-743** | **I-744** | **I-745** | **I-746** | **I-747** | **I-748** |
| R³-69 | **I-749** | **I-750** | **I-751** | **I-752** | **I-753** | **I-754** | **I-755** | **I-756** | **I-757** | **I-758** | **I-759** |
| R³-70 | **I-760** | **I-761** | **I-762** | **I-763** | **I-764** | **I-765** | **I-766** | **I-767** | **I-768** | **I-769** | **I-770** |
| R³-71 | **I-771** | **I-772** | **I-773** | **I-774** | **I-775** | **I-776** | **I-777** | **I-778** | **I-779** | **I-780** | **I-781** |
| R³-72 | **I-782** | **I-783** | **I-784** | **I-785** | **I-786** | **I-787** | **I-788** | **I-789** | **I-790** | **I-791** | **I-792** |
| R³-73 | **I-793** | **I-794** | **I-795** | **I-796** | **I-797** | **I-798** | **I-799** | **I-800** | **I-801** | **I-802** | **I-803** |
| R³-74 | **I-804** | **I-805** | **I-806** | **I-807** | **I-808** | **I-809** | **I-810** | **I-811** | **I-812** | **I-813** | **I-814** |
| R³-75 | **I-815** | **I-816** | **I-817** | **I-818** | **I-819** | **I-820** | **I-821** | **I-822** | **I-823** | **I-824** | **I-825** |
| R³-76 | **I-826** | **I-827** | **I-828** | **I-829** | **I-830** | **I-831** | **I-832** | **I-833** | **I-834** | **I-835** | **I-836** |
| R³-77 | **I-837** | **I-838** | **I-839** | **I-840** | **I-841** | **I-842** | **I-843** | **I-844** | **I-845** | **I-846** | **I-847** |
| R³-78 | **I-848** | **I-849** | **I-850** | **I-851** | **I-852** | **I-853** | **I-854** | **I-855** | **I-856** | **I-857** | **I-858** |
| R³-79 | **I-859** | **I-860** | **I-861** | **I-862** | **I-863** | **I-864** | **I-865** | **I-866** | **I-867** | **I-868** | **I-869** |
| R³-80 | **I-870** | **I-871** | **I-872** | **I-873** | **I-874** | **I-875** | **I-876** | **I-877** | **I-878** | **I-879** | **I-880** |
| R³-81 | **I-881** | **I-882** | **I-883** | **I-884** | **I-885** | **I-886** | **I-887** | **I-888** | **I-889** | **I-890** | **I-891** |
| R³-82 | **I-892** | **I-893** | **I-894** | **I-895** | **I-896** | **I-897** | **I-898** | **I-899** | **I-900*** | **I-901** | **I-902** |
| R³-83 | **I-903** | **I-904** | **I-905** | **I-906** | **I-907** | **I-908** | **I-909** | **I-910** | **I-911** | **I-912** | **I-913** |
| R³-84 | **I-914** | **I-915** | **I-916** | **I-917** | **I-918** | **I-919** | **I-920** | **I-921** | **I-922** | **I-923** | **I-924** |
| R³-85 | **I-925** | **I-926** | **I-927** | **I-928** | **I-929** | **I-930** | **I-931** | **I-932** | **I-933** | **I-934** | **I-935** |
| R³-86 | **I-936** | **I-937** | **I-938** | **I-939** | **I-940** | **I-941** | **I-942** | **I-943** | **I-944** | **I-945** | **I-946** |
| R³-87 | **I-947** | **I-948** | **I-949** | **I-950** | **I-951** | **I-952** | **I-953** | **I-954** | **I-955** | **I-956** | **I-957** |
| R³-88 | **I-958** | **I-959** | **I-960** | **I-961** | **I-962** | **I-963** | **I-964** | **I-965** | **I-966** | **I-967** | **I-968** |
| R³-89 | **I-969** | **I-970** | **I-971** | **I-972** | **I-973** | **I-974** | **I-975** | **I-976** | **I-977** | **I-978** | **I-979** |
| R³-90 | **I-980** | **I-981** | **I-982** | **I-983** | **I-984** | **I-985** | **I-986** | **I-987** | **I-988** | **I-989** | **I-990** |
| R³-91 | **I-991** | **I-992** | **I-993** | **I-994** | **I-995** | **I-996** | **I-997** | **I-998** | **I-999** | **I-1000** | **I-1001** |
| R³-92 | **I-1002** | **I-1003** | **I-1004** | **I-1005** | **I-1006** | **I-1007** | **I-1008** | **I-1009** | **I-1010** | **I-1011** | **I-1012** |
| R³-93 | **I-1013** | **I-1014** | **I-1015** | **I-1016** | **I-1017** | **I-1018** | **I-1019** | **I-1020** | **I-1021** | **I-1022** | **I-1023** |
| R³-94 | **I-1024** | **I-1025** | **I-1026** | **I-1027** | **I-1028** | **I-1029** | **I-1030** | **I-1031** | **I-1032** | **I-1033** | **I-1034** |
| R³-95 | **I-1035** | **I-1036** | **I-1037** | **I-1038** | **I-1039** | **I-1040** | **I-1041** | **I-1042** | **I-1043** | **I-1044** | **I-1045** |
| R³-96 | **I-1046** | **I-1047** | **I-1048** | **I-1049** | **I-1050** | **I-1051** | **I-1052** | **I-1053** | **I-1054** | **I-1055** | **I-1056** |
| R³-97 | **I-1057** | **I-1058** | **I-1059** | **I-1060** | **I-1061** | **I-1062** | **I-1063** | **I-1064** | **I-1065** | **I-1066** | **I-1067** |
| R³-98 | **I-1068** | **I-1069** | **I-1070** | **I-1071** | **I-1072** | **I-1073** | **I-1074** | **I-1075** | **I-1076** | **I-1077** | **I-1078** |
| R³-99 | **I-1079** | **I-1080** | **I-1081** | **I-1082** | **I-1083** | **I-1084** | **I-1085** | **I-1086** | **I-1087** | **I-1088** | **I-1089** |

| R³ | R² | | | | | | | | | | |
|---|---|---|---|---|---|---|---|---|---|---|---|
| | R²-12 | R²-13 | R²-14 | R²-15 | R²-16 | R²-17 | R²-18 | R²-19 | R²-20 | R²-21 | R²-22 |
| R³-1 | **II-1** | **II-2** | **II-3** | **II-4** | **II-5** | **II-6** | **II-7** | **II-8** | **II-9** | **II-10** | **II-11** |
| R³-2 | **II-12** | **II-13** | **II-14** | **II-15** | **II-16** | **II-17** | **II-18** | **II-19** | **II-20** | **II-21** | **II-22** |
| R³-3 | **II-23** | **II-24** | **II-25** | **II-26** | **II-27** | **II-28** | **II-29** | **II-30** | **II-31** | **II-32** | **II-33** |
| R³-4 | **II-34** | **II-35** | **II-36** | **II-37** | **II-38** | **II-39** | **II-40** | **II-41** | **II-42** | **II-43** | **II-44** |
| R³-5 | **II-45** | **II-46** | **II-47** | **II-48** | **II-49** | **II-50** | **II-51** | **II-52** | **II-53** | **II-54** | **II-55** |
| R³-6 | **II-56** | **II-57** | **II-58** | **II-59** | **II-60** | **II-61** | **II-62** | **II-63** | **II-64** | **II-65** | **II-66** |
| R³-7 | **II-67** | **II-68** | **II-69** | **II-70** | **II-71** | **II-72** | **II-73** | **II-74** | **II-75** | **II-76** | **II-77** |
| R³-8 | **II-78** | **II-79** | **II-80** | **II-81** | **II-82** | **II-83** | **II-84** | **II-85** | **II-86** | **II-87** | **II-88** |
| R³-9 | **II-89** | **II-90** | **II-91** | **II-92** | **II-93** | **II-94** | **II-95** | **II-96** | **II-97** | **II-98** | **II-99** |
| R³-10 | **II-100** | **II-101** | **II-102** | **II-103** | **II-104** | **II-105** | **II-106** | **II-107** | **II-108** | **II-109** | **II-110** |
| R³-11 | **II-111** | **II-112** | **II-113** | **II-114** | **II-115** | **II-116** | **II-117** | **II-118** | **II-119** | **II-120** | **II-121** |
| R³-12 | **II-122** | **II-123** | **II-124** | **II-125** | **II-126** | **II-127** | **II-128** | **II-129** | **II-130** | **II-131** | **II-132** |
| R³-13 | **II-133** | **II-134** | **II-135** | **II-136** | **II-137** | **II-138** | **II-139** | **II-140** | **II-141** | **II-142** | **II-143** |
| R³-14 | **II-144** | **II-145** | **II-146** | **II-147** | **II-148** | **II-149** | **II-150** | **II-151** | **II-152** | **II-153** | **II-154** |
| R³-15 | **II-155** | **II-156** | **II-157** | **II-158** | **II-159** | **II-160** | **II-161** | **II-162** | **II-163** | **II-164** | **II-165** |
| R³-16 | **II-166** | **II-167** | **II-168** | **II-169** | **II-170** | **II-171** | **II-172** | **II-173** | **II-174** | **II-175** | **II-176** |
| R³-17 | **II-177** | **II-178** | **II-179** | **II-180** | **II-181** | **II-182** | **II-183** | **II-184** | **II-185** | **II-186** | **II-187** |
| R³-18 | **II-188** | **II-189** | **II-190** | **II-191** | **II-192** | **II-193** | **II-194** | **II-195** | **II-196** | **II-197** | **II-198** |
| R³-19 | **II-199** | **II-200** | **II-201** | **II-202** | **II-203** | **II-204** | **II-205** | **II-206** | **II-207** | **II-208** | **II-209** |
| R³-20 | **II-210** | **II-211** | **II-212** | **II-213** | **II-214** | **II-215** | **II-216** | **II-217** | **II-218** | **II-219** | **II-220** |
| R³-21 | **II-221** | **II-222** | **II-223** | **II-224** | **II-225** | **II-226** | **II-227** | **II-228** | **II-229** | **II-230** | **II-231** |
| R³-22 | **II-232** | **II-233** | **II-234** | **II-235** | **II-236** | **II-237** | **II-238** | **II-239** | **II-240** | **II-241** | **II-242** |
| R³-23 | **II-243** | **II-244** | **II-245** | **II-246** | **II-247** | **II-248** | **II-249** | **II-250** | **II-251** | **II-252** | **II-253** |
| R³-24 | **II-254** | **II-255** | **II-256** | **II-257** | **II-258** | **II-259** | **II-260** | **II-261** | **II-262** | **II-263** | **II-264** |
| R³-25 | **II-265** | **II-266** | **II-267** | **II-268** | **II-269** | **II-270** | **II-271** | **II-272** | **II-273** | **II-274** | **II-275** |
| R³-26 | **II-276** | **II-277** | **II-278** | **II-279** | **II-280** | **II-281** | **II-282** | **II-283** | **II-284** | **II-285** | **II-286** |
| R³-27 | **II-287** | **II-288** | **II-289** | **II-290** | **II-291** | **II-292** | **II-293** | **II-294** | **II-295** | **II-296** | **II-297** |
| R³-28 | **II-298** | **II-299** | **II-300** | **II-301** | **II-302** | **II-303** | **II-304** | **II-305** | **II-306** | **II-307** | **II-308** |
| R³-29 | **II-309** | **II-310** | **II-311** | **II-312** | **II-313** | **II-314** | **II-315** | **II-316** | **II-317** | **II-318** | **II-319** |
| R³-30 | **II-320** | **II-321** | **II-322** | **II-323** | **II-324** | **II-325** | **II-326** | **II-327** | **II-328** | **II-329** | **II-330** |
| R³-31 | **II-331** | **II-332** | **II-333** | **II-334** | **II-335** | **II-336** | **II-337** | **II-338** | **II-339** | **II-340** | **II-341** |
| R³-32 | **II-342** | **II-343** | **II-344** | **II-345** | **II-346** | **II-347** | **II-348** | **II-349** | **II-350** | **II-351** | **II-352** |
| R³-33 | **II-353** | **II-354** | **II-355** | **II-356** | **II-357** | **II-358** | **II-359** | **II-360** | **II-361** | **II-362** | **II-363** |
| R³-34 | **II-364** | **II-365** | **II-366** | **II-367** | **II-368** | **II-369** | **II-370** | **II-371** | **II-372** | **II-373** | **II-374** |
| R³-35 | **II-375** | **II-376** | **II-377** | **II-378** | **II-379** | **II-380** | **II-381** | **II-382** | **II-383** | **II-384** | **II-385** |
| R³-36 | **II-386** | **II-387** | **II-388** | **II-389** | **II-390** | **II-391** | **II-392** | **II-393** | **II-394** | **II-395** | **II-396** |
| R³-37 | **II-397** | **II-398** | **II-399** | **II-400** | **II-401** | **II-402** | **II-403** | **II-404** | **II-405** | **II-406** | **II-407** |
| R³-38 | **II-408** | **II-409** | **II-410** | **II-411** | **II-412** | **II-413** | **II-414** | **II-415** | **II-416** | **II-417** | **II-418** |
| R³-39 | **II-419** | **II-420** | **II-421** | **II-422** | **II-423** | **II-424** | **II-425** | **II-426** | **II-427** | **II-428** | **II-429** |
| R³-40 | **II-430** | **II-431** | **II-432** | **II-433** | **II-434** | **II-435** | **II-436** | **II-437** | **II-438** | **II-439** | **II-440** |
| R³-41 | **II-441** | **II-442** | **II-443** | **II-444** | **II-445** | **II-446** | **II-447** | **II-448** | **II-449** | **II-450** | **II-451** |
| R³-42 | **II-452** | **II-453** | **II-454** | **II-455** | **II-456** | **II-457** | **II-458** | **II-459** | **II-460** | **II-461** | **II-462** |
| R³-43 | **II-463** | **II-464** | **II-465** | **II-466** | **II-467** | **II-468** | **II-469** | **II-470** | **II-471** | **II-472** | **II-473** |
| R³-44 | **II-474** | **II-475** | **II-476** | **II-477** | **II-478** | **II-479** | **II-480** | **II-481** | **II-482** | **II-483** | **II-484** |
| R³-45 | **II-485** | **II-486** | **II-487** | **II-488** | **II-489** | **II-490** | **II-491** | **II-492** | **II-493** | **II-494** | **II-495** |
| R³-46 | **II-496** | **II-497** | **II-498** | **II-499** | **II-500** | **II-501** | **II-502** | **II-503** | **II-504** | **II-505** | **II-506** |
| R³-47 | **II-507** | **II-508** | **II-509** | **II-510** | **II-511** | **II-512** | **II-513** | **II-514** | **II-515** | **II-516** | **II-517** |
| R³-48 | **II-518** | **II-519** | **II-520** | **II-521** | **II-522** | **II-523** | **II-524** | **II-525** | **II-526** | **II-527** | **II-528** |
| R³-49 | **II-529** | **II-530** | **II-531** | **II-532** | **II-533** | **II-534** | **II-535** | **II-536** | **II-537** | **II-538** | **II-539** |
| R³-50 | **II-540** | **II-541** | **II-542** | **II-543** | **II-544** | **II-545** | **II-546** | **II-547** | **II-548** | **II-549** | **II-550** |
| R³-51 | **II-551** | **II-552** | **II-553** | **II-554** | **II-555** | **II-556** | **II-557** | **II-558** | **II-559** | **II-560** | **II-561** |
| R³-52 | **II-562** | **II-563** | **II-564** | **II-565** | **II-566** | **II-567** | **II-568** | **II-569** | **II-570** | **II-571** | **II-572** |
| R³-53 | **II-573** | **II-574** | **II-575** | **II-576** | **II-577** | **II-578** | **II-579** | **II-580** | **II-581** | **II-582** | **II-583** |
| R³-54 | **II-584** | **II-585** | **II-586** | **II-587** | **II-588** | **II-589** | **II-590** | **II-591** | **II-592** | **II-593** | **II-594** |
| R³-55 | **II-595** | **II-596** | **II-597** | **II-598** | **II-599** | **II-600** | **II-601** | **II-602** | **II-603** | **II-604** | **II-605** |
| R³-56 | **II-606** | **II-607** | **II-608** | **II-609** | **II-610** | **II-611** | **II-612** | **II-613** | **II-614** | **II-615** | **II-616** |
| R³-57 | **II-617** | **II-618** | **II-619** | **II-620** | **II-621** | **II-622** | **II-623** | **II-624** | **II-625** | **II-626** | **II-627** |
| R³-58 | **II-628** | **II-629** | **II-630** | **II-631** | **II-632** | **II-633** | **II-634** | **II-635** | **II-636** | **II-637** | **II-638** |
| R³-59 | **II-639** | **II-640** | **II-641** | **II-642** | **II-643** | **II-644** | **II-645** | **II-646** | **II-647** | **II-648** | **II-649** |
| R³-60 | **II-650** | **II-651** | **II-652** | **II-653** | **II-654** | **II-655** | **II-656** | **II-657** | **II-658** | **II-659** | **II-660** |
| R³-61 | **II-661** | **II-662** | **II-663** | **II-664** | **II-665** | **II-666** | **II-667** | **II-668** | **II-669** | **II-670** | **II-671** |
| R³-62 | **II-672** | **II-673** | **II-674** | **II-675** | **II-676** | **II-677** | **II-678** | **II-679** | **II-680** | **II-681** | **II-682** |
| R³-63 | **II-683** | **II-684** | **II-685** | **II-686** | **II-687** | **II-688** | **II-689** | **II-690** | **II-691** | **II-692** | **II-693** |
| R³-64 | **II-694** | **II-695** | **II-696** | **II-697** | **II-698** | **II-699** | **II-700** | **II-701** | **II-702** | **II-703** | **II-704** |
| R³-65 | **II-705** | **II-706** | **II-707** | **II-708** | **II-709** | **II-710** | **II-711** | **II-712** | **II-713** | **II-714** | **II-715** |
| R³-66 | **II-716** | **II-717** | **II-718** | **II-719** | **II-720** | **II-721** | **II-722** | **II-723** | **II-724** | **II-725** | **II-726** |
| R³-67 | **II-727** | **II-728** | **II-729** | **II-730** | **II-731** | **II-732** | **II-733** | **II-734** | **II-735** | **II-736** | **II-737** |
| R³-68 | **II-738** | **II-739** | **II-740** | **II-741** | **II-742** | **II-743** | **II-744** | **II-745** | **II-746** | **II-747** | **II-748** |
| R³-69 | **II-749** | **II-750** | **II-751** | **II-752** | **II-753** | **II-754** | **II-755** | **II-756** | **II-757** | **II-758** | **II-759** |
| R³-70 | **II-760** | **II-761** | **II-762** | **II-763** | **II-764** | **II-765** | **II-766** | **II-767** | **II-768** | **II-769** | **II-770** |
| R³-71 | **II-771** | **II-772** | **II-773** | **II-774** | **II-775** | **II-776** | **II-777** | **II-778** | **II-779** | **II-780** | **II-781** |
| R³-72 | **II-782** | **II-783** | **II-784** | **II-785** | **II-786** | **II-787** | **II-788** | **II-789** | **II-790** | **II-791** | **II-792** |
| R³-73 | **II-793** | **II-794** | **II-795** | **II-796** | **II-797** | **II-798** | **II-799** | **II-800** | **II-801** | **II-802** | **II-803** |
| R³-74 | **II-804** | **II-805** | **II-806** | **II-807** | **II-808** | **II-809** | **II-810** | **II-811** | **II-812** | **II-813** | **II-814** |
| R³-75 | **II-815** | **II-816** | **II-817** | **II-818** | **II-819** | **II-820** | **II-821** | **II-822** | **II-823** | **II-824** | **II-825** |
| R³-76 | **II-826** | **II-827** | **II-828** | **II-829** | **II-830** | **II-831** | **II-832** | **II-833** | **II-834** | **II-835** | **II-836** |
| R³-77 | **II-837** | **II-838** | **II-839** | **II-840** | **II-841** | **II-842** | **II-843** | **II-844** | **II-845** | **II-846** | **II-847** |
| **R³-78** | **II-848** | **II-849** | **II-850** | **II-851** | **II-852** | **II-853** | **II-854** | **II-855** | **II-856** | **II-857** | **II-858** |
| R³-79 | **II-859** | **II-860** | **II-861** | **II-862** | **II-863** | **II-864** | **II-865** | **II-866** | **II-867** | **II-868** | **II-869** |
| R³-80 | **II-870** | **II-871** | **II-872** | **II-873** | **II-874** | **II-875** | **II-876** | **II-877** | **II-878** | **II-879** | **II-880** |
| R³-81 | **II-881** | **II-882** | **II-883** | **II-884** | **II-885** | **II-886** | **II-887** | **II-888** | **II-889** | **II-890** | **II-891** |
| R³-82 | **II-892** | **II-893** | **II-894** | **II-895** | **II-896** | **II-897** | **II-898** | **II-899** | **II-900** | **II-901** | **II-902** |
| R³-83 | **II-903** | **II-904** | **II-905** | **II-906** | **II-907** | **II-908** | **II-909** | **II-910** | **II-911** | **II-912** | **II-913** |
| R³-84 | **II-914** | **II-915** | **II-916** | **II-917** | **II-918** | **II-919** | **II-920** | **II-921** | **II-922** | **II-923** | **II-924** |
| R³-85 | **II-925** | **II-926** | **II-927** | **II-928** | **II-929** | **II-930** | **II-931** | **II-932** | **II-933** | **II-934** | **II-935** |
| R³-86 | **II-936** | **II-937** | **II-938** | **II-939** | **II-940** | **II-941** | **II-942** | **II-943** | **II-944** | **II-945** | **II-946** |
| R³-87 | **II-947** | **II-948** | **II-949** | **II-950** | **II-951** | **II-952** | **II-953** | **II-954** | **II-955** | **II-956** | **II-957** |
| R³-88 | **II-958** | **II-959** | **II-960** | **II-961** | **II-962** | **II-963** | **II-964** | **II-965** | **II-966** | **II-967** | **II-968** |
| R³-89 | **II-969** | **II-970** | **II-971** | **II-972** | **II-973** | **II-974** | **II-975** | **II-976** | **II-977** | **II-978** | **II-979** |
| R³-90 | **II-980** | **II-981** | **II-982** | **II-983** | **II-984** | **II-985** | **II-986** | **II-987** | **II-988** | **II-989** | **II-990** |
| R³-91 | **II-991** | **II-992** | **II-993** | **II-994** | **II-995** | **II-996** | **II-997** | **II-998** | **II-999** | **II-1000** | **II-1001** |
| R³-92 | **II-1002** | **II-1003** | **II-1004** | **II-1005** | **II-1006** | **II-1007** | **II-1008** | **II-1009** | **II-1010** | **II-1011** | **II-1012** |
| R³-93 | **II-1013** | **II-1014** | **II-1015** | **II-1016** | **II-1017** | **II-1018** | **II-1019** | **II-1020** | **II-1021** | **II-1022** | **II-1023** |
| R³-94 | **II-1024** | **II-1025** | **II-1026** | **II-1027** | **II-1028** | **II-1029** | **II-1030** | **II-1031** | **II-1032** | **II-1033** | **II-1034** |
| R³-95 | **II-1035** | **II-1036** | **II-1037** | **II-1038** | **II-1039** | **II-1040** | **II-1041** | **II-1042** | **II-1043** | **II-1044** | **II-1045** |
| R³-96 | **II-1046** | **II-1047** | **II-1048** | **II-1049** | **II-1050** | **II-1051** | **II-1052** | **II-1053** | **II-1054** | **II-1055** | **II-1056** |
| R³-97 | **II-1057** | **II-1058** | **II-1059** | **II-1060** | **II-1061** | **II-1062** | **II-1063** | **II-1064** | **II-1065** | **II-1066** | **II-1067** |
| R³-98 | **II-1068** | **II-1069** | **II-1070** | **II-1071** | **II-1072** | **II-1073** | **II-1074** | **II-1075** | **II-1076** | **II-1077** | **II-1078** |
| R³-99 | **II-1079** | **II-1080** | **II-1081** | **II-1082** | **II-1083** | **II-1084** | **II-1085** | **II-1086** | **II-1087** | **II-1088** | **II-1089** |

| R³ | R² | | | | | | | | | | |
|---|---|---|---|---|---|---|---|---|---|---|---|
| | R²-23 | R²-24 | R²-25 | R²-26 | R²-27 | R²-28 | R²-29 | R²-30 | R²-31 | R²-32 | R²-33 |
| R³-1 | **III-1** | **III-2** | **III-3** | **III-4** | **III-5** | **III-6** | **III-7** | **III-8** | **III-9** | **III-10** | **III-11** |
| R³-2 | **III-12** | **III-13** | **III-14** | **III-15** | **III-16** | **III-17** | **III-18** | **III-19** | **III-20** | **III-21** | **III-22** |
| R³-3 | **III-23** | **III-24** | **III-25** | **III-26** | **III-27** | **III-28** | **III-29** | **III-30** | **III-31** | **III-32** | **III-33** |
| R³-4 | **III-34** | **III-35** | **III-36** | **III-37** | **III-38** | **III-39** | **III-40** | **III-41** | **III-42** | **III-43** | **III-44** |
| R³-5 | **III-45** | **III-46** | **III-47** | **III-48** | **III-49** | **III-50** | **III-51** | **III-52** | **III-53** | **III-54** | **III-55** |
| R³-6 | **III-56** | **III-57** | **III-58** | **III-59** | **III-60** | **III-61** | **III-62** | **III-63** | **III-64** | **III-65** | **III-66** |
| R³-7 | **III-67** | **III-68** | **III-69** | **III-70** | **III-71** | **III-72** | **III-73** | **III-74** | **III-75** | **III-76** | **III-77** |
| R³-8 | **III-78** | **III-79** | **III-80** | **III-81** | **III-82** | **III-83** | **III-84** | **III-85** | **III-86** | **III-87** | **III-88** |
| R³-9 | **III-89** | **III-90** | **III-91** | **III-92** | **III-93** | **III-94** | **III-95** | **III-96** | **III-97** | **III-98** | **III-99** |
| R³-10 | **III-100** | **III-101** | **III-102** | **III-103** | **III-104** | **III-105** | **III-106** | **III-107** | **III-108** | **III-109** | **III-110** |
| R³-11 | **III-111** | **III-112** | **III-113** | **III-114** | **III-115** | **III-116** | **III-117** | **III-118** | **III-119** | **III-120** | **III-121** |
| R³-12 | **III-122** | **III-123** | **III-124** | **III-125** | **III-126** | **III-127** | **III-128** | **III-129** | **III-130** | **III-131** | **III-132** |
| R³-13 | **III-133** | **III-134** | **III-135** | **III-136** | **III-137** | **III-138** | **III-139** | **III-140** | **III-141** | **III-142** | **III-143** |
| R³-14 | **III-144** | **III-145** | **III-146** | **III-147** | **III-148** | **III-149** | **III-150** | **III-151** | **III-152** | **III-153** | **III-154** |
| R³-15 | **III-155** | **III-156** | **III-157** | **III-158** | **III-159** | **III-160** | **III-161** | **III-162** | **III-163** | **III-164** | **III-165** |
| R³-16 | **III-166** | **III-167** | **III-168** | **III-169** | **III-170** | **III-171** | **III-172** | **III-173** | **III-174** | **III-175** | **III-176** |
| R³-17 | **III-177** | **III-178** | **III-179** | **III-180** | **III-181** | **III-182** | **III-183** | **III-184** | **III-185** | **III-186** | **III-187** |
| R³-18 | **III-188** | **III-189** | **III-190** | **III-191** | **III-192** | **III-193** | **III-194** | **III-195** | **III-196** | **III-197** | **III-198** |
| R³-19 | **III-199** | **III-200** | **III-201** | **III-202** | **III-203** | **III-204** | **III-205** | **III-206** | **III-207** | **III-208** | **III-209** |
| R³-20 | **III-210** | **III-211** | **III-212** | **III-213** | **III-214** | **III-215** | **III-216** | **III-217** | **III-218** | **III-219** | **III-220** |
| R³-21 | **III-221** | **III-222** | **III-223** | **III-224** | **III-225** | **III-226** | **III-227** | **III-228** | **III-229** | **III-230** | **III-231** |
| R³-22 | **III-232** | **III-233** | **III-234** | **III-235** | **III-236** | **III-237** | **III-238** | **III-239** | **III-240** | **III-241** | **III-242** |
| R³-23 | **III-243** | **III-244** | **III-245** | **III-246** | **III-247** | **III-248** | **III-249** | **III-250** | **III-251** | **III-252** | **III-253** |
| R³-24 | **III-254** | **III-255** | **III-256** | **III-257** | **III-258** | **III-259** | **III-260** | **III-261** | **III-262** | **III-263** | **III-264** |
| R³-25 | **III-265** | **III-266** | **III-267** | **III-268** | **III-269** | **III-270** | **III-271** | **III-272** | **III-273** | **III-274** | **III-275** |
| R³-26 | **III-276** | **III-277** | **III-278** | **III-279** | **III-280** | **III-281** | **III-282** | **III-283** | **III-284** | **III-285** | **III-286** |
| R³-27 | **III-287** | **III-288** | **III-289** | **III-290** | **III-291** | **III-292** | **III-293** | **III-294** | **III-295** | **III-296** | **III-297** |
| R³-28 | **III-298** | **III-299** | **III-300** | **III-301** | **III-302** | **III-303** | **III-304** | **III-305** | **III-306** | **III-307** | **III-308** |
| R³-29 | **III-309** | **III-310** | **III-311** | **III-312** | **III-313** | **III-314** | **III-315** | **III-316** | **III-317** | **III-318** | **III-319** |
| R³-30 | **III-320** | **III-321** | **III-322** | **III-323** | **III-324** | **III-325** | **III-326** | **III-327** | **III-328** | **III-329** | **III-330** |
| R³-31 | **III-331** | **III-332** | **III-333** | **III-334** | **III-335** | **III-336** | **III-337** | **III-338** | **III-339** | **III-340** | **III-341** |
| R³-32 | **III-342** | **III-343** | **III-344** | **III-345** | **III-346** | **III-347** | **III-348** | **III-349** | **III-350** | **III-351** | **III-352** |
| R³-33 | **III-353** | **III-354** | **III-355** | **III-356** | **III-357** | **III-358** | **III-359** | **III-360** | **III-361** | **III-362** | **III-363** |
| R³-34 | **III-364** | **III-365** | **III-366** | **III-367** | **III-368** | **III-369** | **III-370** | **III-371** | **III-372** | **III-373** | **III-374** |
| R³-35 | **III-375** | **III-376** | **III-377** | **III-378** | **III-379** | **III-380** | **III-381** | **III-382** | **III-383** | **III-384** | **III-385** |
| R³-36 | **III-386** | **III-387** | **III-388** | **III-389** | **III-390** | **III-391** | **III-392** | **III-393** | **III-394** | **III-395** | **III-396** |
| R³-37 | **III-397** | **III-398** | **III-399** | **III-400** | **III-401** | **III-402** | **III-403** | **III-404** | **III-405** | **III-406** | **III-407** |
| R³-38 | **III-408** | **III-409** | **III-410** | **III-411** | **III-412** | **III-413** | **III-414** | **III-415** | **III-416** | **III-417** | **III-418** |
| R³-39 | **III-419** | **III-420** | **III-421** | **III-422** | **III-423** | **III-424** | **III-425** | **III-426** | **III-427** | **III-428** | **III-429** |
| R³-40 | **III-430** | **III-431** | **III-432** | **III-433** | **III-434** | **III-435** | **III-436** | **III-437** | **III-438** | **III-439** | **III-440** |
| R³-41 | **III-441** | **III-442** | **III-443** | **III-444** | **III-445** | **III-446** | **III-447** | **III-448** | **III-449** | **III-450** | **III-451** |
| R³-42 | **III-452** | **III-453** | **III-454** | **III-455** | **III-456** | **III-457** | **III-458** | **III-459** | **III-460** | **III-461** | **III-462** |
| R³-43 | **III-463** | **III-464** | **III-465** | **III-466** | **III-467** | **III-468** | **III-469** | **III-470** | **III-471** | **III-472** | **III-473** |
| R³-44 | **III-474** | **III-475** | **III-476** | **III-477** | **III-478** | **III-479** | **III-480** | **III-481** | **III-482** | **III-483** | **III-484** |
| R³-45 | **III-485** | **III-486** | **III-487** | **III-488** | **III-489** | **III-490** | **III-491** | **III-492** | **III-493** | **III-494** | **III-495** |
| R³-46 | **III-496** | **III-497** | **III-498** | **III-499** | **III-500** | **III-501** | **III-502** | **III-503** | **III-504** | **III-505** | **III-506** |
| R³-47 | **III-507** | **III-508** | **III-509** | **III-510** | **III-511** | **III-512** | **III-513** | **III-514** | **III-515** | **III-516** | **III-517** |
| R³-48 | **III-518** | **III-519** | **III-520** | **III-521** | **III-522** | **III-523** | **III-524** | **III-525** | **III-526** | **III-527** | **III-528** |
| R³-49 | **III-529** | **III-530** | **III-531** | **III-532** | **III-533** | **III-534** | **III-535** | **III-536** | **III-537** | **III-538** | **III-539** |
| R³-50 | **III-540** | **III-541** | **III-542** | **III-543** | **III-544** | **III-545** | **III-546** | **III-547** | **III-548** | **III-549** | **III-550** |
| R³-51 | **III-551** | **III-552** | **III-553** | **III-554** | **III-555** | **III-556** | **III-557** | **III-558** | **III-559** | **III-560** | **III-561** |
| R³-52 | **III-562** | **III-563** | **III-564** | **III-565** | **III-566** | **III-567** | **III-568** | **III-569** | **III-570** | **III-571** | **III-572** |
| R³-53 | **III-573** | **III-574** | **III-575** | **III-576** | **III-577** | **III-578** | **III-579** | **III-580** | **III-581** | **III-582** | **III-583** |
| R³-54 | **III-584** | **III-585** | **III-586** | **III-587** | **III-588** | **III-589** | **III-590** | **III-591** | **III-592** | **III-593** | **III-594** |
| R³-55 | **III-595** | **III-596** | **III-597** | **III-598** | **III-599** | **III-600** | **III-601** | **III-602** | **III-603** | **III-604** | **III-605** |
| R³-56 | **III-606** | **III-607** | **III-608** | **III-609** | **III-610** | **III-611** | **III-612** | **III-613** | **III-614** | **III-615** | **III-616** |
| R³-57 | **III-617** | **III-618** | **III-619** | **III-620** | **III-621** | **III-622** | **III-623** | **III-624** | **III-625** | **III-626** | **III-627** |
| R³-58 | **III-628** | **III-629** | **III-630** | **III-631** | **III-632** | **III-633** | **III-634** | **III-635** | **III-636** | **III-637** | **III-638** |
| R³-59 | **III-639** | **III-640** | **III-641** | **III-642** | **III-643** | **III-644** | **III-645** | **III-646** | **III-647** | **III-648** | **III-649** |
| R³-60 | **III-650** | **III-651** | **III-652** | **III-653** | **III-654** | **III-655** | **III-656** | **III-657** | **III-658** | **III-659** | **III-660** |
| R³-61 | **III-661** | **III-662** | **III-663** | **III-664** | **III-665** | **III-666** | **III-667** | **III-668** | **III-669** | **III-670** | **III-671** |
| R³-62 | **III-672** | **III-673** | **III-674** | **III-675** | **III-676** | **III-677** | **III-678** | **III-679** | **III-680** | **III-681** | **III-682** |
| R³-63 | **III-683** | **III-684** | **III-685** | **III-686** | **III-687** | **III-688** | **III-689** | **III-690** | **III-691** | **III-692** | **III-693** |
| R³-64 | **III-694** | **III-695** | **III-696** | **III-697** | **III-698** | **III-699** | **III-700** | **III-701** | **III-702** | **III-703** | **III-704** |
| R³-65 | **III-705** | **III-706** | **III-707** | **III-708** | **III-709** | **III-710** | **III-711** | **III-712** | **III-713** | **III-714** | **III-715** |
| R³-66 | **III-716** | **III-717** | **III-718** | **III-719** | **III-720** | **III-721** | **III-722** | **III-723** | **III-724** | **III-725** | **III-726** |
| R³-67 | **III-727** | **III-728** | **III-729** | **III-730** | **III-731** | **III-732** | **III-733** | **III-734** | **III-735** | **III-736** | **III-737** |
| R³-68 | **III-738** | **III-739** | **III-740** | **III-741** | **III-742** | **III-743** | **III-744** | **III-745** | **III-746** | **III-747** | **III-748** |
| R³-69 | **III-749** | **III-750** | **III-751** | **III-752** | **III-753** | **III-754** | **III-755** | **III-756** | **III-757** | **III-758** | **III-759** |
| R³-70 | **III-760** | **III-761** | **III-762** | **III-763** | **III-764** | **III-765** | **III-766** | **III-767** | **III-768** | **III-769** | **III-770** |
| R³-71 | **III-771** | **III-772** | **III-773** | **III-774** | **III-775** | **III-776** | **III-777** | **III-778** | **III-779** | **III-780** | **III-781** |
| R³-72 | **III-782** | **III-783** | **III-784** | **III-785** | **III-786** | **III-787** | **III-788** | **III-789** | **III-790** | **III-791** | **III-792** |
| R³-73 | **III-793** | **III-794** | **III-795** | **III-796** | **III-797** | **III-798** | **III-799** | **III-800** | **III-801** | **III-802** | **III-803** |
| R³-74 | **III-804** | **III-805** | **III-806** | **III-807** | **III-808** | **III-809** | **III-810** | **III-811** | **III-812** | **III-813** | **III-814** |
| R³-75 | **III-815** | **III-816** | **III-817** | **III-818** | **III-819** | **III-820** | **III-821** | **III-822** | **III-823** | **III-824** | **III-825** |
| R³-76 | **III-826** | **III-827** | **III-828** | **III-829** | **III-830** | **III-831** | **III-832** | **III-833** | **III-834** | **III-835** | **III-836** |
| R³-77 | **III-837** | **III-838** | **III-839** | **III-840** | **III-841** | **III-842** | **III-843** | **III-844** | **III-845** | **III-846** | **III-847** |
| R³-78 | **III-848** | **III-849** | **III-850** | **III-851** | **III-852** | **III-853** | **III-854** | **III-855** | **III-856** | **III-857** | **III-858** |
| R³-79 | **III-859** | **III-860** | **III-861** | **III-862** | **III-863** | **III-864** | **III-865** | **III-866** | **III-867** | **III-868** | **III-869** |
| R³-80 | **III-870** | **III-871** | **III-872** | **III-873** | **III-874** | **III-875** | **III-876** | **III-877** | **III-878** | **III-879** | **III-880** |
| R³-81 | **III-881** | **III-882** | **III-883** | **III-884** | **III-885** | **III-886** | **III-887** | **III-888** | **III-889** | **III-890** | **III-891** |
| R³-82 | **III-892** | **III-893** | **III-894** | **III-895** | **III-896** | **III-897** | **III-898** | **III-899** | **III-900** | **III-901** | **III-902** |
| R³-83 | **III-903** | **III-904** | **III-905** | **III-906** | **III-907** | **III-908** | **III-909** | **III-910** | **III-911** | **III-912** | **III-913** |
| R³-84 | **III-914** | **III-915** | **III-916** | **III-917** | **III-918** | **III-919** | **III-920** | **III-921** | **III-922** | **III-923** | **III-924** |
| R³-85 | **III-925** | **III-926** | **III-927** | **III-928** | **III-929** | **III-930** | **III-931** | **III-932** | **III-933** | **III-934** | **III-935** |
| R³-86 | **III-936** | **III-937** | **III-938** | **III-939** | **III-940** | **III-941** | **III-942** | **III-943** | **III-944** | **III-945** | **III-946** |
| R³-87 | **III-947** | **III-948** | **III-949** | **III-950** | **III-951** | **III-952** | **111-953** | **III-954** | **III-955** | **III-956** | **III-957** |
| R³-88 | **III-958** | **III-959** | **III-960** | **III-961** | **III-962** | **III-963** | **III-964** | **III-965** | **III-966** | **III-967** | **III-968** |
| R³-89 | **III-969** | **III-970** | **III-971** | **III-972** | **III-973** | **III-974** | **III-975** | **III-976** | **III-977** | **III-978** | **III-979** |
| R³-90 | **III-980** | **III-981** | **III-982** | **III-983** | **III-984** | **III-985** | **III-986** | **III-987** | **III-988** | **III-989** | **III-990** |
| R³-91 | **III-991** | **III-992** | **III-993** | **III-994** | **III-995** | **III-996** | **III-997** | **III-998** | **III-999** | **III-1000** | **III-1001** |
| R³-92 | **III-1002** | **III-1003** | **III-1004** | **III-1005** | **III-1006** | **III-1007** | **III-1008** | **III-1009** | **III-1010** | **III-1011** | **III-1012** |
| R³-93 | **III-1013** | **III-1014** | **III-1015** | **III-1016** | **III-1017** | **III-1018** | **III-1019** | **III-1020** | **III-1021** | **III-1022** | **III-1023** |
| R³-94 | **III-1024** | **III-1025** | **III-1026** | **III-1027** | **III-1028** | **III-1029** | **III-1030** | **III-1031** | **III-1032** | **III-1033** | **III-1034** |
| R³-95 | **III-1035** | **III-1036** | **III-1037** | **III-1038** | **III-1039** | **III-1040** | **III-1041** | **III-1042** | **III-1043** | **III-1044** | **III-1045** |
| R³-96 | **III-1046** | **III-1047** | **III-1048** | **III-1049** | **III-1050** | **III-1051** | **III-1052** | **III-1053** | **III-1054** | **III-1055** | **III-1056** |
| R³-97 | **III-1057** | **III-1058** | **III-1059** | **III-1060** | **III-1061** | **III-1062** | **III-1063** | **III-1064** | **III-1065** | **III-1066** | **III-1067** |
| R³-98 | **III-1068** | **III-1069** | **III-1070** | **III-1071** | **III-1072** | **III-1073** | **III-1074** | **III-1075** | **III-1076** | **III-1077** | **III-1078** |
| R³-99 | **III-1079** | **III-1080** | **III-1081** | **III-1082** | **III-1083** | **III-1084** | **III-1085** | **III-1086** | **III-1087** | **III-1088** | **III-1089** |

| R³ | R² | | | | | | | | | | |
|---|---|---|---|---|---|---|---|---|---|---|---|
| | R²-34 | R²-35 | R²-36 | R²-37 | R²-38 | R²-39 | R²-40 | R²-41 | R²-42 | R²-43 | R²-44 |
| R³-1 | **IV-1** | **IV-2** | **IV-3** | **IV-4** | **IV-5** | **IV-6** | **IV-7** | **IV-8** | **IV-9** | **IV-10** | **IV-11** |
| R³-2 | **IV-12** | **IV-13** | **IV-14** | **IV-15** | **IV-16** | **IV-17** | **IV-18** | **IV-19** | **IV-20** | **IV-21** | **IV-22** |
| R³-3 | **IV-23** | **IV-24** | **IV-25** | **IV-26** | **IV-27** | **IV-28** | **IV-29** | **IV-30** | **IV-31** | **IV-32** | **IV-33** |
| R³-4 | **IV-34** | **IV-35** | **IV-36** | **IV-37** | **IV-38** | **IV-39** | **IV-40** | **IV-41** | **IV-42** | **IV-43** | **IV-44** |
| R³-5 | **IV-45** | **IV-46** | **IV-47** | **IV-48** | **IV-49** | **IV-50** | **IV-51** | **IV-52** | **IV-53** | **IV-54** | **IV-55** |
| R³-6 | **IV-56** | **IV-57** | **IV-58** | **IV-59** | **IV-60** | **IV-61** | **IV-62** | **IV-63** | **IV-64** | **IV-65** | **IV-66** |
| R³-7 | **IV-67** | **IV-68** | **IV-69** | **IV-70** | **IV-71** | **IV-72** | **IV-73** | **IV-74** | **IV-75** | **IV-76** | **IV-77** |
| R³-8 | **IV-78** | **IV-79** | **IV-80** | **IV-81** | **IV-82** | **IV-83** | **IV-84** | **IV-85** | **IV-86** | **IV-87** | **IV-88** |
| R³-9 | **IV-89** | **IV-90** | **IV-91** | **IV-92** | **IV-93** | **IV-94** | **IV-95** | **IV-96** | **IV-97** | **IV-98** | **IV-99** |
| R³-10 | **IV-100** | **IV-101** | **IV-102** | **IV-103** | **IV-104** | **IV-105** | **IV-106** | **IV-107** | **IV-108** | **IV-109** | **IV-110** |
| R³-11 | **IV-111** | **IV-112** | **IV-113** | **IV-114** | **IV-115** | **IV-116** | **IV-117** | **IV-118** | **IV-119** | **IV-120** | **IV-121** |
| R³-12 | **IV-122** | **IV-123** | **IV-124** | **IV-125** | **IV-126** | **IV-127** | **IV-128** | **IV-129** | **IV-130** | **IV-131** | **IV-132** |
| R³-13 | **IV-133** | **IV-134** | **IV-135** | **IV-136** | **IV-137** | **IV-138** | **IV-139** | **IV-140** | **IV-141** | **IV-142** | **IV-143** |
| R³-14 | **IV-144** | **IV-145** | **IV-146** | **IV-147** | **IV-148** | **IV-149** | **IV-150** | **IV-151** | **IV-152** | **IV-153** | **IV-154** |
| R³-15 | **IV-155** | **IV-156** | **IV-157** | **IV-158** | **IV-159** | **IV-160** | **IV-161** | **IV-162** | **IV-163** | **IV-164** | **IV-165** |
| R³-16 | **IV-166** | **IV-167** | **IV-168** | **IV-169** | **IV-170** | **IV-171** | **IV-172** | **IV-173** | **IV-174** | **IV-175** | **IV-176** |
| R³-17 | **IV-177** | **IV-178** | **IV-179** | **IV-180** | **IV-181** | **IV-182** | **IV-183** | **IV-184** | **IV-185** | **IV-186** | **IV-187** |
| R³-18 | **IV-188** | **IV-189** | **IV-190** | **IV-191** | **IV-192** | **IV-193** | **IV-194** | **IV-195** | **IV-196** | **IV-197** | **IV-198** |
| R³-19 | **IV-199** | **IV-200** | **IV-201** | **IV-202** | **IV-203** | **IV-204** | **IV-205** | **IV-206** | **IV-207** | **IV-208** | **IV-209** |
| R³-20 | **IV-210** | **IV-211** | **IV-212** | **IV-213** | **IV-214** | **IV-215** | **IV-216** | **IV-217** | **IV-218** | **IV-219** | **IV-220** |
| R³-21 | **IV-221** | **IV-222** | **IV-223** | **IV-224** | **IV-225** | **IV-226** | **IV-227** | **IV-228** | **IV-229** | **IV-230** | **IV-231** |
| R³-22 | **IV-232** | **IV-233** | **IV-234** | **IV-235** | **IV-236** | **IV-237** | **IV-238** | **IV-239** | **IV-240** | **IV-241** | **IV-242** |
| R³-23 | **IV-243** | **IV-244** | **IV-245** | **IV-246** | **IV-247** | **IV-248** | **IV-249** | **IV-250** | **IV-251** | **IV-252** | **IV-253** |
| R³-24 | **IV-254** | **IV-255** | **IV-256** | **IV-257** | **IV-258** | **IV-259** | **IV-260** | **IV-261** | **IV-262** | **IV-263** | **IV-264** |
| R³-25 | **IV-265** | **IV-266** | **IV-267** | **IV-268** | **IV-269** | **IV-270** | **IV-271** | **IV-272** | **IV-273** | **IV-274** | **IV-275** |
| R³-26 | **IV-276** | **IV-277** | **IV-278** | **IV-279** | **IV-280** | **IV-281** | **IV-282** | **IV-283** | **IV-284** | **IV-285** | **IV-286** |
| R³-27 | **IV-287** | **IV-288** | **IV-289** | **IV-290** | **IV-291** | **IV-292** | **IV-293** | **IV-294** | **IV-295** | **IV-296** | **IV-297** |
| R³-28 | **IV-298** | **IV-299** | **IV-300** | **IV-301** | **IV-302** | **IV-303** | **IV-304** | **IV-305** | **IV-306** | **IV-307** | **IV-308** |
| R³-29 | **IV-309** | **IV-310** | **IV-311** | **IV-312** | **IV-313** | **IV-314** | **IV-315** | **IV-316** | **IV-317** | **IV-318** | **IV-319** |
| R³-30 | **IV-320** | **IV-321** | **IV-322** | **IV-323** | **IV-324** | **IV-325** | **IV-326** | **IV-327** | **IV-328** | **IV-329** | **IV-330** |
| R³-31 | **IV-331** | **IV-332** | **IV-333** | **IV-334** | **IV-335** | **IV-336** | **IV-337** | **IV-338** | **IV-339** | **IV-340** | **IV-341** |
| R³-32 | **IV-342** | **IV-343** | **IV-344** | **IV-345** | **IV-346** | **IV-347** | **IV-348** | **IV-349** | **IV-350** | **IV-351** | **IV-352** |
| R³-33 | **IV-353** | **IV-354** | **IV-355** | **IV-356** | **IV-357** | **IV-358** | **IV-359** | **IV-360** | **IV-361** | **IV-362** | **IV-363** |
| R³-34 | **IV-364** | **IV-365** | **IV-366** | **IV-367** | **IV-368** | **IV-369** | **IV-370** | **IV-371** | **IV-372** | **IV-373** | **IV-374** |
| R³-35 | **IV-375** | **IV-376** | **IV-377** | **IV-378** | **IV-379** | **IV-380** | **IV-381** | **IV-382** | **IV-383** | **IV-384** | **IV-385** |
| R³-36 | **IV-386** | **IV-387** | **IV-388** | **IV-389** | **IV-390** | **IV-391** | **IV-392** | **IV-393** | **IV-394** | **IV-395** | **IV-396** |
| R³-37 | **IV-397** | **IV-398** | **IV-399** | **IV-400** | **IV-401** | **IV-402** | **IV-403** | **IV-404** | **IV-405** | **IV-406** | **IV-407** |
| R³-38 | **IV-408** | **IV-409** | **IV-410** | **IV-411** | **IV-412** | **IV-413** | **IV-414** | **IV-415** | **IV-416** | **IV-417** | **IV-418** |
| R³-39 | **IV-419** | **IV-420** | **IV-421** | **IV-422** | **IV-423** | **IV-424** | **IV-425** | **IV-426** | **IV-427** | **IV-428** | **IV-429** |
| R³-40 | **IV-430** | **IV-431** | **IV-432** | **IV-433** | **IV-434** | **IV-435** | **IV-436** | **IV-437** | **IV-438** | **IV-439** | **IV-440** |
| R³-41 | **IV-441** | **IV-442** | **IV-443** | **IV-444** | **IV-445** | **IV-446** | **IV-447** | **IV-448** | **IV-449** | **IV-450** | **IV-451** |
| R³-42 | **IV-452** | **IV-453** | **IV-454** | **IV-455** | **IV-456** | **IV-457** | **IV-458** | **IV-459** | **IV-460** | **IV-461** | **IV-462** |
| R³-43 | **IV-463** | **IV-464** | **IV-465** | **IV-466** | **IV-467** | **IV-468** | **IV-469** | **IV-470** | **IV-471** | **IV-472** | **IV-473** |
| R³-44 | **IV-474** | **IV-475** | **IV-476** | **IV-477** | **IV-478** | **IV-479** | **IV-480** | **IV-481** | **IV-482** | **IV-483** | **IV-484** |
| R³-45 | **IV-485** | **IV-486** | **IV-487** | **IV-488** | **IV-489** | **IV-490** | **IV-491** | **IV-492** | **IV-493** | **IV-494** | **IV-495** |
| R³-46 | **IV-496** | **IV-497** | **IV-498** | **IV-499** | **IV-500** | **IV-501** | **IV-502** | **IV-503** | **IV-504** | **IV-505** | **IV-506** |
| R³-47 | **IV-507** | **IV-508** | **IV-509** | **IV-510** | **IV-511** | **IV-512** | **IV-513** | **IV-514** | **IV-515** | **IV-516** | **IV-517** |
| R³-48 | **IV-518** | **IV-519** | **IV-520** | **IV-521** | **IV-522** | **IV-523** | **IV-524** | **IV-525** | **IV-526** | **IV-527** | **IV-528** |
| R³-49 | **IV-529** | **IV-530** | **IV-531** | **IV-532** | **IV-533** | **IV-534** | **IV-535** | **IV-536** | **IV-537** | **IV-538** | **IV-539** |
| R³-50 | **IV-540** | **IV-541** | **IV-542** | **IV-543** | **IV-544** | **IV-545** | **IV-546** | **IV-547** | **IV-548** | **IV-549** | **IV-550** |
| R³-51 | **IV-551** | **IV-552** | **IV-553** | **IV-554** | **IV-555** | **IV-556** | **IV-557** | **IV-558** | **IV-559** | **IV-560** | **IV-561** |
| R³-52 | **IV-562** | **IV-563** | **IV-564** | **IV-565** | **IV-566** | **IV-567** | **IV-568** | **IV-569** | **IV-570** | **IV-571** | **IV-572** |
| R³-53 | **IV-573** | **IV-574** | **IV-575** | **IV-576** | **IV-577** | **IV-578** | **IV-579** | **IV-580** | **IV-581** | **IV-582** | **IV-583** |
| R³-54 | **IV-584** | **IV-585** | **IV-586** | **IV-587** | **IV-588** | **IV-589** | **IV-590** | **IV-591** | **IV-592** | **IV-593** | **IV-594** |
| R³-55 | **IV-595** | **IV-596** | **IV-597** | **IV-598** | **IV-599** | **IV-600** | **IV-601** | **IV-602** | **IV-603** | **IV-604** | **IV-605** |
| R³-56 | **IV-606** | **IV-607** | **IV-608** | **IV-609** | **IV-610** | **IV-611** | **IV-612** | **IV-613** | **IV-614** | **IV-615** | **IV-616** |
| R³-57 | **IV-617** | **IV-618** | **IV-619** | **IV-620** | **IV-621** | **IV-622** | **IV-623** | **IV-624** | **IV-625** | **IV-626** | **IV-627** |
| R³-58 | **IV-628** | **IV-629** | **IV-630** | **IV-631** | **IV-632** | **IV-633** | **IV-634** | **IV-635** | **IV-636** | **IV-637** | **IV-638** |
| R³-59 | **IV-639** | **IV-640** | **IV-641** | **IV-642** | **IV-643** | **IV-644** | **IV-645** | **IV-646** | **IV-647** | **IV-648** | **IV-649** |
| R³-60 | **IV-650** | **IV-651** | **IV-652** | **IV-653** | **IV-654** | **IV-655** | **IV-656** | **IV-657** | **IV-658** | **IV-659** | **IV-660** |
| R³-61 | **IV-661** | **IV-662** | **IV-663** | **IV-664** | **IV-665** | **IV-666** | **IV-667** | **IV-668** | **IV-669** | **IV-670** | **IV-671** |
| R³-62 | **IV-672** | **IV-673** | **IV-674** | **IV-675** | **IV-676** | **IV-677** | **IV-678** | **IV-679** | **IV-680** | **IV-681** | **IV-682** |
| R³-63 | **IV-683** | **IV-684** | **IV-685** | **IV-686** | **IV-687** | **IV-688** | **IV-689** | **IV-690** | **IV-691** | **IV-692** | **IV-693** |
| R³-64 | **IV-694** | **IV-695** | **IV-696** | **IV-697** | **IV-698** | **IV-699** | **IV-700** | **IV-701** | **IV-702** | **IV-703** | **IV-704** |
| R³-65 | **IV-705** | **IV-706** | **IV-707** | **IV-708** | **IV-709** | **IV-710** | **IV-711** | **IV-712** | **IV-713** | **IV-714** | **IV-715** |
| R³-66 | **IV-716** | **IV-717** | **IV-718** | **IV-719** | **IV-720** | **IV-721** | **IV-722** | **IV-723** | **IV-724** | **IV-725** | **IV-726** |
| R³-67 | **IV-727** | **IV-728** | **IV-729** | **IV-730** | **IV-731** | **IV-732** | **IV-733** | **IV-734** | **IV-735** | **IV-736** | **IV-737** |
| R³-68 | **IV-738** | **IV-739** | **IV-740** | **IV-741** | **IV-742** | **IV-743** | **IV-744** | **IV-745** | **IV-746** | **IV-747** | **IV-748** |
| R³-69 | **IV-749** | **IV-750** | **IV-751** | **IV-752** | **IV-753** | **IV-754** | **IV-755** | **IV-756** | **IV-757** | **IV-758** | **IV-759** |
| R³-70 | **IV-760** | **IV-761** | **IV-762** | **IV-763** | **IV-764** | **IV-765** | **IV-766** | **IV-767** | **IV-768** | **IV-769** | **IV-770** |
| R³-71 | **IV-771** | **IV-772** | **IV-773** | **IV-774** | **IV-775** | **IV-776** | **IV-777** | **IV-778** | **IV-779** | **IV-780** | **IV-781** |
| R³-72 | **IV-782** | **IV-783** | **IV-784** | **IV-785** | **IV-786** | **IV-787** | **IV-788** | **IV-789** | **IV-790** | **IV-791** | **IV-792** |
| R³-73 | **IV-793** | **IV-794** | **IV-795** | **IV-796** | **IV-797** | **IV-798** | **IV-799** | **IV-800** | **IV-801** | **IV-802** | **IV-803** |
| R³-74 | **IV-804** | **IV-805** | **IV-806** | **IV-807** | **IV-808** | **IV-809** | **IV-810** | **IV-811** | **IV-812** | **IV-813** | **IV-814** |
| R³-75 | **IV-815** | **IV-816** | **IV-817** | **IV-818** | **IV-819** | **IV-820** | **IV-821** | **IV-822** | **IV-823** | **IV-824** | **IV-825** |
| R³-76 | **IV-826** | **IV-827** | **IV-828** | **IV-829** | **IV-830** | **IV-831** | **IV-832** | **IV-833** | **IV-834** | **IV-835** | **IV-836** |
| R³-77 | **IV-837** | **IV-838** | **IV-839** | **IV-840** | **IV-841** | **IV-842** | **IV-843** | **IV-844** | **IV-845** | **IV-846** | **IV-847** |
| R³-78 | **IV-848** | **IV-849** | **IV-850** | **IV-851** | **IV-852** | **IV-853** | **IV-854** | **IV-855** | **IV-856** | **IV-857** | **IV-858** |
| R³-79 | **IV-859** | **IV-860** | **IV-861** | **IV-862** | **IV-863** | **IV-864** | **IV-865** | **IV-866** | **IV-867** | **IV-868** | **IV-869** |
| R³-80 | **IV-870** | **IV-871** | **IV-872** | **IV-873** | **IV-874** | **IV-875** | **IV-876** | **IV-877** | **IV-878** | **IV-879** | **IV-880** |
| R³-81 | **IV-881** | **IV-882** | **IV-883** | **IV-884** | **IV-885** | **IV-886** | **IV-887** | **IV-888** | **IV-889** | **IV-890** | **IV-891** |
| R³-82 | **IV-892** | **IV-893** | **IV-894** | **IV-895** | **IV-896** | **IV-897** | **IV-898** | **IV-899** | **IV-900** | **IV-901** | **IV-902** |
| R³-83 | **IV-903** | **IV-904** | **IV-905** | **IV-906** | **IV-907** | **IV-908** | **IV-909** | **IV-910** | **IV-911** | **IV-912** | **IV-913** |
| R³-84 | **IV-914** | **IV-915** | **IV-916** | **IV-917** | **IV-918** | **IV-919** | **IV-920** | **IV-921** | **IV-922** | **IV-923** | **IV-924** |
| R³-85 | **IV-925** | **IV-926** | **IV-927** | **IV-928** | **IV-929** | **IV-930** | **IV-931** | **IV-932** | **IV-933** | **IV-934** | **IV-935** |
| R³-86 | **IV-936** | **IV-937** | **IV-938** | **IV-939** | **IV-940** | **IV-941** | **IV-942** | **IV-943** | **IV-944** | **IV-945** | **IV-946** |
| R³-87 | **IV-947** | **IV-948** | **IV-949** | **IV-950** | **IV-951** | **IV-952** | **IV-953** | **IV-954** | **IV-955** | **IV-956** | **IV-957** |
| R³-88 | **IV-958** | **IV-959** | **IV-960** | **IV-961** | **IV-962** | **IV-963** | **IV-964** | **IV-965** | **IV-966** | **IV-967** | **IV-968** |
| R³-89 | **IV-969** | **IV-970** | **IV-971** | **IV-972** | **IV-973** | **IV-974** | **IV-975** | **IV-976** | **IV-977** | **IV-978** | **IV-979** |
| R³-90 | **IV-980** | **IV-981** | **IV-982** | **IV-983** | **IV-984** | **IV-985** | **IV-986** | **IV-987** | **IV-988** | **IV-989** | **IV-990** |
| R³-91 | **IV-991** | **IV-992** | **IV-993** | **IV-994** | **IV-995** | **IV-996** | **IV-997** | **IV-998** | **IV-999** | **IV-1000** | **IV-1001** |
| R³-92 | **IV-1002** | **IV-1003** | **IV-1004** | **IV-1005** | **IV-1006** | **IV-1007** | **IV-1008** | **IV-1009** | **IV-1010** | **IV-1011** | **IV-1012** |
| R³-93 | **IV-1013** | **IV-1014** | **IV-1015** | **IV-1016** | **IV-1017** | **IV-1018** | **IV-1019** | **IV-1020** | **IV-1021** | **IV-1022** | **IV-1023** |
| R³-94 | **IV-1024** | **IV-1025** | **IV-1026** | **IV-1027** | **IV-1028** | **IV-1029** | **IV-1030** | **IV-1031** | **IV-1032** | **IV-1033** | **IV-1034** |
| R³-95 | **IV-1035** | **IV-1036** | **IV-1037** | **IV-1038** | **IV-1039** | **IV-1040** | **IV-1041** | **IV-1042** | **IV-1043** | **IV-1044** | **IV-1045** |
| R³-96 | **IV-1046** | **IV-1047** | **IV-1048** | **IV-1049** | **IV-1050** | **IV-1051** | **IV-1052** | **IV-1053** | **IV-1054** | **IV-1055** | **IV-1056** |
| R³-97 | **IV-1057** | **IV-1058** | **IV-1059** | **IV-1060** | **IV-1061** | **IV-1062** | **IV-1063** | **IV-1064** | **IV-1065** | **IV-1066** | **IV-1067** |
| R³-98 | **IV-1068** | **IV-1069** | **IV-1070** | **IV-1071** | **IV-1072** | **IV-1073** | **IV-1074** | **IV-1075** | **IV-1076** | **IV-1077** | **IV-1078** |
| R³-99 | **IV-1079** | **IV-1080** | **IV-1081** | **IV-1082** | **IV-1083** | **IV-1084** | **IV-1085** | **IV-1086** | **IV-1087** | **IV-1088** | **IV-1089** |

| R³ | R² | | | | | | | | | | |
|---|---|---|---|---|---|---|---|---|---|---|---|
| | R²-45 | R²-46 | R²-47 | R²-48 | R²-49 | R²-50 | R²-51 | R²-52 | R²-53 | R²-54 | R²-55 |
| R³-1 | **V-1** | **V-2** | **V-3** | **V-4** | **V-5** | **V-6** | **V-7** | **V-8** | **V-9** | **V-10** | **V-11** |
| R³-2 | **V-12** | **V-13** | **V-14** | **V-15** | **V-16** | **V-17** | **V-18** | **V-19** | **V-20** | **V-21** | **V-22** |
| R³-3 | **V-23** | **V-24** | **V-25** | **V-26** | **V-27** | **V-28** | **V-29** | **V-30** | **V-31** | **V-32** | **V-33** |
| R³-4 | **V-34** | **V-35** | **V-36** | **V-37** | **V-38** | **V-39** | **V-40** | **V-41** | **V-42** | **V-43** | **V-44** |
| R³-5 | **V-45** | **V-46** | **V-47** | **V-48** | **V-49** | **V-50** | **V-51** | **V-52** | **V-53** | **V-54** | **V-55** |
| R³-6 | **V-56** | **V-57** | **V-58** | **V-59** | **V-60** | **V-61** | **V-62** | **V-63** | **V-64** | **V-65** | **V-66** |
| R³-7 | **V-67** | **V-68** | **V-69** | **V-70** | **V-71** | **V-72** | **V-73** | **V-74** | **V-75** | **V-76** | **V-77** |
| R³-8 | **V-78** | **V-79** | **V-80** | **V-81** | **V-82** | **V-83** | **V-84** | **V-85** | **V-86** | **V-87** | **V-88** |
| R³-9 | **V-89** | **V-90** | **V-91** | **V-92** | **V-93** | **V-94** | **V-95** | **V-96** | **V-97** | **V-98** | **V-99** |
| R³-10 | **V-100** | **V-101** | **V-102** | **V-103** | **V-104** | **V-105** | **V-106** | **V-107** | **V-108** | **V-109** | **V-110** |
| R³-11 | **V-111** | **V-112** | **V-113** | **V-114** | **V-115** | **V-116** | **V-117** | **V-118** | **V-119** | **V-120** | **V-121** |
| R³-12 | **V-122** | **V-123** | **V-124** | **V-125** | **V-126** | **V-127** | **V-128** | **V-129** | **V-130** | **V-131** | **V-132** |
| R³-13 | **V-133** | **V-134** | **V-135** | **V-136** | **V-137** | **V-138** | **V-139** | **V-140** | **V-141** | **V-142** | **V-143** |
| R³-14 | **V-144** | **V-145** | **V-146** | **V-147** | **V-148** | **V-149** | **V-150** | **V-151** | **V-152** | **V-153** | **V-154** |
| R³-15 | **V-155** | **V-156** | **V-157** | **V-158** | **V-159** | **V-160** | **V-161** | **V-162** | **V-163** | **V-164** | **V-165** |
| R³-16 | **V-166** | **V-167** | **V-168** | **V-169** | **V-170** | **V-171** | **V-172** | **V-173** | **V-174** | **V-175** | **V-176** |
| R³-17 | **V-177** | **V-178** | **V-179** | **V-180** | **V-181** | **V-182** | **V-183** | **V-184** | **V-185** | **V-186** | **V-187** |
| R³-18 | **V-188** | **V-189** | **V-190** | **V-191** | **V-192** | **V-193** | **V-194** | **V-195** | **V-196** | **V-197** | **V-198** |
| R³-19 | **V-199** | **V-200** | **V-201** | **V-202** | **V-203** | **V-204** | **V-205** | **V-206** | **V-207** | **V-208** | **V-209** |
| R³-20 | **V-210** | **V-211** | **V-212** | **V-213** | **V-214** | **V-215** | **V-216** | **V-217** | **V-218** | **V-219** | **V-220** |
| R³-21 | **V-221** | **V-222** | **V-223** | **V-224** | **V-225** | **V-226** | **V-227** | **V-228** | **V-229** | **V-230** | **V-231** |
| R³-22 | **V-232** | **V-233** | **V-234** | **V-235** | **V-236** | **V-237** | **V-238** | **V-239** | **V-240** | **V-241** | **V-242** |
| R³-23 | **V-243** | **V-244** | **V-245** | **V-246** | **V-247** | **V-248** | **V-249** | **V-250** | **V-251** | **V-252** | **V-253** |
| R³-24 | **V-254** | **V-255** | **V-256** | **V-257** | **V-258** | **V-259** | **V-260** | **V-261** | **V-262** | **V-263** | **V-264** |
| R³-25 | **V-265** | **V-266** | **V-267** | **V-268** | **V-269** | **V-270** | **V-271** | **V-272** | **V-273** | **V-274** | **V-275** |
| R³-26 | **V-276** | **V-277** | **V-278** | **V-279** | **V-280** | **V-281** | **V-282** | **V-283** | **V-284** | **V-285** | **V-286** |
| R³-27 | **V-287** | **V-288** | **V-289** | **V-290** | **V-291** | **V-292** | **V-293** | **V-294** | **V-295** | **V-296** | **V-297** |
| R³-28 | **V-298** | **V-299** | **V-300** | **V-301** | **V-302** | **V-303** | **V-304** | **V-305** | **V-306** | **V-307** | **V-308** |
| R³-29 | **V-309** | **V-310** | **V-311** | **V-312** | **V-313** | **V-314** | **V-315** | **V-316** | **V-317** | **V-318** | **V-319** |
| R³-30 | **V-320** | **V-321** | **V-322** | **V-323** | **V-324** | **V-325** | **V-326** | **V-327** | **V-328** | **V-329** | **V-330** |
| R³-31 | **V-331** | **V-332** | **V-333** | **V-334** | **V-335** | **V-336** | **V-337** | **V-338** | **V-339** | **V-340** | **V-341** |
| R³-32 | **V-342** | **V-343** | **V-344** | **V-345** | **V-346** | **V-347** | **V-348** | **V-349** | **V-350** | **V-351** | **V-352** |
| R³-33 | **V-353** | **V-354** | **V-355** | **V-356** | **V-357** | **V-358** | **V-359** | **V-360** | **V-361** | **V-362** | **V-363** |
| R³-34 | **V-364** | **V-365** | **V-366** | **V-367** | **V-368** | **V-369** | **V-370** | **V-371** | **V-372** | **V-373** | **V-374** |
| R³-35 | **V-375** | **V-376** | **V-377** | **V-378** | **V-379** | **V-380** | **V-381** | **V-382** | **V-383** | **V-384** | **V-385** |
| R³-36 | **V-386** | **V-387** | **V-388** | **V-389** | **V-390** | **V-391** | **V-392** | **V-393** | **V-394** | **V-395** | **V-396** |
| R³-37 | **V-397** | **V-398** | **V-399** | **V-400** | **V-401** | **V-402** | **V-403** | **V-404** | **V-405** | **V-406** | **V-407** |
| R³-38 | **V-408** | **V-409** | **V-410** | **V-411** | **V-412** | **V-413** | **V-414** | **V-415** | **V-416** | **V-417** | **V-418** |
| R³-39 | **V-419** | **V-420** | **V-421** | **V-422** | **V-423** | **V-424** | **V-425** | **V-426** | **V-427** | **V-428** | **V-429** |
| R³-40 | **V-430** | **V-431** | **V-432** | **V-433** | **V-434** | **V-435** | **V-436** | **V-437** | **V-438** | **V-439** | **V-440** |
| R³-41 | **V-441** | **V-442** | **V-443** | **V-444** | **V-445** | **V-446** | **V-447** | **V-448** | **V-449** | **V-450** | **V-451** |
| R³-42 | **V-452** | **V-453** | **V-454** | **V-455** | **V-456** | **V-457** | **V-458** | **V-459** | **V-460** | **V-461** | **V-462** |
| R³-43 | **V-463** | **V-464** | **V-465** | **V-466** | **V-467** | **V-468** | **V-469** | **V-470** | **V-471** | **V-472** | **V-473** |
| R³-44 | **V-474** | **V-475** | **V-476** | **V-477** | **V-478** | **V-479** | **V-480** | **V-481** | **V-482** | **V-483** | **V-484** |
| R³-45 | **V-485** | **V-486** | **V-487** | **V-488** | **V-489** | **V-490** | **V-491** | **V-492** | **V-493** | **V-494** | **V-495** |
| R³-46 | **V-496** | **V-497** | **V-498** | **V-499** | **V-500** | **V-501** | **V-502** | **V-503** | **V-504** | **V-505** | **V-506** |
| R³-47 | **V-507** | **V-508** | **V-509** | **V-510** | **V-511** | **V-512** | **V-513** | **V-514** | **V-515** | **V-516** | **V-517** |
| R³-48 | **V-518** | **V-519** | **V-520** | **V-521** | **V-522** | **V-523** | **V-524** | **V-525** | **V-526** | **V-527** | **V-528** |
| R³-49 | **V-529** | **V-530** | **V-531** | **V-532** | **V-533** | **V-534** | **V-535** | **V-536** | **V-537** | **V-538** | **V-539** |
| R³-50 | **V-540** | **V-541** | **V-542** | **V-543** | **V-544** | **V-545** | **V-546** | **V-547** | **V-548** | **V-549** | **V-550** |
| R³-51 | **V-551** | **V-552** | **V-553** | **V-554** | **V-555** | **V-556** | **V-557** | **V-558** | **V-559** | **V-560** | **V-561** |
| R³-52 | **V-562** | **V-563** | **V-564** | **V-565** | **V-566** | **V-567** | **V-568** | **V-569** | **V-570** | **V-571** | **V-572** |
| R³-53 | **V-573** | **V-574** | **V-575** | **V-576** | **V-577** | **V-578** | **V-579** | **V-580** | **V-581** | **V-582** | **V-583** |
| R³-54 | **V-584** | **V-585** | **V-586** | **V-587** | **V-588** | **V-589** | **V-590** | **V-591** | **V-592** | **V-593** | **V-594** |
| R³-55 | **V-595** | **V-596** | **V-597** | **V-598** | **V-599** | **V-600** | **V-601** | **V-602** | **V-603** | **V-604** | **V-605** |
| R³-56 | **V-606** | **V-607** | **V-608** | **V-609** | **V-610** | **V-611** | **V-612** | **V-613** | **V-614** | **V-615** | **V-616** |
| R³-57 | **V-617** | **V-618** | **V-619** | **V-620** | **V-621** | **V-622** | **V-623** | **V-624** | **V-625** | **V-626** | **V-627** |
| R³-58 | **V-628** | **V-629** | **V-630** | **V-631** | **V-632** | **V-633** | **V-634** | **V-635** | **V-636** | **V-637** | **V-638** |
| R³-59 | **V-639** | **V-640** | **V-641** | **V-642** | **V-643** | **V-644** | **V-645** | **V-646** | **V-647** | **V-648** | **V-649** |
| R³-60 | **V-650** | **V-651** | **V-652** | **V-653** | **V-654** | **V-655** | **V-656** | **V-657** | **V-658** | **V-659** | **V-660** |
| R³-61 | **V-661** | **V-662** | **V-663** | **V-664** | **V-665** | **V-666** | **V-667** | **V-668** | **V-669** | **V-670** | **V-671** |
| R³-62 | **V-672** | **V-673** | **V-674** | **V-675** | **V-676** | **V-677** | **V-678** | **V-679** | **V-680** | **V-681** | **V-682** |
| R³-63 | **V-683** | **V-684** | **V-685** | **V-686** | **V-687** | **V-688** | **V-689** | **V-690** | **V-691** | **V-692** | **V-693** |
| R³-64 | **V-694** | **V-695** | **V-696** | **V-697** | **V-698** | **V-699** | **V-700** | **V-701** | **V-702** | **V-703** | **V-704** |
| R³-65 | **V-705** | **V-706** | **V-707** | **V-708** | **V-709** | **V-710** | **V-711** | **V-712** | **V-713** | **V-714** | **V-715** |
| R³-66 | **V-716** | **V-717** | **V-718** | **V-719** | **V-720** | **V-721** | **V-722** | **V-723** | **V-724** | **V-725** | **V-726** |
| R³-67 | **V-727** | **V-728** | **V-729** | **V-730** | **V-731** | **V-732** | **V-733** | **V-734** | **V-735** | **V-736** | **V-737** |
| R³-68 | **V-738** | **V-739** | **V-740** | **V-741** | **V-742** | **V-743** | **V-744** | **V-745** | **V-746** | **V-747** | **V-748** |
| R³-69 | **V-749** | **V-750** | **V-751** | **V-752** | **V-753** | **V-754** | **V-755** | **V-756** | **V-757** | **V-758** | **V-759** |
| R³-70 | **V-760** | **V-761** | **V-762** | **V-763** | **V-764** | **V-765** | **V-766** | **V-767** | **V-768** | **V-769** | **V-770** |
| R³-71 | **V-771** | **V-772** | **V-773** | **V-774** | **V-775** | **V-776** | **V-777** | **V-778** | **V-779** | **V-780** | **V-781** |
| R³-72 | **V-782** | **V-783** | **V-784** | **V-785** | **V-786** | **V-787** | **V-788** | **V-789** | **V-790** | **V-791** | **V-792** |
| R³-73 | **V-793** | **V-794** | **V-795** | **V-796** | **V-797** | **V-798** | **V-799** | **V-800** | **V-801** | **V-802** | **V-803** |
| R³-74 | **V-804** | **V-805** | **V-806** | **V-807** | **V-808** | **V-809** | **V-810** | **V-811** | **V-812** | **V-813** | **V-814** |
| R³-75 | **V-815** | **V-816** | **V-817** | **V-818** | **V-819** | **V-820** | **V-821** | **V-822** | **V-823** | **V-824** | **V-825** |
| R³-76 | **V-826** | **V-827** | **V-828** | **V-829** | **V-830** | **V-831** | **V-832** | **V-833** | **V-834** | **V-835** | **V-836** |
| R³-77 | **V-837** | **V-838** | **V-839** | **V-840** | **V-841** | **V-842** | **V-843** | **V-844** | **V-845** | **V-846** | **V-847** |
| R³-78 | **V-848** | **V-849** | **V-850** | **V-851** | **V-852** | **V-853** | **V-854** | **V-855** | **V-856** | **V-857** | **V-858** |
| R³-79 | **V-859** | **V-860** | **V-861** | **V-862** | **V-863** | **V-864** | **V-865** | **V-866** | **V-867** | **V-868** | **V-869** |
| R³-80 | **V-870** | **V-871** | **V-872** | **V-873** | **V-874** | **V-875** | **V-876** | **V-877** | **V-878** | **V-879** | **V-880** |
| R³-81 | **V-881** | **V-882** | **V-883** | **V-884** | **V-885** | **V-886** | **V-887** | **V-888** | **V-889** | **V-890** | **V-891** |
| R³-82 | **V-892** | **V-893** | **V-894** | **V-895** | **V-896** | **V-897** | **V-898** | **V-899** | **V-900** | **V-901** | **V-902** |
| R³-83 | **V-903** | **V-904** | **V-905** | **V-906** | **V-907** | **V-908** | **V-909** | **V-910** | **V-911** | **V-912** | **V-913** |
| R³-84 | **V-914** | **V-915** | **V-916** | **V-917** | **V-918** | **V-919** | **V-920** | **V-921** | **V-922** | **V-923** | **V-924** |
| R³-85 | **V-925** | **V-926** | **V-927** | **V-928** | **V-929** | **V-930** | **V-931** | **V-932** | **V-933** | **V-934** | **V-935** |
| R³-86 | **V-936** | **V-937** | **V-938** | **V-939** | **V-940** | **V-941** | **V-942** | **V-943** | **V-944** | **V-945** | **V-946** |
| R³-87 | **V-947** | **V-948** | **V-949** | **V-950** | **V-951** | **V-952** | **V-953** | **V-954** | **V-955** | **V-956** | **V-957** |
| R³-88 | **V-958** | **V-959** | **V-960** | **V-961** | **V-962** | **V-963** | **V-964** | **V-965** | **V-966** | **V-967** | **V-968** |
| R³-89 | **V-969** | **V-970** | **V-971** | **V-972** | **V-973** | **V-974** | **V-975** | **V-976** | **V-977** | **V-978** | **V-979** |
| R³-90 | **V-980** | **V-981** | **V-982** | **V-983** | **V-984** | **V-985** | **V-986** | **V-987** | **V-988** | **V-989** | **V-990** |
| R³-91 | **V-991** | **V-992** | **V-993** | **V-994** | **V-995** | **V-996** | **V-997** | **V-998** | **V-999** | **V-1000** | **V-1001** |
| R³-92 | **V-1002** | **V-1003** | **V-1004** | **V-1005** | **V-1006** | **V-1007** | **V-1008** | **V-1009** | **V-1010** | **V-1011** | **V-1012** |
| R³-93 | **V-1013** | **V-1014** | **V-1015** | **V-1016** | **V-1017** | **V-1018** | **V-1019** | **V-1020** | **V-1021** | **V-1022** | **V-1023** |
| R³-94 | **V-1024** | **V-1025** | **V-1026** | **V-1027** | **V-1028** | **V-1029** | **V-1030** | **V-1031** | **V-1032** | **V-1033** | **V-1034** |
| R³-95 | **V-1035** | **V-1036** | **V-1037** | **V-1038** | **V-1039** | **V-1040** | **V-1041** | **V-1042** | **V-1043** | **V-1044** | **V-1045** |
| R³-96 | **V-1046** | **V-1047** | **V-1048** | **V-1049** | **V-1050** | **V-1051** | **V-1052** | **V-1053** | **V-1054** | **V-1055** | **V-1056** |
| R³-97 | **V-1057** | **V-1058** | **V-1059** | **V-1060** | **V-1061** | **V-1062** | **V-1063** | **V-1064** | **V-1065** | **V-1066** | **V-1067** |
| R³-98 | **V-1068** | **V-1069** | **V-1070** | **V-1071** | **V-1072** | **V-1073** | **V-1074** | **V-1075** | **V-1076** | **V-1077** | **V-1078** |
| R³-99 | **V-1079** | **V-1080** | **V-1081** | **V-1082** | **V-1083** | **V-1084** | **V-1085** | **V-1086** | **V-1087** | **V-1088** | **V-1089** |

| | R² | | | | | | | |
|---|---|---|---|---|---|---|---|---|
| R³ | R²-56 | R²-57 | R²-58 | R²-59 | R²-60 | R²-61 | R²-62 | R²-63 |
| R³-1 | **VI-1** | **VI-2** | **VI-3** | **VI-4** | **VI-5** | **VI-6** | **VI-7** | **VI-8** |
| R³-2 | **VI-12** | **VI-13** | **VI-14** | **VI-15** | **VI-16** | **VI-17** | **VI-18** | **VI-19** |
| R³-3 | **VI-23** | **VI-24** | **VI-25** | **VI-26** | **VI-27** | **VI-28** | **VI-29** | **VI-30** |
| R³-4 | **VI-34** | **VI-35** | **VI-36** | **VI-37** | **VI-38** | **VI-39** | **VI-40** | **VI-41** |
| R³-5 | **VI-45** | **VI-46** | **VI-47** | **VI-48** | **VI-49** | **VI-50** | **VI-51** | **VI-52** |
| R³-6 | **VI-56** | **VI-57** | **VI-58** | **VI-59** | **VI-60** | **VI-61** | **VI-62** | **VI-63** |
| R^{3_}7 | **VI-67** | **VI-68** | **VI-69** | **VI-70** | **VI-71** | **VI-72** | **VI-73** | **VI-74** |
| R^{3_}8 | **VI-78** | **VI-79** | **VI-80** | **VI-81** | **VI-82** | **VI-83** | **VI-84** | **VI-85** |
| R^{3_}9 | **VI-89** | **VI-90** | **VI-91** | **VI-92** | **VI-93** | **VI-94** | **VI-95** | **VI-96** |
| R³-10 | **VI-100** | **VI-101** | **VI-102** | **VI-103** | **VI-104** | **VI-105** | **VI-106** | **VI-107** |
| R³-11 | **VI-111** | **VI-112** | **VI-113** | **VI-114** | **VI-115** | **VI-116** | **VI-117** | **VI-118** |
| R³-12 | **VI-122** | **VI-123** | **VI-124** | **VI-125** | **VI-126** | **VI-127** | **VI-128** | **VI-129** |
| R³-13 | **VI-133** | **VI-134** | **VI-135** | **VI-136** | **VI-137** | **VI-138** | **VI-139** | **VI-140** |
| R³-14 | **VI-144** | **VI-145** | **VI-146** | **VI-147** | **VI-148** | **VI-149** | **VI-150** | **VI-151** |
| R³-15 | **VI-155** | **VI-156** | **VI-157** | **VI-158** | **VI-159** | **VI-160** | **VI-161** | **VI-162** |
| R³-16 | **VI-166** | **VI-167** | **VI-168** | **VI-169** | **VI-170** | **VI-171** | **VI-172** | **VI-173** |
| R³-17 | **VI-177** | **VI-178** | **VI-179** | **VI-180** | **VI-181** | **VI-182** | **VI-183** | **VI-184** |
| R³-18 | **VI-188** | **VI-189** | **VI-190** | **VI-191** | **VI-192** | **VI-193** | **VI-194** | **VI-195** |
| R³-19 | **VI-199** | **VI-200** | **VI-201** | **VI-202** | **VI-203** | **VI-204** | **VI-205** | **VI-206** |
| R³-20 | **VI-210** | **VI-211** | **VI-212** | **VI-213** | **VI-214** | **VI-215** | **VI-216** | **VI-217** |
| R³-21 | **VI-221** | **VI-222** | **VI-223** | **VI-224** | **VI-225** | **VI-226** | **VI-227** | **VI-228** |
| R³-22 | **VI-232** | **VI-233** | **VI-234** | **VI-235** | **VI-236** | **VI-237** | **VI-238** | **VI-239** |
| R³-23 | **VI-243** | **VI-244** | **VI-245** | **VI-246** | **VI-247** | **VI-248** | **VI-249** | **VI-250** |
| R³-24 | **VI-254** | **VI-255** | **VI-256** | **VI-257** | **VI-258** | **VI-259** | **VI-260** | **VI-261** |
| R³-25 | **VI-265** | **VI-266** | **VI-267** | **VI-268** | **VI-269** | **VI-270** | **VI-271** | **VI-272** |
| R³-26 | **VI-276** | **VI-277** | **VI-278** | **VI-279** | **VI-280** | **VI-281** | **VI-282** | **VI-283** |
| R³-27 | **VI-287** | **VI-288** | **VI-289** | **VI-290** | **VI-291** | **VI-292** | **VI-293** | **VI-294** |
| R³-28 | **VI-298** | **VI-299** | **VI-300** | **VI-301** | **VI-302** | **VI-303** | **VI-304** | **VI-305** |
| R³-29 | **VI-309** | **VI-310** | **VI-311** | **VI-312** | **VI-313** | **VI-314** | **VI-315** | **VI-316** |
| R³-30 | **VI-320** | **VI-321** | **VI-322** | **VI-323** | **VI-324** | **VI-325** | **VI-326** | **VI-327** |
| R³-31 | **VI-331** | **VI-332** | **VI-333** | **VI-334** | **VI-335** | **VI-336** | **VI-337** | **VI-338** |
| R³-32 | **VI-342** | **VI-343** | **VI-344** | **VI-345** | **VI-346** | **VI-347** | **VI-348** | **VI-349** |
| R³-33 | **VI-353** | **VI-354** | **VI-355** | **VI-356** | **VI-357** | **VI-358** | **VI-359** | **VI-360** |
| R³-34 | **VI-364** | **VI-365** | **VI-366** | **VI-367** | **VI-368** | **VI-369** | **VI-370** | **VI-371** |
| R³-35 | **VI-375** | **VI-376** | **VI-377** | **VI-378** | **VI-379** | **VI-380** | **VI-381** | **VI-382** |
| R³-36 | **VI-386** | **VI-387** | **VI-388** | **VI-389** | **VI-390** | **VI-391** | **VI-392** | **VI-393** |
| R³-37 | **VI-397** | **VI-398** | **VI-399** | **VI-400** | **VI-401** | **VI-402** | **VI-403** | **VI-404** |
| R³-38 | **VI-408** | **VI-409** | **VI-410** | **VI-411** | **VI-412** | **VI-413** | **VI-414** | **VI-415** |
| R³-39 | **VI-419** | **VI-420** | **VI-421** | **VI-422** | **VI-423** | **VI-424** | **VI-425** | **VI-426** |
| R³-40 | **VI-430** | **VI-431** | **VI-432** | **VI-433** | **VI-434** | **VI-435** | **VI-436** | **VI-437** |
| R³-41 | **VI-441** | **VI-442** | **VI-443** | **VI-444** | **VI-445** | **VI-446** | **VI-447** | **VI-448** |
| R³-42 | **VI-452** | **VI-453** | **VI-454** | **VI-455** | **VI-456** | **VI-457** | **VI-458** | **VI-459** |
| R³-43 | **VI-463** | **VI-464** | **VI-465** | **VI-466** | **VI-467** | **VI-468** | **VI-469** | **VI-470** |
| R³-44 | **VI-474** | **VI-475** | **VI-476** | **VI-477** | **VI-478** | **VI-479** | **VI-480** | **VI-481** |
| R³-45 | **VI-485** | **VI-486** | **VI-487** | **VI-488** | **VI-489** | **VI-490** | **VI-491** | **VI-492** |
| R³-46 | **VI-496** | **VI-497** | **VI-498** | **VI-499** | **VI-500** | **VI-501** | **VI-502** | **VI-503** |
| R³-47 | **VI-507** | **VI-508** | **VI-509** | **VI-510** | **VI-511** | **VI-512** | **VI-513** | **VI-514** |
| R³-48 | **VI-518** | **VI-519** | **VI-520** | **VI-521** | **VI-522** | **VI-523** | **VI-524** | **VI-525** |
| R³-49 | **VI-529** | **VI-530** | **VI-531** | **VI-532** | **VI-533** | **VI-534** | **VI-535** | **VI-536** |
| R³-50 | **VI-540** | **VI-541** | **VI-542** | **VI-543** | **VI-544** | **VI-545** | **VI-546** | **VI-547** |
| R³-51 | **VI-551** | **VI-552** | **VI-553** | **VI-554** | **VI-555** | **VI-556** | **VI-557** | **VI-558** |
| R³-52 | **VI-562** | **VI-563** | **VI-564** | **VI-565** | **VI-566** | **VI-567** | **VI-568** | **VI-569** |
| R³-53 | **VI-573** | **VI-574** | **VI-575** | **VI-576** | **VI-577** | **VI-578** | **VI-579** | **VI-580** |
| R³-54 | **VI-584** | **VI-585** | **VI-586** | **VI-587** | **VI-588** | **VI-589** | **VI-590** | **VI-591** |
| R³-55 | **VI-595** | **VI-596** | **VI-597** | **VI-598** | **VI-599** | **VI-600** | **VI-601** | **VI-602** |
| R³-56 | **VI-606** | **VI-607** | **VI-608** | **VI-609** | **VI-610** | **VI-611** | **VI-612** | **VI-613** |
| R³-57 | **VI-617** | **VI-618** | **VI-619** | **VI-620** | **VI-621** | **VI-622** | **VI-623** | **VI-624** |
| R³-58 | **VI-628** | **VI-629** | **VI-630** | **VI-631** | **VI-632** | **VI-633** | **VI-634** | **VI-635** |
| R³-59 | **VI-639** | **VI-640** | **VI-641** | **VI-642** | **VI-643** | **VI-644** | **VI-645** | **VI-646** |
| R³-60 | **VI-650** | **VI-651** | **VI-652** | **VI-653** | **VI-654** | **VI-655** | **VI-656** | **VI-657** |
| R³-61 | **VI-661** | **VI-662** | **VI-663** | **VI-664** | **VI-665** | **VI-666** | **VI-667** | **VI-668** |
| R³-62 | **VI-672** | **VI-673** | **VI-674** | **VI-675** | **VI-676** | **VI-677** | **VI-678** | **VI-679** |
| R³-63 | **VI-683** | **VI-684** | **VI-685** | **VI-686** | **VI-687** | **VI-688** | **VI-689** | **VI-690** |
| R³-64 | **VI-694** | **VI-695** | **VI-696** | **VI-697** | **VI-698** | **VI-699** | **VI-700** | **VI-701** |

| | R² | | | | | | | |
|---|---|---|---|---|---|---|---|---|
| R³ | R²-56 | R²-57 | R²-58 | R²-59 | R²-60 | R²-61 | R²-62 | R²-63 |
| R³-65 | **VI-705** | **VI-706** | **VI-707** | **VI-708** | **VI-709** | **VI-710** | **VI-711** | **VI-712** |
| R³-66 | **VI-716** | **VI-717** | **VI-718** | **VI-719** | **VI-720** | **VI-721** | **VI-722** | **VI-723** |
| R³-67 | **VI-727** | **VI-728** | **VI-729** | **VI-730** | **VI-731** | **VI-732** | **VI-733** | **VI-734** |
| R³-68 | **VI-738** | **VI-739** | **VI-740** | **VI-741** | **VI-742** | **VI-743** | **VI-744** | **VI-745** |
| R³-69 | **VI-749** | **VI-750** | **VI-751** | **VI-752** | **VI-753** | **VI-754** | **VI-755** | **VI-756** |
| R³-70 | **VI-760** | **VI-761** | **VI-762** | **VI-763** | **VI-764** | **VI-765** | **VI-766** | **VI-767** |
| R³-71 | **VI-771** | **VI-772** | **VI-773** | **VI-774** | **VI-775** | **VI-776** | **VI-777** | **VI-778** |
| R³-72 | **VI-782** | **VI-783** | **VI-784** | **VI-785** | **VI-786** | **VI-787** | **VI-788** | **VI-789** |
| R³-73 | **VI-793** | **VI-794** | **VI-795** | **VI-796** | **VI-797** | **VI-798** | **VI-799** | **VI-800** |
| R³-74 | **VI-804** | **VI-805** | **VI-806** | **VI-807** | **VI-808** | **VI-809** | **VI-810** | **VI-811** |
| R³-75 | **VI-815** | **VI-816** | **VI-817** | **VI-818** | **VI-819** | **VI-820** | **VI-821** | **VI-822** |
| R³-76 | **VI-826** | **VI-827** | **VI-828** | **VI-829** | **VI-830** | **VI-831** | **VI-832** | **VI-833** |
| R³-77 | **VI-837** | **VI-838** | **VI-839** | **VI-840** | **VI-841** | **VI-842** | **VI-843** | **VI-844** |
| R³-78 | **VI-848** | **VI-849** | **VI-850** | **VI-851** | **VI-852** | **VI-853** | **VI-854** | **VI-855** |
| R³-79 | **VI-859** | **VI-860** | **VI-861** | **VI-862** | **VI-863** | **VI-864** | **VI-865** | **VI-866** |
| R³-80 | **VI-870** | **VI-871** | **VI-872** | **VI-873** | **VI-874** | **VI-875** | **VI-876** | **VI-877** |
| R³-81 | **VI-881** | **VI-882** | **VI-883** | **VI-884** | **VI-885** | **VI-886** | **VI-887** | **VI-888** |
| R³-82 | **VI-892** | **VI-893** | **VI-894** | **VI-895** | **VI-896** | **VI-897** | **VI-898** | **VI-899** |
| R³-83 | **VI-903** | **VI-904** | **VI-905** | **VI-906** | **VI-907** | **VI-908** | **VI-909** | **VI-910** |
| R³-84 | **VI-914** | **VI-915** | **VI-916** | **VI-917** | **VI-918** | **VI-919** | **VI-920** | **VI-921** |
| R³-85 | **VI-925** | **VI-926** | **VI-927** | **VI-928** | **VI-929** | **VI-930** | **VI-931** | **VI-932** |
| R³-86 | **VI-936** | **VI-937** | **VI-938** | **VI-939** | **VI-940** | **VI-941** | **VI-942** | **VI-943** |
| R³-87 | **VI-947** | **VI-948** | **VI-949** | **VI-950** | **VI-951** | **VI-952** | **VI-953** | **VI-954** |
| R³-88 | **VI-958** | **VI-959** | **VI-960** | **VI-961** | **VI-962** | **VI-963** | **VI-964** | **VI-965** |
| R³-89 | **VI-969** | **VI-970** | **VI-971** | **VI-972** | **VI-973** | **VI-974** | **VI-975** | **VI-976** |
| R³-90 | **VI-980** | **VI-981** | **VI-982** | **VI-983** | **VI-984** | **VI-985** | **VI-986** | **VI-987** |
| R³-91 | **VI-991** | **VI-992** | **VI-993** | **VI-994** | **VI-995** | **VI-996** | **VI-997** | **VI-998** |
| R³-92 | **VI-1002** | **VI-1003** | **VI-1004** | **VI-1005** | **VI-1006** | **VI-1007** | **VI-1008** | **VI-1009** |
| R³-93 | **VI-1013** | **VI-1014** | **VI-1015** | **VI-1016** | **VI-1017** | **VI-1018** | **VI-1019** | **VI-1020** |
| R³-94 | **VI-1024** | **VI-1025** | **VI-1026** | **VI-1027** | **VI-1028** | **VI-1029** | **VI-1030** | **VI-1031** |
| R³-95 | **VI-1035** | **VI-1036** | **VI-1037** | **VI-1038** | **VI-1039** | **VI-1040** | **VI-1041** | **VI-1042** |
| R³-96 | **VI-1046** | **VI-1047** | **VI-1048** | **VI-1049** | **VI-1050** | **VI-1051** | **VI-1052** | **VI-1053** |
| R³-97 | **VI-1057** | **VI-1058** | **VI-1059** | **VI-1060** | **VI-1061** | **VI-1062** | **VI-1063** | **VI-1064** |
| R³-98 | **VI-1068** | **VI-1069** | **VI-1070** | **VI-1071** | **VI-1072** | **VI-1073** | **VI-1074** | **VI-1075** |
| R³-99 | **VI-1079** | **VI-1080** | **VI-1081** | **VI-1082** | **VI-1083** | **VI-1084** | **VI-1085** | **VI-1086** |

In a preferred embodiment, the compound of formula la corresponds to the following compound of Formula la': 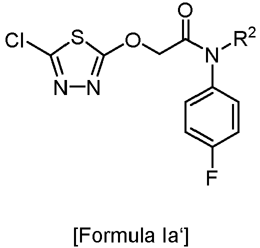 wherein R² is selected from (i) allyl, (ii) propargyl, (iii) 1-butyn-3-yl (iv) 1-butyn-4-yl, (v) 2-butyn-1-yl, (vi) prop-1-yl and (vii) 3-methyl-1-butyn-3-yl.

Alternatively, the compound of formula la corresponds to the following compound: 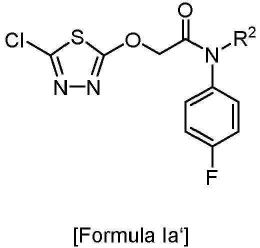 wherein R² is selected from: (i) allyl, (ii) propargyl, (iii) 1-butyn-4-yl and (iv) 2-butyn-1-yl.

In another preferred embodiment, the compound is represented by the Formula !, wherein:
- R² is isopropyl;
   and
- R3 is selected from:
   (i) 3,4-difluorophenyl;
   (ii) 2,4-difluorophenyl;
   (iii) 3-fluoro-4-chlorophenyl; and
   (iv) 3-chloro-4-fluorophenyl;
or an agrochemically acceptable salt thereof, a stereoisomer thereof, an enantiomer thereof, or a tautomer thereof.

Most preferably, the compound of formula I corresponds to a compound of formula la where:
- R² is propargyl (R²-7) and R³ is 4-fluorophenyl (R³-1) (i.e. compound 1-7); or
- R² is isopropyl (R²-9) and R³ is 2,4-difluorophenyl (R³-26) (i.e. compound 1-284).

In some embodiments, the compound of formula I may contain asymmetric centres and may be present as a single enantiomer, a pair of enantiomers in any proportion or, where more than one asymmetric center is present, contain diastereomers in all possible ratios. In case the compound of formula I contains one or more double bonds, the one or more double bonds may each independently exist in (E)- or (Z)-configuration. Any stereoisomers, enantiomers, geometric isomers, as well as tautomers of the compound of formula I are also encompassed by the present invention.

In addition, the compound of formula I may form agrochemically acceptable salts which are also covered by the present invention. Suitable salts include, but are not limited to, salts of acceptable inorganic acids such as hydrochloric, sulfuric, phosphoric, nitric, carbonic, boric, sulfamic, and hydrobromic acids, or salts of agronomically acceptable organic acids such as acetic, propionic, butyric, tartaric, maleic, hydroxymaleic, fumaric, malic, citric, lactic, mucic, gluconic, benzoic, succinic, oxalic, phenylacetic, methanesulfonic, toluenesulfonic, benzenesulfonic, salicylic, sulfanilic, aspartic, glutamic, edetic, stearic, palmitic, oleic, lauric, pantothenic, tannic, ascorbic and valeric acids. Suitable salts also include salts of inorganic and organic bases, e.g. counterions such as Na, Ca, K, Li, Mg, ammonium, and trimethylsulfonium. In addition, any isotopes including deuterated compounds are covered by the compound of formula I.

### Compositions comprising one or more compounds of formula I

The compounds of formula I according to the invention can be used as herbicides by themselves, but they can also be formulated into compositions. Therefore, in a second aspect, the present invention provides a composition comprising one or more compounds of formula I or an agrochemically acceptable salt thereof, a stereoisomer thereof, an enantiomer thereof or a tautomer thereof, and one or more auxiliary and/or adjuvant, and optionally one or more further active ingredients. In a certain embodiment, the composition comprising one or more compounds of formula I is an agrochemical composition.

The compositions can be in various physical forms, e.g., in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates. Such compositions can either be used directly or diluted prior to use. The dilutions can be made, for example, with water, liquid fertilisers, micronutrients, biological organisms, oil or solvents.

The compositions according to the second aspect of the invention are prepared in a known manner, by mixing the compounds of the formula I with the one or more adjuvants, and optionally one or more other active ingredients. In addition, the composition may comprise further additives that are conventional in this technical field, including but not limited to colorants, wetting agents, dispersants, emulsifiers, antifoams, preservatives, secondary thickeners, adhesives, gibberellins and water.

For example, the compositions can be prepared e.g., by mixing a compound according the first aspect of the present invention as the active ingredient with the adjuvant(s) in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof. The active ingredients can also be contained in very fine microcapsules. Microcapsules contain the active ingredients in a porous carrier. This enables the active ingredients to be released into the environment in controlled amounts (e.g., slow-release).

Microcapsules usually have a diameter of from 0.1 to 500 microns. They contain active ingredients in an amount of about from 25 to 95% by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

### (a) Adjuvants

The adjuvants that are suitable for the preparation of the compositions according to the second aspect of the present invention are known in this technical field.

As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1 ,2-dichloropropane, diethanolamine, *p*-diethylbenzene, diethylene glycol, diethylene glycol abietate, diethylene glycol butyl ether, diethylene glycol ethyl ether, diethylene glycol methyl ether, *N,N-*dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxy-propanol, methyl isoamyl ketone, methyl isobutyl ketone, methyl laurate, methyl octanoate, methyl oleate, methylene chloride, *m*-xylene, *n*-hexane, *n*-octylamine, octadecanoic acid, octylamine acetate, oleic acid, oleylamine, *o*-xylene, phenol, polyethylene glycol, propionic acid, propyl lactate, propylene carbonate, propylene glycol, propylene glycol methyl ether, *p*-xylene, toluene, triethyl phosphate, triethylene glycol, xylene sulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, N-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described *e.g*., in McCutcheon's Detergents and Emulsifiers Annual, MC Publishing Corp., Ridgewood New Jersey (1981).

Further adjuvants that can be used in herbicidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the second aspect of the present invention can further include an additive comprising an oil of vegetable or animal origin, a mineral oil, alkyl esters of such oils or mixtures of such oils and oil derivatives. The amount of oil additive in the composition according to the invention is generally from 0.01 to 10%, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99% by weight, especially from 0.1 to 95% by weight, of compounds of the present invention and from 1 to 99.9% by weight of a formulation adjuvant which preferably includes from 0 to 25% by weight of a surface-active substance.

Formulation types for the composition of the invention include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants. Whereas commercial products may preferably be formulated as concentrates, the end user will normally employ dilute formulations.

The rates of application vary within wide limits and depend on the nature of the soil, the method of application, the crop plant, the species to be controlled, the prevailing climatic conditions, and other factors governed by the method of application, the time of application and the target crop. As a general guideline, compounds may be applied at a rate of from 1 to 2000 l/ha, especially from 10 to 1000 l/ha.

### Agrochemical combinations

The compounds according to the first aspect of the present invention and the composition according to the second aspect of the present invention may also be used with further active ingredients. Therefore, in another aspect of the present invention, it is provided an agrochemical combination comprising the compound according to the first aspect of the present invention or the composition according to the second aspect of the present invention and one or more additional herbicides and/or plant growth regulators.

Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, benquitrione, bensulfuron (including bensulfuron-methyl), bentazone, bicyclopyrone, bifenox, bilanafos, bipyrazone, bispyribac-sodium, bixlozone, bromacil, bromoxynil, butachlor, butafenacil, carfentrazone (including carfentrazone-ethyl), cloransulam (including cloransulam-methyl), chlomethoxyfen, chlorimuron (including chlorimuron-ethyl), chlorotoluron, chlorsulfuron, cinmethylin, cinflubrolin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, dioxopyritrione, diquat dibromide, diuron, epyrifenacil, ethalfluralin,ethofumesate, fenoxaprop (including fenoxaprop-P-ethyl), fenoxasulfone, fenpyrazone, fenquinotrione, fentrazamide, feproxydim, flazasulfuron, florasulam, florpyrauxifen (including florpyrauxifen-benzyl), fluazifop (including fluazifop-P-butyl), flucarbazone (including flucarbazone-sodium), , flumetsulam, flumioxazin, fluometuron,fomesafen flupyrsulfuron (including flupyrsulfuron-methyl-sodium), fluroxypyr (including fluroxypyr-meptyl), fomesafen, foramsulfuron, glufosinate (including L-glufosinate and the ammonium salts of both), glyphosate (including the diammonium, isopropylammonium and potassium salts thereof), halauxifen (including halauxifen-methyl), haloxyfop (including haloxyfop-methyl), hexazinone, hydantocidin, imazamox (including R-imazamox), imazapic, imazapyr, imazethapyr, indaziflam, iodosulfuron (including iodosulfuron-methyl-sodium), iofensulfuron (including iofensulfuron-sodium), ioxynil, isoproturon, isoxafenacil, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, nonanoic acid, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyraquinate, pyrasulfotole, pyridate, pyriflubenzoxim, pyriftalid, pyrimisulfan, pyroxasulfone, pyroxsulam, quinclorac, quinmerac, quizalofop (including quizalofop-P-ethyl and quizalofop-P-tefuryl), rimisoxafen, rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, tripyrasulfone, 3-(2- chloro-4-fluoro-5-(3-methyl-2,6-dioxo-4-trifluoromethyl-3,6-dihydropyrimidin-1 (2H)-yl)phenyl)-5-methyl-4,5-dihydroisoxazole-5-carboxylic acid ethyl ester,4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1 H-indol-6-yl)pyridine-2-carboxylate, prop-2-ynyl 4-amino-3-chloro-5-fluoro-6-(7-fluoro-1 H-indol-6-yl)pyridine-2-carboxylate and cyanomethyl 4-amino-3-chloro-5-fluoro-6-(7-fluoro-1 H-indol-6-yl)pyridine-2-carboxylate), 3-ethylsulfanyl-N-(1,3,4-oxadiazol-2-yl)-5-(trifluoromethyl)-[1,2,4]triazolo[4,3-a]pyridine-8-carboxamide, 3-(isopropylsulfanylmethyl)-N-(5-methyl-1,3,4-oxadiazol-2-yl)-5-(trifluoromethyl)-[1,2,4]triazolo[4,3-a]pyridine-8-carboxamide, 3-(isopropylsulfonylmethyl)-N-(5-methyl-'1,3,4-oxadiazol-2-yl)-5-(trifluoromethyl)-[1,2,4]triazolo[4,3-a]pyridine-8-carboxamide, 3-(ethylsulfonylmethyl)-N-(5-methyl-1,3,4-oxadiazol-2-yl)-5-(trifluoromethyl)-[1,2,4]triazolo[4,3-a]pyridine-8-carboxamide, ethyl-2-[[3-[[3-chloro-5-fluoro-6-[3-methyl-2,6-dioxo-4-(trifluoromethyl)pyrimidin-1-yl]-2-pyridyl]oxy]acetate,6-chloro-4-(2,7-dimethyl-1-naphthyl)-5-hydroxy-2-methyl-pyridazin-3-one, tetrahydrofuran-2-ylmethyl (2R)-2-[(4-amino-3,5-dichloro-6-fluoro-2-pyridyl)oxy]propanoate, tetrahydrofuran-2-ylmethyl (2R)-2-[(4-amino-3,5-dichloro-6-fluoro-2-pyridyl)oxy]propanoate, tetrahydrofuran-2-ylmethyl 2-[(4-amino-3,5-dichloro-6-fluoro-2-pyridyl)oxy]propanoate, 2-[(4-amino-3,5-dichloro-6-fluoro-2-pyridyl)oxy]propanoic acid, 2-fluoro-N-(5-methyl-1,3,4-oxadiazol-2-yl)-3-[(R)-propylsulfinyl]-4-(trifluoromethyl)benzamide, 2-fluoro-N-(5-methyl-1,3,4-oxadiazol-2-yl)-3-propylsulfinyl-4-(trifluoromethyl)benzamide, (2-fluorophenyl)methyl 6-amino-5-chloro-2-(4-chloro-2-fluoro-3-methoxyphenyl)pyrimidine-4-carboxylate and 6-amino-5-chloro-2-(4-chloro-2-fluoro-3-methoxyphenyl)pyrimidine-4-carboxylic acid.

### Use of the compounds and compositions

It was surprisingly found that compounds of formula I exhibit a desirable effect acting as a herbicide. Therefore, in a third aspect, the present invention provides the use of the compound according to the first aspect or the composition according to the second aspect in agriculture, preferably as a herbicide.

For example, the compound of the first aspect or an agrochemically acceptable salt thereof, a stereoisomer thereof, an enantiomer thereof or a tautomer thereof, or a composition according to the second aspect may be used as a herbicide on crops selected from cereals including barley and wheat, cotton, oilseed rape, sunflower, maize, rice, soybeans, sugar beet, sugar cane, turf, trees including fruit trees, palm trees, coconut trees or other nuts, vines including grapes, fruit bushes, fruit plants, and vegetables including potatoes and tomatoes.

Preferably, the compound of the first aspect or an agrochemically acceptable salt thereof, a stereoisomer thereof, an enantiomer thereof, a deuteromer thereof or a tautomer thereof, or a composition according to the second aspect is used as a herbicide, more preferably a pre-emergence herbicide, even more preferably a broad-spectrum herbicide, against a monocotyledonous plant (i.e. from the plant clade Monocots) and/or a dicotyledonous plant (i.e. from the plant clade Eudicots). In an exemplary embodiment, the compound of the first aspect or an agrochemically acceptable salt thereof, a stereoisomer thereof, an enantiomer thereof or a tautomer thereof, or a composition according to the second aspect is used against species selected from blackgrass, wild oat, Fat Hen, Crabgrass, Barnyard grass, cut-leaved crane's-bill, Italian ryegrass, Common Millet, green foxtail and Black nightshade. For example, it may be used against species selected from *Alopecurus myosuroidpes* (ALOMY), *Apera spica-venti* (APESV), *Avena fatua* (AVEFA), *Chenopodium album* (CHEAL), *Digitaria sanguinalis* (DIGSA), *Echinochloa crus-galli* (ECHCG), *Geranium dissectum* (GERDI), *Lolium multiflorum* (LOLMU), *Panicum miliaceum* (PANMI), *Poa annua* (POAAN), *Setaria viridis* (SETVI) and *Solanum nigrum* (SOLNI). In a more preferred embodiment, the compound of the first aspect or an agrochemically acceptable salt thereof, a stereoisomer thereof, an enantiomer thereof or a tautomer thereof, or a composition according to the second aspect is used as a broad-spectrum herbicide, preferably a pre-emergence herbicide, against monocotyledonous plants, and as a specific herbicide against *Chenopodium album* (CHEAL), *Geranium dissectum* (GERDI), and/or *Solanum nigrum* (SOLNI).

In one specific embodiment, there is the use of a compound of formula !, wherein R² is selected from 1-butyn-4-yl, 2-butyn-1-yl, propargyl, 1-butyn-3-yl, prop-1-yl, 3-methyl-1-butyn-3-yl and allyl, and R³ is 4-fluorophenyl, or wherein R² is isopropyl, and R³ is selected from 4-chlorophenyl, 4-nitrophenyl, 4-(trifluoromethyl)phenyl, 3,4-difluorophenyl, 2,4-difluorophenyl, 3-fluoro-4-chlorophenyl and 3-chloro-4-fluorophenyl, against species selected from *Alopecurus myosuroidpes* (ALOMY), *Apera spica-venti* (APESV), *Avena fatua* (AVEFA), *Chenopodium album* (CHEAL), *Digitaria sanguinalis* (DIGSA), *Echinochloa crus-galli* (ECHCG), *Geranium dissectum* (GERDI), *Lolium multiflorum* (LOLMU), *Panicum miliaceum* (PANMI), *Poa annua* (POAAN), *Setaria viridis* (SETVI) and *Solanum nigrum* (SOLNI). Preferably, there is the use of a compound of formula !, wherein R² is selected from 1-butyn-4-yl, 2-butyn-1-yl, propargyl, 1-butyn-3-yl, prop-1-yl, 3-methyl-1-butyn-3-yl and allyl, and R³ is 4-fluorophenyl against one or more species selected from ALOMY, APESV, AVEFA, DIGSA, ECHCG, LOLMU, PANMI, POAAN and SETVI, and optionally also against CHEAL, GERDI and/or SOLNI. More preferably, there is the use of said compound of formula I against ALOMY, LOLMU and APESV, and as a pre-emergence herbicide against monocotyledonous plants, and as a specific herbicide against *Chenopodium album* (CHEAL), *Geranium dissectum* (GERDI), and/or *Solanum nigrum* (SOLNI).

In another embodiment, there is the use of a compound of formula !, wherein R² is iso-propyl and R³ is selected from 3,4-difluorophenyl, 2,4-difluorophenyl, 3-fluoro-4-chlorophenyl and 3-chloro-4-fluorophenyl against species selected from ALOMY, APESV, AVEFA, DIGSA, ECHCG, GERDI, LOLMU, PANMI, POAAN and SETVI, and optionally also against CHEAL, GERDI and/or SOLNI.

In a further embodiment, there is the use of a compound of formula !, wherein R² is iso-butyl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, AVEFA, CHEAL, DIGSA, ECHCG, LOLMU, PANMI and SOLNI, more preferably against one or more species selected from AVEFA, DIGSA, ECHCG and LOLMU, even more preferably one or more species selected from DIGSA, ECHCG and LPLMU.

In yet another embodiment, there is the use of a compound of formula !, wherein R² is propargyl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, APESV, AVEFA, CHEAL, DIGSA, ECHCG, GERDI, LOLMU, PANMI, POAAN, SETVI and SOLNI, more preferably against one or more species selected from ALOMY, APESV, CHEAL, DIGSA, ECHCG, LOLMU, PANMI, POAAN, SETVI and SOLNI, even more preferably one or more species selected from CHEAL, LOLMU and SOLNI.

In yet another embodiment, there is the use of a compound of formula !, wherein R² is cyclopentyl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from AVEFA, CHEAL, DIGSA, ECHCG, LOLMU, PANMI and SOLNI, more preferably against one or more species selected from DIGSA and ECHCG.

In a further embodiment, there is the use of a compound of formula !, wherein R² is tert-butyl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from AVEFA, CHEAL, DIGSA, ECHCG, LOLMU, PANMI and SOLNI, more preferably against one or more species selected from CHEAL, DIGSA and ECHCG, even more preferably against the species ECHCG.

In another embodiment, there is the use of a compound of formula I, wherein R² is allyl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably ALOMY, APESV, DIGSA, ECHCG, GERDI, LOLMU, PANMI, POAAN, SETVI and SOLNI, more preferably against one or more species selected from ALOMY, APESV, DIGSA, LOLMU, POAAN, SETVI and SOLNI, even more preferably against one or more species selected from ALOMY and LOLMU.

In yet a further embodiment, there is the use of a compound of formula I, wherein R² is sec-butyl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, AVEFA, CHEAL, DIGSA, ECHCG, LOLMU and PANMI, more preferably against the species ALOMY.

In another embodiment, there is the use of a compound of formula I, wherein R² is cyclopropyl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, AVEFA, CHEAL, DIGSA, ECHCG, LOLMU and PANMI, more preferably against one or more species selected from ALOMY, DIGSA, ECHCG, LOLMU and PANMI.

Also, in a further embodiment, there is the use of a compound of formula I, wherein R² is cyanomethyl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, AVEFA, CHEAL, DIGSA, ECHCG, LOLMU and PANMI, more preferably against one or more species selected from ALOMY, DIGSA and LOLMU, and even more preferably against the species DIGSA.

In another embodiment, there is the use of a compound of formula I, wherein R² is 1-butyn-4-yl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, APESV, CHEAL, DIGSA, ECHCG, LOLMU, PANMI, POAAN, SETVI and SOLNI, more preferably against one or more species selected from ALOMY, APESV, CHEAL, DIGSA, PANMI, POAAN, SETVI and SOLNI, even more preferably against one or more species selected from ALOMY and SOLNI.

In another embodiment, there is the use of a compound of formula I, wherein R² is 2-butyn-1-yl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, APESV, AVEFA, CHEAL, DIGSA, ECHCG, LOLMU, PANMI, POAAN, SETVI and SOLNI, more preferably against one or more species selected from ALOMY, APESV, DIGSA, ECHCG, POAAN, SETVI and SOLNI.

In another embodiment, there is the use of a compound of formula I, wherein R² is isopropyl and R³ is 3,4-difluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, APESV, CHEAL, DIGSA, ECHCG, GERDI, PANMI, POAAN, SETVI and SOLNI, more preferably against one or more species selected from APESV, CHEAL, DIGSA, ECHCG, GERDI, PANMI, SETVI and SOLNI, even more preferably against one or more species selected from CHEAL and GERDI.

In another embodiment, there is the use of a compound of formula I, wherein R² is isopropyl and R³ is 2,4-difluorophenyl against one or more species selected from ALOMY, APESV, DIGSA, ECHCG, GERDI, LOLMU, PANMI, POAAN, SETVI and SOLNI, preferably against one or more species selected from ALOMY, APESV, DIGSA, ECHCG, LOLMU, PANMI, POAAN, SETVI and SOLNI, more preferably against one or more species selected from APESV, DIGSA, ECHCG, LOLMU, PANMI, POAAN, SETVI and SOLNI, even more preferably against LOLMU.

In another embodiment, there is the use of a compound of formula I, wherein R² is isopropyl and R³ is 3-fluoro-4-chlorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, AVEFA, DIGSA, ECHCG, PANMI, POAAN and SETVI, more preferably against one or more species selected from DIGSA, ECHCG, PANMI, POAAN and SETVI.

In another embodiment, there is the use of a compound of formula I, wherein R² is isopropyl and R³ is 3-chloro-4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY, CHEAL, DIGSA, ECHCG, LOLMU, PANMI and POAAN, more preferably against one or more species selected from CHEAL and LOLMU.

In another embodiment, there is the use of a compound of formula I, wherein R² is prop-1-yl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY and LOLMU, more preferably against LOLMU.

In another embodiment, there is the use of a compound of formula I, wherein R² is 1-butyn-3-yl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY and LOLMU, more preferably against ALOMY.

In another embodiment, there is the use of a compound of formula I, wherein R² is 3-methyl-1-butyn-3-yl and R³ is 4-fluorophenyl against one or more species selected from ALOMY, APESV, AVEFA, CHEAL, DIGSA, ECHCG, GERDI, LOLMU, PANMI, POAAN, SETVI and SOLNI, preferably against one or more species selected from ALOMY and LOLMU, more preferably against ALOMY.

In another embodiment, there is the use of a compound of formula I, wherein R² is isopropyl and R³ is 3-cyano-4-fluorophenyl against one or more species selected from ALOMY, AVEFA, CHEAL, ECHCG, LOLMU, PANMI and POAAN, preferably against one or more species selected from ALOMY, CHEAL, LOLMU and PANMI, even more preferably against ALOMY.

In another embodiment, there is the use of a compound of formula I, wherein R² is 3 isopropyl and R³ is 4-cyano-3-fluorophenyl against one or more species selected from ALOMY, AVEFA, CHEAL, ECHCG, LOLMU, PANMI and POAAN, preferably against one or more species selected from ALOMY, CHEAL, ECHCG, LOLMU and POAAN, more preferably against ALOMY, CHEAL and POAAN.

In another embodiment, there is the use of a compound of formula I, wherein R² is propargyl and R³ is 2,4-difluorophenyl against one or more species selected from ALOMY, AVEFA, CHEAL, ECHCG, LOLMU, PANMI and POAAN, preferably against one or more species selected from ALOMY, CHEAL, ECHCG, LOLMU, PANMI and POAAN, more preferably against ALOMY, CHEAL, ECHCG and POAAN.

In another embodiment, there is the use of a compound of formula I, wherein R² is 2-butyn-1-yl and R³ is 2,4-difluorophenyl against one or more species selected from ALOMY, AVEFA, CHEAL, ECHCG, LOLMU, PANMI and POAAN, preferably against one or more species selected from ALOMY, CHEAL, ECHCG, LOLMU and POAAN, more preferably against one or more species selected from ALOMY, ECHCG and POAAN, even more preferably against ALOMY and POAAN.

In another embodiment, there is the use of a compound of formula !, wherein R² is allyl and R³ is 2,4-difluorophenyl against one or more species selected from ALOMY, AVEFA, CHEAL, ECHCG, LOLMU, PANMI and POAAN, preferably against one or more species selected from ALOMY, AVEFA, CHEAL, ECHCG, LOLMU and POAAN, more preferably against one or more species selected from ALOMY, CHEAL, ECHCG, LOLMU and POAAN, even more preferably against ALOMY and CHEAL.

In another embodiment, there is the use of a compound of formula I, wherein R² is isopropyl and R³ is 2,3,4-trifluorophenyl against one or more species selected from ALOMY, AVEFA, CHEAL, ECHCG, LOLMU, PANMI and POAAN, preferably against one or more species selected from ALOMY, CHEAL, ECHCG, LOLMU, PANMI and POAAN, more preferably against ALOMY, ECHCG, PANMI and POAAN.

In another embodiment, there is the use of a compound of formula I, wherein R² is isopropyl and R³ is 4-chloro-2-fluorophenyl against one or more species selected from AVEFA, ECHCG, LOLMU, PANMI and POAAN, preferably against one or more species selected from ECHCG, LOLMU, PANMI and POAAN.

In another embodiment, there is the use of a compound of formula I, wherein R² is isopropyl and R³ is 2,4-chlorophenyl against one or more species selected from AVEFA, ECHCG, LOLMU, PANMI and POAAN, preferably against one or more species selected from ECHCG, LOLMU and POAAN, or from AVEFA, LOLMU and POAAN.

In another embodiment, there is the use of a compound of formula I, wherein R² is isopropyl and R³ is 2-methyl-4-fluorophenyl against one or more species selected from AVEFA, ECHCG, LOLMU, PANMI and POAAN, preferably against one or more species selected from ECHCG, LOLMU, PANMI and POAAN, more preferably against ECHCG, LOLMU and PANMI and.

In another embodiment, there is the use of a compound of formula I, wherein R² is isopropyl and R³ is 2,4,5-trifluorophenyl against one or more species selected from AVEFA, ECHCG, LOLMU, PANMI and POAAN, preferably against one or more species selected from AVEFA, ECHCG, LOLMU and PANMI, more preferably against AVEFA, ECHCG and PANMI.

In another embodiment, there is the use of a compound of formula I, wherein R² is isopropyl and R³ is 3-bromo-4-fluorophenyl against one or more species selected from AVEFA, ECHCG, LOLMU, PANMI and POAAN, preferably against one or more species selected from AVEFA, ECHCG, LOLMU and POAAN, or from ECHCG, LOLMU, PANMI and POAAN.

In some embodiments, the compound of formula I is applied pre-emergence at no more than 250 g/ha, preferably at no more than 125 g/ha or at no more than 63 g/ha (i.e. about 62.5 g/ha).

In one embodiment, the compound according to the first aspect of the present invention or the composition according to the second aspect are used in combination with one or more additional herbicides and/or plant growth regulators as listed above, for example, on the above-mentioned crops and/or against the above-mentioned species. The compound according to the first aspect of the present invention or the composition according to the second aspect and the one or more additional herbicides and/or plant growth regulators may either be used separately or may be formulated into an agrochemical composition.

As the compounds of the present invention are useful as herbicides, the present invention further provides a method for controlling unwanted plants comprising applying to said plants or a locus comprising them, an effective amount of the compound of formula (I) or a herbicidal composition containing said compound.

In another exemplary embodiment, a plant propagation material, such as a seed, comprises or is treated with or adhered to a compound according to the first aspect of the present invention or the composition according to the second aspect of the present invention.

### Preparation of compounds of formula I

In a fourth aspect, the present invention further provides methods for producing the compound according to the first aspect of the invention, i.e., compounds of formula I.

In a particular embodiment, the method for producing a compound of formula I comprises a step which is selected from the following (i) to (iv):
(i) 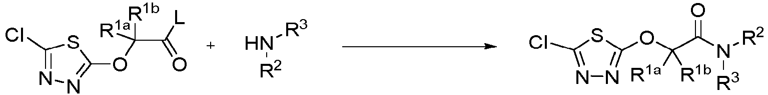
(ii) 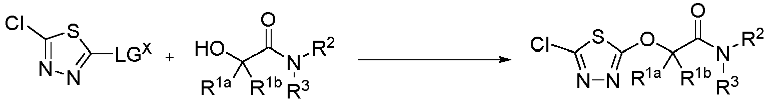
(iii) 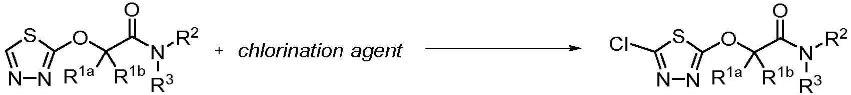
(iv) 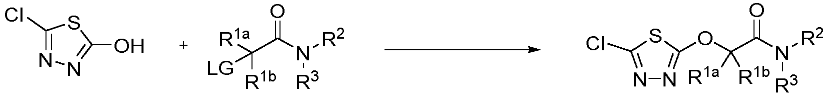
wherein L, LG and LG^{X} are each independently leaving groups such as the ones described below, and the remaining groups are as defined for the compound of formula I above.

Exemplary methods for producing exemplary compounds will be set out in the following. The skilled person will appreciate that these methods for the manufacture of the compounds of the present invention, as well as the methods for producing intermediates used for preparing the compounds of the present invention may be adapted depending on the compound to be synthesized.

For example, the skilled person will be immediately familiar with standard textbooks such as "Comprehensive Organic Transformations - A Guide to Functional Group Transformations", RC Larock, Wiley-VCH (1999 or later editions); "March's Advanced Organic Chemistry - Reactions, Mechanisms and Structure", MB Smith, J. March, Wiley, (5th edition or later editions); "Advanced Organic Chemistry, Part B, Reactions and Synthesis", FA Carey, RJ Sundberg, Kluwer Academic/Plenum Publications, (2001 or later editions); "Organic Synthesis - The Disconnection Approach", S Warren (Wiley), (1982 or later editions); "Designing Organic Syntheses" S Warren (Wiley) (1983 or later editions); "Heterocyclic Chemistry", J. Joule (Wiley 2010 edition or later editions); "Guidebook To Organic Synthesis" RK Mackie and DM Smith (Longman) (1982 or later editions), etc., and the references therein as a guide.

The skilled person is familiar with a range of strategies for synthesising organic and particularly heterocyclic molecules and these represent common general knowledge as set out in textbooks such as Warren "Organic Synthesis: The Disconnection Approach"; Mackie and Smith "Guidebook to Organic Chemistry"; and Clayden, Greeves, Warren and Wothers "Organic Chemistry".

The skilled person will exercise his/her judgement and skill as to the most efficient sequence of reactions for the synthesis of a given target compound and will employ protecting groups as necessary. This will depend inter alia on factors such as the nature of other functional groups present in a particular substrate. Clearly, the type of chemistry involved will influence the choice of reagent that is used in the said synthetic steps, the need, and type, of protecting groups that are employed, and the sequence for accomplishing the protection/deprotection steps. These and other reaction parameters will be evident to the skilled person by reference to standard textbooks and to the examples provided herein.

Sensitive functional groups may need to be protected and deprotected during synthesis of a compound of the invention. This may be achieved by conventional methods using protecting groups (sometimes abbreviated as "PG" hereinafter), for example as described in "Protective Groups in Organic Synthesis" by TW Greene and PGM Wuts, John Wiley & Sons Inc. (1999), and references therein. For example, compounds of formula I can be made, as shown in Scheme 1 below by coupling a carboxylic acid derivative with an amine. In the carboxylic acid derivative shown below, L is a suitable leaving group which can include: OH, halogen, Oalkyl, Oaryl, OCOalkyl or OCOaryl. A suitable amide forming procedure involves treatment of the carboxylic acid derivative with a secondary amine at 0 - 120 °C in a suitable solvent. The presence of a base, or, when L = OH, a coupling agent, may also be necessary for the reaction to occur. Suitable bases for the reaction include 4-(*N,N*-dimethylamino)pyridine, pyridine, triethylamine or *N,N-*diisopropylethylamine. Suitable coupling agents, when L = OH include: carbodiimides, for example 1,3-dicyclohexylcarbodiimide or 1-(3-dimethylaminopropyl-3-ethylcarbodiimide hydrochloride; phosphonium reagents, for example (benzotriazol-1-yloxy)tris(dimethylamino)phosphonium hexafluorophosphate or benzotriazol-1-yloxytripyrrolidinophosphonium hexafluorophosphate; and uronium reagents, for example *O-*(benzotriazol-1-yl)-*N,N,N',N*'-tetramethyluronium tetrafluoroborate. Suitable solvents for the reaction include *N,N-*dimethylformamide, dimethyl sulfoxide or acetonitrile.

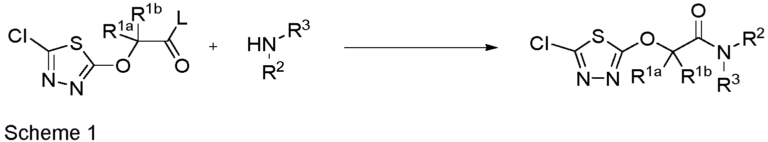

A preferred embodiment of this method is shown in the below Scheme 2:

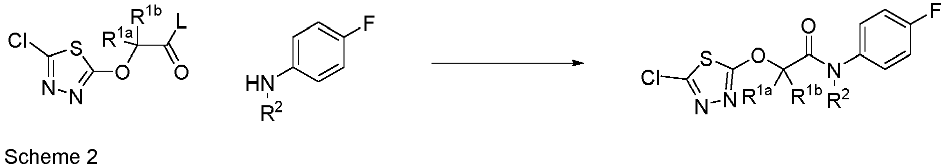

Secondary amines can be made, for example, as shown in Scheme 3 below by reductive amination of an amine derivative with a ketone or aldehyde derivative. A suitable secondary amine forming procedure involves treatment of an amine compound with a ketone or aldehyde compound in the presence of a reducing agent, for example, sodium triacetoxyborohydride, sodium cyanoborohydride or hydrogen and a metal catalyst, for example, palladium on carbon (10 wt.%) at 0 - 120 °C in a suitable solvent. Suitable solvents for the reaction include methanol or toluene.

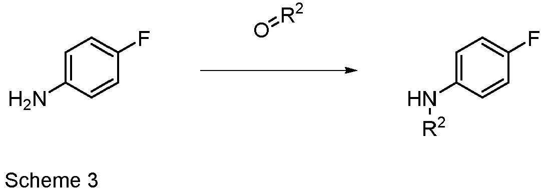

Alternatively, secondary amines can be made, for example, as shown in Scheme 4 below by substitution of an alkyl derivative, where LG^{X} is a suitable leaving group, for example, C!, Br, !, OTf, with an amine derivative. A suitable secondary amine forming procedure involves treatment of an amine compound with an alkyl derivative at 0 -120 °C in a suitable solvent. The presence of a base may also be necessary for the reaction to occur. Suitable bases for the reaction include sodium hydroxide, potassium *tert-*butoxide or potassium carbonate. Suitable solvents for the reaction include *N,N*-dimethylformamide, dimethyl sulfoxide or acetonitrile.

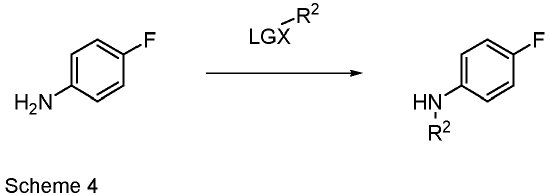

Secondary amines can be made, for example, as shown in Scheme 5 below by Smiles rearrangement of an amide. A suitable secondary amine forming procedure involves treatment of an amide with a base, for example, potassium hydroxide, or potassium tert-butoxide at 0 - 120 °C in a suitable solvent. Suitable solvents for the reaction include *N,N-*dimethylformamide, *N*-methyl-2-pyrrolidone or toluene.

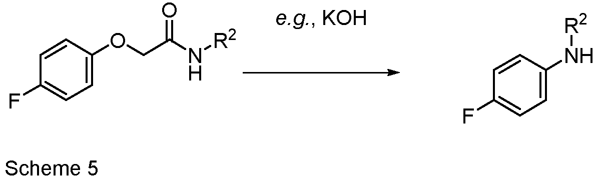

Secondary amides can be made, for example, as shown in Scheme 6 below by substitution of an amide derivative, where LG is a suitable leaving group which can include: OH, halogen, Oalkyl, Oaryl, OCOalkyl, SO₂alkyl, SO₂aryl or OCOaryl, with a phenol derivative. A suitable ether forming procedure involves treatment of a phenol derivative with an amide derivative at 0 - 120 °C in a suitable solvent. The presence of a base, or, when LG = OH, a coupling agent, may also be necessary for the reaction to occur. Suitable bases for the reaction include sodium hydroxide or potassium carbonate among others. Suitable coupling agents when LG = OH include: azodicarboxylates, for example diethyl azodicarboxylate or diisopropyl azodicarboxylate, alongside a trisubstituted phosphine reagent, for example triphenylphosphine; and phosphorane ylides, for example, (cyanomethylene)trimethylphosphorane or (cyanomethylene)tributylphosphorane. Suitable solvents for the reaction include acetone, water, toluene or isopropyl alcohol.

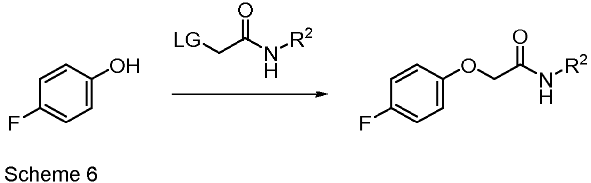

Secondary amines can be made, for example, as shown in Scheme 7 below by a metal-catalysed cross-coupling reaction of an amine derivative and an aryl-halide derivative, where X is a halogen, or pseudo-halogen including: C!, Br, !, OTs. A suitable metal-catalysed cross-coupling procedure involves treatment of an aryl-(pseudo)halide compound and an amine with a suitable catalyst, for example, copper (I) iodide, copper (I) oxide or copper (I) bromide and a suitable base, for example, potassium carbonate, potassium tert-butoxide, tripotassium phosphate or caesium carbonate at 0 - 120 °C in a suitable solvent. Suitable solvents for the reaction include *N,N-*dimethylformamide, dimethyl sulfoxide or acetonitrile. The presence of a ligand may also be required. Suitable ligands include: 2-(diphenylphosphinyl)benzaldehyde oxime or *N¹,N²*-bis(2,4,6-trimethoxyphenyl)ethanediamide.

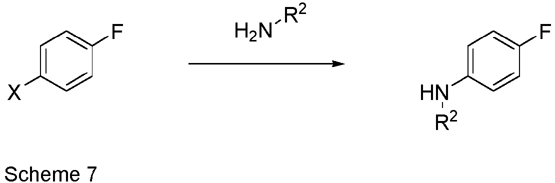

Alkoxy-thiadiazole derivatives can be made, for example, as shown in Scheme 8 below by nucleophilic aromatic substitution of a thiadiazole derivative where L is a suitable leaving group which can include: OH, halogen, Oalkyl, Oaryl, OCOalkyl, SO₂alkyl, SO₂aryl or OCOaryl, with an alcohol derivative, where LG is a suitable leaving group which can include: OH, halogen, Oalkyl, Oaryl, OCOalkyl, SO₂alkyl, SO₂aryl or OCOaryl.. A suitable ether forming procedure involves treatment of an alkoxy-thiadiazole with an alcohol in the presence of a base, for example, sodium hydroxide, potassium carbonate or potassium tert-butoxide at 0 - 120 °C in a suitable solvent. Suitable solvents for the reaction include acetone, water, acetonitrile or toluene.

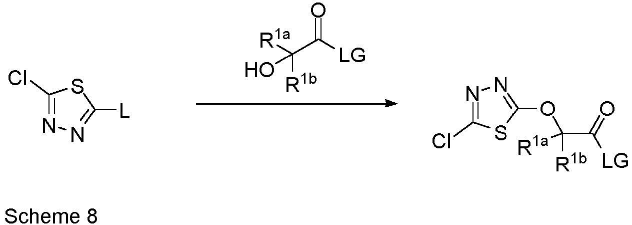

Sulfone derivatives, where R^{X} is defined as C₁₋ₙ alkyl, can be made, for example, as shown in Scheme 9 below by oxidation of a thioether derivative, where R^{X} is defined as C₁₋ₙ alkyl. A suitable oxidation procedure involves treatment of a thioether derivative with a suitable oxidising agent, for example, hydrogen peroxide, *meta*-chloroperbenzoic acid or potassium persulfate at 0 - 120 °C in a suitable solvent. Suitable solvents for the reaction include methanol, water or toluene.

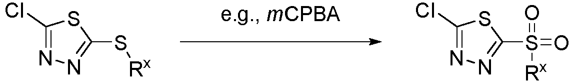

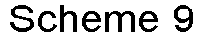

Chlorinated thiadiazoles, where R^{X} is defined as C₁₋ₙ alkyl, can be made, for example, as shown in Scheme 10 below by Sandmeyer reaction of an amine derivative, where R^{X} is defined as C₁₋ₙ alkyl. A suitable Sandmeyer reaction procedure involves treatment of an amine derivative with a suitable diazonium forming reagent, for example, sodium nitrite or *tert*-butyl nitrite and a suitable halogen source, for example, hydrochloric acid or copper (I) chloride at 0 - 120 °C in a suitable solvent. Suitable solvents for the reaction include acetonitrile or water.

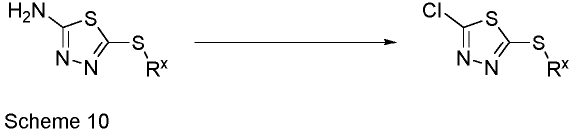

Thioether derivatives, where R^{X} is defined as C₁₋ₙ alkyl can be made, for example, as shown in Scheme 11 below by alkylation of a thiol derivative, with an alkylating agent of, where R^{X} is defined as C₁₋ₙ alkyl and where LG^{X} is a suitable leaving group which can include: halogen, Oalkyl, Oaryl, OCOalkyl, SO₂alkyl, SO₂aryl or OCOaryl. A suitable alkylation procedure involves treatment of a thiol derivative with an alkylating agent in the presence of a base, for example, potassium hydroxide or potassium carbonate at 0 - 120 °C in a suitable solvent. Suitable solvents for the reaction include *N,N-*dimethylformamide, water or isopropyl alcohol.

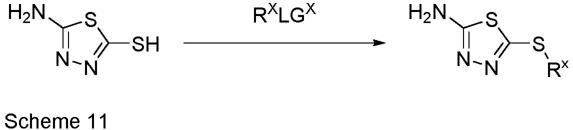

Alternatively, ether derivatives can be made, for example, as shown in Scheme 12: 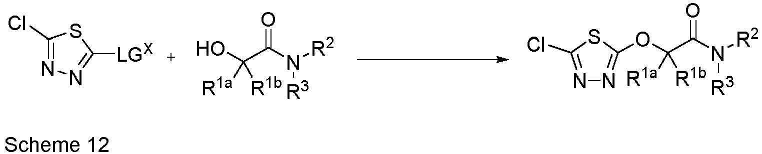 A preferred embodiment of this method is shown in the below Scheme 13 below by nucleophilic aromatic substitution of a thiadiazole derivative, where LG^{X} is a suitable leaving group which can include: OH, halogen, Oalkyl, Oaryl, OCOalkyl, SO₂alkyl, SO₂aryl or OCOaryl, with an alcohol derivative. A suitable ether forming procedure involves treatment of a thiadiazole derivative with an alcohol derivative at -50 - 120 °C in a suitable solvent. The presence of a base, or, when L = OH, a coupling agent, may also be necessary for the reaction to occur. Suitable bases for the reaction include sodium hydroxide or potassium carbonate among others. Suitable coupling agents when L = OH include: azodicarboxylates, for example diethyl azodicarboxylate or diisopropyl azodicarboxylate, alongside a trisubstituted phosphine reagent, for example triphenylphosphine; and phosphorane ylides, for example, (cyanomethylene)trimethylphosphorane or (cyanomethylene)tributylphosphorane. Suitable solvents for the reaction include acetone, water, toluene or isopropyl alcohol.

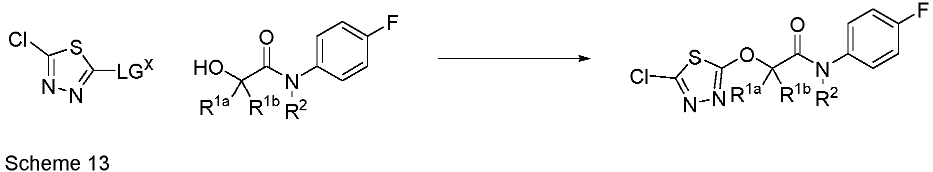

Alcohol derivatives can be made, for example, as shown in Scheme 14 below by hydrolysis of an ester derivatives where R^{X} is defined as C₁₋ₙ alkyl. A suitable hydrolysis procedure involves treatment of an ester derivative with a base, for example, sodium hydroxide or potassium carbonate at 0 - 120 °C in a suitable solvent. Suitable solvents for the reaction include methanol or water.

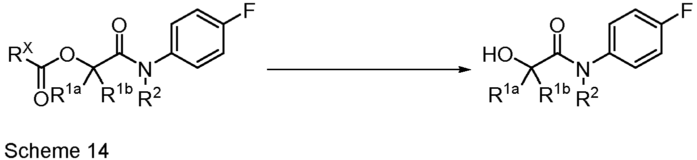

Ester derivatives can be made, for example, as shown in Scheme 15 below by substitution of amide derivative, where LG^{X} is a suitable leaving group which can include: OH, halogen, Oalkyl, Oaryl, OCOalkyl, SO₂alkyl, SO₂aryl or OCOaryl, with a carboxylic acid derivative, where R^{X} is defined as C₁₋ₙ alkyl. A suitable carbon-oxygen bond forming procedure involves treatment of an amide derivative with a carboxylic acid derivative at 0 - 120 °C in a suitable solvent. The presence of a base or, when L = OH, a coupling agent, may also be necessary for the reaction to occur. Suitable bases for the reaction include sodium hydroxide, potassium tert-butoxide or potassium carbonate. Suitable coupling agents when L = OH include: azodicarboxylates, for example diethyl azodicarboxylate or diisopropyl azodicarboxylate, alongside a trisubstituted phosphine reagent, for example triphenylphosphine; and phosphorane ylides, for example, (cyanomethylene)trimethylphosphorane or (cyanomethylene)tributylphosphorane. Suitable solvents for the reaction include *N,N-*dimethylformamide, toluene or acetonitrile.

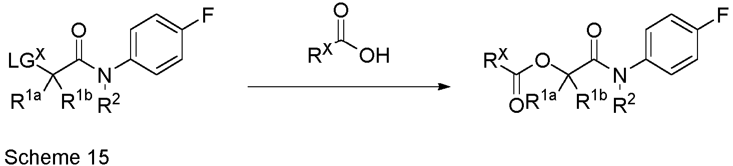

Amides can be made, for example, as shown in Scheme 16 below by coupling a carboxylic acid derivative with an aniline. In the carboxylic acid derivative, LG and LG^{X} are a suitable leaving group which can include: OH, halogen, Oalkyl, Oaryl, OCOalkyl or OCOaryl. A suitable amide forming procedure involves treatment of a carboxylic acid derivative with an aniline at 0 - 120 °C in a suitable solvent. The presence of a base, or, when L = OH, a coupling agent, may also be necessary for the reaction to occur. Suitable bases for the reaction include 4-(*N,N*-dimethylamino)pyridine, pyridine, triethylamine or *N,N*-diisopropylethylamine. Suitable coupling agents when L = OH include: carbodiimides, for example 1,3-dicyclohexylcarbodiimide or 1-(3-dimethylaminopropyl)-3-ethylcarbodiimide hydrochloride; phosphonium reagents, for example (benzotriazol-1-yloxy)tris(dimethylamino)phosphonium hexafluorophosphate or benzotriazol-1-yloxytripyrrolidinophosphonium hexafluorophosphate; and uronium reagents, for example *O-*(benzotriazol-1-yl)-*N,N,N',N'*-tetramethyluronium tetrafluoroborate. Suitable solvents for the reaction include *N,N-*dimethylformamide, dimethyl sulfoxide or dichloromethane.

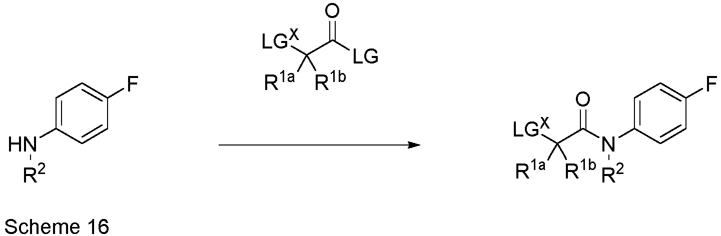

Alternatively, chlorinated thioethers, where R^{x} is defined as C₁₋ₙ alkyl, can be made, for example, as shown in Scheme 17 below by chlorination of a thiadiazole derivative. A suitable chlorination procedure involves treatment of a thiadiazole derivative with a suitable chlorinating agent, for example, chlorine, *N*-chlorosuccinimide, thionyl chloride or 1 ,3-dichloro-5,5-dimethylhydantoin at 0 - 120 °C in a suitable solvent. Suitable solvents for the reaction include *N,N-*dimethylformamide, acetic acid or *N,N-*dimethylacetamide.

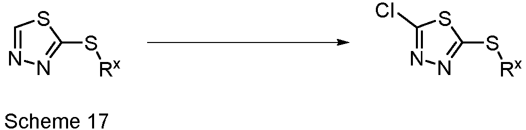

Thioether derivatives, where R^{X} are defined as C₁₋ₙ alkyl can be made, for example, as shown in Scheme 18 below by alkylation of a thiol derivative, with an alkylating agent, where R^{X} is defined as C₁₋ₙ alkyl, and where LG^{X} is a suitable leaving group which can include: halogen, Oalkyl, Oaryl, OCOalkyl, SO₂alkyl, SO₂aryl or OCOaryl. A suitable alkylation procedure involves treatment of a thiol derivative with an alkylating agent in the presence of a base, for example, potassium hydroxide or potassium carbonate at 0 - 120 °C in a suitable solvent. Suitable solvents for the reaction include *N,N-*dimethylformamide, water or isopropyl alcohol.

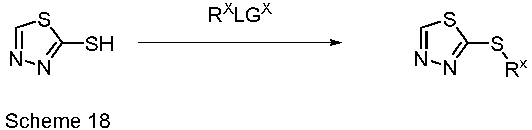

Thio derivatives can be made, for example, as shown in Scheme 19 below by cyclisation of a hydrazine derivative, with carbon disulfide. A suitable cyclisation procedure involves treatment of a hydrazine derivative with carbon disulfide in the presence of a base, for example, potassium hydroxide or potassium carbonate at 0 - 120 °C in a suitable solvent followed by treatment with a suitable acid, for example sulfuric acid or hydrochloric acid, at 0 - 120 °C in a suitable solvent. Suitable solvents for the reaction include ethanol, water or isopropyl alcohol.

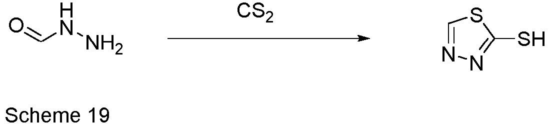

Sulfone derivatives can be made, for example, as shown in Scheme 20 below by oxidation of a thioether derivative, where R^{X} is defined as C₁₋ₙ alkyl. A suitable oxidation procedure involves treatment of a thioether derivative with a suitable oxidising agent, for example, hydrogen peroxide, *meta-*chloroperbenzoic acid (mCBPA) or potassium persulfate at 0 - 120 °C in a suitable solvent. Suitable solvents for the reaction include methanol, water or toluene.

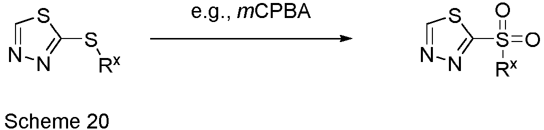

Amide derivatives can be made, for example, as shown in Scheme 21 below by nucleophilic aromatic substitution of thiadiazole derivative, where LG^{X} is a suitable leaving group which can include: OH, halogen, Oalkyl, Oaryl, OCOalkyl, SO₂alkyl, SO₂aryl or OCOaryl, with an alcohol derivative, where R^{1a}, R^{1b} and R² are defined as above. A suitable ether forming procedure involves treatment of a thiadiazole derivative with an alcohol at 0 - 120 °C in a suitable solvent. The presence of a base, or, when LG^{x} = OH, a coupling agent, may also be necessary for the reaction to occur. Suitable bases for the reaction include sodium hydroxide or potassium carbonate among others. Suitable coupling agents when LG^{x} = OH include: azodicarboxylates, for example diethyl azodicarboxylate or diisopropyl azodicarboxylate, alongside a trisubstituted phosphine reagent, for example triphenylphosphine; and phosphorane ylides, for example, (cyanomethylene)trimethylphosphorane or (cyanomethylene)tributylphosphorane. Suitable solvents for the reaction include acetone, water, toluene or isopropyl alcohol.

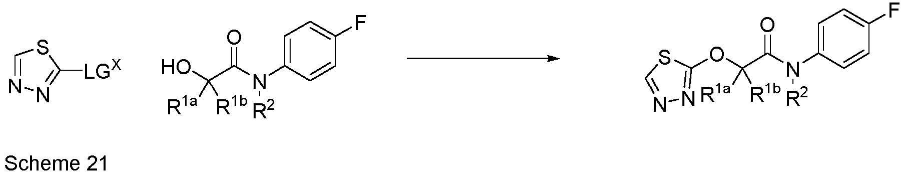

Chlorinated thiadiazoles can be made, for example, as shown in Scheme 22 below, by chlorination of a thiadiazole derivative:

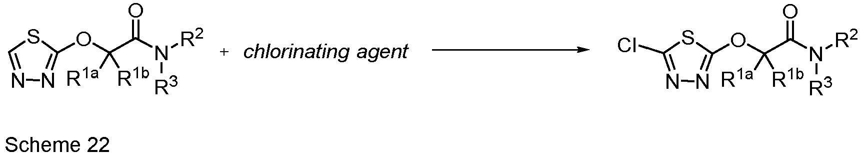

A suitable chlorination procedure involves treatment of a thiadiazole derivative with a suitable chlorinating agent, for example, chlorine, *N*-chlorosuccinimide, thionyl chloride or 1,3-dichloro-5,5-dimethylhydantoin at 0 - 120 °C in a suitable solvent. Suitable solvents for the reaction include *N,N-*dimethylformamide, acetic acid or *N,N*-dimethylacetamide. A preferred embodiment of this method is shown in Scheme 23 below.

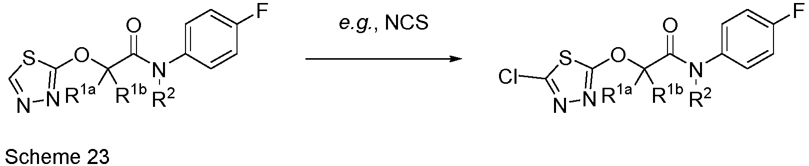

Ester derivatives can be made, for example, as shown in Scheme 24 below by nucleophilic aromatic substitution of a thiadiazole derivative, where L is a suitable leaving group which can include: OH, halogen, Oalkyl, Oaryl, OCOalkyl, SO₂alkyl, SO₂aryl or OCOaryl, with an alcohol derivative, where R^{'} is defined as C₁₋ₙ alkyl. A suitable ether forming procedure involves treatment of a thiadiazole derivative with an alcohol in the presence of a base, for example, sodium hydroxide, potassium carbonate or potassium *tert*-butoxide at 0 - 120 °C in a suitable solvent. Suitable solvents for the reaction include acetone, water, acetonitrile or toluene.

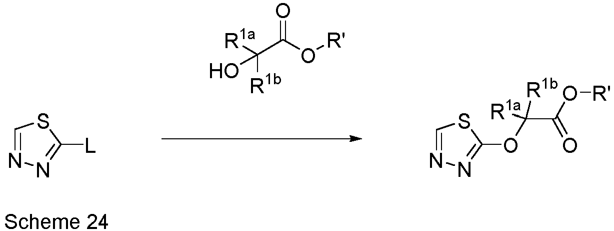

Chlorinated thiadiazoles can be made, for example, as shown in Scheme 25 below by chlorination of a thiadiazole derivative, where R^{'} is defined as C₁₋ₙ alkyl. A suitable chlorination procedure involves treatment of a thiadiazole derivative with a suitable chlorinating agent, for example, chlorine, *N-*chlorosuccinimide (NCS), thionyl chloride or 1,3-dichloro-5,5-dimethylhydantoin at 0 - 120 °C in a suitable solvent. Suitable solvents for the reaction include *N,N*-dimethylformamide, acetic acid or *N,N-*dimethylacetamide.

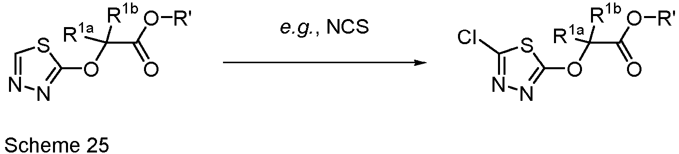

Carboxylic acids can be made, for example, as shown in Scheme 26 below by hydrolysis of ester derivatives, where R^{'} is defined as C₁₋ₙ alkyl. A suitable hydrolysis procedure involves treatment of an ester derivative with a base, for example, sodium hydroxide or potassium carbonate at 0 - 120 °C in a suitable solvent. Suitable solvents for the reaction include methanol or water.

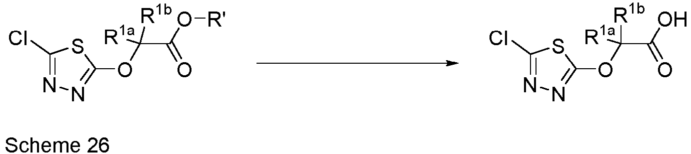

Aryl chlorides can be made, for example, as shown in Scheme 27 below by chlorination of a thiadiazole derivative, where R^{'} is defined as H. A suitable chlorination procedure involves treatment of a thiadiazole derivative with a suitable chlorinating agent, for example, chlorine, oxalyl chloride or thionyl chloride at 0-120 °C in a suitable solvent. Suitable solvents for the reaction include *N,N*-dimethylformamide, acetic acid or *N,N*-dimethylacetamide.

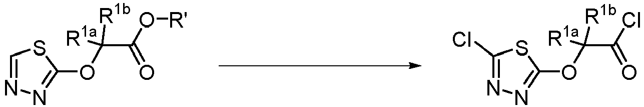

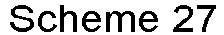

Chlorinated thiadiazoles can be made, for example, as shown in Scheme 28 below by chlorination of a thiadiazole derivative, where R^{x} is defined as C₁₋₃ alkyl, chloro or OH. A suitable chlorination procedure involves treatment of a thiadiazole derivative with a suitable chlorinating agent, for example, chlorine, oxalyl chloride or thionyl chloride at 0 - 120 °C in a suitable solvent. Suitable solvents for the reaction include *N,N*-dimethylformamide, acetic acid or *N,N*-dimethylacetamide.

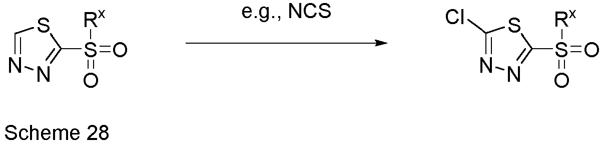

Chlorinated thiadiazoles can be made, for example, as shown in Scheme 29 below by chlorination of a thiadiazole derivative. A suitable chlorination procedure involves treatment of a thiadiazole compound with a suitable chlorinating agent, for example, chlorine, oxalyl chloride or thionyl chloride at 0 - 120 °C in a suitable solvent. Suitable solvents for the reaction include *N,N*-dimethylformamide or toluene.

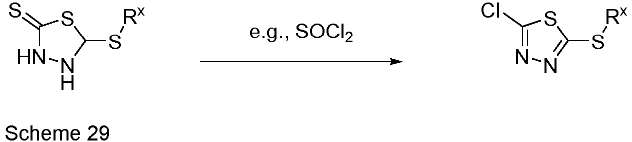

Thioethers can be made, for example, as shown in Scheme 30 below by alkylation of a thiadiazole derivative, with an alkylating agent, where R^{X} is C₁₋₃ alkyl and where LG^{X} is a suitable leaving group which can include: halogen, Oalkyl, Oaryl, OCOalkyl, SO₂alkyl, SO₂aryl or OCOaryl. A suitable alkylation procedure involves treatment of a thiadiazole derivative with an alkylating agent in the presence of a base, for example, potassium hydroxide or potassium carbonate at 0 - 120 °C in a suitable solvent. Suitable solvents for the reaction include *N,N*-dimethylformamide, water or isopropyl alcohol.

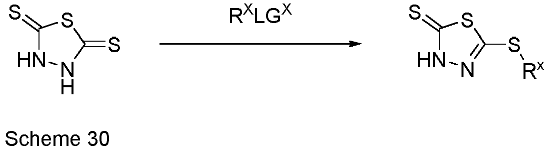

Dichlorothiadiazoles can be made, for example, as shown in Scheme 31 below by chlorination of a thiadiazole derivative. A suitable chlorination procedure involves treatment of a thiadiazole derivative with a suitable chlorinating agent, for example, chlorine, oxalyl chloride, thionyl chloride, *N-*chlorosuccinimide, DCDMH 1,3-dichloro-5,5-dimethylhydantoin, trichloroisocyanuric acid, or thionyl chloride/hydrogen peroxide at 0 - 120 °C in a suitable solvent. Suitable solvents for the reaction include *N,N*-dimethylformamide, toluene or acetonitrile.

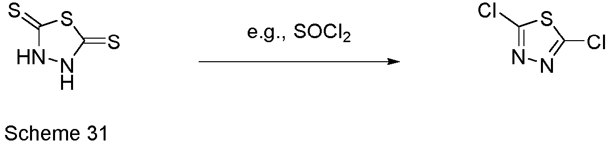

Thiols can be made, for example, as shown in Scheme 32 below by cyclisation of carbon disulfide, with hydrazine hydrate. A suitable cyclisation procedure involves treatment of carbon disulfide with hydrazine hydrate or hydrazine in the presence of a base, for example, sodium hydroxide or potassium hydroxide at 0 - 120 °C in a suitable solvent followed by treatment with a suitable acid, for example sulfuric acid or hydrochloric acid at 0 - 120 °C in a suitable solvent. Suitable solvents for the reaction include ethanol, water or pyridine.

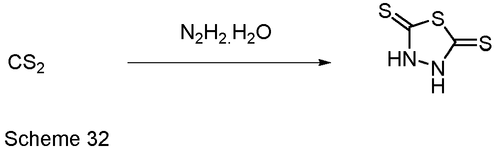

Ether derivatives can be made, for example, as shown in Scheme 33 below by substitution of an amide derivative:

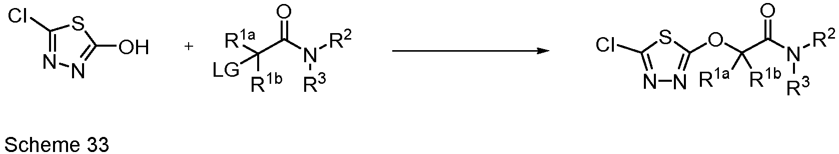

In the amide, LG is a suitable leaving group which can include: OH, halogen, Oalkyl, Oaryl, OCOalkyl, SO₂alkyl, SO₂aryl or OCOaryl. A suitable ether forming procedure involves treatment of an amide derivative with an alcohol at 0 - 120 °C in a suitable solvent. The presence of a base, or, when LG = OH, a coupling agent, may also be necessary for the reaction to occur. Suitable bases for the reaction include sodium hydroxide or potassium carbonate among others. Suitable coupling agents when L = OH include: azodicarboxylates, for example diethyl azodicarboxylate or diisopropyl azodicarboxylate, alongside a trisubstituted phosphine reagent, for example triphenylphosphine; and phosphorane ylides, for example, (cyanomethylene)trimethylphosphorane or (cyanomethylene)tributylphosphorane. Suitable solvents for the reaction include acetone, water, toluene or isopropyl alcohol.

A preferred embodiment of this method is shown in Scheme 34 below:

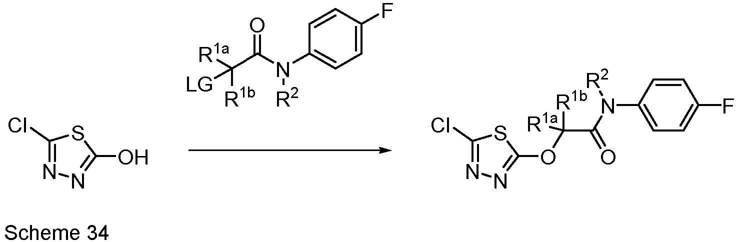

Chlorinated thiadiazole derivates can be made, for example, as shown in Scheme 35 below by chlorination of a thiadiazole derivative. A suitable chlorination procedure involves treatment of a compound thiadiazole derivative with a suitable chlorinating agent, for example, chlorine or *N-*chlorosuccinimide at 0 - 120 °C in a suitable solvent. Suitable solvents for the reaction include *N,N-*dimethylformamide, acetic acid or *N,N*-dimethylacetamide.

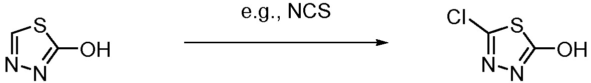

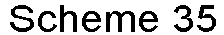

Thioethers can be made, for example, as shown in Scheme 36 below by alkylation of a thiadiazole derivative, with an alkylating agent of, where R^{X} are defined as C₁₋₃ alkyl and where LG^{X} is a suitable leaving group which can include: halogen, Oalkyl, Oaryl, OCOalkyl, SO₂alkyl, SO₂aryl or OCOaryl. A suitable alkylation procedure involves treatment of a thiadiazole derivative with an alkylating agent in the presence of a base, for example, potassium hydroxide or potassium carbonate at 0 - 120 °C in a suitable solvent. Suitable solvents for the reaction include *N,N*-dimethylformamide, water or isopropyl alcohol.

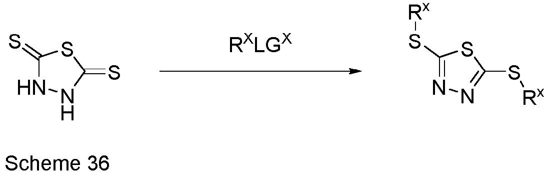

Thiadiazole sulfone derivatives can be made, for example, as shown in Scheme 37 below by oxidation of thioether derivatives, where R^{X} are defined as H or C₁₋₅ alkyl. A suitable oxidation procedure involves treatment of a thioether derivative with a suitable oxidising agent, for example, hydrogen peroxide, *meta-*chloroperbenzoic acid (*m*CBPA) or potassium persulfate at 0 - 120 °C in a suitable solvent. Suitable solvents for the reaction include methanol, water or toluene.

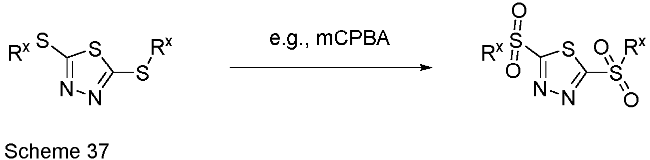

Dichlorothiadiazole compounds can be made, for example, as shown in Scheme 38 below by chlorination of a thiadiazole derivative where R^{X} are defined as OH (sulfonic acid) or Cl (sulfonyl chloride). A suitable chlorination procedure involves treatment of a thiadiazole derivative with a suitable chlorinating agent, for example, sulfuryl chloride, thionyl chloride or HCl at 0 - 120 °C in a suitable solvent. Suitable solvents for the reaction include *N,N*-dimethylformamide, acetic acid or toluene.

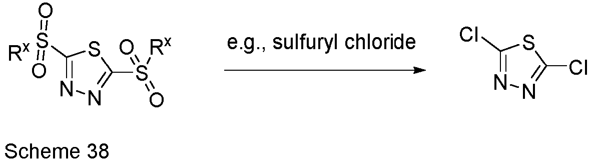

Sulfonic acids or sulfonyl chlorides can be made, for example, as shown in Scheme 39 below by oxidation of thiol derivatives where R^{X} are defined as OH (sulfonic acid) or Cl (sulfonyl chloride). A suitable oxidation procedure involves treatment of a thiol derivative with a suitable oxidising agent, for example, N-chlorosuccinimide (NCS), 1,3-dichloro-5,5-dimethylhydantoin, permanganic acid or trichloroisocyanuric acid at 0 - 120 °C in a suitable solvent. Suitable solvents for the reaction include dichloromethane, water or toluene.

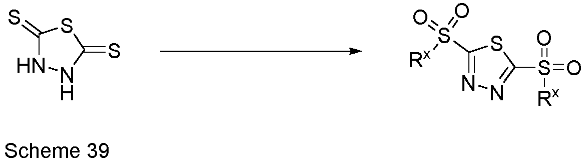

Ether compounds can be made, for example, as shown in Scheme 40 below by cyclisation of a hydrazinecarbothioate derivative with a suitable reagent. A suitable cyclisation procedure involves treatment of a hydrazinecarbothioate compound with phosgene or oxalyl chloride at 0 - 120 °C in a suitable solvent. Suitable solvents for the reaction include dichloromethane, toluene or *N,N-*dimethylformamide.

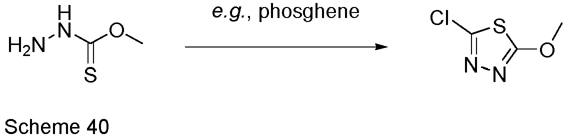

Chlorothiadiazolone compounds can be made, for example, as shown in Scheme 41 below by demethylation of an ether derivative. A suitable demethylation procedure involves treatment of an ether compound with a suitable reagent, for example, boron tribromide, hydrogen bromide, aluminium bromide at 0 - 120 °C in a suitable solvent. Suitable solvents for the reaction include dichloromethane or water.

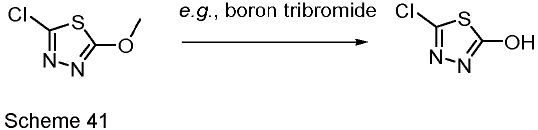

Thiadiazolone compounds can be made, for example, as shown in Scheme 42 below by demethylation of an alkylated thiadiazolone derivative A suitable demethylation procedure involves treatment of an *O-*alkylated thiadiazolone compound with a suitable reagent, for example, boron tribromide, hydrogen chloride, hydrogen iodide at 0 - 120 °C in a suitable solvent. Suitable solvents for the reaction include 1 ,4-dioxane or water.

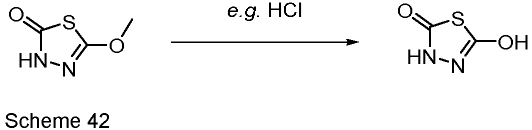

Dichlorothiadiazole compounds can be made, for example, as shown in Scheme 43 below by chlorination of a thiadiazolone derivative. A suitable chlorination procedure involves treatment of a thiadiazolone compound with a suitable chlorinating agent, for example, phosphorus oxychloride, oxalyl chloride or thionyl chloride at 0 - 120 °C in a suitable solvent. Suitable solvents for the reaction include *N,N*-dimethylformamide, water or toluene.

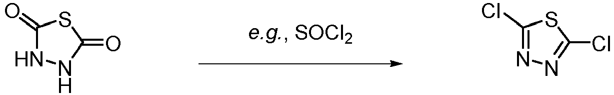

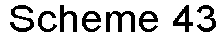

In a further aspect, the present invention also relates to novel compounds which are intermediates in the preparation of compounds according to formula I. Preferred intermediates useful in methods for producing compounds of formula I, preferably useful in one or more of the above methods, include:

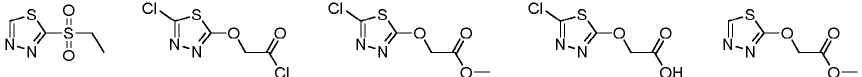

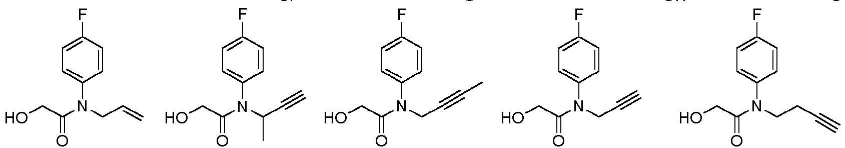

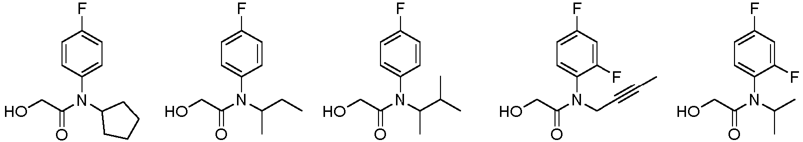

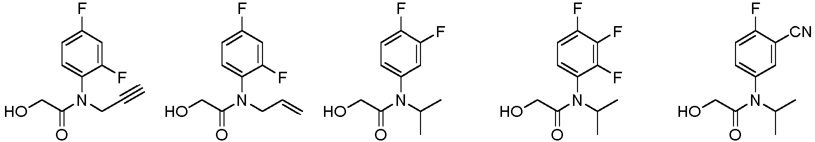

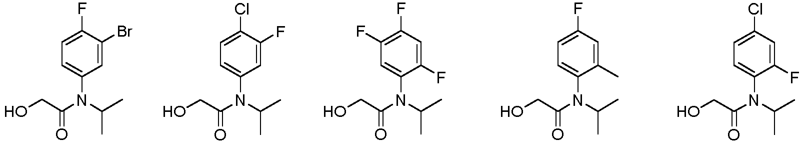

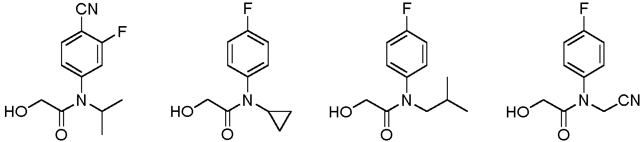

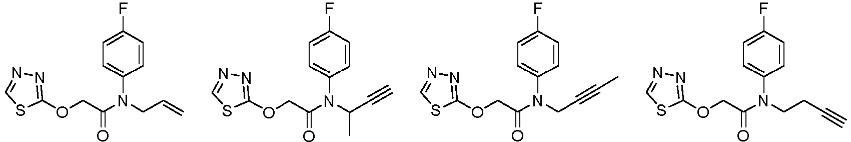

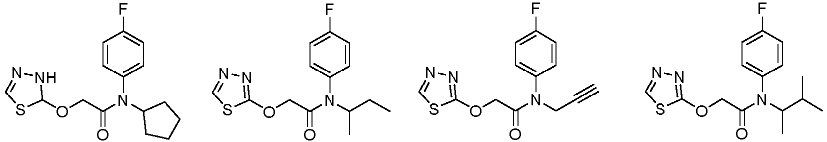

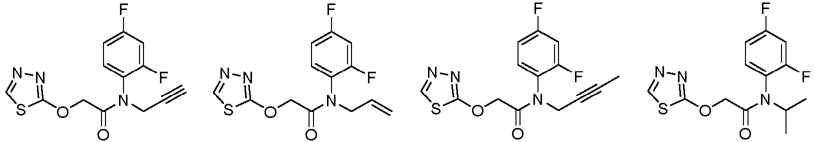

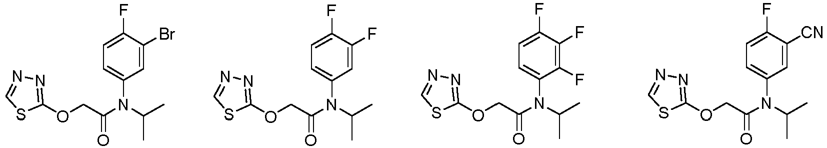

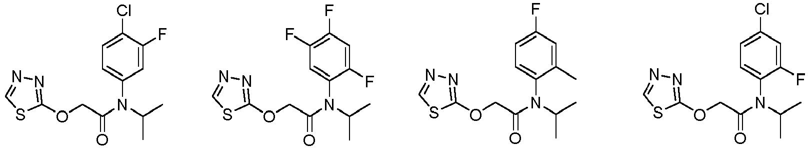

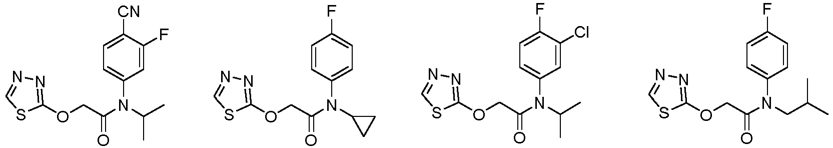

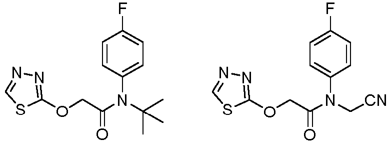

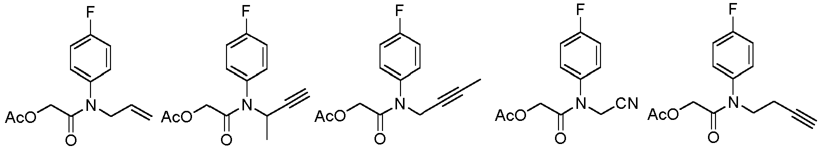

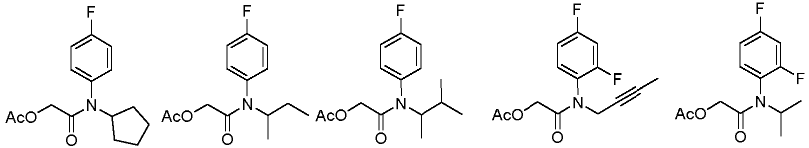

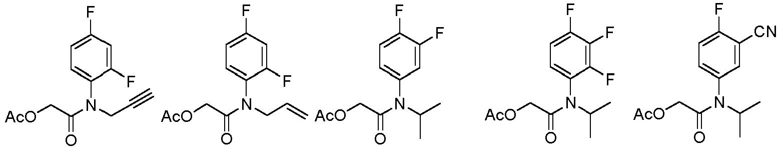

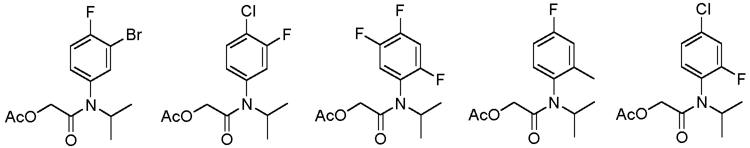

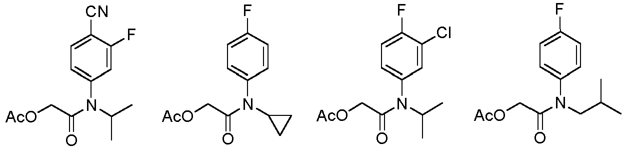

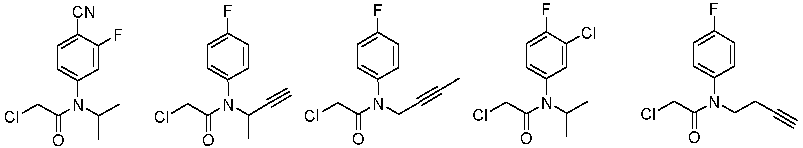

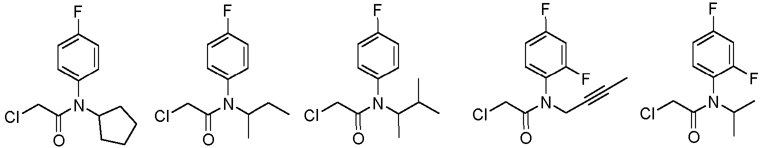

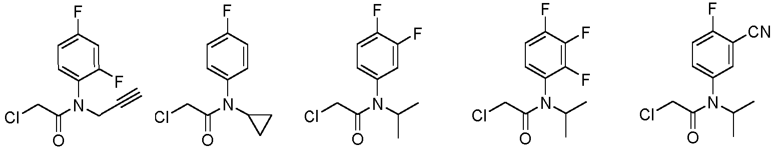

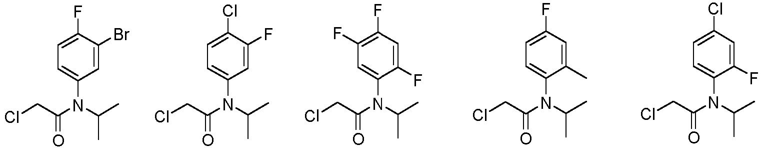

In one embodiment, a method for producing a compound according to the first aspect of the invention involves using one or more intermediates as depicted above in at least one step.

In another aspect of the invention, it is provided the use of any of the above intermediate compounds in the preparation of a compound according to the first aspect of the invention or any other uses and it is further provided a process for preparing a compound according to the first aspect of the invention involving any of the above intermediate compounds.

### EXAMPLES

The present invention will be demonstrated in more detail by way of examples which are not intended to be limiting.

### General Methods

Flash chromatography was carried out using a CombiFlash^{®} NextGen 100 (flow rate 1 to 100 mL/min) with RediSep^{®} cartridges packed with 40 - 63 µm silica particles with a surface area of 500 m²/g. Visualization was carried out with UV light (254 nm) and by staining with either potassium permanganate, phosphomolybdic acid (PMA) or ninhydrin solutions.

### LCMS Methods:

### Method A (5 min basic pH)

Spectra were recorded on a Waters HClass UPLC-QDA UV-MS Mass Spectrometerequipped with a column: Waters XSelect HSS T3 Column, 100 Å, 2.5 µm, 3 mm x 50 mm. Injection volume: 3 µL. Temperature: 37 °C. Mobile phases: Water (A) / Acetonitrile (B) / 49.5% Water, 49.5% Acetonitrile, 1% Formic acid (C)

| Time (min) | %A | %B | %C | Flow rate (mL/min) |
|---|---|---|---|---|
| 0.0 | 90 | 0 | 10 | 1.0 |
| 3.0 | 0 | 90 | 10 | 1.0 |
| 4.0 | 0 | 90 | 10 | 1.0 |
| 4.1 | 90 | 0 | 10 | 1.0 |
| 5.0 | 90 | 0 | 10 | 1.0 |

### Method B (5 min basic pH)

Spectra were recorded on a Mass Spectrometer from Applied Biosystems (API 2000, triple quadrupole mass spectrometer) equipped with electrospray ionisation using an atmospheric pressure ionisation source (Polarity: positive or negative ions, Declustering Potential: 50 V, Ion Source: Turbo spray, Ion Spray Voltage: 5500 V [positive], -4500 V [negative], Source Temperature: 200 °C, Mass range: 200 to 1700 Da and a Prominence HPLC from Shimadzu equipped with a column: Waters XBridge C18, 5.0 µm, 50 x 4.6 mm, UV Wavelengths: 220 and 260 nm. Mobile phases: 10 mM Ammonium Acetate in Water (A) / Acetonitrile (B):

| Time (min) | %A | %B | Flow rate (mL/min) |
|---|---|---|---|
| 0.01 | 90 | 10 | 1.2 |
| 1.50 | 70 | 30 | 1.2 |
| 3.00 | 10 | 90 | 1.2 |
| 4.00 | 10 | 90 | 1.2 |
| 5.00 | 90 | 10 | 1.2 |

### Method C (12 min basic pH):

Spectra were recorded on a Mass Spectrometer from Waters (SQ Detector 2, single quadrupole mass spectrometer) equipped with an electrospray source (Polarity: positive or negative ions, Capillary: 3.50 kV, Cone: 25.00 V, Source Temperature: 150 °C, Desolvation Temperature: 350 °C, Cone Gas Flow: 50 L/Hr, Desolvation Gas Flow: 750 L/Hr, Mass range: 100 to 900 Da) and an Acquity H-Class UPLC from Waters equipped with a column: Waters XBridge C18, 3.5 µm, 50 x 3.0 mm, Temp: 40 °C, DAD Wavelength range: 200 to 400 nm. Mobile phases: 5 mM Ammonium Acetate in Water (A) / 5 mM Ammonium Acetate in Acetonitrile:Water (9:1) (B):

| Time | %A | %B | Flow rate (mL/min) |
|---|---|---|---|
| 0.00 | 98 | 2 | 1.0 |
| 1.00 | 98 | 2 | 1.0 |
| 5.00 | 50 | 50 | 1.0 |
| 8.00 | 2 | 98 | 1.0 |
| 10.00 | 2 | 98 | 1.0 |
| 12.00 | 98 | 2 | 1.0 |

### Method D (7 min acidic pH):

Spectra were recorded on an Alliance Waters e2695 series separation module HPLC System with 2998 PDA Detector with an Acquity QDa equipped with a column: Agilent, Zorbax Eclipse XDB C18, 5 µm, 4.6 mm x 50 mm. Injection volume: 5 µL. Temperature: 35 °C. Mobile phases: Formic acid (0.1%) in water (A) / Acetonitrile:Methanol (9:1) (B)

| Time (min) | %A | %B | Flow rate (mL/min) |
|---|---|---|---|
| 0.00 | 90 | 10 | 1.0 |
| 2.50 | 5 | 95 | 1.0 |
| 5.00 | 5 | 95 | 1.0 |
| 5.10 | 90 | 10 | 1.0 |
| 7.00 | 90 | 10 | 1.0 |

### GCMS Method

### Method E (15 min):

GC-Mass was recorded on an Agilent 7890B series instrument with MSD 5977B. Column: HP-5MS (30 m x 250 µm x 0.25 µm). Carrier Gas: Helium. Inlet Temperature: 250 °C. Split ratio: 30:1. Gas flow: 1.0 mL/min. Ramp Profile: Oven temperature initial from 60 °C held for 2 min, then to 100 °C increasing at the rate of 20 °C and held for 2 min, then to 310 °C increasing at the rate of 40 °C and held for 4 min. Total run time is 15.25 min.

All reagents were obtained from commercial suppliers and used as supplied unless otherwise stated.

All compounds are named using ChemDraw Professional 23.0.1.10.

The abbreviations used in the following are the abbreviations commonly used in this technical field. For example, the following abbreviations are used:
- BrettPhos: 2-(Dicyclohexylphosphino)3,6-dimethoxy-2',4',6'-triisopropyl-1,1'-biphenyl
- dba: dibenzylideneacetone
- DCM: Dichloromethane
- DEAD: Diethyl Azodicarboxylate
- DIPEA: *N*,*N*-Diisopropylethylamine
- DMF: *N*,*N*-Dimethylformamide
- DMSO: Dimethyl Sulfoxide
- equiv.: equivalents
- HPLC: High-performance Liquid Chromatography
- MeOH: Methanol
- NMP: *N*-Methyl-2-pyrrolidone
- STAB: Sodium Triacetoxyborohydride
- THF: Tetrahydrofuran
- Rt: Retention time

### Synthesis of Exemplary Compounds of Formula I

### General Procedure A: Aniline Alkylation

To a stirred solution of an aniline and an alkyl halide in DMF at 0 °C was added potassium carbonate. The reaction mixture was warmed to ambient temperature and stirred for 4 h. The reaction mixture was diluted with water and extracted with EtOAc. The organic layer was washed with water and brine, dried over sodium sulfate and concentrated under reduced pressure. The crude material was purified by column chromatography (silica gel, ethyl acetate in hexane) to afford the product.

### General Procedure B: Reductive Amination

To a stirred solution of an aniline and acetone in DCM or THF at 0 °C was added acetic acid. STAB was then added portion wise. The reaction mixture was allowed to warm to ambient temperature and stirred for 3 h. After cooling to 0 °C, the reaction mixture was quenched with water and extracted with DCM. The combined organic phases were washed with water and aqueous potassium carbonate and concentrated under reduced pressure to afford the product.

### General Procedure C: Acetamide Synthesis

To a stirred solution of an aniline in DCM, THF or toluene at 0 °C was added triethylamine. After stirring for 5 min, 2-chloroacetyl chloride was added dropwise and the resulting reaction mixture was warmed to ambient temperature and was allowed to stir for a further 2 h. The reaction mixture was diluted with water and extracted with DCM. The organic layer was washed with water and brine, dried over sodium sulfate and concentrated under reduced pressure. The crude material was purified by column chromatography (silica gel, ethyl acetate in hexane) to afford the product.

### General Procedure D: Acetamide Synthesis

To a stirred solution of an aniline in DMF at 0 °C was added sodium hydride (60% dispersion in mineral oil). After stirring for 10 min, 2-chloroacetyl chloride was added dropwise and the resulting reaction mixture was allowed to stir for a further 10 min. The reaction mixture was diluted with saturated aqueous sodium hydrogen carbonate and extracted with EtOAc. The organic layer was washed with water and brine, dried over sodium sulfate and concentrated under reduced pressure afford the product.

### General Procedure E: Acetate Synthesis

To a stirred solution of an alkyl chloride in DMF at ambient temperature was added sodium acetate. The resulting reaction mixture was warmed to 80 °C and was allowed to stir for 2 h. After completion, the reaction mixture was allowed to cool to ambient temperature and was diluted with water. The aqueous phase was extracted with EtOAc and the organic layer was then washed with water. The organic phase was dried over sodium sulfate and concentrated under reduced pressure. The crude material was purified by column chromatography (silica gel, ethyl acetate in hexane) to afford the product.

### General Procedure F: Ester Hydrolysis

To a stirred solution of an acetate in MeOH or THF at ambient temperature was added a solution of lithium or sodium hydroxide in water. The resulting reaction mixture was warmed to 80 °C and stirred for 2 h. After cooling, the reaction mixture was concentrated under reduced pressure. The crude residue was diluted with water and extracted with ethyl acetate. The organic phase was washed with brine, dried over sodium sulfate and concentrated under reduced pressure. The crude material was purified by column chromatography (silica gel, ethyl acetate in hexane) to afford the product.

### General Procedure G: Ester Hydrolysis

To a stirred solution of an acetate in MeOH at ambient temperature was added potassium carbonate and water. The resulting reaction mixture was stirred at ambient temperature for 3 h and then concentrated under reduced pressure. The crude residue was diluted with EtOAc and washed with aqueous HCl. The organic phase was concentrated under reduced pressure to afford the product.

### General Procedure H: Ester Hydrolysis

To a stirred solution of an acetate in MeOH at ambient temperature was added sodium methoxide. The resulting reaction mixture was stirred at ambient temperature for 1 h. The reaction mixture was concentrated under reduced pressure. The organic phase was concentrated under reduced pressure to afford the product. The crude material was purified by column chromatography (silica gel, ethyl acetate in hexane) to afford the product.

### General Procedure I: Nucleophilic Aromatic Substitution of 2,5-dichloro-1 ,3,4-thiadiazole

To a stirred solution of an alcohol and 2,5-dichloro-1 ,3,4-thiadiazole in isopropanol at -10 °C was added dropwise a solution of sodium or potassium hydroxide in water over 30 minutes. The resulting reaction mixture was stirred at -10 °C and then warmed to ambient temperature. The reaction was concentrated under reduced pressure. The crude material was purified by column chromatography (silica gel, ethyl acetate in hexane) to afford the product.

### General Procedure J: Nucleophilic Aromatic Substitution of 2,5-dichloro-1 ,3,4-thiadiazole

To a stirred solution of an alcohol and sodium hydride (60% dispersion in mineral oil) in toluene at 0 °C was added 2,5-dichloro-1,3,4-thiadiazole. The resulting reaction mixture was warmed to ambient temperature and stirred for 30 min. The reaction mixture was quenched with water and extracted with EtOAc. The organic phase was washed with water and brine, dried over sodium sulfate and concentrated under reduced pressure. The crude material was purified by Prep HPLC (C₁₈-silica, acetonitrile in water) to afford the product.

General Procedure K: Nucleophilic Aromatic Substitution of 2-chloro-5-(ethylsulfonyl)-1 ,3,4-thiadiazole To a stirred solution of an alcohol and 2-chloro-5-(ethylsulfonyl)-1,3,4-thiadiazole in isopropanol at -10 °C was added dropwise a solution of sodium hydroxide in water over 30 min. The resulting reaction mixture was stirred at -10 °C and then warmed to ambient temperature. The reaction was concentrated under reduced pressure. The crude material was purified by column chromatography (silica gel, ethyl acetate in hexane) to afford the product.

### General Procedure L: Nucleophilic Aromatic Substitution of 2-chloro-5-(methylsulfonyl)-1,3,4-thiadiazole

To a stirred solution of an alcohol and potassium hydroxide in isopropanol at ambient temperature was added 2-chloro-5-(methylsulfonyl)-1,3,4-thiadiazole. The resulting reaction mixture was stirred at 80 °C for 2 h. The reaction was quenched with water and extracted with EtOAc. The organic phase was washed with water and brine, dried over sodium sulfate and concentrated under reduced pressure. The crude material was purified by Prep HPLC (C₁₈-silica, acetonitrile in water) to afford the product.

| **Example** | **Compound** | **R^{1a}** | **R^{1b}** | **R²** | **R³** | **Melting Point (°C)** |
|---|---|---|---|---|---|---|
| 1 | I-7 | H | H | 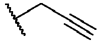 | 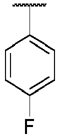 | 68-71 |
| 2 | I-4 | H | H | 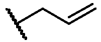 | 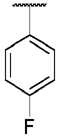 | 62-64 |
| 3 | IV-11 | H | H | 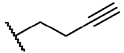 | 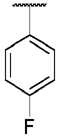 | 83-85 |
| 4 | II-6 | H | H | 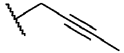 | 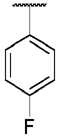 | 148-150 |
| 5 | I-42 | H | H | 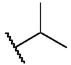 | 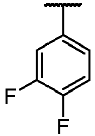 | 107-109 |
| 6 | I-284 | H | H | 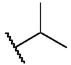 | 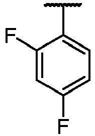 | 74-76 |
| 7 | I-53 | H | H | 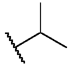 | 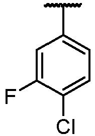 | 58-61 |
| 8 | I-31 | H | H | 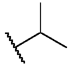 | 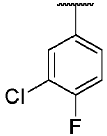 | 106-108 |
| 9 | I-5 | H | H | 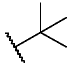 | 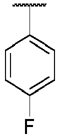 | 100-102 |
| 10 | I-8 | H | H | 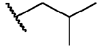 | 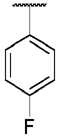 | N/A (oil) |
| 11 | I-6 | H | H | 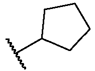 | 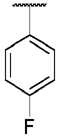 | 96-99 |
| 12 | I-1 | H | H | 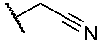 | 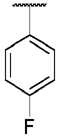 | 102-105 |
| 13 | I-2 | H | H | 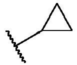 | 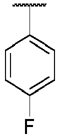 | 88-90 |
| 14 | I-10 | H | H | 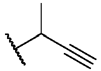 | 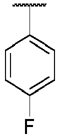 | 68-71 |
| 15 | I-3 | H | H | 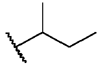 | 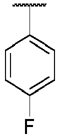 | 49-52 |
| 16 | I-1054 | H | H | 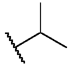 | 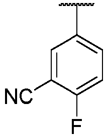 | 111-114 |
| 17 | I-207 | H | H | 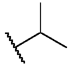 | 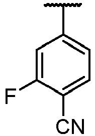 | 149-150 |
| 18 | I-282 | H | H | 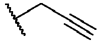 | 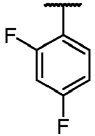 | N/A (oil) |
| 19 | I-281 | H | H | 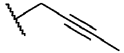 | 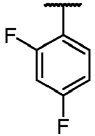 | 98-101 |
| 20 | I-279 | H | H | 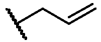 | 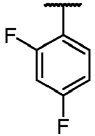 | N/A (oil) |
| 21 | I-361 | H | H | 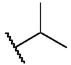 | 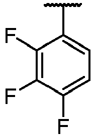 | 67-69 |
| 22 | I-273 | H | H | 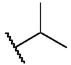 | 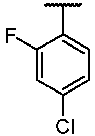 | 120-123 |
| 23 | I-295 | H | H | 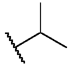 | 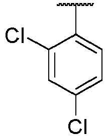 | 107-109 |
| 24 | I-317 | H | H | 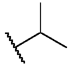 | 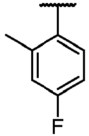 | 96-97 |
| 25 | I-339 | H | H | 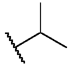 | 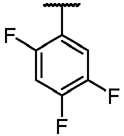 | 109-111 |
| 26 | I-1032 | H | H | 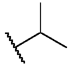 | 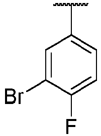 | N/A (oil) |

### Example 1: Preparation of 2-((5-chloro-1,3,4-thiadiazol-2-yl)oxy)-N-(4-fluorophenyl)-N-(prop-2-yn-1-yl)acetamide (I-7).

### Step 1: Preparation of intermediate I-7a, 4-fluoro-N-(prop-2-yn-1-yl)aniline.

Prepared according to general procedure A using 4-fluoroaniline (2.00 g, 18.0 mmol) and 3-bromoprop-1-yne (2.30 mL, 27.0 mmol), DMF (20 mL) and potassium carbonate (2.90 g, 21.3 mmol) to yield 4-fluoro-*N*-(prop-2-yn-1-yl)aniline (1.80 g). GCMS (Method E): Rt = 9.04 min, m/z = 148.1 [M+H]⁺.

### Step 2: Preparation of intermediate I-7b, 2-chloro-N-(4-fluorophenyl)-N-(prop-2-yn-1-yl)acetamide.

Prepared according to general procedure C using 4-fluoro-*N*-(prop-2-yn-1-yl)aniline (1.80 g, 12.1 mmol), triethylamine (2.88 mL, 20.5 mmol), DCM (20 mL) and 2-chloroacetyl chloride (1.30 mL, 16.9 mmol) to yield 2-chloro-*N*-(4-fluorophenyl)-*N*-(prop-2-yn-1-yl)acetamide (1.40 g). LCMS (Method B): Rt = 3.17 min, m/z = 226.0 [M+H]⁺.

### Step 3: Preparation of intermediate I-7c, 2-((4-fluorophenyl)(prop-2-yn-1-yl)amino)-2-oxoethyl acetate.

Prepared according to general procedure E using 2-chloro-*N*-(4-fluorophenyl)-*N*-(prop-2-yn-1-yl)acetamide (1.40 g, 6.22 mmol), DMF (15 mL) and sodium acetate (0.67 g 8.09 mmol) to yield 2-((4-fluorophenyl)(prop-2-yn-1-yl)amino)-2-oxoethyl acetate (0.90 g). LCMS (Method B): Rt = 3.07 min, m/z = 250.2 [M+H]⁺.

### Step 4: Preparation of intermediate I-7d, N-(4-fluorophenyl)-2-hydroxy-N-(prop-2-yn-1-yl)acetamide.

Prepared according to general procedure F using 2-((4-fluorophenyl)(prop-2-yn-1-yl)amino)-2-oxoethyl acetate (0.90 g, 3.63 mmol), MeOH (10 mL), sodium hydroxide (0.72 g, 18.2 mmol) and water (4.0 mL) to yield *N*-(4-fluorophenyl)-2-hydroxy-*N*-(prop-2-yn-1-yl)acetamide (0.60 g). LCMS (Method B): Rt = 2.61 min, m/z = 208.2 [M+H]⁺.

### Step 5: Preparation of 2-((5-chloro-1,3,4-thiadiazol-2-yl)oxy)-N-(4-fluorophenyl)-N-(prop-2-yn-1-yl)acetamide (I-7).

Prepared according to general procedure K using *N*-(4-fluorophenyl)-2-hydroxy-*N*-(prop-2-yn-1-yl)acetamide (0.10 g, 0.48 mmol), 2-chloro-5-(ethylsulfonyl)-1,3,4-thiadiazole (0.10 g, 0.48 mmol), isopropanol (2.0 mL), potassium hydroxide (0.08 g, 1.45 mmol) and water (0.6 mL) to yield 2-((5-chloro-1,3,4-thiadiazol-2-yl)oxy)-*N*-(4-fluorophenyl)-*N*-(prop-2-yn-1-yl)acetamide (0.02 g). LCMS (Method C): Rt = 2.53 min, m/z = 326.1 [M+H]⁺.

### Example 2: Preparation of N-allyl-2-((5-chloro-1,3,4-thiadiazol-2-yl)oxy)-N-(4-fluorophenyl)acetamide (I-4).

### Step 1: Preparation of intermediate I-4a, N-allyl-4-fluoroaniline.

Prepared according to general procedure A using 4-fluoroaniline (1.00 g, 9.00 mmol), allyl bromide (1.20 g, 9.92 mmol), DMF (10 mL) and potassium carbonate (1.24 g, 8.97 mmol) to yield N-allyl-4-fluoroaniline (0.25 g). LCMS (Method B): Rt = 1.37 min, m/z = 152.2 [M+H]⁺.

### Step 2: Preparation of intermediate I-4b, N-allyl-2-chloro-N-(4-fluorophenyl)acetamide.

Prepared according to general procedure C using *N*-allyl-4-fluoroaniline (0.24 g, 1.59 mmol), DCM (5.0 mL), triethylamine (0.33 mL, 2.37 mmol) and 2-chloroacetyl chloride (0.23 mL, 2.89 mmol) to yield *N-*allyl-2-chloro-*N*-(4-fluorophenyl)acetamide (0.30 g). LCMS (Method B): Rt = 1.81 min, m/z = 228.3 [M+H]⁺.

### Step 3: Preparation of intermediate I-4c, 2-(allyl(4-fluorophenyl)amino)-2-oxoethyl acetate.

Prepared according to general procedure E using *N*-allyl-2-chloro-*N*-(4-fluorophenyl)acetamide (0.38 g, 1.67 mmol), DMF (3.0 mL) and sodium acetate (0.13 g, 1.58 mmol) to yield 2-(allyl(4-fluorophenyl)amino)-2-oxoethyl acetate (0.25 g). LCMS (Method B): Rt = 3.07 min, m/z = 252.2 [M+H]⁺.

### Step 4: Preparation of intermediate I-4d, N-allyl-N-(4-fluorophenyl)-2-hydroxyacetamide.

Prepared according to general procedure F using 2-(allyl(4-fluorophenyl)amino)-2-oxoethyl acetate (0.25 g, 0.99 mmol), MeOH (3.0 mL), sodium hydroxide (0.20 g, 4.98 mmol) and water (1.0 mL) to yield *N*-allyl-*N*-(4-fluorophenyl)-2-hydroxyacetamide (0.10 g). LCMS (Method B): Rt = 2.77 min, m/z = 209.9 [M+H]⁺.

### Step 5: Preparation of N-allyl-2-((5-chloro-1,3,4-thiadiazol-2-yl)oxy)-N-(4-fluorophenyl)acetamide (I-4).

Prepared according to general procedure K using *N*-allyl-*N*-(4-fluorophenyl)-2-hydroxyacetamide (0.13 g, 0.62 mmol), 2-chloro-5-(ethylsulfonyl)-1,3,4-thiadiazole (0.13 g, 0.62 mmol), isopropanol (2.6 mL), potassium hydroxide (0.10 g, 1.85 mmol) and water (1.0 mL) to yield *N*-allyl-2-((5-chloro-1,3,4-thiadiazol-2-yl)oxy)-*N*-(4-fluorophenyl)acetamide (0.10 g). LCMS (Method B): Rt = 2.63 min, m/z = 328.1, 330.1 [M+H]⁺.

### Example 3: Preparation of N-(but-3-yn-1-yl)-2-((5-chloro-1,3,4-thiadiazol-2-yl)oxy)-N-(4-fluorophenyl)acetamide (IV-11).

### Step 1: Preparation of intermediate IV-11a, N-(but-3-yn-1-yl)-4-fluoroaniline.

Prepared according to general procedure A using 4-fluoroaniline (2.00 g, 18.0 mmol), 4-bromobut-1-yne (2.8 g, 21.6 mmol), DMF (40 mL) and potassium carbonate (3.7 g, 27.0 mmol) to yield *N*-(but-3-yn-1-yl)-4-fluoroaniline (1.8 g).

### Step 2: Preparation of intermediate IV-11b, N-(but-3-yn-1-yl)-2-chloro-N-(4-fluorophenyl)acetamide.

Prepared according to general procedure D using *N*-(but-3-yn-1-yl)-4-fluoroaniline (1.8 g, 11.0 mmol), DMF (18 mL), sodium hydride (0.66 mg, 16.5 mmol) and 2-chloroacetyl chloride (1.06 mL, 13.2 mmol) to yield *N*-(but-3-yn-1-yl)-2-chloro-*N*-(4-fluorophenyl)acetamide (1.9 g).

### Step 3: Preparation of intermediate IV-11c, 2-(but-3-yn-1-yl(4-fluorophenyl)amino)-2-oxoethyl acetate.

Prepared according to general procedure E using *N*-(but-3-yn-1-yl)-2-chloro-*N*-(4-fluorophenyl)acetamide (1.9 g, 7.92 mmol), DMF (19 mL) and sodium acetate (0.98 g, 11.9 mmol) to yield 2-(but-3-yn-1-yl(4-fluorophenyl)amino)-2-oxoethyl acetate (0.60 g).

### Step 4: Preparation of intermediate IV-11d, N-(but-3-yn-1-yl)-N-(4-fluorophenyl)-2-hydroxyacetamide.

Prepared according to general procedure H using 2-(but-3-yn-1-yl(4-fluorophenyl)amino)-2-oxoethyl acetate (0.60 g, 2.27 mmol), MeOH (6.0 mL) and sodium methoxide (0.18 g, 3.41 mmol) to yield *N*-(but-3-yn-1-yl)-*N*-(4-fluorophenyl)-2-hydroxyacetamide (0.25 g).

### Step 5: Preparation of N-(but-3-yn-1-yl)-2-((5-chloro-1,3,4-thiadiazol-2-yl)oxy)-N-(4-fluorophenyl)acetamide (IV-11)

Prepared according to general procedure L using *N*-(but-3-yn-1-yl)-*N*-(4-fluorophenyl)-2-hydroxyacetamide (0.25 g, 1.13 mmol), 2-chloro-5-(methylsulfonyl)-1,3,4-thiadiazole (0.22 g, 1.13 mmol), isopropanol (2.5 mL) and potassium hydroxide (0.10 g, 1.69 mmol) to yield *N*-(but-3-yn-1-yl)-2-((5-chloro-1,3,4-thiadiazol-2-yl)oxy)-*N*-(4-fluorophenyl)acetamide (0.05 g). LCMS (Method D): Rt = 3.11 min, m/z = 339.9, 341.9 [M+H]⁺.

### Example 4: Preparation of N-(but-2-yn-1-yl)-2-((5-chloro-1,3,4-thiadiazol-2-yl)oxy)-N-(4-fluorophenyl)acetamide (II-6).

### Step 1: Preparation of intermediate II-6a, N-(but-2-yn-1-yl)-4-fluoroaniline.

Prepared according to general procedure A using 4-fluoroaniline (2.0 g, 18.0 mmol), 1-bromobut-2-yne (2.8 g, 21.6 mmol), DMF (40 mL) and potassium carbonate (3.7 g, 27.0 mmol) stirring for 3 h to yield N-(but-2-yn-1-yl)-4-fluoroaniline (2.4 g).

### Step 2: Preparation of intermediate II-6b, N-(but-2-yn-1-yl)-2-chloro-N-(4-fluorophenyl)acetamide.

Prepared according to general procedure D using *N*-(but-2-yn-1-yl)-4-fluoroaniline (2.4 g, 14.7 mmol), DMF (24 mL), sodium hydride (0.88 g, 22.1 mmol) and 2-chloroacetyl chloride (1.41 mL, 17.6 mmol) to yield *N*-(but-2-yn-1-yl)-2-chloro-*N*-(4-fluorophenyl)acetamide (2.6 g). LCMS (Method D): Rt = 3.09 min, m/z = 239.9, 241.9 [M+H]⁺.

### Step 3: Preparation of intermediate II-6c, 2-(but-2-yn-1-yl(4-fluorophenyl)amino)-2-oxoethyl acetate.

Prepared according to general procedure E using *N*-(but-2-yn-1-yl)-2-chloro-*N*-(4-fluorophenyl)acetamide (2.6 g, 10.8 mmol), DMF (26 mL) and sodium acetate (1.0 g, 16.3 mmol) to yield 2-(but-2-yn-1-yl(4-fluorophenyl)amino)-2-oxoethyl acetate (1.2 g).

### Step 4: Preparation of intermediate II-6d, N-(but-2-yn-1-yl)-N-(4-fluorophenyl)-2-hydroxyacetamide.

Prepared according to general procedure H using 2-(but-2-yn-1-yl(4-fluorophenyl)amino)-2-oxoethyl acetate (1.2 g, 4.55 mmol), MeOH (12 mL) and sodium methoxide (0.27 g, 6.83 mmol) to yield *N*-(but-2-yn-1-yl)-*N*-(4-fluorophenyl)-2-hydroxyacetamide (0.80 g).

### Step 5: Preparation of N-(but-2-yn-1-yl)-2-((5-chloro-1,3,4-thiadiazol-2-yl)oxy)-N-(4-fluorophenyl)acetamide (II-6)

Prepared according to general procedure L using *N*-(but-2-yn-1-yl)-*N*-(4-fluorophenyl)-2-hydroxyacetamide (0.30 g, 1.35 mmol), 2-chloro-5-(methylsulfonyl)-1,3,4-thiadiazole (0.27 g, 1.35 mmol), isopropanol (3.0 mL) and potassium hydroxide (0.13 g, 2.02 mmol) to yield *N*-(but-2-yn-1-yl)-2-((5-chloro-1,3,4-thiadiazol-2-yl)oxy)-*N*-(4-fluorophenyl)acetamide (0.05 g). LCMS (Method D): Rt = 3.09 min, m/z = 340.0 [M+H]⁺.

### Example 5: Preparation of 2-((5-chloro-1,3,4-thiadiazol-2-yl)oxy)-N-(3,4-difluorophenyl)-N-isopropylacetamide (I-42).

### Step 1: Preparation of intermediate I-42a, 3,4-difluoro-N-isopropylaniline.

Prepared according to general procedure B using 3,4-difluoroaniline (2.0 g, 15.5 mmol), acetone (3.44 mL, 46.5 mmol), acetic acid (1.71 mL, 30.9 mmol), STAB (4.9 g, 23.2 mmol) and DCM (20 mL) stirring for 24 h. Extraction was performed with EtOAc, washing with water and brine. The crude material was purified by column chromatography (silica gel, ethyl acetate in hexane) to yield 3,4-difluoro-*N-*isopropylaniline (2.3 g). LCMS (Method D): Rt = 3.19 min, m/z = 172.0 [M+H]⁺.

### Step 2: Preparation of intermediate I-42b, 2-chloro-N-(3,4-difluorophenyl)-N-isopropylacetamide.

Prepared according to general procedure C using 3,4-difluoro-*N*-isopropylaniline (2.3 g, 13.4 mmol), DCM (23 mL), triethylamine (2.75 mL, 20.1 mmol) and 2-chloroacetyl chloride (1.76 mL, 22.8 mmol) stirring for 30 min at ambient temperature and quenching with a saturated solution of sodium hydrogen carbonate to yield 2-chloro-*N*-(3,4-difluorophenyl)-*N*-isopropylacetamide (1.4 g).

### Step 3: Preparation of intermediate I-42c, 2-((3,4-difluorophenyl)(isopropyl)amino)-2-oxoethyl acetate.

Prepared according to general procedure E using 2-chloro-*N*-(3,4-difluorophenyl)-*N*-isopropylacetamide (1.4 g, 5.65 mmol), DMF (14 mL) and sodium acetate (0.56 g, 6.78 mmol) stirring for 1 h to yield 2-((3,4-difluorophenyl)(isopropyl)amino)-2-oxoethyl acetate (1.5 g). LCMS (Method D): Rt = 3.02 min, m/z = 272.0 [M+H]⁺.

### Step 4: Preparation of intermediate I-42d, N-(3,4-difluorophenyl)-2-hydroxy-N-isopropylacetamide.

Prepared according to general procedure H using 2-((3,4-difluorophenyl)(isopropyl)amino)-2-oxoethyl acetate (1.5 g, 5.52 mmol), MeOH (15 mL) and sodium methoxide (0.45 g, 8.29 mmol) to yield *N*-(3,4-difluorophenyl)-2-hydroxy-*N*-isopropylacetamide (1.2 g). LCMS (Method D): Rt = 2.75 min, m/z = 230.0 [M+H]⁺.

### Step 5: Preparation of 2-((5-chloro-1,3,4-thiadiazol-2-yl)oxy)-N-(3,4-difluorophenyl)-N-isopropylacetamide (I-42).

Prepared according to general procedure I using *N*-(3,4-difluorophenyl)-2-hydroxy-*N-*isopropylacetamide (1.28 g, 5.57 mmol), 2,5-dichloro-1,3,4-thiadiazole (0.87 g, 5.57 mmol), isopropanol (24 mL), potassium hydroxide (0.31 g, 5.57 mmol) and water (6 mL) stirring for 30 min at -10 °C and then for 2 h at ambient temperature. The reaction mixture was quenched with water and extracted with EtOAc. The organic layer was washed with water, brine, dried over sodium sulfate, filtered and concentrated under reduced pressure. The crude material was purified by Prep HPLC (C₁₈-silica, acetonitrile in water) to yield 2-((5-chloro-1,3,4-thiadiazol-2-yl)oxy)-*N*-(3,4-difluorophenyl)-*N*-isopropylacetamide (0.05 g). LCMS (Method D): Rt = 3.34 min, m/z = 347.9, 349.9 [M+H]⁺.

### Example 6: 2-((5-chloro-1,3,4-thiadiazol-2-yl)oxy)-N-(2,4-difluorophenyl)-N-isopropylacetamide (I-284).

### Step 1: Preparation of intermediate I-284a, 2,4-difluoro-N-isopropylaniline.

Prepared according to general procedure B using 2,4-difluoroaniline (3.0 g, 23.2 mmol), acetone (6.89 mL, 92.9 mmol), acetic acid (2.67 mL, 46.5 mmol), STAB (11.8 g, 55.8 mmol) and DCM (30 mL) stirring for 24 h. Extraction was performed with EtOAc, washing with water and brine. The crude material was purified by column chromatography (silica gel, ethyl acetate in hexane) to yield 2,4-difluoro-*N-*isopropylaniline (2.3 g).

### Step 2: Preparation of intermediate I-284b, 2-chloro-N-(2,4-difluorophenyl)-N-isopropylacetamide.

Prepared according to general procedure C using 2,4-difluoro-*N*-isopropylaniline (2.3 g, 13.4 mmol), DCM (23 mL), triethylamine (2.75 mL, 20.1 mmol) and 2-chloroacetyl chloride (1.76 mL, 22.8 mmol) stirring for 30 min at ambient temperature and quenching with a saturated solution of sodium hydrogen carbonate to yield 2-chloro-*N*-(2,4-difluorophenyl)-*N*-isopropylacetamide (1.9 g).

### Step 3: Preparation of intermediate I-284c, 2-((2,4-difluorophenyl)(isopropyl)amino)-2-oxoethyl acetate.

Prepared according to general procedure E using 2-chloro-*N*-(2,4-difluorophenyl)-*N*-isopropylacetamide (1.9 g, 7.67 mmol), DMF (19 mL) and sodium acetate (0.76 g, 9.2 mmol) stirring for 1 h to yield 2-((2,4-difluorophenyl)(isopropyl)amino)-2-oxoethyl acetate (1.8 g). LCMS (Method D): Rt = 3.01 min, m/z = 272.0 [M+H]⁺.

### Step 4: Preparation of intermediate I-284d, N-(2,4-difluorophenyl)-2-hydroxy-N-isopropylacetamide.

Prepared according to general procedure H using 2-((2,4-difluorophenyl)(isopropyl)amino)-2-oxoethyl acetate (1.8 g, 6.63 mmol), MeOH (18 mL) and sodium methoxide (0.54 g, 9.95 mmol) to yield *N*-(2,4-difluorophenyl)-2-hydroxy-*N*-isopropylacetamide (1.5 g).

### Step 5: Preparation of 2-((5-chloro-1,3,4-thiadiazol-2-yl)oxy)-N-(2,4-difluorophenyl)-N-isopropylacetamide (I-284).

Prepared according to general procedure J using *N*-(2,4-difluorophenyl)-2-hydroxy-*N-*isopropylacetamide (0.15 g, 0.69 mmol), 2,5-dichloro-1,3,4-thiadiazole (0.11 g, 0.69 mmol), toluene (1.5 mL) and sodium hydride (0.04 g, 1.03 mmol) to yield 2-((5-chloro-1,3,4-thiadiazol-2-yl)oxy)-*N*-(2,4-difluorophenyl)-*N*-isopropylacetamide (0.10 g). LCMS (Method D): Rt = 3.34 min, m/z = 347.9, 349.9 [M+H]⁺.

### Example 7: Preparation of 2-((5-chloro-1,3,4-thiadiazol-2-yl)oxy)-N-(4-chloro-3-fluorophenyl)-N-isopropylacetamide (I-53).

### Step 1: Preparation of intermediate I-53a, 4-chloro-3-fluoro-N-isopropylaniline.

Prepared according to general procedure B using 4-chloro-3-fluoroaniline (0.50 g, 3.44 mmol), acetone (1.02 mL, 13.70 mmol), acetic acid (0.39 mL, 6.87 mmol), STAB (1.75 g, 8.24 mmol) and DCM (8.0 mL) to yield 4-chloro-3-fluoro-*N*-isopropylaniline (0.57 g). LCMS (Method A): Rt = 2.77 min, m/z = 188.1, 190.1 [M+H]⁺.f

### Step 2: Preparation of intermediate I-53b, 2-chloro-N-(4-chloro-3-fluorophenyl)-N-isopropylacetamide.

Prepared according to general procedure C using 4-chloro-3-fluoro-*N*-isopropylaniline (0.57 g, 3.02 mmol), toluene (6.1 mL), triethylamine (1.26 mL, 9.06 mmol) and 2-chloroacetyl chloride (0.48 mL, 6.04 mmol) to yield 2-chloro-*N*-(4-chloro-3-fluorophenyl)-*N*-isopropylacetamide (0.71 g). LCMS (Method A): Rt = 2.55 min, m/z = 264.0, 266.0 [M+H]⁺.

### Step 3: Preparation of intermediate I-53c, 2-((4-chloro-3-fluorophenyl)(isopropyl)amino)-2-oxoethyl acetate.

Prepared according to general procedure E using 2-chloro-*N*-(4-chloro-3-fluorophenyl)-*N-*isopropylacetamide (0.71 g, 2.67 mmol), DMF (4.0 mL) and sodium acetate (0.24 g, 2.94 mmol) stirring for 3 h to yield 2-((4-chloro-3-fluorophenyl)(isopropyl)amino)-2-oxoethyl acetate (0.68 g). LCMS (Method A): Rt = 2.36 min, m/z = 288.1 [M+H]⁺.

### Step 4: Preparation of intermediate I-53d, N-(4-chloro-3-fluorophenyl)-2-hydroxy-N-isopropylacetamide.

Prepared according to general procedure G using 2-((4-chloro-3-fluorophenyl)(isopropyl)amino)-2-oxoethyl acetate (0.68 g, 2.36 mmol), MeOH (6.5 mL), potassium carbonate (0.06 g, 0.43 mmol) and water (0.15 mL) to yield *N*-(4-chloro-3-fluorophenyl)-2-hydroxy-*N*-isopropylacetamide (0.26 g). LCMS (Method A): Rt = 2.11 min, m/z = 246.1, 248.1 [M+H]⁺.

### Step 5: Preparation of 2-((5-chloro-1,3,4-thiadiazol-2-yl)oxy)-N-(4-chloro-3-fluorophenyl)-N-isopropylacetamide (I-53).

Prepared according to general procedure I using *N*-(4-chloro-3-fluorophenyl)-2-hydroxy-*N-*isopropylacetamide (0.13 g, 0.51 mmol), 2,5-dichloro-1,3,4-thiadiazole (0.08 g, 0.51 mmol), isopropanol (1.5 mL), sodium hydroxide (0.06 g, 1.52 mmol) and water (0.76 mL) stirring for 30 min at -10 °C and 3.5 h at ambient temperature to yield 2-((5-chloro-1,3,4-thiadiazol-2-yl)oxy)-*N*-(4-chloro-3-fluorophenyl)-*N*-isopropylacetamide (0.08 g). LCMS (Method A): Rt = 2.67 min, m/z = 364.0, 366.0, 368.0 [M+H]⁺.

### Example 8: Preparation of 2-((5-chloro-1,3,4-thiadiazol-2-yl)oxy)-N-(3-chloro-4-fluorophenyl)-N-isopropylacetamide (I-31).

### Step 1: Preparation of intermediate I-31a, 3-chloro-4-fluoro-N-isopropylaniline.

Prepared according to general procedure B using 3-chloro-4-fluoroaniline (0.50 g, 3.44 mmol), acetone (1.02 mL, 13.70 mmol), acetic acid (0.39 mL, 6.87 mmol), STAB (1.75 g, 8.24 mmol) and DCM (8 mL) to yield 3-chloro-4-fluoro-*N*-isopropylaniline (0.57 g). LCMS (Method A): Rt = 2.53 min, m/z = 188.1, 190.1 [M+H]⁺.

### Step 2: Preparation of intermediate I-31b, 2-chloro-N-(3-chloro-4-fluorophenyl)-N-isopropylacetamide.

Prepared according to general procedure C using 3-chloro-4-fluoro-*N*-isopropylaniline (0.57 g, 3.03 mmol), toluene (6.1 mL), triethylamine (0.84 mL, 6.05 mmol) and 2-chloroacetyl chloride (1.33 mL, 9.08 mmol) to yield 2-chloro-*N*-(3-chloro-4-fluorophenyl)-*N*-isopropylacetamide (0.80 g). LCMS (Method A): Rt = 2.52 min, m/z = 264.0, 266.0 [M+H]⁺.

### Step 3: Preparation of intermediate I-31c, 2-((3-chloro-4-fluorophenyl)(isopropyl)amino)-2-oxoethyl acetate.

Prepared according to general procedure E using 2-chloro-*N*-(3-chloro-4-fluorophenyl)-*N-*isopropylacetamide (0.80 g, 2.86 mmol), DMF (4.0 mL) and sodium acetate (0.26 g, 3.15 mmol) stirring for 3 h to yield 2-((3-chloro-4-fluorophenyl)(isopropyl)amino)-2-oxoethyl acetate (0.69 g). LCMS (Method A): Rt = 2.32 min, m/z = 288.1 [M+H]⁺.

### Step 4: Preparation of intermediate I-31d, N-(3-chloro-4-fluorophenyl)-2-hydroxy-N-isopropylacetamide.

Prepared according to general procedure G using 2-((3-chloro-4-fluorophenyl)(isopropyl)amino)-2-oxoethyl acetate (0.21 g, 0.39 mmol), MeOH (6.5 mL), potassium carbonate (0.01 g, 0.08 mmol) and water (0.15 mL) to yield *N*-(3-chloro-4-fluorophenyl)-2-hydroxy-*N*-isopropylacetamide (0.16 g). LCMS (Method A): Rt = 2.08 min, m/z = 246.1, 248.1 [M+H]⁺.

### Step 5: Preparation of 2-((5-chloro-1,3,4-thiadiazol-2-yl)oxy)-N-(3-chloro-4-fluorophenyl)-N-isopropylacetamide (I-31).

Prepared according to general procedure I using *N*-(3-chloro-4-fluorophenyl)-2-hydroxy-*N-*isopropylacetamide (0.08 g, 0.31 mmol), 2,5-dichloro-1,3,4-thiadiazole (0.05 g, 0.31 mmol), isopropanol (1.5 mL), sodium hydroxide (0.04 g, 0.93 mmol) and water (0.46 mL) stirring for 30 min at -10 °C and 4.5 h at ambient temperature to yield 2-((5-chloro-1,3,4-thiadiazol-2-yl)oxy)-*N*-(3-chloro-4-fluorophenyl)-*N*-isopropylacetamide (0.08 g). LCMS (Method A): Rt = 2.62 min, m/z = 364.0, 366.0, 368.0 [M+H]⁺.

### Example 9: Preparation of N-(tert-butyl)-2-((5-chloro-1,3,4-thiadiazol-2-yl)oxy)-N-(4-fluorophenyl)acetamide (I-5).

### Step 1: Preparation of intermediate I-5a, N-(tert-butyl)-2-(4-fluorophenoxy)acetamide.

To a stirred solution of 4-fluorophenol (2.00 g, 17.85 mmol) and N-tert-butyl-2-chloroacetamide (3.20 g, 21.41 mmol) in DMF (20 mL) at 0 °C was added potassium carbonate (4.90 g, 35.46 mmol). The resulting reaction mixture was heated to 110 °C and allowed to stir for 8 h. The reaction mixture was diluted with water and extracted with ethyl acetate. The organic layer was washed water and brine, dried over sodium sulfate and concentrated under reduced pressure. The crude material was purified by column chromatography (silica gel, ethyl acetate in hexane) to afford N-(tert-butyl)-2-(4-fluorophenoxy)acetamide (3.20 g). LCMS (Method B): Rt = 3.23 min, m/z = 226.2 [M+H]⁺.

### Step 2: Preparation of intermediate I-5b, N-(tert-butyl)-4-fluoroaniline.

To a stirred solution of *N*-(*tert*-butyl)-2-(4-fluorophenoxy)acetamide (3.20 g, 14.21 mmol) in NMP:toluene (1:2) (50 mL) at ambient temperature was added potassium hydroxide (2.90 g, 51.69 mmol). The reaction mixture was heated to 120 °C and stirred for 16 h. The reaction mixture was diluted with water and extracted with EtOAc. The organic layer was washed with water and brine, dried over sodium sulfate and concentrated under reduced pressure. The crude material was purified by column chromatography (silica gel, ethyl acetate in hexane) to afford N-(tert-butyl)-4-fluoroaniline (0.60 g). LCMS (Method B); Rt = 3.42 min, m/z = 168.0 [M+H]⁺.

### Step 3: Preparation of intermediate I-5c, N-(tert-butyl)-2-chloro-N-(4-fluorophenyl)acetamide.

Prepared according to general procedure C using N-(tert-butyl)-4-fluoroaniline (0.60 g, 3.59 mmol), DCM (15 mL), triethylamine (0.85 mL, 6.10 mmol) and 2-chloroacetyl chloride (0.30 mL, 3.77 mmol) to yield *N*-(*tert*-butyl)-2-chloro-*N*-(4-fluorophenyl)acetamide (0.40 g). LCMS (Method B): Rt = 3.48 min, m/z = 244.2 [M+H]⁺.

### Step 4: Preparation of intermediate I-5d, 2-(tert-butyl(4-fluorophenyl)amino)-2-oxoethyl acetate.

Prepared according to general procedure E using *N*-*tert*-butyl-2-chloro-*N*-(4-fluorophenyl)acetamide (0.40 g, 1.64 mmol), DMF (3 mL) and sodium acetate (0.20 g, 2.46 mmol) to yield 2-(tert-butyl(4-fluorophenyl)amino)-2-oxoethyl acetate (0.27 g). LCMS (Method B): Rt = 3.18 min, m/z = 268.2 [M+H]⁺.

### Step 5: Preparation of intermediate I-5e, N-(tert-butyl)-N-(4-fluorophenyl)-2-hydroxyacetamide.

Prepared according to general procedure F using 2-(tert-butyl(4-fluorophenyl)amino)-2-oxoethyl acetate (0.27 g, 1.01 mmol), MeOH (2.5 mL), sodium hydroxide (0.20 g, 5.05 mmol) and water (0.6 mL) to yield *N*-(*tert*-butyl)-*N*-(4-fluorophenyl)-2-hydroxyacetamide (0.20 g). LCMS (Method B): Rt = 3.30 min, m/z = 226.2 [M+H]⁺.

### Step 6: Preparation of N-(tert-butyl)-2-((5-chloro-1,3,4-thiadiazol-2-yl)oxy)-N-(4-fluorophenyl)acetamide (I-5).

Prepared according to general procedure K using *N*-(*tert*-butyl)-*N*-(4-fluorophenyl)-2-hydroxyacetamide (0.20 g, 0.89 mmol), 2-chloro-5-(ethylsulfonyl)-1,3,4-thiadiazole (0.19 g, 0.89 mmol), isopropanol (3 mL), potassium hydroxide (0.15 g, 2.67 mmol) and water (0.6 mL) stirring for 30 min at -10 °C to yield N-(tert-butyl)-2-((5-chloro-1,3,4-thiadiazol-2-yl)oxy)-*N*-(4-fluorophenyl)acetamide (0.12 g). LCMS (Method C): Rt = 2.83 min, m/z = 344.1 [M+H]⁺.

### Example 10: Preparation of 2-((5-chloro-1,3,4-thiadiazol-2-yl)oxy)-N-(4-fluorophenyl)-N-isobutylacetamide (I-8).

### Step 1: Preparation of intermediate I-8a, 4-fluoro-N-isobutylaniline.

Prepared according to general procedure B using 4-fluoroaniline (2.00 g, 18.0 mmol), isobutyl aldehyde (1.20 g, 18.0 mmol), THF (20 mL) and STAB (5.70 g, 27.0 mmol) to yield 4-fluoro-*N*-isobutylaniline (1.50 g). LCMS (Method B): Rt = 3.67 min, m/z = 168.1 [M+H]⁺.

### Step 2: Preparation of intermediate I-8b, 2-chloro-N-(4-fluorophenyl)-N-isobutylacetamide.

Prepared according to general procedure C using 4-fluoro-*N*-isobutylaniline (1.50 g, 8.97 mmol), DCM (15 mL), triethylamine (2.13 mL, 15.25 mmol) and 2-chloroacetyl chloride (0.92 mL, 12.57 mmol) to yield 2-chloro-*N*-(4-fluorophenyl)-*N*-isobutylacetamide (1.20 g). LCMS (Method B): Rt = 3.46 min, m/z = 244.0 [M+H]⁺.

### Step 3: Preparation of intermediate I-8c, 2-((4-fluorophenyl)(isobutyl)amino)-2-oxoethyl acetate.

Prepared according to general procedure E using 2-chloro-*N*-(4-fluorophenyl)-*N*-isobutylacetamide (1.20 g, 4.94 mmol), DMF (20 mL) and sodium acetate (0.50 g, 6.41 mmol) to yield 2-((4-fluorophenyl)(isobutyl)amino)-2-oxoethyl acetate (1.00 g). LCMS (Method B): Rt = 3.24 min, m/z = 268.1 [M+H]⁺.

### Step 4: Preparation of intermediate I-8d, N-(4-fluorophenyl)-2-hydroxy-N-isobutylacetamide.

Prepared according to general procedure F using 2-((4-fluorophenyl)(isobutyl)amino)-2-oxoethyl acetate (1.00 g, 3.73 mmol), MeOH (10 mL), sodium hydroxide (0.76 g, 18.17 mmol) and water (4 mL) to yield *N*-(4-fluorophenyl)-2-hydroxy-*N*-isobutylacetamide (0.80 g). LCMS (Method B): Rt = 3.09 min, m/z = 226.2 [M+H]⁺.

### Step 5: Preparation of 2-((5-chloro-1 ,3,4-thiadiazol-2-yl)oxy)-N-(4-fluorophenyl)-N-isobutylacetamide (I-8).

Prepared according to general procedure K using *N*-(4-fluorophenyl)-2-hydroxy-*N*-isobutylacetamide (0.25 g, 1.11 mmol), 2-chloro-5-(ethylsulfonyl)-1,3,4-thiadiazole (0.24 g, 1.11 mmol), isopropanol (2 mL), potassium hydroxide (0.19 g, 3.33 mmol) and water (1 mL) at -20 °C for 2 h to yield 2-((5-chloro-1,3,4-thiadiazol-2-yl)oxy)-*N*-(4-fluorophenyl)-*N*-isobutylacetamide (0.14 g). LCMS (Method B): Rt = 2.80 min, m/z = 344.1 [M+H]⁺.

### Example 11: Preparation of 2-((5-chloro-1,3,4-thiadiazol-2-yl)oxy)-N-cyclopentyl-N-(4-fluorophenyl)acetamide (I-6).

### Step 1: Preparation of intermediate I-6a, N-cyclopentyl-4-fluoroaniline.

To a degassed, stirred solution of 1-chloro-4-fluorobenzene (1.00 g, 7.66 mmol) and cyclopentamine (0.98 g, 11.49 mmol) in DMSO (4 mL), copper iodide (0.22 g, 1.15 mmol), *N*¹,*N*²-bis(2,4,6-trimethoxyphenyl)ethanediamide (0.48 g, 1.14 mmol) and potassium phosphate tribasic (4.88 g, 22.98 mmol) were added. The reaction mixture was heated to 120 °C and stirred for 24 h. The reaction mixture was cooled to ambient temperature, diluted with water and extracted with EtOAc. The organic phase was dried over sodium sulfate and concentrated under reduced pressure. The crude material was purified by column chromatography (silica gel, ethyl acetate in petroleum ether) to afford *N*-cyclopentyl-4-fluoroaniline (0.60 g). LCMS (Method C): Rt = 3.69 min, m/z = 179.8 [M+H]⁺.

### Step 2: Preparation of intermediate I-6b, 2-chloro-N-cyclopentyl-N-(4-fluorophenyl)acetamide.

Prepared according to general procedure C using *N*-cyclopentyl-4-fluoroaniline (0.60 g, 3.35 mmol), DCM (10 mL), triethylamine (0.75 mL, 5.36 mmol) and 2-chloroacetyl chloride (0.32 mL, 4.02 mmol) to yield 2-chloro-*N*-cyclopentyl-*N*-(4-fluorophenyl)acetamide (0.55 g). LCMS (Method B): Rt = 3.33 min, m/z = 256.1 [M+H]⁺.

### Step 3: Preparation of intermediate I-6c, 2-(cyclopentyl(4-fluorophenyl)amino)-2-oxoethyl acetate.

Prepared according to general procedure E using 2-chloro-*N*-cyclopentyl-*N*-(4-fluorophenyl)acetamide (0.55 g, 2.16 mmol), DMF (4 mL) and sodium acetate (0.21 g, 2.56 mmol) to yield 2-(cyclopentyl(4-fluorophenyl)amino)-2-oxoethyl acetate (0.50 g). LCMS (Method B): Rt = 3.35 min, m/z = 280.2 [M+H]⁺.

### Step 4: Preparation of intermediate I-6d, N-cyclopentyl-N-(4-fluorophenyl)-2-hydroxyacetamide.

Prepared according to general procedure F using 2-(cyclopentyl(4-fluorophenyl)amino)-2-oxoethyl acetate (0.50 g, 1.79 mmol), MeOH (6 mL), sodium hydroxide (0.36 g, 8.95 mmol) and water (4 mL) to yield *N*-cyclopentyl-*N*-(4-fluorophenyl)-2-hydroxyacetamide (0.35 g). LCMS (Method B): Rt = 3.13 min, m/z = 238.2 [M+H]⁺.

### Step 5: Preparation of 2-((5-chloro-1,3,4-thiadiazol-2-yl)oxy)-N-cyclopentyl-N-(4-fluorophenyl)acetamide (I-6).

Prepared according to general procedure K using *N*-cyclopentyl-*N*-(4-fluorophenyl)-2-hydroxyacetamide (0.15 g, 0.63 mmol), 2-chloro-5-(ethylsulfonyl)-1,3,4-thiadiazole (0.13 g, 0.63 mmol), isopropanol (2 mL), potassium hydroxide (0.10 g, 1.85 mmol) and water (1 mL) at -20 °C for 2 h to yield 2-((5-chloro-1,3,4-thiadiazol-2-yl)oxy)-*N*-cyclopentyl-*N*-(4-fluorophenyl)acetamide (0.10 g). LCMS (Method C): Rt = 2.80 min, m/z = 356.1 [M+H]⁺.

### Example 12: Preparation of 2-((5-chloro-1,3,4-thiadiazol-2-yl)oxy)-N-(cyanomethyl)-N-(4-fluorophenyl)acetamide (I-1).

### Step 1: Preparation of intermediate I-1a, 2-((4-fluorophenyl)amino)acetonitrile.

To a solution 4-fluoroaniline (1.00 g, 9.00 mmol) and bromoacetonitrile (1.08 g, 9.00 mmol) in anhydrous EtOH (10 mL) at ambient temperature was added sodium acetate (1.23 g, 14.99 mmol). The resulting reaction mixture was heated to 80 °C and stirred for 4 h. After cooling, the solvent was removed under reduced pressure and the crude residue was diluted with water. The aqueous phase was extracted with EtOAc and the organic phase was dried over sodium sulfate and concentrated under reduced pressure. The crude material was purified by column chromatography (silica gel, ethyl acetate in hexane) to afford 2-((4-fluorophenyl)amino)acetonitrile (1.00 g). LCMS (Method B): Rt = 1.81 min, m/z = 151.1 [M+H]⁺.

### Step 2: Preparation of intermediate I-1b, 2-chloro-N-(cyanomethyl)-N-(4-fluorophenyl)acetamide.

Prepared according to general procedure C using 2-((4-fluorophenyl)amino)acetonitrile (1.00 g, 6.66 mmol), DCM (10 mL), triethylamine (1.38 mL, 9.89 mmol) and 2-chloroacetyl chloride (0.58 mL, 7.29 mmol) to yield 2-chloro-*N*-(cyanomethyl)-*N*-(4-fluorophenyl)acetamide (0.90 g). LCMS (Method B): Rt = 2.98 min, m/z = 227.0 [M+H]⁺.

### Step 3: Preparation of intermediate I-1c, 2-((cyanomethyl)(4-fluorophenyl)amino)-2-oxoethyl acetate.

Prepared according to general procedure E using 2-chloro-*N*-(cyanomethyl)-*N*-(4-fluorophenyl)acetamide (0.90 g, 3.97 mmol), DMF (10 mL) and sodium acetate (0.36 g, 4.36 mmol) stirring for 1 h at 80 °C to yield 2-((cyanomethyl)(4-fluorophenyl)amino)-2-oxoethyl acetate (0.80 g). LCMS (Method B): Rt = 2.87 min, m/z = 251.0 [M+H]⁺.

### Step 4: Preparation of intermediate I-1d, N-(cyanomethyl)-N-(4-fluorophenyl)-2-hydroxyacetamide.

Prepared according to general procedure G using 2-((cyanomethyl)(4-fluorophenyl)amino)-2-oxoethyl acetate (0.80 g, 3.20 mmol), MeOH (30 mL), potassium carbonate (0.53 g, 3.83 mmol) stirring at 0 °C for 15 min to yield *N*-(cyanomethyl)-*N*-(4-fluorophenyl)-2-hydroxyacetamide (0.15 g). LCMS (Method B): Rt = 2.33 min, m/z = 209.2 [M+H]⁺.

### Step 5: Preparation of 2-((5-chloro-1,3,4-thiadiazol-2-yl)oxy)-N-(cyanomethyl)-N-(4-fluorophenyl)acetamide (I-1).

Prepared according to general procedure K using *N*-(cyanomethyl)-*N*-(4-fluorophenyl)-2-hydroxyacetamide (0.15 g, 0.72 mmol), 2-chloro-5-(ethylsulfonyl)-1,3,4-thiadiazole (0.15 g, 0.72 mmol), isopropanol (6 mL), potassium hydroxide (0.12 g, 2.16 mmol) and water (0.5 mL) at -20 °C for 45 min to yield 2-((5-chloro-1,3,4-thiadiazol-2-yl)oxy)-*N*-(cyanomethyl)-*N*-(4-fluorophenyl)acetamide (0.02 g). LCMS (Method C): Rt = 2.45 min, m/z = 327.0, 329.0 [M+H]⁺.

### Example 13: Preparation of 2-((5-chloro-1,3,4-thiadiazol-2-yl)oxy)-N-cyclopropyl-N-(4-fluorophenyl)acetamide (I-2).

### Step 1: Preparation of intermediate I-2a, N-cyclopropyl-4-fluoroaniline.

A solution of 1-bromo-4-fluorobenzene (1.00 g, 5.71 mmol) and cyclopropylamine (0.79 mL, 11.40 mmol) in toluene (10.0 mL) was degassed with argon for 20 min. Potassium tert-butoxide (2.53 g, 22.55 mmol) followed by BrettPhos (0.27 g, 0.50 mmol) and Pd(dba)² (0.03 g, 0.06 mmol) were then added and the reaction was warmed to 90 °C and stirred for 18 h. After cooling, the reaction mixture was diluted with water and extracted with EtOAc. The organic phase was washed with brine, dried over sodium sulfate and concentrated under reduced pressure. The crude material was purified by column chromatography (silica gel, ethyl acetate in hexane) to afford *N*-cyclopropyl-4-fluoroaniline (0.80 g). LCMS (Method C): Rt = 8.30 min, m/z = 150.2 [MH]⁻.

### Step 2: Preparation of intermediate I-2b, 2-chloro-N-cyclopropyl-N-(4-fluorophenyl)acetamide.

Prepared according to general procedure C using *N*-cyclopropyl-4-fluoroaniline (0.80 g, 5.29 mmol), DCM (10 mL), triethylamine (1.20 mL, 8.60 mmol) and 2-chloroacetyl chloride (0.56 mL, 7.04 mmol) to yield 2-chloro-*N*-cyclopropyl-*N*-(4-fluorophenyl)acetamide (0.81 g). LCMS (Method B): Rt = 3.02 min, m/z = 228.2 [MH]⁻.

### Step 3: Preparation of intermediate I-2c, 2-(cyclopropyl(4-fluorophenyl)amino)-2-oxoethyl acetate.

Prepared according to general procedure E using 2-chloro-*N*-cyclopropyl-*N*-(4-fluorophenyl)acetamide (0.81 g, 3.56 mmol), DMF (10 mL) and sodium acetate (0.38 g, 4.63 mmol) stirring for 1 h at 80 °C to yield 2-(cyclopropyl(4-fluorophenyl)amino)-2-oxoethyl acetate (0.55 g). LCMS (Method B): Rt = 3.05 min, m/z = 252.2 [M+H]⁺.

### Step 4: Preparation of intermediate I-2d, N-cyclopropyl-N-(4-fluorophenyl)-2-hydroxyacetamide.

Prepared according to general procedure F using 2-(cyclopropyl(4-fluorophenyl)amino)-2-oxoethyl acetate (0.55 g, 2.19 mmol), MeOH (10 mL), sodium hydroxide (0.44 g, 10.93 mmol) and water (3 mL) to yield *N*-cyclopropyl-*N*-(4-fluorophenyl)-2-hydroxyacetamide (0.28 g). LCMS (Method B): Rt = 1.48 min, m/z = 210.3 [M+H]⁺.

### Step 5: Preparation of 2-((5-chloro-1,3,4-thiadiazol-2-yl)oxy)-N-cyclopropyl-N-(4-fluorophenyl)acetamide (I-2).

Prepared according to general procedure K using *N*-cyclopropyl-*N*-(4-fluorophenyl)-2-hydroxyacetamide (0.15 g, 0.72 mmol), 2-chloro-5-(ethylsulfonyl)-1,3,4-thiadiazole (0.15 g, 0.72 mmol), isopropanol (6 mL), potassium hydroxide (0.12 g, 2.16 mmol) and water (0.5 mL) at -20 °C for 45 min to yield 2-((5-chloro-1,3,4-thiadiazol-2-yl)oxy)-*N*-cyclopropyl-*N*-(4-fluorophenyl)acetamide (0.02 g). LCMS (Method C): Rt = 2.54 min, m/z = 328.1, 330.1 [M+H]⁺.

### Example 14: Preparation of N-(but-3-yn-2-yl)-2-((5-chloro-1,3,4-thiadiazol-2-yl)oxy)-N-(4-fluorophenyl)acetamide (I-10).

### Step 1: Preparation of intermediate I-10a, N-(but-3-yn-2-yl)-4-fluoroaniline.

To a stirred solution of triphenylphosphine (8.50 g, 32.41 mmol) in THF (10 mL) at 0 °C under an argon atmosphere was added DEAD (5.03 mL, 32.06 mmol) in THF (5 mL). The mixture was allowed to stir at 0 °C for 20 min, after which but-3-yn-2-ol (2.11 mL, 26.91 mmol) in THF (5 mL) was added. The resulting reaction mixture was stirred for 10 min at 0 °C, followed by the addition of a solution of 4-fluoroaniline (2.0 g, 18.00 mmol) and DIPEA (3.76 mL, 21.59 mmol) in THF (5 mL). The reaction mixture was stirred for 20 min at 0 °C. The reaction mixture was allowed to warm to ambient temperature and was stirred for 2 h. The reaction mixture was diluted with water and extracted with EtOAc. The organic phase was washed with water and brine, dried over sodium sulfate and concentrated under reduced pressure. The crude material was purified by column chromatography (silica gel, ethyl acetate in hexane) to afford N-(but-3-yn-2-yl)-4-fluoroaniline (0.51 g). GCMS (Method E): Rt = 8.27 min, m/z = 163.2 [MH]⁻.

### Step 2: Preparation of intermediate I-10b, N-(but-3-yn-2-yl)-2-chloro-N-(4-fluorophenyl)acetamide.

Prepared according to general procedure C using *N*-(but-3-yn-2-yl)-4-fluoroaniline (0.51 g, 3.13 mmol), DCM (10 mL), triethylamine (0.74 mL, 5.31 mmol) and 2-chloroacetyl chloride (0.33 mL, 4.15 mmol) to yield *N*-(but-3-yn-2-yl)-2-chloro-*N*-(4-fluorophenyl)acetamide (0.38 g). LCMS (Method B): Rt = 3.27 min, m/z = 240.0 [M+H]⁺.

### Step 3: Preparation of intermediate I-10c, 2-(but-3-yn-2-yl(4-fluorophenyl)amino)-2-oxoethyl acetate.

Prepared according to general procedure E using *N*-(but-3-yn-2-yl)-2-chloro-*N*-(4-fluorophenyl)acetamide (0.38 g, 1.59 mmol), DMF (4 mL) and sodium acetate (0.17 g, 2.06 mmol) stirring for 1 h at 80 °C to yield 2-(but-3-yn-2-yl(4-fluorophenyl)amino)-2-oxoethyl acetate (0.36 g). LCMS (Method B): Rt = 3.15 min, m/z = 264.2 [M+H]⁺.

### Step 4: Preparation of intermediate I-10d, N-(but-3-yn-2-yl)-N-(4-fluorophenyl)-2-hydroxyacetamide.

Prepared according to general procedure F using 2-(but-3-yn-2-yl(4-fluorophenyl)amino)-2-oxoethyl acetate (0.36 g, 1.37 mmol), MeOH (6 mL), sodium hydroxide (0.27 g, 6.83 mmol) and water (1 mL) stirring at 60 °C for 30 min to yield *N*-(but-3-yn-2-yl)-*N*-(4-fluorophenyl)-2-hydroxyacetamide (0.28 g). LCMS (Method B): Rt = 2.85 min, m/z = 222.0 [M+H]⁺.

### Step 5: Preparation of N-(but-3-yn-2-yl)-2-((5-chloro-1,3,4-thiadiazol-2-yl)oxy)-N-(4-fluorophenyl)acetamide (I-10).

Prepared according to general procedure K using *N*-(but-3-yn-2-yl)-*N*-(4-fluorophenyl)-2-hydroxyacetamide (0.28 g, 1.27 mmol), 2-chloro-5-(ethylsulfonyl)-1,3,4-thiadiazole (0.27 g, 1.29 mmol), isopropanol (6 mL), potassium hydroxide (0.21 g, 3.81 mmol) and water (1 mL) at -20 °C for 30 min to yield *N*-(but-3-yn-2-yl)-2-((5-chloro-1,3,4-thiadiazol-2-yl)oxy)-*N*-(4-fluorophenyl)acetamide (0.14 g). LCMS (Method B): Rt = 2.66 min, m/z = 340.1, 342.1 [M+H]⁺.

### Example 15: Preparation of N-(sec-butyl)-2-((5-chloro-1,3,4-thiadiazol-2-yl)oxy)-N-(4-fluorophenyl)acetamide (I-3).

### Step 1: Preparation of intermediate I3-a, N-(sec-butyl)-4-fluoroaniline.

Prepared according to general procedure A using 4-fluoroaniline (1.50 g, 13.50 mmol), 2-bromobutane (2.30 mL, 20.25 mmol), DMF (15 mL) and potassium carbonate (2.90 g, 20.98 mmol) stirring at 70 °C for 4 h to yield *N*-(*sec*-butyl)-4-fluoroaniline (0.90 g). LCMS (Method B): Rt = 3.59 min, m/z = 168.1 [M+H]⁺.

### Step 2: Preparation of intermediate I-3b, N-(sec-butyl)-2-chloro-N-(4-fluorophenyl)acetamide.

Prepared according to general procedure C using *N*-(*sec*-butyl)-4-fluoroaniline (0.90 g, 5.38 mmol), DCM (10 mL), triethylamine (1.09 mL, 7.81 mmol) and 2-chloroacetyl chloride (0.40 mL, 5.03 mmol) to yield *N*-(*sec*-butyl)-2-chloro-*N*-(4-fluorophenyl)acetamide (0.80 g). LCMS (Method B): Rt = 1.90 min, m/z = 244.3 [M+H]⁺.

### Step 3: Preparation of intermediate I-3c, 2-(sec-butyl(4-fluorophenyl)amino)-2-oxoethyl acetate.

Prepared according to general procedure E using *N*-(*sec*-butyl)-2-chloro-*N*-(4-fluorophenyl)acetamide (0.80 g, 3.28 mmol), DMF (10 mL) and sodium acetate (0.35 g, 4.27 mmol) to yield 2-(sec-butyl(4-fluorophenyl)amino)-2-oxoethyl acetate (0.80 g). GCMS (Method E): Rt = 8.30 min.

### Step 4: Preparation of intermediate I-3d, N-(sec-butyl)-N-(4-fluorophenyl)-2-hydroxyacetamide.

Prepared according to general procedure F using 2-(sec-butyl(4-fluorophenyl)amino)-2-oxoethyl acetate (0.80 g, 2.99 mmol), MeOH (10 mL), sodium hydroxide (0.57 g, 14.25 mmol) and water (3 mL) stirring at 60 °C for 30 min to yield *N*-(*sec*-butyl)-*N*-(4-fluorophenyl)-2-hydroxyacetamide (0.65 g). LCMS (Method B): Rt = 3.12 min, m/z = 226.1 [M+H]⁺.

### Step 5: Preparation of N-(sec-butyl)-2-((5-chloro-1,3,4-thiadiazol-2-yl)oxy)-N-(4-fluorophenyl)acetamide (I-3).

Prepared according to general procedure K using *N*-(*sec*-butyl)-*N*-(4-fluorophenyl)-2-hydroxyacetamide (0.30 g, 1.33 mmol), 2-chloro-5-(ethylsulfonyl)-1,3,4-thiadiazole (0.28 g, 1.33 mmol), isopropanol (6 mL), potassium hydroxide (0.22 g, 3.97 mmol) and water (1.5 mL) at -20 °C for 30 min to yield *N*-(*sec*-butyl)-2-((5-chloro-1,3,4-thiadiazol-2-yl)oxy)-*N*-(4-fluorophenyl)acetamide (0.29 g). LCMS (Method B): Rt = 2.76 min, m/z = 344.2 [M+H]⁺.

### Example 16: 2-((5-chloro-1,3,4-thiadiazol-2-yl)oxy)-N-(3-cyano-4-fluorophenyl)-N-isopropylacetamide (I-1054).

### Step 1: Preparation of intermediate I-1054a, 2-fluoro-5-(isopropylamino)benzonitrile.

Prepared according to general procedure B using 5-amino-2-fluorobenzonitrile (3.0 g, 22.0 mmol), acetone (6.53 mL, 88.2 mmol), acetic acid (2.65 mL, 44.1 mmol), STAB (11.2 g, 52.9 mmol) and DCM (30 mL) stirring for 24 h. Extraction was performed with EtOAc, washing with water and brine. The crude material was purified by column chromatography (silica gel, ethyl acetate in hexane) to yield 2-fluoro-5-(isopropylamino)benzonitrile (1.8 g).

### Step 2: Preparation of intermediate I-1054b, 2-chloro-N-(3-cyano-4-fluorophenyl)-N-isopropylacetamide.

Prepared according to general procedure C using 2-fluoro-5-(isopropylamino)benzonitrile (1.5 g, 8.42 mmol), DCM (15 mL), triethylamine (1.76 mL, 12.6 mmol) and 2-chloroacetyl chloride (1.14 mL, 14.3 mmol) stirring for 30 min at ambient temperature and quenching with a saturated solution of sodium hydrogen carbonate to yield 2-chloro-*N*-(3-cyano-4-fluorophenyl)-*N*-isopropylacetamide (2.1 g).

### Step 3: Preparation of intermediate I-1054c, 2-((3-cyano-4-fluorophenyl)(isopropyl)amino)-2-oxoethyl acetate.

Prepared according to general procedure E using 2-chloro-*N*-(3-cyano-4-fluorophenyl)-*N-*isopropylacetamide (1.0 g, 3.93 mmol), DMF (10 mL) and sodium acetate (0.32 g, 3.93 mmol) stirring for 1 h at 50 °C to yield 2-((3-cyano-4-fluorophenyl)(isopropyl)amino)-2-oxoethyl acetate (0.9 g).

### Step 4: Preparation of intermediate I-1054d, N-(3-cyano-4-fluorophenyl)-2-hydroxy-N-isopropylacetamide.

Prepared according to general procedure F using 2-((3-cyano-4-fluorophenyl)(isopropyl)amino)-2-oxoethyl acetate (1.00 g, 3.59 mmol), THF (4.5 mL) and lithium hydroxide monohydrate (0.15 g, 3.59 mmol) stirring for 10 min at ambient temperature to yield *N*-(3-cyano-4-fluorophenyl)-2-hydroxy-*N-*isopropylacetamide (0.80 g).

### Step 5: Preparation of 2-((5-chloro-1,3,4-thiadiazol-2-yl)oxy)-N-(3-cyano-4-fluorophenyl)-N-isopropylacetamide (I-1054).

Prepared according to general procedure J using *N*-(3-cyano-4-fluorophenyl)-2-hydroxy-*N-*isopropylacetamide (0.25 g, 1.06 mmol), 2,5-dichloro-1,3,4-thiadiazole (0.25 g, 1.59 mmol), toluene (2.5 mL) and sodium hydride (0.06 g, 1.38 mmol) stirring for 1 h to yield 2-((5-chloro-1,3,4-thiadiazol-2-yl)oxy)-*N*-(3-cyano-4-fluorophenyl)-*N-*isopropylacetamide (0.04 g). LCMS (Method D): Rt = 3.19 min, m/z = 354.9, 356.9 [M+H]⁺.

### Example 17: 2-((5-chloro-1,3,4-thiadiazol-2-yl)oxy)-N-(4-cyano-3-fluorophenyl)-N-isopropylacetamide (I-207).

### Step 1: Preparation of intermediate I-207a, 2-fluoro-4-(isopropylamino)benzonitrile.

Prepared according to general procedure B using 4-amino-2-fluorobenzonitrile (3.0 g, 22.0 mmol), acetone (6.53 mL, 88.2 mmol), acetic acid (2.65 mL, 44.1 mmol), STAB (11.2 g, 52.9 mmol) and DCM (30 mL) stirring for 24 h. Extraction was performed with EtOAc, washing with water and brine. The crude material was purified by column chromatography (silica gel, ethyl acetate in hexane) to yield 2-fluoro-4-(isopropylamino)benzonitrile (1.8 g).

### Step 2: Preparation of intermediate I-207b, 2-chloro-N-(4-cyano-3-fluorophenyl)-N-isopropylacetamide.

Prepared according to general procedure C using 2-fluoro-4-(isopropylamino)benzonitrile (1.5 g, 8.42 mmol), DCM (15 mL), triethylamine (1.76 mL, 12.6 mmol) and 2-chloroacetyl chloride (1.14 mL, 14.3 mmol) stirring for 30 min at ambient temperature and quenching with a saturated solution of sodium hydrogen carbonate to yield 2-chloro-*N*-(4-cyano-3-fluorophenyl)-*N*-isopropylacetamide (2.1 g).

### Step 3: Preparation of intermediate I-207c, 2-((4-cyano-3-fluorophenyl)(isopropyl)amino)-2-oxoethyl acetate.

Prepared according to general procedure E using 2-chloro-*N*-(4-cyano-3-fluorophenyl)-*N-*isopropylacetamide (2.1 g, 8.25 mmol), DMF (21 mL) and sodium acetate (0.81 g, 9.89 mmol) stirring for 1 h to yield 2-((4-cyano-3-fluorophenyl)(isopropyl)amino)-2-oxoethyl acetate (0.3 g).

### Step 4: Preparation of intermediate I-207d, N-(2,4-difluorophenyl)-2-hydroxy-N-isopropylacetamide.

Prepared according to general procedure F using 2-((4-cyano-3-fluorophenyl)(isopropyl)amino)-2-oxoethyl acetate (0.35 g, 1.30 mmol), THF (1.75 mL) and lithium hydroxide monohydrate (0.05 g, 1.30 mmol) stirring for 10 min at ambient temperature to yield *N*-(4-cyano-3-fluorophenyl)-2-hydroxy-*N-*isopropylacetamide (0.22 g).

### Step 5: Preparation of 2-((5-chloro-1,3,4-thiadiazol-2-yl)oxy)-N-(4-cyano-3-fluorophenyl)-N-isopropylacetamide (I-207).

Prepared according to general procedure J using *N*-(4-cyano-3-fluorophenyl)-2-hydroxy-*N-*isopropylacetamide (0.30 g, 1.27 mmol), 2,5-dichloro-1,3,4-thiadiazole (0.30 g, 1.90 mmol), toluene (3 mL) and sodium hydride (0.07 g, 1.65 mmol) stirring for 1 h to yield 2-((5-chloro-1,3,4-thiadiazol-2-yl)oxy)-*N*-(4-cyano-3-fluorophenyl)-*N-*isopropylacetamide (0.08 g). LCMS (Method D): Rt = 3.15 min, m/z = 354.9, 356.9 [M+H]⁺.

### Example 18: Preparation of 2-((5-chloro-1,3,4-thiadiazol-2-yl)oxy)-N-(2,4-difluorophenyl)-N-(prop-2-yn-1-yl)acetamide (I-282).

### Step 1: Preparation of intermediate I-282a, 2,4-difluoro-N-(prop-2-yn-1-yl)aniline.

Prepared according to general procedure A using 2,4-difluoroaniline (0.39 mL, 3.87 mmol), propargyl bromide (0.43 mL, 3.87 mmol), DMF (5 mL) and potassium carbonate (0.64 g, 4.65 mmol) to yield 2,4-difluoro-*N*-(prop-2-yn-1-yl)aniline (0.46 g). LCMS (Method A): Rt = 2.50 min, m/z = 168.0 [M+H]⁺.

### Step 2: Preparation of intermediate I-282b, 2-chloro-N-(2,4-difluorophenyl)-N-(prop-2-yn-1-yl)acetamide.

Prepared according to general procedure C using 2,4-difluoro-*N*-(prop-2-yn-1-yl)aniline (0.37 g, 2.24 mmol), triethylamine (0.34 mL, 2.47 mmol), DCM (5 mL) and 2-chloroacetyl chloride (0.20 mL, 2.47 mmol) to yield 2-chloro-*N*-(2,4-difluorophenyl)-*N*-(prop-2-yn-1-yl)acetamide (0.52 g). LCMS (Method A): Rt = 2.46 min, m/z = 244.1 [M+H]⁺.

### Step 3: Preparation of intermediate I-282c, 2-((2,4-difluorophenyl)(prop-2-yn-1-yl)amino)-2-oxoethyl acetate.

Prepared according to general procedure E using 2-chloro-*N*-(2,4-difluorophenyl)-*N*-(prop-2-yn-1-yl)acetamide (0.52 g, 2.14 mmol), DMF (5 mL) and sodium acetate (0.19 g, 2.36 mmol) to yield 2-((2,4-difluorophenyl)(prop-2-yn-1-yl)amino)-2-oxoethyl acetate (0.57 g). LCMS (Method A): Rt = 2.32 min.

### Step 4: Preparation of intermediate I-282d, N-(2,4-difluorophenyl)-2-hydroxy-N-(prop-2-yn-1-yl)acetamide.

Prepared according to general procedure G using 2-((2,4-difluorophenyl)(prop-2-yn-1-yl)amino)-2-oxoethyl acetate (0.46 g, 1.71 mmol), MeOH (5 mL) and potassium carbonate (0.05 g, 0.36 mmol) to yield *N*-(2,4-difluorophenyl)-2-hydroxy-*N*-(prop-2-yn-1-yl)acetamide (0.26 g). LCMS (Method A): Rt = 1.98 min, m/z = 226.1 [M+H]⁺.

### Step 5: Preparation of 2-((5-chloro-1,3,4-thiadiazol-2-yl)oxy)-N-(2,4-difluorophenyl)-N-(prop-2-yn-1-yl)acetamide (I-282).

Prepared according to general procedure J using *N*-(2,4-difluorophenyl)-2-hydroxy-*N*-(prop-2-yn-1-yl)acetamide (0.26 g, 1.13 mmol), 2,5-dichloro-1,3,4-thiadiazole (0.18 g, 1.13 mmol), toluene (2 mL) and sodium hydride (0.07 g, 1.70 mmol) to yield 2-((5-chloro-1,3,4-thiadiazol-2-yl)oxy)-*N*-(2,4-difluorophenyl)-*N*-(prop-2-yn-1-yl)acetamide (0.18 g). LCMS (Method C): Rt = 2.63 min, m/z = 344.0 [M+H]⁺.

### Example 19: Preparation of N-(but-2-yn-1-yl)-2-((5-chloro-1,3,4-thiadiazol-2-yl)oxy)-N-(2,4-difluorophenyl)acetamide (II-281).

### Step 1: Preparation of intermediate II-281a, N-(but-2-yn-1-yl)-2,4-difluoroaniline.

Prepared according to general procedure A using 2,4-difluoroaniline (0.39 mL, 3.87 mmol), 1-bromobut-2-yne (0.34 mL, 3.87 mmol), DMF (5 mL) and potassium carbonate (0.64 g, 4.65 mmol) to yield *N*-(but-2-yn-1-yl)-2,4-difluoroaniline (0.57 g). LCMS (Method A): Rt = 2.68 min, m/z = 182.1 [M+H]⁺.

### Step 2: Preparation of intermediate II-281b, N-(but-2-yn-1-yl)-2-chloro-N-(2,4-difluorophenyl)acetamide.

Prepared according to general procedure C using 2,4-difluoro-*N*-(prop-2-yn-1-yl)aniline (0.57 g, 3.12 mmol), triethylamine (0.61 mL, 4.37 mmol), DCM (5 mL) and 2-chloroacetyl chloride (0.35 mL, 4.37 mmol) stirring for 20 min to yield *N*-(but-2-yn-1-yl)-2-chloro-*N*-(2,4-difluorophenyl)acetamide (0.49 g). LCMS (Method A): Rt = 2.57 min, m/z = 258.1 [M+H]⁺.

### Step 3: Preparation of intermediate II-281c, 2-(but-2-yn-1-yl(2,4-difluorophenyl)amino)-2-oxoethyl acetate.

Prepared according to general procedure E using *N*-(but-2-yn-1-yl)-2-chloro-*N*-(2,4-difluorophenyl)acetamide (0.49 g, 1.90 mmol), DMF (5 mL) and sodium acetate (0.33 g, 3.99 mmol) stirring for 38 h to yield 2-(but-2-yn-1-yl(2,4-difluorophenyl)amino)-2-oxoethyl acetate (0.85 g). LCMS (Method A): Rt = 2.43 min, m/z = 282.1 [M+H]⁺.

### Step 4: Preparation of intermediate II-281d, N-(but-2-yn-1-yl)-N-(2,4-difluorophenyl)-2-hydroxyacetamide.

Prepared according to general procedure G using 2-(but-2-yn-1-yl(2,4-difluorophenyl)amino)-2-oxoethyl acetate (0.47 g, 1.69 mmol), MeOH (5 mL) and potassium carbonate (0.05 g, 0.36 mmol) to yield N-(but-2-yn-1-yl)-*N*-(2,4-difluorophenyl)-2-hydroxyacetamide (0.40 g). LCMS (Method A): Rt = 2.19 min, m/z = 240.1 [M+H]⁺.

### Step 5: Preparation of N-(but-2-yn-1-yl)-2-((5-chloro-1,3,4-thiadiazol-2-yl)oxy)-N-(2,4-difluorophenyl)acetamide (II-281).

Prepared according to general procedure J using *N*-(but-2-yn-1-yl)-*N*-(2,4-difluorophenyl)-2-hydroxyacetamide (0.37 g, 1.55 mmol), 2,5-dichloro-1,3,4-thiadiazole (0.24 g, 1.55 mmol), toluene (2 mL) and sodium hydride (0.09 g, 1.70 mmol) to yield *N*-(but-2-yn-1-yl)-2-((5-chloro-1,3,4-thiadiazol-2-yl)oxy)-*N*-(2,4-difluorophenyl)acetamide (0.28 g). LCMS (Method A): Rt = 2.76 min, m/z = 358.0 [M+H]⁺.

### Example 20: Preparation of N-allyl-2-((5-chloro-1,3,4-thiadiazol-2-yl)oxy)-N-(2,4-difluorophenyl)acetamide (I-279).

### Step 1: Preparation of intermediate I-279a, N-allyl-2,4-difluoroaniline.

Prepared according to general procedure A using 2,4-difluoroaniline (0.39 mL, 3.87 mmol), allyl bromide (0.40 mL, 4.65 mmol), DMF (5 mL) and potassium carbonate (0.64 g, 4.65 mmol) stirring to 42 h to yield *N*-allyl-2,4-difluoroaniline (0.42 g). LCMS (Method A): Rt = 2.73 min, m/z = 170.1 [M+H]⁺.

### Step 2: Preparation of intermediate I-279b, N-allyl-2-chloro-N-(2,4-difluorophenyl)acetamide.

Prepared according to general procedure C using *N*-allyl-2,4-difluoroaniline (0.42 g, 2.49 mmol), triethylamine (0.38 mL, 2.74 mmol), DCM (5 mL) and 2-chloroacetyl chloride (0.22 mL, 2.74 mmol) stirring for 10 min to yield *N*-allyl-2-chloro-*N*-(2,4-difluorophenyl)acetamide (0.36 g). LCMS (Method A): Rt = 2.54 min, m/z = 246.1 [M+H]⁺.

### Step 3: Preparation of intermediate I-279c, 2-(allyl(2,4-difluorophenyl)amino)-2-oxoethyl acetate.

Prepared according to general procedure E using *N*-allyl-2-chloro-*N*-(2,4-difluorophenyl)acetamide (0.34 g, 1.36 mmol), DMF (5 mL) and sodium acetate (0.24 g, 2.87 mmol) stirring for 18 h to 2-(allyl(2,4-difluorophenyl)amino)-2-oxoethyl acetate (0.36 g). LCMS (Method A): Rt = 2.43 min, m/z = 270.1 [M+H]⁺.

### Step 4: Preparation of intermediate I-279d, N-allyl-N-(2,4-difluorophenyl)-2-hydroxyacetamide.

Prepared according to general procedure G using 2-(allyl(2,4-difluorophenyl)amino)-2-oxoethyl acetate (0.36 g, 1.33 mmol), MeOH (5 mL) and potassium carbonate (0.04 g, 0.30 mmol) stirring for 2 h to yield *N*-allyl-*N*-(2,4-difluorophenyl)-2-hydroxyacetamide (0.20 g). LCMS (Method A): Rt = 2.17 min, m/z = 228.1 [M+H]⁺.

### Step 5: Preparation of N-allyl-2-((5-chloro-1,3,4-thiadiazol-2-yl)oxy)-N-(2,4-difluorophenyl)acetamide (I-279).

Prepared according to general procedure J using *N*-allyl-*N*-(2,4-difluorophenyl)-2-hydroxyacetamide (0.22 g, 0.96 mmol), 2,5-dichloro-1,3,4-thiadiazole (0.15 g, 0.96 mmol), toluene (2 mL) and sodium hydride (0.06 g, 1.44 mmol) to yield *N*-allyl-2-((5-chloro-1,3,4-thiadiazol-2-yl)oxy)-*N*-(2,4-difluorophenyl)acetamide (0.15 g). LCMS (Method A): Rt = 2.70 min, m/z = 346.0 [M+H]⁺.

### Example 21: Preparation of 2-((5-chloro-1,3,4-thiadiazol-2-yl)oxy)-N-isopropyl-N-(2,3,4-trifluorophenyl)acetamide (I-361).

### Step 1: Preparation of intermediate I-361a, 2,3,4-trifluoro-N-isopropylaniline.

Prepared according to general procedure B using 2,3,4-trifluoroaniline (0.36 mL, 3.40 mmol), acetone (1.01 mL, 13.6 mmol), acetic acid (0.39 mL, 6.80 mmol), STAB (1.73 g, 8.16 mmol) and DCM (6 mL) to yield 2,3,4-trifluoro-*N*-isopropylaniline (0.61 g). LCMS (Method A): Rt = 2.99 min, m/z = 190.1 [M+H]⁺.

### Step 2: Preparation of intermediate I-361b, 2-chloro-N-isopropyl-N-(2,3,4-trifluorophenyl)acetamide.

Prepared according to general procedure C using 2,3,4-trifluoro-*N*-isopropylaniline (0.61 g, 3.21 mmol), DCM (6 mL), triethylamine (1.34 mL, 9.63 mmol) and 2-chloroacetyl chloride (0.77 mL, 9.63 mmol) stirring for 70 min at ambient temperature, washing with 6 M HCl (aqueous) to yield 2-chloro-*N*-isopropyl-*N*-(2,3,4-trifluorophenyl)acetamide (0.72 g). LCMS (Method A): Rt = 2.72 min, m/z = 266.1 [M+H]⁺.

### Step 3: Preparation of intermediate I-361c, 2-(isopropyl(2,3,4-trifluorophenyl)amino)-2-oxoethyl acetate.

Prepared according to general procedure E using 2-chloro-*N*-isopropyl-*N*-(2,3,4-trifluorophenyl)acetamide (0.73 g, 2.73 mmol), DMF (8 mL) and sodium acetate (0.34 g, 4.01 mmol) stirring for 22 h to yield 2-(isopropyl(2,3,4-trifluorophenyl)amino)-2-oxoethyl acetate (0.52 g). LCMS (Method D): Rt = 2.55 min, m/z = 290.1 [M+H]⁺.

### Step 4: Preparation of intermediate I-361d, 2-hydroxy-N-isopropyl-N-(2,3,4-trifluorophenyl)acetamide.

Prepared according to general procedure G using 2-(isopropyl(2,3,4-trifluorophenyl)amino)-2-oxoethyl acetate (0.52 g, 1.81 mmol), potassium carbonate (0.05 g, 0.36 mmol), MeOH (6.5 mL) and water (0.15 mL) to yield 2-hydroxy-*N*-isopropyl-*N*-(2,3,4-trifluorophenyl)acetamide (0.40 g). LCMS (Method A): Rt = 2.32 min, m/z = 248.1 [M+H]⁺.

### Step 5: Preparation of 2-((5-chloro-1,3,4-thiadiazol-2-yl)oxy)-N-isopropyl-N-(2,3,4-trifluorophenyl)acetamide (I-361).

Prepared according to general procedure I using 2-hydroxy-*N*-isopropyl-*N*-(2,3,4-trifluorophenyl)acetamide (0.26 g, 1.04 mmol), 2,5-dichloro-1,3,4-thiadiazole (0.16 g, 1.04 mmol), isopropanol (1.5 mL), sodium hydroxide (0.13 g, 3.13 mmol) and water (1.6 mL) stirring for 30 min at -10 °C and then for 3.5 h at ambient temperature to yield 2-((5-chloro-1,3,4-thiadiazol-2-yl)oxy)-*N*-isopropyl-*N*-(2,3,4-trifluorophenyl)acetamide (0.19 g). LCMS (Method A): Rt = 2.85 min, m/z = 366.0, 368.0 [M+H]⁺.

### Example 22: Preparation of 2-((5-chloro-1,3,4-thiadiazol-2-yl)oxy)-N-(4-chloro-2-fluorophenyl)-N-isopropylacetamide (I-273).

### Step 1: Preparation of intermediate I-273a, 4-chloro-2-fluoro-N-isopropylaniline.

Prepared according to general procedure B using 4-chloro-2-fluoroaniline (0.38 mL, 3.44 mmol), acetone (1.02 mL, 13.7 mmol), acetic acid (0.39 mL, 6.87 mmol), STAB (1.76 g, 8.24 mmol) and DCM (6 mL) to yield 4-chloro-2-fluoro-*N*-isopropylaniline (0.61 g). LCMS (Method A): Rt = 3.12 min, m/z = 188.1, 190.1 [M+H]⁺.

### Step 2: Preparation of intermediate I-273b, 2-chloro-N-isopropyl-N-(2,3,4-trifluorophenyl)acetamide.

Prepared according to general procedure C using 4-chloro-2-fluoro-*N*-isopropylaniline (0.60 g, 3.21 mmol), DCM (6 mL), triethylamine (1.79 mL, 12.84 mmol) and 2-chloroacetyl chloride (0.77 mL, 9.63 mmol) stirring for 20 min at ambient temperature, washing with HCl (aqueous) to yield 2-chloro-*N*-(4-chloro-2-fluorophenyl)-*N*-isopropylacetamide (0.54 g). LCMS (Method A): Rt = 2.79 min, m/z = 264.0, 266.0 [M+H]⁺.

### Step 3: Preparation of intermediate I-273c, 2-((4-chloro-2-fluorophenyl)(isopropyl)amino)-2-oxoethyl acetate.

Prepared according to general procedure E using 2-chloro-*N*-(4-chloro-2-fluorophenyl)-*N-*isopropylacetamide (0.51 g, 1.94 mmol), DMF (4 mL) and sodium acetate (0.24 g, 2.91 mmol) stirring for 63 h to yield 2-((4-chloro-2-fluorophenyl)(isopropyl)amino)-2-oxoethyl acetate (0.54 g). LCMS (Method A): Rt = 2.61 min, m/z = 288.1 [M+H]⁺.

### Step 4: Preparation of intermediate I-273d, N-(4-chloro-2-fluorophenyl)-2-hydroxy-N-isopropylacetamide.

Prepared according to general procedure G using 2-(isopropyl(2,3,4-trifluorophenyl)amino)-2-oxoethyl acetate (0.52 g, 1.81 mmol), potassium carbonate (0.31 g, 2.24 mmol), MeOH (6.5 mL) and water (0.15 mL) to yield *N*-(4-chloro-2-fluorophenyl)-2-hydroxy-*N*-isopropylacetamide (0.23 g). LCMS (Method A): Rt = 2.37 min, m/z = 246.1, 248.1 [M+H]⁺.

### Step 5: Preparation of 2-((5-chloro-1,3,4-thiadiazol-2-yl)oxy)-N-(4-chloro-2-fluorophenyl)-N-isopropylacetamide (I-273).

Prepared according to general procedure I using *N*-(4-chloro-2-fluorophenyl)-2-hydroxy-*N-*isopropylacetamide (0.22 g, 0.91 mmol), 2,5-dichloro-1,3,4-thiadiazole (0.14 g, 0.91 mmol), isopropanol (1.5 mL), sodium hydroxide (0.11 g, 2.73 mmol) and water (1.4 mL) stirring for 30 min at -10 °C and then for 5 h at ambient temperature to yield 2-((5-chloro-1,3,4-thiadiazol-2-yl)oxy)-*N*-(4-chloro-2-fluorophenyl)-*N*-isopropylacetamide (0.18 g). LCMS (Method A): Rt = 2.91 min, m/z = 364.0, 366.0 [M+H]⁺.

### Example 23: Preparation of 2-((5-chloro-1,3,4-thiadiazol-2-yl)oxy)-N-(2,4-dichlorophenyl)-N-isopropylacetamide (I-295).

### Step 1: Preparation of intermediate I-295a, 2,4-dichloro-N-isopropylaniline.

Prepared according to general procedure B using 2,4-dichloroaniline (0.50 g, 3.09 mmol), acetone (0.92 mL, 12.3 mmol), acetic acid (0.35 mL, 6.17 mmol), STAB (1.57 g, 7.41 mmol) and DCM (5 mL) to yield 2,4-dichloro-*N*-isopropylaniline (0.54 g). LCMS (Method A): Rt = 3.37 min, m/z = 204.1, 206.1, 208.1 [M+H]⁺.

### Step 2: Preparation of intermediate I-295b, 2-chloro-N-(2,4-dichlorophenyl)-N-isopropylacetamide.

Prepared according to general procedure C using 2,4-dichloro-*N*-isopropylaniline (0.54 g, 2.67 mmol), DCM (5 mL), triethylamine (1.49 mL, 10.68 mmol) and 2-chloroacetyl chloride (0.85 mL, 10.68 mmol) stirring for 30 min at ambient temperature, washing with HCl (aqueous) to yield 2-chloro-*N*-(2,4-dichlorophenyl)-*N*-isopropylacetamide (0.49 g). LCMS (Method A): Rt = 2.92 min, m/z = 280.0, 284.0 [M+H]⁺.

### Step 3: Preparation of intermediate I-295c, 2-((2,4-dichlorophenyl)(isopropyl)amino)-2-oxoethyl acetate.

Prepared according to general procedure E using 2-chloro-*N*-(2,4-dichlorophenyl)-*N-*isopropylacetamide (0.49 g, 1.74 mmol), DMF (6 mL) and sodium acetate (0.30 g, 3.65 mmol) stirring for 18 h to yield 2-((2,4-dichlorophenyl)(isopropyl)amino)-2-oxoethyl acetate (0.44 g). LCMS (Method A): Rt = 2.77 min, m/z = 304.0 [M+H]⁺.

### Step 4: Preparation of intermediate I-295d, N-(2,4-dichlorophenyl)-2-hydroxy-N-isopropylacetamide.

Prepared according to general procedure G using 2-((2,4-dichlorophenyl)(isopropyl)amino)-2-oxoethyl acetate (0.44 g, 1.44 mmol), potassium carbonate (0.04 g, 0.29 mmol) and MeOH (6.5 mL) to yield N-(2,4-dichlorophenyl)-2-hydroxy-*N*-isopropylacetamide (0.29 g). LCMS (Method A): Rt = 2.52 min, m/z = 262.1, 264.0, 266.0 [M+H]⁺.

### Step 5: Preparation of 2-((5-chloro-1,3,4-thiadiazol-2-yl)oxy)-N-(2,4-dichlorophenyl)-N-isopropylacetamide (I-295).

Prepared according to general procedure J using *N*-(2,4-dichlorophenyl)-2-hydroxy-*N-*isopropylacetamide (0.22 g, 0.83 mmol), 2,5-dichloro-1,3,4-thiadiazole (0.13 g, 0.83 mmol), toluene (2 mL) and sodium hydride (0.05 g, 1.25 mmol) to yield 2-((5-chloro-1,3,4-thiadiazol-2-yl)oxy)-*N*-(2,4-dichlorophenyl)-*N*-isopropylacetamide (0.14 g). LCMS (Method A): Rt = 3.01 min, m/z = 382.0, 384.0, 386.0 [M+H]⁺.

### Example 24: Preparation of 2-((5-chloro-1,3,4-thiadiazol-2-yl)oxy)-N-(4-fluoro-2-methylphenyl)-N-isopropylacetamide (I-317).

### Step 1: Preparation of intermediate I-317a, 4-fluoro-N-isopropyl-2-methylaniline.

Prepared according to general procedure B using 4-fluoro-2-methylaniline (0.44 mL, 4.00 mmol), acetone (1.18 mL, 16.0 mmol), acetic acid (0.46 mL, 7.99 mmol), STAB (2.03 g, 9.59 mmol) and DCM (5 mL) stirring for 2 h to yield 4-fluoro-*N*-isopropyl-2-methylaniline (0.52 g). LCMS (Method A): Rt = 1.68 min, m/z = 168.0 [M+H]⁺.

### Step 2: Preparation of intermediate I-317b, 2-chloro-N-(4-fluoro-2-methylphenyl)-N-isopropylacetamide.

Prepared according to general procedure C using 4-fluoro-*N*-isopropyl-2-methylaniline (0.55 g, 3.26 mmol), toluene (5 mL), triethylamine (0.91 mL, 6.52 mmol) and 2-chloroacetyl chloride (0.52 mL, 6.52 mmol) stirring for 20 min at ambient temperature, washing with HCl (aqueous) to yield 2-chloro-*N*-(4-fluoro-2-methylphenyl)-*N*-isopropylacetamide (0.66 g). LCMS (Method A): Rt = 2.67 min, m/z = 244.1, 246.1 [M+H]⁺.

### Step 3: Preparation of intermediate I-317c, 2-((4-fluoro-2-methylphenyl)(isopropyl)amino)-2-oxoethyl acetate.

Prepared according to general procedure E using 2-chloro-*N*-(4-fluoro-2-methylphenyl)-*N-*isopropylacetamide (0.66 g, 2.69 mmol), DMF (6 mL) and sodium acetate (0.46 g, 5.64 mmol) stirring for 18 h to yield 2-((4-fluoro-2-methylphenyl)(isopropyl)amino)-2-oxoethyl acetate (0.45 g). LCMS (Method A): Rt = 2.51 min, m/z = 268.1 [M+H]⁺.

### Step 4: Preparation of intermediate I-317d, N-(4-fluoro-2-methylphenyl)-2-hydroxy-N-isopropylacetamide.

Prepared according to general procedure G using 2-((4-fluoro-2-methylphenyl)(isopropyl)amino)-2-oxoethyl acetate (0.45 g, 1.69 mmol), potassium carbonate (0.05 g, 0.38 mmol) and MeOH (5 mL) stirring for 4 h to yield *N*-(4-fluoro-2-methylphenyl)-2-hydroxy-*N*-isopropylacetamide (0.20 g). LCMS (Method A): Rt = 2.29 min, m/z = 226.0 [M+H]⁺.

### Step 5: Preparation of 2-((5-chloro-1,3,4-thiadiazol-2-yl)oxy)-N-(4-fluoro-2-methylphenyl)-N-isopropylacetamide (I-317).

Prepared according to general procedure J using *N*-(4-fluoro-2-methylphenyl)-2-hydroxy-*N-*isopropylacetamide (0.20 g, 0.89 mmol), 2,5-dichloro-1,3,4-thiadiazole (0.14 g, 0.89 mmol), toluene (2 mL) and sodium hydride (0.05 g, 1.34 mmol) to yield 2-((5-chloro-1 ,3,4-thiadiazol-2-yl)oxy)-*N*-(4-fluoro-2-methylphenyl)-*N*-isopropylacetamide (0.17 g). LCMS (Method A): Rt = 3.86 min, m/z = 344.0, 346.0 [M+H]⁺.

### Example 25: Preparation of 2-((5-chloro-1,3,4-thiadiazol-2-yl)oxy)-N-isopropyl-N-(2,4,5-trifluorophenyl)acetamide (I-339).

### Step 1: Preparation of intermediate I-339a, 2,4,5-trifluoro-N-isopropylaniline.

Prepared according to general procedure B using 2,4,5-trifluoroaniline (0.50 g, 3.40 mmol), acetone (1.01 mL, 13.6 mmol), acetic acid (0.39 mL, 6.80 mmol), STAB (1.74 g, 8.16 mmol) and DCM (6 mL) to yield 2,4,5-trifluoro-*N*-isopropylaniline (0.58 g). LCMS (Method A): Rt = 2.96 min, m/z = 190.1 [M+H]⁺.

### Step 2: Preparation of intermediate I-339b, 2-chloro-N-isopropyl-N-(2,4,5-trifluorophenyl)acetamide.

Prepared according to general procedure C using 2,4,5-trifluoro-*N*-isopropylaniline (0.60 g, 3.18 mmol), DCM (5 mL), triethylamine (1.33 mL, 9.55 mmol) and 2-chloroacetyl chloride (0.76 mL, 9.55 mmol) stirring for 10 min at ambient temperature, washing with HCl (aqueous) to yield 2-chloro-*N*-isopropyl-*N-*(2,4,5-trifluorophenyl)acetamide (0.65 g). LCMS (Method A): Rt = 2.65 min, m/z = 266.1 [M+H]⁺.

### Step 3: Preparation of intermediate I-339c, 2-(isopropyl(2,4,5-trifluorophenyl)amino)-2-oxoethyl acetate.

Prepared according to general procedure E using 2-chloro-*N*-isopropyl-*N*-(2,4,5-trifluorophenyl)acetamide (0.61 g, 2.30 mmol), DMF (4 mL) and sodium acetate (0.28 g, 3.45 mmol) to yield 2-(isopropyl(2,4,5-trifluorophenyl)amino)-2-oxoethyl acetate (0.62 g). LCMS (Method A): Rt = 2.52 min, m/z = 290.1 [M+H]⁺.

### Step 4: Preparation of intermediate I-339d, 2-hydroxy-N-isopropyl-N-(2,4,5-trifluorophenyl)acetamide.

Prepared according to general procedure G using 2-(isopropyl(2,4,5-trifluorophenyl)amino)-2-oxoethyl acetate (0.62 g, 2.13 mmol), potassium carbonate (0.06 g, 0.43 mmol), MeOH (6.5 mL) and water (0.15 mL) stirring for 1 h to yield 2-hydroxy-*N*-isopropyl-*N*-(2,4,5-trifluorophenyl)acetamide (0.32 g). LCMS (Method A): Rt = 2.31 min, m/z = 248.1 [M+H]⁺.

### Step 5: Preparation of 2-((5-chloro-1,3,4-thiadiazol-2-yl)oxy)-N-isopropyl-N-(2,4,5-trifluorophenyl)acetamide (I-339).

Prepared according to general procedure I using 2-hydroxy-*N*-isopropyl-*N*-(2,4,5-trifluorophenyl)acetamide (0.32 g, 1.28 mmol), 2,5-dichloro-1,3,4-thiadiazole (0.20 g, 1.28 mmol), isopropanol (1.5 mL), sodium hydroxide (0.15 g, 3.85 mmol) and water (1.9 mL) stirring for 3.5 h to yield 2-((5-chloro-1,3,4-thiadiazol-2-yl)oxy)-*N*-isopropyl-*N-*(2,4,5-trifluorophenyl)acetamide (0.23 g). LCMS (Method A): Rt = 2.80 min, m/z = 366.0, 368.0 [M+H]⁺.

### Example 26: Preparation of N-(3-bromo-4-fluorophenyl)-2-((5-chloro-1,3,4-thiadiazol-2-yl)oxy)-N-isopropylacetamide (I-1032).

### Step 1: Preparation of intermediate I-1032a, 3-bromo-4-fluoro-N-isopropylaniline.

Prepared according to general procedure B using 3-bromo-4-fluoroaniline (0.70 g, 3.68 mmol), acetone (1.09 mL, 14.7 mmol), acetic acid (0.42 mL, 7.37 mmol), STAB (1.88 g, 8.44 mmol) and DCM (6 mL) to yield 3-bromo-4-fluoro-*N*-isopropylaniline (0.81 g). LCMS (Method A): Rt = 2.77 min, m/z = 232.0 [M+H]⁺.

### Step 2: Preparation of intermediate I-1032b, N-(3-bromo-4-fluorophenyl)-2-chloro-N-isopropylacetamide.

Prepared according to general procedure C using 3-bromo-4-fluoro-*N*-isopropylaniline (0.80 g, 3.43 mmol), DCM (6 mL), triethylamine (1.91 mL, 13.72 mmol) and 2-chloroacetyl chloride (1.10 mL, 13.72 mmol), washing with HCl (aqueous) to yield *N*-(3-bromo-4-fluorophenyl)-2-chloro-*N*-isopropylacetamide (1.07 g). LCMS (Method A): Rt = 2.80 min, m/z = 308.0, 310.0, 312.0 [M+H]⁺.

### Step 3: Preparation of intermediate I-1032c, 2-((3-bromo-4-fluorophenyl)(isopropyl)amino)-2-oxoethyl acetate.

Prepared according to general procedure E using *N*-(3-bromo-4-fluorophenyl)-2-chloro-*N-*isopropylacetamide (0.96 g, 3.11 mmol), DMF (4 mL) and sodium acetate (0.38 g, 4.67 mmol) stirring for 3 h to yield 2-((3-bromo-4-fluorophenyl)(isopropyl)amino)-2-oxoethyl acetate (0.78 g). LCMS (Method A): Rt = 2.62 min, m/z = 332.0 [M+H]⁺.

### Step 4: Preparation of intermediate I-1032d, N-(3-bromo-4-fluorophenyl)-2-hydroxy-N-isopropylacetamide.

Prepared according to general procedure G using 2-((3-bromo-4-fluorophenyl)(isopropyl)amino)-2-oxoethyl acetate (0.58 g, 1.76 mmol), potassium carbonate (0.05 g, 0.35 mmol), MeOH (6.5 mL) and water (0.15 mL) stirring for 1 h to yield *N*-(3-bromo-4-fluorophenyl)-2-hydroxy-*N*-isopropylacetamide (0.43 g). LCMS (Method A): Rt = 2.39 min, m/z = 290.0, 292.0 [M+H]⁺.

### Step 5: Preparation of N-(3-bromo-4-fluorophenyl)-2-((5-chloro-1,3,4-thiadiazol-2-yl)oxy)-N-isopropylacetamide (I-1032).

Prepared according to general procedure I using *N*-(3-bromo-4-fluorophenyl)-2-hydroxy-*N-*isopropylacetamide (0.31 g, 1.08 mmol), 2,5-dichloro-1,3,4-thiadiazole (0.17 g, 1.08 mmol), isopropanol (1.5 mL), sodium hydroxide (0.13 g, 3.24 mmol) and water (1.6 mL) stirring for 3.5 h to yield *N*-(3-bromo-4-fluorophenyl)-2-((5-chloro-1,3,4-thiadiazol-2-yl)oxy)-*N*-isopropylacetamide (0.19 g). LCMS (Method A): Rt = 2.94 min, m/z = 408.0, 410.0, 412.0 [M+H]⁺.

### Biological Examples

The herbicidal effect of some of the above synthesized compounds was tested in greenhouse experiments on different plants.

For the plants employed in these experiments, the following abbreviations are used:
- ALOMY:: *Alopecurus myosuroides*
- APESV:: *Apera spica-venti*
- AVEFA:: *Avena fatua*
- CHEAL:: *Chenopodium album*
- DIGSA:: *Digitaria sanguinalis*
- ECHCG:: *Echinochloa crus-galli*
- GERDI:: *Geranium dissectum*
- LOLMU:: *Lolium multiflorum*
- PANMI:: *Panicum miliaceum*
- POAAN:: *Poa annua*
- SETVI:: *Setaria viridis*
- SOLNI:: *Solanum nigrum*

### Herbicidal Effect in Pre-Emergence

Seeds of monocotyledonous or dicotyledonous weeds and crop plants are designed in plastic trays and covered with soil. Aqueous solutions are then pipetted onto each individual plant cells (within plastic trays), each derived from the formulation of the technical active ingredient in acetone / water (3.5% Acetone) solution containing (0.1%) Tween 20 (polyoxyethelyene (20) sorbitan monolaurate, CAS RN 9005-64-5). Compound(s) are applied pre-emergence at 250 g/ha (Table 5), 125 g/ha (Table 6) or 62.5 g/ha (Table 7).

The test plants are then grown in a glasshouse under controlled conditions (at 20 °C/12 °C, day/night; photoperiod 16:8 hours light:dark; 65 % humidity) and watered daily. After 14 or 21 days the test is evaluated for the percentage damage caused to the plant. The biological activities are shown in the following Tables on a five-point scale (5 = 81-100%; 4 = 61-80%; 3=41-60%; 2=21-40%; 1=0-20%; - = not tested) and for some compounds are listed in parentheses.

**Table 5**

| Compound | ALOMY | APESV | AVEFA | CHEAL | DIGSA | ECHCG |
|---|---|---|---|---|---|---|
| Flufenacet | 5 (98%) | 5 (100%) | 5 (86%) | 4 (74%) | 5 (100%) | 5 (99%) |
| I-8 | - | - | 3 | 1 | 5 | 5 |
| I-7 | 5 | 5 (100%) | 5 | 5 (81%) | 5 (100%) | 5 (99%) |
| I-6 | - | - | 2 | 1 | 5 | 5 |
| I-5 | - | - | 1 | 3 | 3 | 4 |
| I-4 | 5 (98%) | 5 (100%) | 4 | 1 | 5 | 5 |
| I-3 | 5 | - | 1 | 1 | 5 | 1 |
| I-2 | 5 | - | 2 | 1 | 5 | 5 |
| I-1 | 4 | - | 3 | 1 | 5 | 2 |
| IV-11 | 5 (100%) | 5 (100%) | 5 | 2 | 5 (100%) | 5 (100%) |
| II-6 | 5 (100%) | 5 (100%) | 5 (93%) | 4 (74%) | 5 (100%) | 5 (100%) |
| I-42 | 5 | 5 | 5 | 5 | 5 | 5 |
| I-284 | 5 (100%) | 5 (100%) | 3 | 4 | 5 (100%) | 3 |
| I-53 | 5 | - | 2 | 2 | 5 | 2 |
| I-31 | 5 (98%) | - | 5 | 5 (85%) | 5 (100%) | 5 (100%) |
| I-1054 | 5 (100%) | - | 5 (98%) | 5 (100%) | - | 5 (100%) |
| I-207 | 5 (100%) | - | 3 | 5 (93%) | - | 5 (100%) |
| I-282 | 5 (100%) | - | 3 | 5 (100%) | - | 5 (100%) |
| II-281 | 5 (100%) | - | 2 | 5 (93%) | - | 5 (100%) |
| I-279 | 5 (100%) | - | 5 (100%) | 5 (100%) | - | 5 (100%) |
| I-361 | 5 (100%) | - | 5 (99%) | 5 (100%) | - | 5 (100%) |
| I-273 | - | - | 5 (100%) | - | - | 5 (100%) |
| I-295 | - | - | 5 (93%) | - | - | 5 (100%) |
| I-317 | - | - | 5 (100%) | - | - | 5 (100%) |
| I-339 | - | - | 5 (100%) | - | - | 5 (100%) |
| I-1032 | - | - | 5 (100%) | - | - | 5 (100%) |

| Compound | GERDI | LOLMU | PANMI | POAAN | SETVI | SOLNI |
|---|---|---|---|---|---|---|
| Flufenacet | 2 (34%) | 5 (99%) | 5 (99%) | 5 (100%) | 5 (100%) | 2 (34%) |
| I-8 | - | 5 | 1 | - | - | 1 |
| I-7 | 3 (46%) | 5 | 5 (100%) | 5 (100%) | 5 (100%) | 3 (57%) |
| I-6 | - | 1 | 1 | - | - | 1 |
| I-5 | - | 1 | 2 | - | - | 1 |
| I-4 | 1 | 5 | 5 | - | 5 | 2 |
| I-3 | - | 5 | 1 | - | 2 | - |
| I-2 | - | 5 | 5 | - | 2 | - |
| I-1 | - | 4 | 2 | - | 3 | - |
| IV-11 | 2 | 5 | 5 (99%) | 5 (100%) | 5 (100%) | 2 |
| II-6 | 2 | 5 | 5 (100%) | 5 | 5 | 2 |
| I-42 | 4 | 4 | 5 | 5 | 5 | 2 |
| I-284 | 1 | 5 | 5 | 5 (100%) | 5 (100%) | 1 |
| I-53 | - | 5 | 5 | 5 | 5 | - |
| I-31 | - | 5 (100%) | 5 (100%) | 5 (100%) | 5 (100%) | - |
| I-1054 | - | 5 (100%) | 5 (100%) | 5 (100%) | - | - |
| I-207 | - | 5 (100%) | 5 | 5 (100%) | - | - |
| I-282 | - | 5 | 5 (100%) | 5 (100%) | - | - |
| II-281 | - | 5 (100%) | 5 | 5 (100%) | - | - |
| I-279 | - | 5 (100%) | 3 | 5 (100%) | - | - |
| I-361 | - | 5 (100%) | 5 (100%) | 5 (100%) | - | - |
| I-273 | - | 5 (100%) | 5 (100%) | 5 (100%) | - | - |
| I-295 | - | 5 | 5 | 5 (100%) | - | - |
| I-317 | - | 5 (100%) | 5 (100%) | 5 (100%) | - | - |
| I-339 | - | 5 (100%) | 5 (100%) | 5 (100%) | - | - |
| I-1032 | - | 5 (100%) | 5 (100%) | 5 (100%) | - | - |

As can be seen from Table 5, compounds I-9, I-8, I-7, I-6, I-5, I-4, I-3, I-2, IV-11, II-6, I-42, I-284, I-53, I-31, I-1054, I-207, I-282, II-281, I-279, I-361, I-273, I-295, I-317, I-339, I-1032 and I-1 gave an effect of at least 61 % and up to 80% against at least one of the test plants. Even more, compounds I-9, I-8, I-7, I-6, I-4, I-3, I-2, IV-11, II-6, I-42, I-284, I-53, I-31 and I-1 gave an effect of at least 81% against at least one of the test plants.

**Table 6**

| Compound | ALOMY | APESV | AVEFA | CHEAL | DIGSA | ECHCG |
|---|---|---|---|---|---|---|
| Flufenacet | 5 (99%) | 5 (100%) | 4 (78%) | 3 (58%) | 5 (100%) | 5 (97%) |
| I-7 | 5 | 5 (100%) | 4 | 4 (69%) | 5 (100%) | 5 (99%) |
| I-4 | 4 | 5 (100%) | 3 | 2 | 5 | 5 |
| IV-11 | 5 (99%) | 5 (100%) | 3 | 3 | 5 (100%) | 5 |
| II-6 | 5 | 5 (100%) | 4 | 3 | 5 | 5 |
| I-42 | 5 | 5 | 3 | 5 | 5 | 5 |
| I-284 | 5 | 5 (100%) | 3 | 2 | 5 | 5 |
| I-53 | 5 | - | 2 | 2 | 5 | 5 |
| I-31 | 5 | - | 2 | 5 (100%) | 5 (100%) | 5 (98%) |
| V-2 | 4 | - | - | - | - | - |
| I-10 | 5 (100%) | - | - | - | - | - |
| II-8 | 5 (100%) | - | - | - | - | - |
| I-1054 | 5 (100%) | - | 1 | 5 (100%) | - | 4 |
| I-207 | 5 (100%) | - | 2 | 4 (80%) | - | 5 (100%) |
| I-282 | 5 (100%) | - | 4 (80%) | 5 (100%) | - | 5 (100%) |
| II-281 | 5 (100%) | - | - | 3 | - | 5 (100%) |
| I-279 | 5 (100%) | - | 3 | 5 (100%) | - | 5 (100%) |
| I-361 | 5 (100%) | - | 3 | 5 (100%) | - | 5 (100%) |
| I-273 | - | - | 4 | - | - | 5 (100%) |
| I-295 | - | - | 3 | - | - | 5 (100%) |
| I-317 | - | - | 3 | - | - | 5 (100%) |
| I-339 | - | - | 5 (95%) | - | - | 5 (100%) |
| I-1032 | - | - | 4 | - | - | 5 (100%) |

| Compound | GERDI | LOLMU | PANMI | POAAN | SETVI | SOLNI |
|---|---|---|---|---|---|---|
| Flufenacet | 2 | 5 (96%) | 5 (98%) | 5 (100%) | 5 (100%) | 1 (16%) |
| I-7 | 2 | 5 | 5 | 5 | 5 (100%) | 2 (33%) |
| I-4 | 2 | 5 (98%) | 5 | 5 (100%) | 5 | 1 |
| IV-11 | 1 | 5 | 5 (100%) | 5 | 5 (100%) | 3 (43%) |
| II-6 | 1 | 5 | 5 | 5 | 5 (100%) | 1 |
| I-42 | 5 | 3 | 5 | 5 | 5 | 1 |
| I-284 | 1 | 5 (99%) | 5 | 5 | 5 | 1 |
| I-53 | - | 4 | 5 | 5 | 5 | - |
| I-31 | - | 5 (98%) | 5 | 5 (100%) | 5 (100%) | - |
| V-2 | - | 5 | - | - | - | - |
| I-10 | - | 4 | - | - | - | - |
| II-8 | - | 5 (98%) | - | - | - | - |
| I-1054 | - | 5 (100%) | 5 (100%) | 4 | - | - |
| I-207 | - | 2 | 2 | 5 (100%) | - | - |
| I-282 | - | 5 (100%) | 5 (100%) | 5 | - | - |
| II-281 | - | 2 | 2 | 5 (100%) | - | - |
| I-279 | - | 5 (100%) | 5 | 5 (100%) | - | - |
| I-361 | - | 5 | 5 (100%) | 5 (100%) | - | - |
| I-273 | - | 5 (100%) | 5 (100%) | 5 (100%) | - | - |
| I-295 | - | 5 (100%) | 5 | 5 (100%) | - | - |
| I-317 | - | 5 (100%) | 5 (100%) | 5 (100%) | - | - |
| I-339 | - | 5 | 5 (100%) | 3 | - | - |
| I-1032 | - | 5 (100%) | 5 (100%) | 5 (100%) | - | - |

As can be seen from Table 6, compounds I-7, I-4, IV-11, !!-6, I-42, I-284, I-53, I-31, V-2, I-10, I-1054, I-207, I-282, I1-281, I-279, I-361, I-273, I-295, I-317, I-339, I-1032 and II-8 gave an effect of at least 81% against at least one of the test plants at an application rate of 125 g/ha.

**Table 7**

| Compound | ALOMY | APESV | AVEFA | CHEAL | DIGSA | ECHCG |
|---|---|---|---|---|---|---|
| Flufenacet | 5 (97%) | 5 (90%) | 3 (50%) | 3 (42%) | 5 (98%) | 5 (96%) |
| I-7 | 5 | 5 (100%) | 3 | 3 (57%) | 5 (100%) | 5 (96%) |
| I-4 | 5 (98%) | 5 (100%) | 2 | 1 | 5 | 4 |
| IV-11 | 5 | 5 (95%) | 2 | 3 (55%) | 5 | 4 |
| II-6 | 5 | 5 (100%) | 2 | 2 | 5 | 5 |
| I-42 | 4 | 5 | 1 | 4 | 5 | 5 |
| I-284 | 4 | 5 (100%) | 2 | 1 | 5 (98%) | 5 (97%) |
| I-53 | 4 | - | 3 | 1 | 5 | 5 |
| I-31 | 5 | - | 3 | 5 (85%) | 5 (100%) | 5 (100%) |
| I-1054 | 5 (100%) | - | - | - | - | 5 |
| I-207 | 5 (100%) | - | 2 | 3 (43%) | - | 4 |
| I-282 | 5 (100%) | - | 4 (65%) | 5 (95%) | - | 5 (100%) |
| II-281 | 5 (100%) | - | 2 | 1 | - | 5 |
| I-279 | 5 | - | 5 (85%) | 4 (65%) | - | 3 |
| I-361 | 5 (100%) | - | 4 (70%) | 5 (90%) | - | 5 (100%) |
| I-273 | - | - | - | - | - | 5 (100%) |
| I-295 | - | - | 3 (50%) | - | - | 5 |
| I-317 | - | - | 3 (50%) | - | - | 5 (100%) |
| I-339 | - | - | 4 (78%) | - | - | 5 (98%) |
| I-1032 | - | - | 5 (95%) | - | - | 5 (100%) |

| Compound | GERDI | LOLMU | PANMI | POAAN | SETVI | SOLNI |
|---|---|---|---|---|---|---|
| Flufenacet | 2 | 5 (83%) | 5 (95%) | 5 (100%) | 5 (100%) | 1 (8%) |
| I-7 | 1 | 5 (88%) | 5 | 5 | 5 | 1 (9%) |
| I-4 | 1 | 5 (86%) | 4 | 5 | 5 | 2 (22%) |
| IV-11 | 1 | 4 | 5 | 5 (100%) | 5 | 2 (22%) |
| II-6 | 1 | 3 | 3 | 5 | 5 | 1 |
| I-42 | 3 | 2 | 5 | 4 | 5 | 1 |
| I-284 | 1 | 5 | 5 | 5 | 5 | 1 (9%) |
| I-53 | - | 4 | 5 | 5 | 5 | - |
| I-31 | - | 5 (100%) | 5 | 5 (100%) | 5 (100%) | - |
| I-1054 | - | 4 | 4 | 5 | - | - |
| I-207 | - | 1 | 1 | 5 (100%) | - | - |
| I-282 | - | 4 | 3 | 5 (100%) | - | - |
| II-281 | - | 2 | 1 | 5 (100%) | - | - |
| I-279 | - | 2 | 3 | 4 | - | - |
| I-361 | - | 5 (100%) | 5 (100%) | 5 (100%) | - | - |
| I-273 | - | 5 (100%) | 5 (100%) | 5 (100%) | - | - |
| I-295 | - | 5 (100%) | 1 | 5 (100%) | - | - |
| I-317 | - | 5 (100%) | 5 (95%) | 5 (100%) | - | - |
| I-339 | - | 5 (100%) | 5 | 5 (100%) | - | - |
| I-1032 | - | 5 (90%) | 5 (95%) | 5 (100%) | - | - |

As can be seen from Table 7, compounds I-7, I-4, IV-11, II-6, I-42, I-284, I-53, I-31, I-1054, I-207, I-282, II-281, I-279, I-361, I-273, I-295, I-317, I-339 and I-1032 gave an effect of at least 81% against at least one of the test plants at an application rate of 62.5 g/ha.

### Herbicidal Effect in Post-Emergence

Monocotyledonous or dicotyledonous weeds and crop test plants are grown in a glasshouse in plastic trays under controlled conditions (at 20 °C/12 °C, day/night; photoperiod 16:8 hours light:dark; 65 % humidity) and watered daily. Individual plant cells (within plastic trays) are then sprayed with aqueous solutions, each derived from the formulation of the technical active ingredient in acetone / water (3.5% Acetone) solution containing (0.1%) Tween 20 (polyoxyethelyene (20) sorbitan monolaurate, CAS RN 9005-64-5). Compound(s) are applied post-emergence at 250 g/ha (Table 8), 125 g/ha (Table 9) or 62.5 g/ha (Table 10) and are evaluated after 14 days for the percentage damage caused to the plant. The biological activities are shown in the following Tables on a five-point scale (5 = 81-100%; 4 = 61-80%; 3=41-60%; 2=21-40%; 1=0-20%; - = not tested) and for some compounds are listed in parentheses.

**Table 8**

| Compound | ALOMY | APESV | AVEFA | DIGSA | ECHCG |
|---|---|---|---|---|---|
| Flufenacet | 3 | 5 (95%) | 1 | 5 | 5 (89%) |
| I-7 | 1 | 5 (100%) | 1 | 4 | 5 (90%) |
| I-4 | 1 | 4 | 1 | 4 | 4 |

| Compound | GERDI | LOLMU | PANMI | POAAN | SETVI | SOLNI |
|---|---|---|---|---|---|---|
| Flufenacet | 2 (28%) | 3 (50%) | 3 (53%) | 5 | 4 (65%) | 1 |
| I-7 | 2 | 4 (73%) | 4 (75%) | 4 | 3 | 1 |
| I-4 | 2 (33%) | 4 (78%) | 2 | 1 | 4 (78%) | 1 |

As can be seen from Table 8, compounds I-7 and I-4 gave an improved effect over flufenacet against at least one of the test plants at an application rate of 250 g/ha.

**Table 9**

| Compound | ALOMY | APESV | AVEFA | DIGSA | ECHCG |
|---|---|---|---|---|---|
| Flufenacet | 3 | 4 (65%) | 1 | 5 | 4 |
| I-7 | 1 | 5 (98%) | 1 | 3 | 2 |
| I-4 | 1 | 4 | 1 | 2 | 1 |

| Compound | GERDI | LOLMU | PANMI | POAAN | SETVI | SOLNI |
|---|---|---|---|---|---|---|
| Flufenacet | 1 | 1 (14%) | 2 | 5 | 1 (20%) | 1 |
| I-7 | 1 | 1 (18%) | 2 | 1 | 5 (93%) | 1 |
| I-4 | 1 | 2 (28%) | 1 | 1 | 2 (28%) | 1 |

As can be seen from Table 9, compound I-7 gave an improved effect over flufenacet against at least one of the test plants at an application rate of 125 g/ha.

**Table 10**

| Compound | ALOMY | APESV | AVEFA | DIGSA | ECHCG |
|---|---|---|---|---|---|
| Flufenacet | 2 | 1 (13%) | 1 | 4 | 3 |
| I-7 | 1 | 2 (35%) | 1 | 1 | 1 |
| I-4 | 1 | 1 | 1 | 1 | 1 |

| Compound | GERDI | LOLMU | PANMI | POAAN | SETVI | SOLNI |
|---|---|---|---|---|---|---|
| Flufenacet | 1 | 1 (10%) | 2 (28%) | 4 | 2 (28%) | 1 |
| I-7 | 1 | 1 | 2 (30%) | 1 | 3 (53%) | 1 |
| I-4 | 1 | 1 (15%) | 1 | 1 | 2 (35%) | 1 |

As can be seen from Table 10, compounds I-7 and I-4 gave an improved effect over flufenacet against at least one of the test plants at an application rate of 62.5 g/ha.

## Claims

1. A compound of formula I: 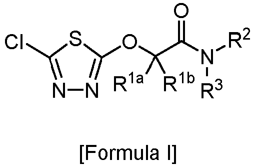 wherein
each of R^{1a} and R^{1b} is H;
R² is selected from C₁₋₃ alkyl, C₄₋₈ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₁₋₆ alkylsulfonyl, C₂₋₈ heterocyclyl and (C₂₋₈ heterocyclyl)C₁₋₃ alkyl, each of which may be optionally substituted; and
R³ is C₆₋₁₀ aryl which is substituted;
or an agrochemically acceptable salt thereof, a stereoisomer thereof, an enantiomer thereof, a deuteromer thereof, or a tautomer thereof,
wherein:
- when R^{1a} = H, R^{1b} = H and R² = isopropyl, R³ is not phenyl, 4-fluorophenyl, 2-chlorophenyl, 3-chlorophenyl, 4-chlorophenyl, 3-methylphenyl, 4-methylphenyl, 2-methoxyphenyl, 4-methoxyphenyl, 4-ethoxyphenyl, 4-thiomethylphenyl, 3-chloro-4-thiomethylphenyl, 3,5-dimethylphenyl, 3,5-dichlorophenyl, 3-chloro-4-methoxyphenyl, 3,5-di(trifluoromethyl)phenyl, 3,4-dichlorophenyl, 5-chloro-2-methylphenyl; or
- when R^{1a} = H, R^{1b} = H and R² = methyl, R³ is not phenyl, 4-(trifluoromethyl)phenyl, 3-(trifluoromethyl)phenyl, 2-methyl-5-nitrophenyl, 4-thiomethylphenyl, 3-thiomethylphenyl, 4-fluorophenyl, 3-fluorophenyl, 3,4-dichlorophenyl, 2-methylphenyl, 3-methylphenyl, 4-methylphenyl, 3-chlorophenyl.

2. The compound of claim 1, wherein:
each of the groups listed for R² is optionally substituted by one or more substituents selected from F, C!, Br, I, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiR₃, wherein each R is independently selected from H, OH, C₁₋₆ alkoxy and C₁₋₆ alkyl, and wherein two Rs and the atom to which they are bound may form a cyclic group;
each of the groups listed for R³ is substituted by one or more substituents selected from F, C!, Br, I, CN, NO₂, C₁₋₆ alkyl, C₁₋₆ alkoxy and C₃₋₆ cycloalkyl;
or an agrochemically acceptable salt thereof, a stereoisomer thereof, an enantiomer thereof, a deuteromer thereof, or a tautomer thereof.

3. The compound of claim 1, with the proviso that R³ is C₆ aryl substituted by at least one of halogen, CN, NO₂, C₁₋₆ alkyl, halogeno-C₁₋₆ alkyl, C₁₋₆ alkoxy, halogeno-C₁₋₆ alkoxy, C₁₋₆ alkylthio and halogeno-C₁₋₆ alkylthio, preferably C₆ aryl substituted by at least one of F, Cl, CH₃, OCH₃, OC₂H₅, SCH₃ and CF₃;
or an agrochemically acceptable salt thereof, a stereoisomer thereof, an enantiomer thereof, a deuteromer thereof, or a tautomer thereof.

4. The compound of any one of claims 1 to 3, wherein R² is selected from C₄₋₈ alkyl, (cyano)C₁₋₆ alkyl, C₁₋₆ alkoxy, C₂₋₆ alkenyl, C₂₋₆ alkynyl, C₃₋₆ cycloalkyl, (C₃₋₆ cycloalkyl)C₁₋₃ alkyl, C₂₋₈ heterocyclyl and (C₂₋₈ heterocyclyl)C₁₋₃ alkyl, each of which may be optionally substituted by one or more groups selected from F, Cl, Br, I, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiR₃, wherein each R is independently selected from H, OH and C₁₋₆ alkyl,
or an agrochemically acceptable salt thereof, a stereoisomer thereof, an enantiomer thereof, a deuteromer thereof, or a tautomer thereof.

5. The compound of any one of claims 1 to 3, wherein R² is selected from cyanomethyl, cyclopropyl, sec-butyl, 1-butyn-4-yl, 2-butyn-1-yl, prop-1-yl, 3-methyl-1-butyn-3-yl, cyclopropylmethyl, 1-trimethylsilyl-2-butyn-4-yl, 1-buten-4-yl, 1-buten-3-yl, 2-methyl-1-propen-3-yl, allyl, tert-butyl, cyclopentyl, propargyl, iso-butyl and 1-butyn-3-yl,
or an agrochemically acceptable salt thereof, a stereoisomer thereof, an enantiomer thereof, a deuteromer thereof, or a tautomer thereof.

6. The compound of claims 1 to 5, wherein R³ is C₆ aryl substituted by at least one F, preferably in the 3-position or 4-position relative to the attachment point of R³ to the remainder of the molecule, and optionally further substituted by one or more groups selected from F, Cl, Br, I, CN, NO₂, C₁₋₆ alkyl, C₁₋₆ alkoxy and C₃₋₆ cycloalkyl;
or an agrochemically acceptable salt thereof, a stereoisomer thereof, an enantiomer thereof, a deuteromer thereof, or a tautomer thereof.

7. The compound of any one of claims 1 to 6, wherein
R² is selected from C₄₋₈ alkyl, (cyano)C₁₋₆ alkyl, C₂₋₆ alkenyl, C₂₋₆ alkynyl, (C₃₋₆ cycloalkyl)C₁₋₃ alkyl and C₃₋₆ cycloalkyl, each of which may be optionally substituted by one or more groups selected from F, C!, Br, I, CN, oxo, C₁₋₆ alkyl, C₁₋₆ alkoxy, (C₁₋₆ alkoxy)C₁₋₆alkyl, SO₂R, C(O)R, CO₂R, C(O)NR₂, NR₂ and SiR₃, wherein each R is independently selected from H, OH and C₁₋₆ alkyl;
or an agrochemically acceptable salt thereof, a stereoisomer thereof, an enantiomer thereof, a deuteromer thereof, or a tautomer thereof.

8. The compound of claim 7, wherein
R³ is selected from 4-fluorophenyl, 2,5-difluorophenyl, 3,4-difluorophenyl, 2,4-difluorophenyl, 3-fluoro-4-chlorophenyl, 3-chloro-4-fluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl,3-chloro-2,4-difluorophenyl, 2,6-dichloro-4-fluorophenyl and 2-chloro-4-fluoro-6-methylphenyl,
preferably, wherein:
R² is selected from cyanomethyl, cyclopropyl, sec-butyl, 1-butyn-4-yl, 2-butyn-1-yl, 3-methyl-1-butyn-3-yl, allyl, tert-butyl, cyclopentyl, propargyl, iso-butyl, 1-butyn-3-yl and 3-methylbut-2-yl, and
R³ is 4-fluorophenyl,
more preferably, wherein:
R² is selected from 1-butyn-4-yl, 2-butyn-1-yl, propargyl, allyl, 3-methyl-1-butyn-3-yl and 1-butyn-3-yl, and
R³ is 4-fluorophenyl;
or an agrochemically acceptable salt thereof, a stereoisomer thereof, an enantiomer thereof, a deuteromer thereof, or a tautomer thereof.

9. The compound of any one of claims 1 to 8, having a structure selected from Table 3;
or an agrochemically acceptable salt thereof, a stereoisomer thereof, an enantiomer thereof, a deuteromer thereof, or a tautomer thereof, with the proviso that said compound is not compound I-9, I-20, I-108, I-141, I-174 or I-900.

10. The compound of any one of claims 1 to 9, represented by the following Formula la', 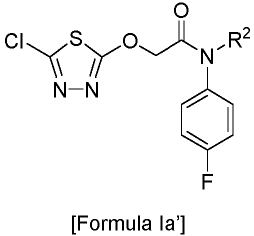 wherein R² is:
(i) 1-butyn-4-yl;
(ii) 2-butyn-1-yl;
(iii) propargyl; or
(iv) allyl;
or an agrochemically acceptable salt thereof, a stereoisomer thereof, an enantiomer thereof, a deuteromer thereof, or a tautomer thereof.

11. The compound of any one of claims 1 to 3, 6 and 9, wherein
R² is isopropyl, and
R³ is selected from 3,4-difluorophenyl, 2,4-difluorophenyl, 3-fluoro-4-chlorophenyl, 3-chloro-4-fluorophenyl, 4-chlorophenyl, 4-nitrophenyl, 4-(trifluoromethoxy)phenyl, 2,5-difluorophenyl, 2-fluorophenyl, 3-fluorophenyl, 2,3-difluorophenyl, 2,5-difluorophenyl, 3,5-difluorophenyl, 2,6-difluorophenyl, 2-chloro-4-fluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl, 3-chloro-2,4-difluorophenyl, 2,6-dichloro-4-fluorophenyl and 2-chloro-4-fluoro-6-methylphenyl,
preferably wherein R³ is selected from 3,4-difluorophenyl, 2,4-difluorophenyl, 3-fluoro-4-chlorophenyl, 3-chloro-4-fluorophenyl, 4-chlorophenyl, 4-nitrophenyl, 4-(trifluoromethoxy)phenyl, 2,5-difluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl, 3-chloro-2,4-difluorophenyl, 2,6-dichloro-4-fluorophenyl and 2-chloro-4-fluoro-6-methylphenyl,
more preferably wherein R³ is selected from 3,4-difluorophenyl, 2,4-difluorophenyl, 3-fluoro-4-chlorophenyl, 3-chloro-4-fluorophenyl and 4-chlorophenyl,
or
R² is prop-1-yl, and
R³ is selected from 4-fluorophenyl, 3,4-difluorophenyl, 2,4-difluorophenyl, 3-fluoro-4-chlorophenyl, 3-chloro-4-fluorophenyl, 4-fluoro-2-nitrophenyl, 4-fluoro-2-methylphenyl, 4-fluoro-2-methoxyphenyl, 2,4,5-trifluorophenyl, 2,3,4-trifluorophenyl, 2,4,6-trifluorophenyl, 2-chloro-4,6-difluorophenyl, 3-chloro-2,4-difluorophenyl, 2,6-dichloro-4-fluorophenyl and 2-chloro-4-fluoro-6-methylphenyl, preferably wherein R³ is selected from 3,4-difluorophenyl, 2,4-difluorophenyl, 3-fluoro-4-chlorophenyl, 3-chloro-4-fluorophenyl and 4-chlorophenyl;
or an agrochemically acceptable salt thereof, a stereoisomer thereof, an enantiomer thereof, a deuteromer thereof, or a tautomer thereof.

12. Use of the compound of any one of claims 1 to 11 or an agrochemically acceptable salt thereof, a stereoisomer thereof, an enantiomer thereof, a deuteromer thereof, or a tautomer thereof as a herbicide, for example on cereals including barley and wheat, cotton, oilseed rape, sunflower, maize, rice, soybeans, sugar beet, sugar cane, turf, trees including fruit trees, palm trees, coconut trees or other nuts, vines including grapes, fruit bushes, fruit plants and vegetables including potatoes and tomatoes.

13. The use of claim 12, wherein the compound is used against species selected from *Alopecurus myosuroidpes, Avena fatua, Chenopodium album, Digitaria sanguinalis, Echinochloa crus-galli, Geranium dissectum, Lolium multiflorum, Panicum miliaceum, Setaria viridis* and *Solanum nigrum.*

14. An intermediate selected from:

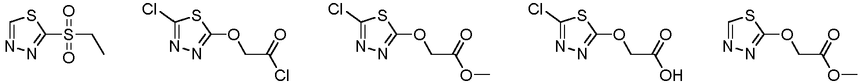

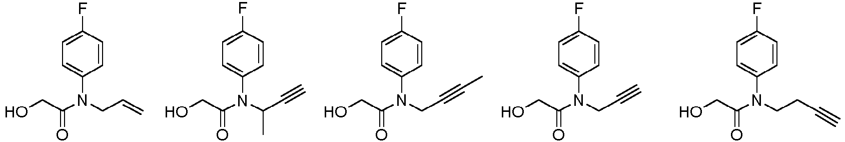

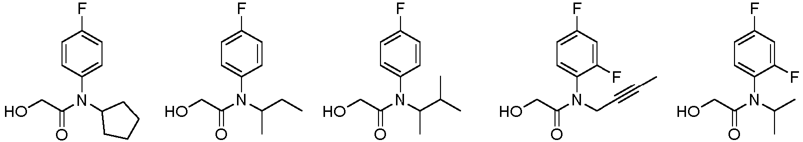

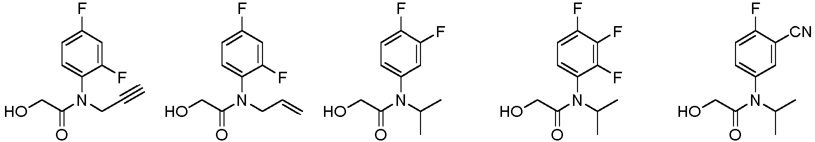

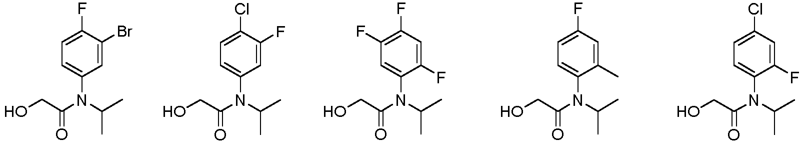

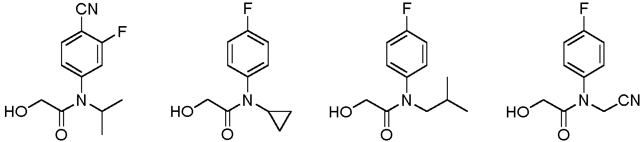

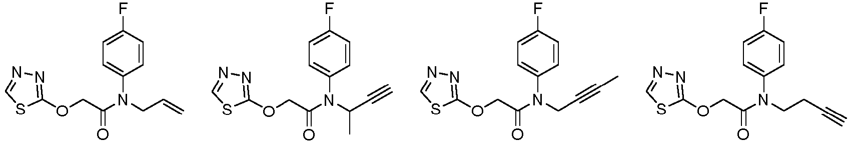

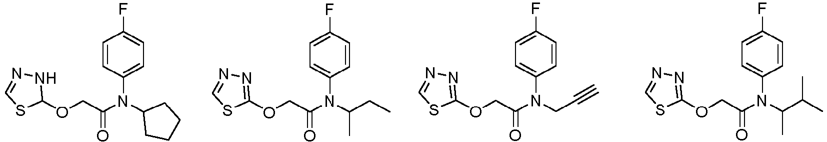

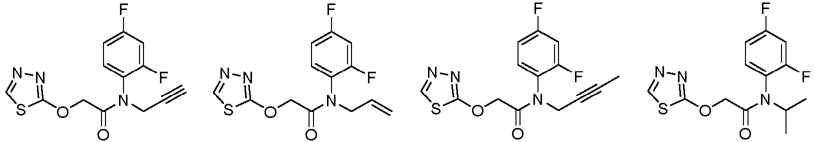

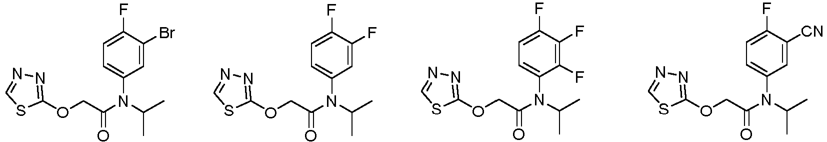

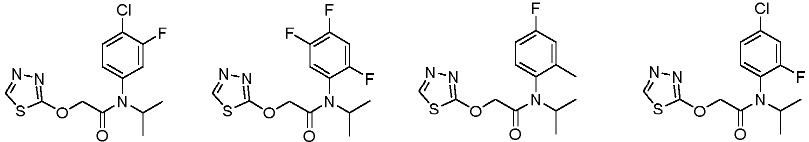

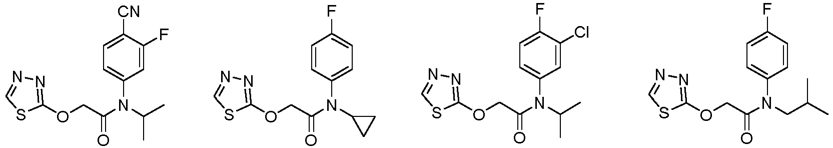

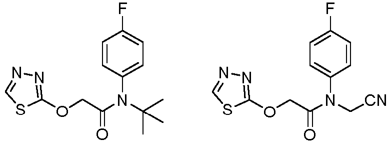

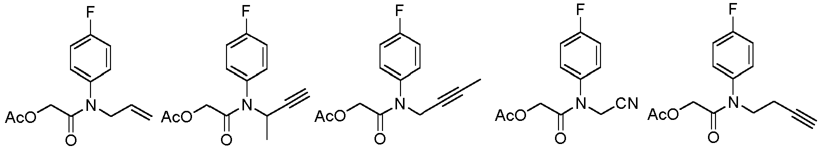

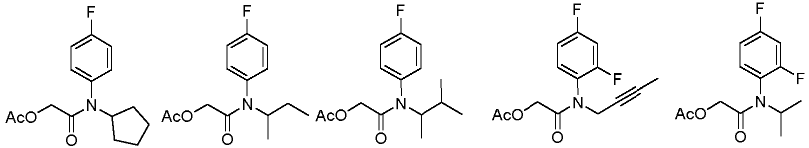

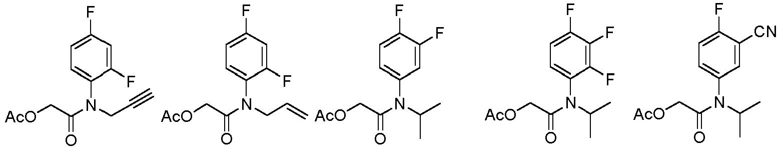

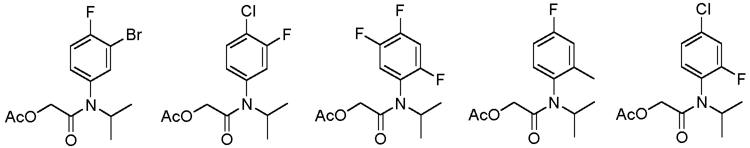

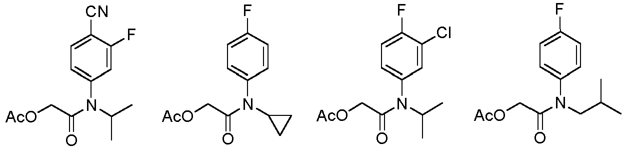

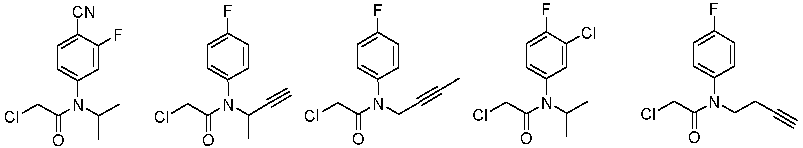

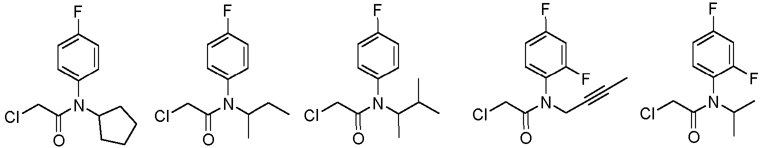

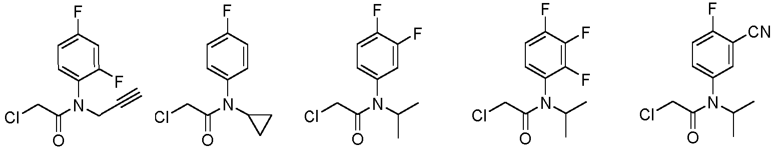

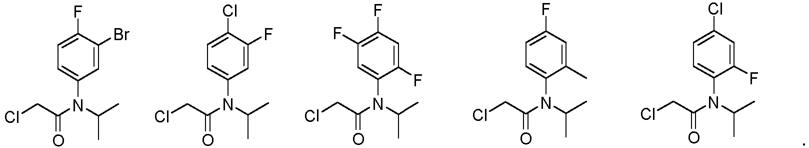

15. A method for producing a compound according to any one of claims 1 to 11, involving using one or more intermediates according to claim 14 in at least one step.

16. A method for producing compound according to any one of claims 1 to 11, comprising a step selected from the following (i) to (iv):
(i) 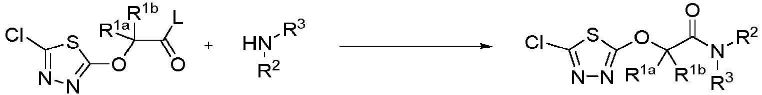
(ii) 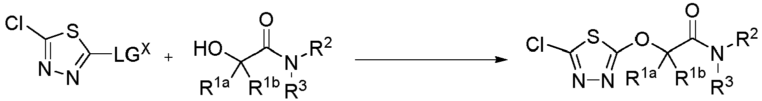
(iii) 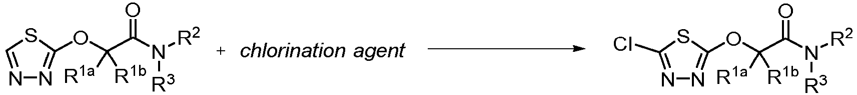
(iv) 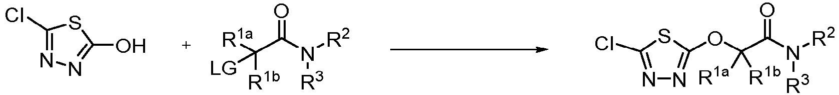 wherein L, LG and LG^{X} are each independently leaving groups, and the remaining groups are as defined hereinabove.
